# EUROPEAN PATENT APPLICATION

(11) **EP 4 477 225 A1**
(43) Date of publication of application: **18.12.2024**
(21) Application number: 23755759.0
(22) Date of filing: 13.02.2023
(51) Int. Cl.: A61K 31/58, A61K 45/06, A61P 25/16, A61P 25/22, A61P 25/24, A61P 35/00, A61P 3/10, A61P 29/00

(54) **USE OF SARSASAPOGENIN STRUCTURE-BASED DERIVATIVE AND PHARMACEUTICAL COMPOSITION THEREOF**

(30) Priority: 18.02.2022 CN 202210152310; 10.02.2023 CN 202310093727
(71) Applicant: Beijing Phytovent Pharmaceutical Technology Co., Ltd., Beijing 102200 (CN)
(72) Inventor: WANG, Na, Beijing 102200 (CN); WENG, Jianyang, Beijing 102200 (CN); NAN, Zhiyuan, Beijing 102200 (CN); KONG, Fansheng, Beijing 102200 (CN)
(74) Representative: Stolmár & Partner Patentanwälte PartG mbB
(86) International application number: PCT/CN2023/075727
(87) International publication number: WO 2023/155757

(57) **Abstract**

The present invention relates to a use of sarsasapogenin-based compounds in preparation of drugs for treating related diseases caused by mitochondrial dysfunction, wherein the structural formula of the compounds is represented by general formula I:

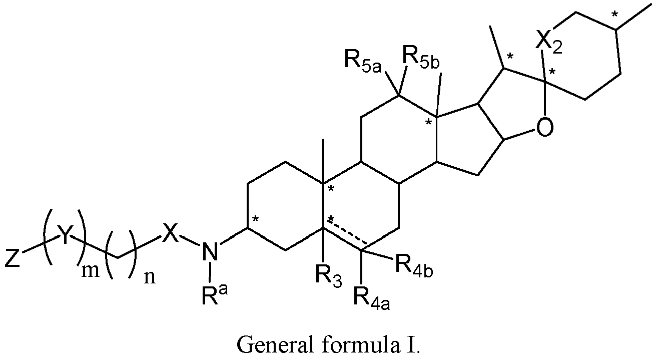

Through related in-vivo and in-vitro model activity tests for sarsasapogenin-based compounds, it is accidentally found that many derivative compounds thereof have superior cytoprotective activity, especially have unexpected protection activity for a plurality of indications associated with mitochondria, more specifically, for a plurality of brain neuron cells. In addition, such compounds have excellent blood brain permeability, making them potentially applicable and valuable for treating various diseases caused by mitochondrial dysfunction, addressing the limitations of sarsasapogenin-based compounds in the prior art, and providing important scientific and commercial values.

## Description

### CROSS-REFERENCE TO RELATED APPLICATIONS

This application is based on and claims priority to Chinese patent application No. 202210152310.X filed on February 18, 2022 and Chinese patent application No. 202310093727.8 filed on February 10, 2023, the disclosures of which are herein incorporated by reference in their entireties.

### FIELD OF THE INVENTION

The present invention relates to the field of medical technology, in particular to the field of medicinal chemical synthesis, and more particularly, to a novel use of derivatives based on sarsasapogenin structure and pharmaceutical compositions thereof in preparation of drugs for treating related diseases caused by mitochondrial dysfunction.

### BACKGROUND OF THE INVENTION

Rhizoma Anemarrhenae, as one of the common traditional Chinese medicinal materials in China, is dried tubers of a liliaceous plant, Anemarrhena asphodeloides Bunge, which has the effects of relieving thirst and warming Middle-Jiao, removing pathogenic qi and eliminating edema of limbs, and thus functions as one of the commonly used yin-nourishing drugs. Its extract has been shown to have biological activity of facilitating urination, resisting diabetes, platelet aggregation and fungi, regulating metabolism, etc., and also to exhibit inhibitory effects on cyclic adenosine monophosphate phosphodiesterase. The main chemical components of the extract are steroidal saponins, diphenylpyranones, polysaccharides and lignins. Among them, the steroidal saponins include sarsasapogenins A-I, A-II, A-III, A-IV, B-I, B-II and B-III, as well as markogenin3-O-β-D-glucopyranosyl(1→2)-β-D-galactopyranoside B, desgalactotigonin, F-gitogenin, smilagenin, etc. The steroidal saponins also contain Rhizoma Anemarrhenae polysaccharides A/B/C/D, cis-hinokiresinol, monomethyl-cis-hinokiresinol, oxy-cis-himokiresinol, 2,6,4'-trihydroxy-4-methoxybenzophenone, p-hydroxyphenyl crotonic acid, vinyl pentacosanoate, β-sitosterol, mangiferin, niacin, niacinamide, pantothenic acid, etc.

Mitochondrial functions and behaviors are central to the human physiology, and mitochondria perform a plurality of interrelated functions to produce ATP and many biosynthesis, meanwhile contribute to cellular stress responses such as autophagy and apoptosis. The mitochondria form a dynamic, interrelated network and are tightly bound together with other cellular compartments. In addition, the mitochondrial functions exceed cell borders and affect the physiology and tissues of organisms by regulating intercellular communications. A mitochondrial respiratory chain is mainly composed of mitochondrial respiratory chain enzymes, so defects in a mitochondrial respiratory chain enzyme complex are important causes of mitochondrial diseases (about 30% to 40% of mitochondrial diseases are due to defects in mitochondrial respiratory chain enzymes). According to structures and functions of human respiratory chain megacomplexes, oxidative phosphorylation is completed in steps by five respiratory chain protein complexes located on a mitochondrial inner membrane, which are complex I (NADH dehydrogenase), complex II (succinate dehydrogenase), complex III (cytochrome c reductase), complex IV (cytochrome c oxidase), and complex V (ATP synthase).

Therefore, it is not surprising that mitochondrial dysfunction is a key factor, including pathogenic factors of a variety of diseases, including neurodegenerative diseases and metabolic disorders. Advances in mitochondrial biology and their correlation to human disease were also discussed in the literature (doi: 10.1016/j.cell.2012.02.035.). At present, there are a variety of small molecule drugs that target respiratory chain complexes and act on a variety of indications, providing a variety of options for disease treatment. At present, existing drugs that target respiratory chain complexes are applicable for various indications, including: tumor cancers, cardiovascular diseases, endocrine diseases, immune diseases, coronavirus infections, inflammations, metabolic diseases, neurodegenerative diseases, intestinal diseases, autism, schizophrenia, Alzheimer's disease, Parkinson's disease, epilepsy, stroke, chronic fatigue syndrome, etc.

Under the action of small molecules on mitochondria, the complex can be maintained in an active state, and allosteric regulation for the activity and electron transport efficiency of the complex is also conducive to stabilizing the overall structure of the complex, thereby reducing the production of mitochondrial ROS (superoxide radicals) caused by electron leakage. Based on the regulation for the complex activity, it is possible to implement the overall regulation for energy metabolism pathways in cells and even the body, as well as the impact on epigenetic modification of genomes.

Stroke, commonly known as apoplexia, includes ischemic stroke (cerebral infarction) and hemorrhagic stroke (intraparenchymal hemorrhage, ventricular hemorrhage, and subarachnoid hemorrhage). As defined by the World Health Organization, a plurality of causes of stroke leads to cerebrovascular injuries and focal (or global) brain tissue damages, resulting in clinical symptoms for more than 24 h or death. Stroke has the characteristics of high morbidity, disability, recurrence and mortality. Stroke is the leading cause of death in China.

Amyotrophic lateral sclerosis, also known as a motor neuron disease, is a chronic, progressive degenerative disease for upper and lower motor neurons and their innervations of the trunk, limbs, as well as head and face muscles. It often manifests as progressive aggravation of muscle weakness, muscle atrophy, fasciculation and the like caused by merged damages of upper and lower motor neurons. Its clinical manifestations include: progressive aggravation of skeletal muscle weakness, muscle atrophy, fasciculation, bulbar palsy and pyramidal tract signs. Since early symptoms of patients are mild, and easy to be confused with other diseases, the patients may just have some weakness, muscle beats, easy fatigue and other symptoms, which gradually progress to generalized muscle atrophy and dysphagia, and finally produce respiratory failure. At present, the main theories of the causes of motor neuron damages include: 1, the accumulation of neurotoxic substances and the accumulation of glutamate in nerve cells cause damages to the nerve cells over time; 2, free radicals damage nerve cell membranes; and 3, the lack of nerve growth factors makes it impossible for the nerve cells to continue to grow and develop. At present, the only internationally recognized drug approved by the U.S. Food and Drug Administration for treating amyotrophic lateral sclerosis is Rilutek, which needs be used as soon as possible. Meanwhile, neurotrophic factors, antioxidants such as vitamin E, vitamin C and creatine, and CoQ10 are also being tested internationally in combination with Rilutek to provide protective treatment for amyotrophic lateral sclerosis, but it has yet to be confirmed by clinical trials.

Neurodegenerative diseases are divided into acute neurodegenerative diseases and chronic neurodegenerative diseases. The acute neurodegenerative diseases mainly include stroke, brain injury (BI), epilepsy, etc. The chronic neurodegenerative diseases mainly include Alzheimer's disease (AD), Parkinson's disease (PD), Huntington's disease (HD), amyotrophic lateral sclerosis (ALS), different types of spinocerebellar ataxia (SCA), and Pick disease. The causes of neurodegenerative diseases mainly lie in the following four aspects: 1, oxidative stress. The oxidative stress is caused by excessive production and/or failure of timely removal of free radicals, imbalance between oxidation and antioxidation effects in vivo, and damages to body's cells and tissues. Free radicals are atoms or groups with unpaired electrons, including hydroxyl radicals, superoxide anions, nitric oxide, etc. In recent years, oxidative damages to nerve tissue have been found in neurodegenerative diseases such as AD, PD, and ALS. 2, Mitochondrial dysfunctions. There are mtDNA defects and abnormal oxidative phosphorylation in the brains of AD patients. It has been found by polymerase chain reaction (PCR) and blot hybridization detection that mtDNA fragmentation, base deletion and mistranslation mutations appear in brain tissues of sporadic AD patients. Electron microscopy observations have confirmed the increase in the number of mitochondria and the structural abnormality, accompanied with lamellar and crystalline inclusions. In addition, the neuronal mitochondrial dysfunctions in AD patients will lead to insufficient supply of neuronal energy and release of a large amount of ROSs, which can induce oxidative stress damages and imbalance of calcium regulation, and ultimately trigger neuronal apoptosis. 3, Excitant toxins. If a glutamate concentration in an intercellular space is too high, toxins will be produced in neurons, leading to degeneration, aging and death of neurons. This excitotoxic effect of glutamate is closely related to the occurrence and development of a variety of neurodegenerative diseases, and is thus regarded as one of the important mechanisms leading to nerve cell death in neurodegenerative diseases. 4, Immune inflammations. Many evidences have shown that inflammations play an important role in the pathogenesis of AD. An innate immune system is an innate immune defense function that is formed during germline development and evolution. In contrast to another specific immune response of the body, this system responds quickly to a variety of harmful substances to protect the body. The activation of the innate immune system itself is a double-edged sword. Long-term and uncontrollable stimulation of harmful substances (such as aggregated forms of Aβ) activates the innate immune system, which can have a damaging effect on the brain.

Corona Virus Disease 2019 named by the World Health Organization, also known as "COVID-19", refers to an acute respiratory infectious disease caused by COVID-19 infections. On March 11, 2020, the World Health Organization assessed the outbreak of COVID-19 epidemic at the time as a global pandemic. The clinical manifestations of pneumonia patients infected by COVID-19 are as follows: fever, fatigue, dry cough and other main manifestations may lead to anoxia and hypoxia, about half of the patients suffer dyspnea after one week, and severe cases rapidly progress to acute respiratory distress syndromes, septic shock, metabolic acidosis that is difficult to correct, and coagulation disorders. At present, prevention methods for COVID-19 are mainly vaccines and isolation, while treatment drugs include Paxlovid, Azvudine Tablets, Molnupiravir Capsules, San Han Hua Shi Granules, Lung Cleaner, etc.

Finding novel urgent drugs with high efficiency, high selectivity, low toxicity and good brain permeability is still extremely challenging, and it is quite urgent to prevent, reduce and treat COVID-19 infections, reduce the mortality and eliminate sequelae. Lowering the free radical level in the brain is considered treatment means. Sarsasapogenin-based compounds have been shown to reduce the free radical level and protect mitochondria for better and more efficient use of oxygen.

Therefore, it is of great practical value to develop and rationally use compounds for treating diseases caused by mitochondrial respiratory chain abnormalities.

### SUMMARY OF THE INVENTION

An object of the present invention is to overcome the defects in the prior art. To fulfill said object, a first aspect of the present invention provides a use of derivatives based on a sarsasapogenin structure in preparation of drugs for treating related diseases caused by mitochondrial dysfunction, and is mainly characterized in that the structural formula of the derivatives is represented by general formula I, 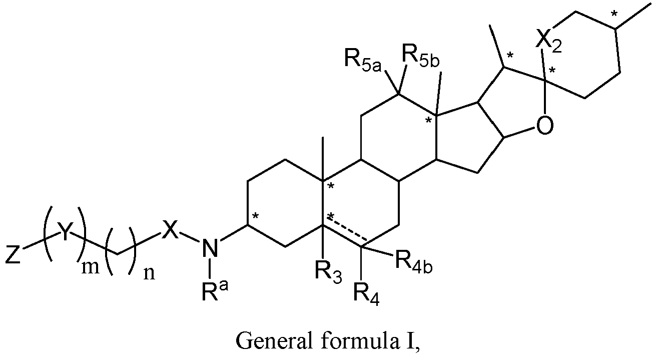 the derivatives represented by general formula I are formed by connecting the following fragment A and fragment B, 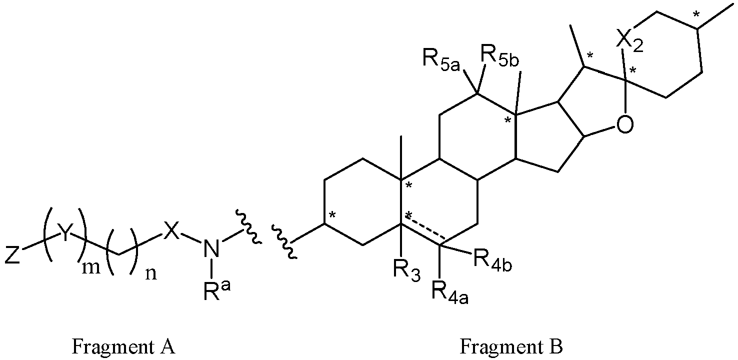
wherein Z is NR¹R²; R¹ and R² are each independently hydrogen, substituted or unsubstituted C₁-C₁₀ alkyl, and the substituents of C₁-C₁₀ alkyl are selected from halogen, hydroxyl, amino, nitro, cyano, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, C₃-C₁₄ heterocyclyl, or C₃-C₁₄ heteroaryl, and the heteroatoms are selected from one or more of N, O, S; or R¹ and R² together form a three- to eight-membered ring, the three- to eight-membered ring has one or more substituents selected from C₁-C₁₀ alkyl, C₃-C₁₀ cycloalkyl, C₆-C₂₀ aryl, C₃-C₁₄ heteroaryl, halogen, hydroxyl, amino, alkoxyl, -CF₃, -SF₅ or three- to eight-membered heterocyclyl whose heteroatoms are S, O, NH or NR^{a}, and the heteroatoms are selected from one or more of N, O, S;
X is C(O) or S(O)₂;
Y is C(R^{d})(R^{e}), C(O), or S(O)₂; R^{d} and R^{e} are each independently hydrogen, or C₁-C₁₀ alkyl, C₃-C₁₀ cycloalkyl, C₆-C₂₀ aryl or C₃-C₁₄ heteroaryl with at least one substituent, wherein the substituents are selected from halogen, hydroxyl, amino, nitro, cyano, aldehyde group, carboxyl, alkoxyl, -CF₃, or - SF₅, and the heteroatoms are selected from one or more of N, O, S; or R^{d} and R^{e} together form a three- to eight-membered ring, the three- to eight-membered ring has one or more substituents selected from C₁-C₁₀ alkyl, C₃-C₁₀ cycloalkyl, C₆-C₂₀ aryl, C₃-C₁₄ heteroaryl, halogen, hydroxyl, amino, alkoxyl, -CF₃, -SF₅, or three- to eight-membered heterocyclyl whose heteroatoms are S, O, NH or NR^{a}, the heteroatoms are selected from one or more of N, O, S;
X₂ is O, S, or NH;
R^{a} is independently hydrogen or C₁-C₁₀ alkyl, C₃-C₁₀ cycloalkyl, C₆-C₂₀ aryl, or C₃-C₁₄ heteroaryl with at least one substituent, wherein the substituents are selected from halogen, hydroxyl, amino, nitro, cyano, alkoxyl, -CF₃ or -SF₅, and the heteroatoms are selected from one or more of N, O, S;
n is an integer from 0 to 10, n is not 0 and m is 1, or n is 0 and m is 1, or n is an integer from 0 to 10, n is not 0 and m is 0;
R₃, R₄ₐ, R_{4b}, R₅ₐ, and R_{5b} are each independently hydrogen or selected from halogen, substituted alkyl, hydroxyl, and amino;
" ------" represents a single bond or double bond; and
each "*" independently represents a racemic, S or R configuration.

Preferably, the structural formula of the derivatives is represented by general formula II, 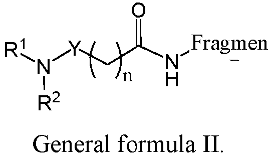 preferably, the structural formula of the derivatives is represented by general formula III, 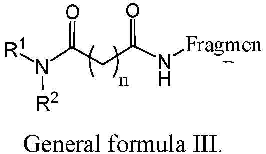 preferably, the structural formula of the derivatives is represented by general formula IV,

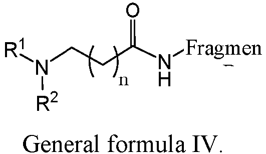

Preferably, the structural formula of the derivatives is represented by general formula V, 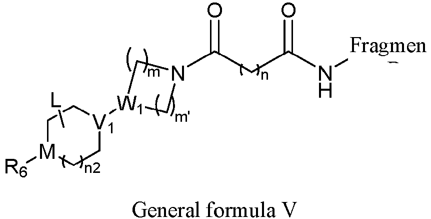
wherein, R₆ is hydrogen, substituted or unsubstituted C₁-C₁₀ alkyl, the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, pyridyl, -CO alkyl, -CO aryl, -SO₂ alkyl, -SO₂ aryl, -CO₂ alkyl, C₂-C₄(CO) alkenyl, - CO₂ aryl, -SO₃H;
L is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl;
n is an integer from 0 to 10;
n2 is 0, 1, 2, or 3;
m, m' are independently an integer from 1 to 4;
W₁ is C or NH;
V₁ is C or NH; and
Mis C, S, O or NH;
preferably, the structural formula of the derivatives is represented by general formula VI, 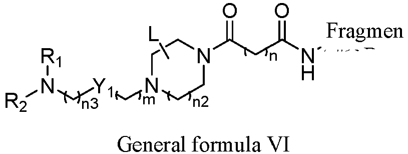
Y₁ is C(Rd)(Re), C(O) or S(O)₂, Rd and Re are independently hydrogen or C₁-C₁₀ alkyl, C₃-C₁₀ cycloalkyl, C₆-C₂₀ aryl, or C₃-C₁₄ heteroaryl with at least one substituent, wherein the substituents are selected from halogen, hydroxyl, amino, nitro, cyano, aldehyde, carboxyl, alkoxyl, -CF₃ or -SF₅, the heteroatoms are selected from one or more of N, O, S, or R^{d} and R^{e} together form a three- to eight-membered ring, the three- to eight-membered ring has one or more substituents selected from C₁-C₁₀ alkyl, C₃-C₁₀ cycloalkyl, C₆-C₂₀ aryl, or C₃-C₁₄ heteroaryl, halogen, hydroxyl, amino, alkoxyl, -CF₃, -SF₅ or three to eight-membered heterocyclyl whose heteroatoms are sulfur, oxygen, NH or NR^{a}, and the heteroatoms are selected from one or more of N, O, S;
L is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl;
n is an integer from 0 to 10;
n2 is 0, 1, 2, or 3;
n3 is an integer from 1 to 10; and
m is an integer from 0 to 10.
preferably, the structural formula of the derivatives is represented by general formula VII, 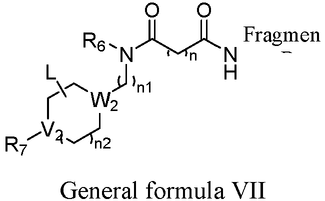
wherein, R₆ and R₇ are independently hydrogen, substituted or unsubstituted C₁-C₁₀ alkyl, the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, pyridyl, -CO alkyl, -CO aryl, -SO₂ alkyl, -SO₂ aryl, -CO₂ alkyl, C₂-C₄(CO) alkenyl, -CO₂ aryl, -SO₃H;
L is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl;
W₂ is C or NH;
V₂ is C, O, S, or NH;
n is an integer from 0 to 10;
n1 is an integer from 1 to 10; and
n2 is 0, 1, 2, or 3.
preferably, the structural formula of the derivatives is represented by general formula VIII, 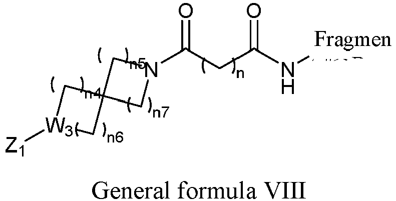
Z₁ is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, pyridyl, -CO alkyl, -CO aryl, -SO₂ alkyl, -SO₂ aryl, -CO₂ alkyl, C₂-C₄ (CO) alkenyl, -CO₂ aryl, -SO₃H;
W₃ is C, S, O, or NH;
n is an integer from 0 to 10; and
n4, n5, n6, and n7 are integers from 1 to 4.

In another preferred embodiment, the fragment B in the structural formula of the derivatives is as follow: 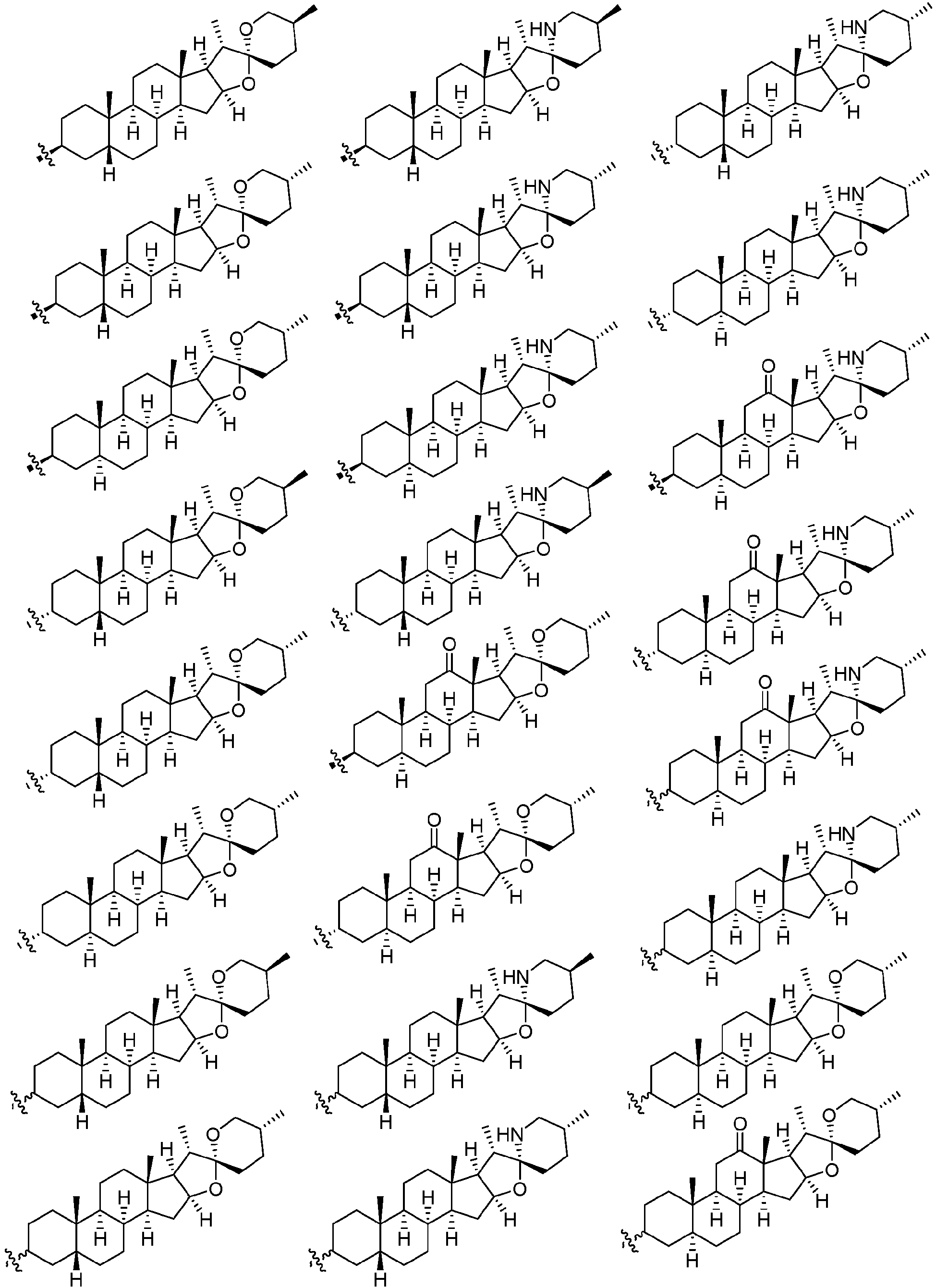 preferably, the derivatives are selected from one of the following compounds, mixtures of diastereomers of the following compounds, or enantiomers of the following compounds: 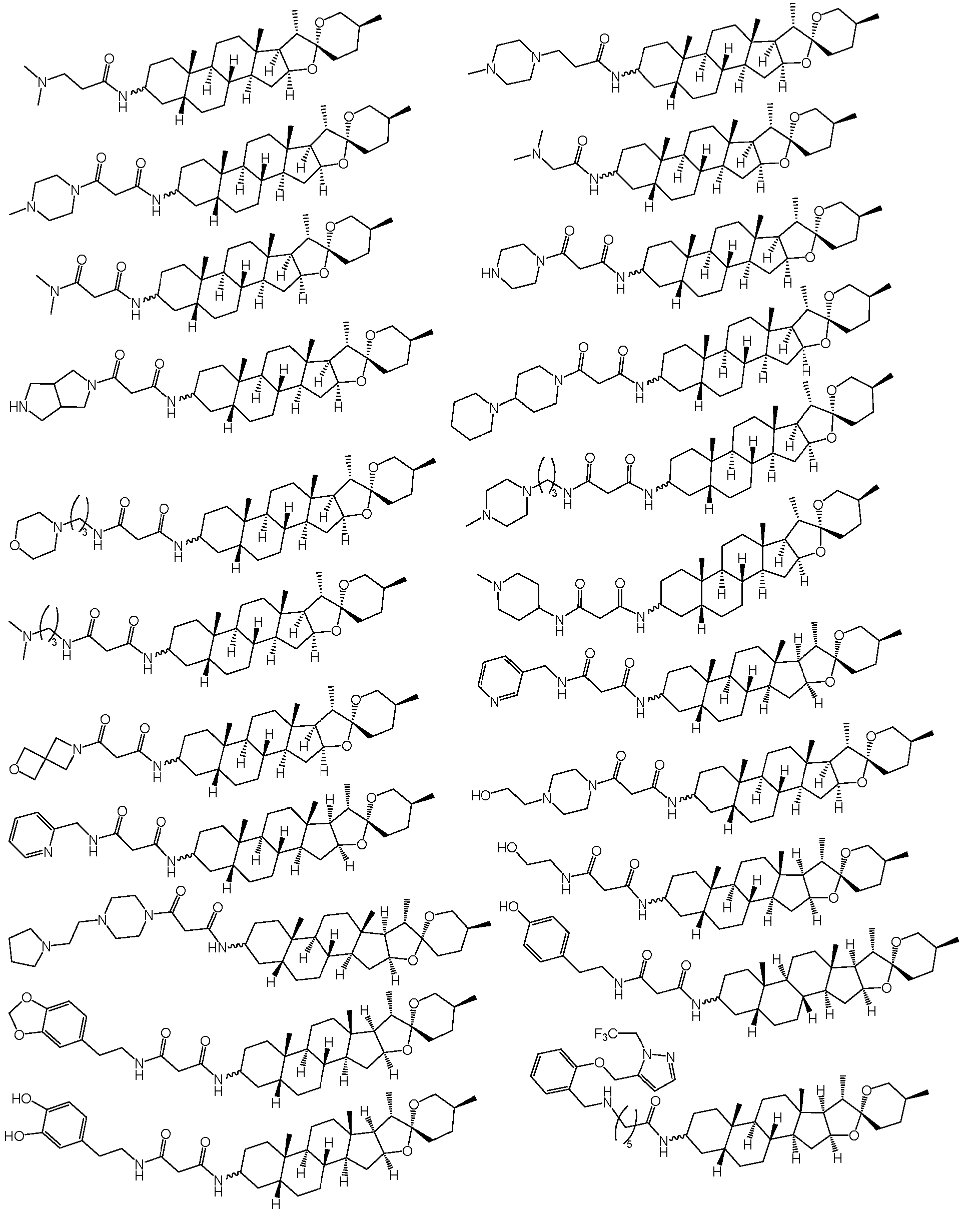 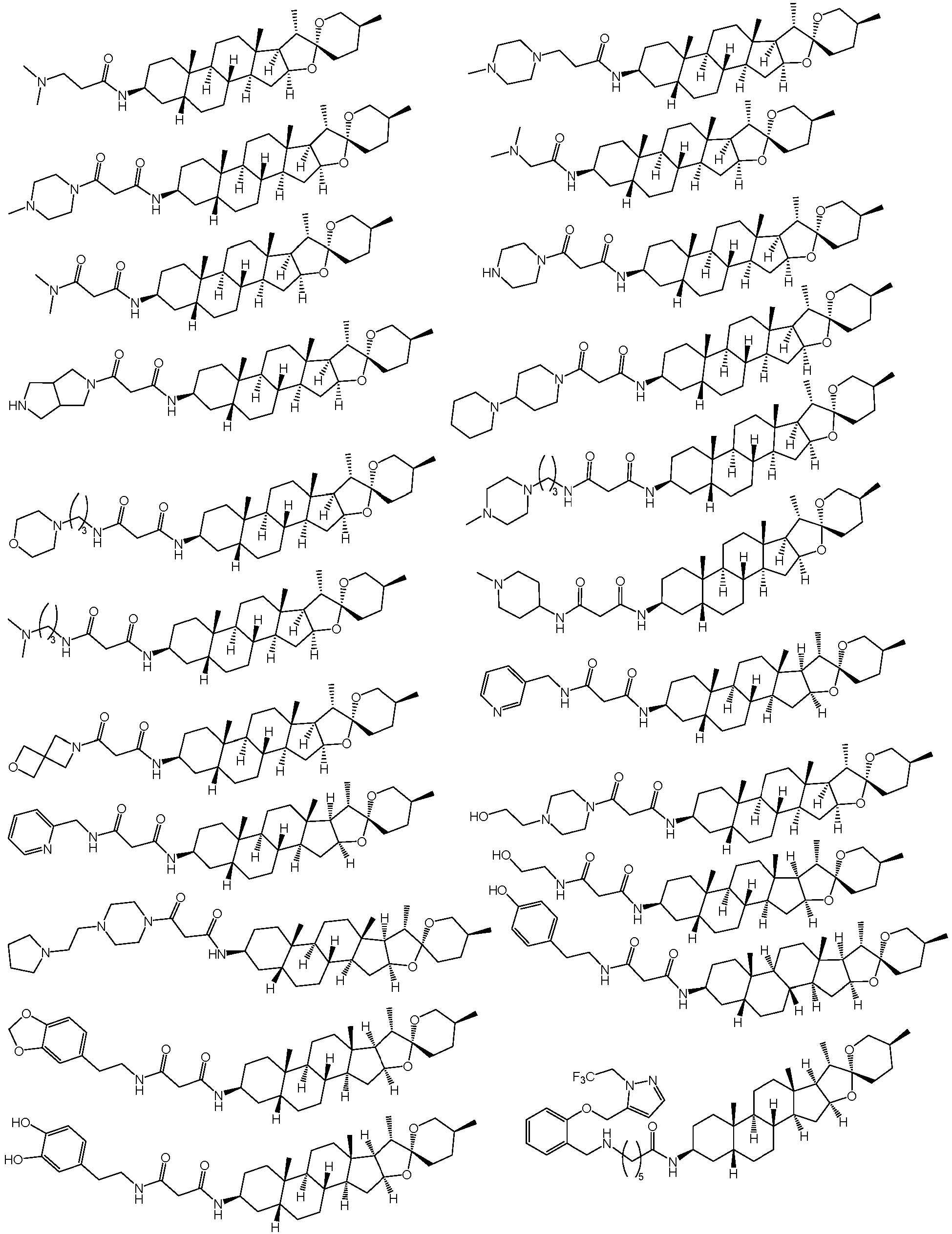 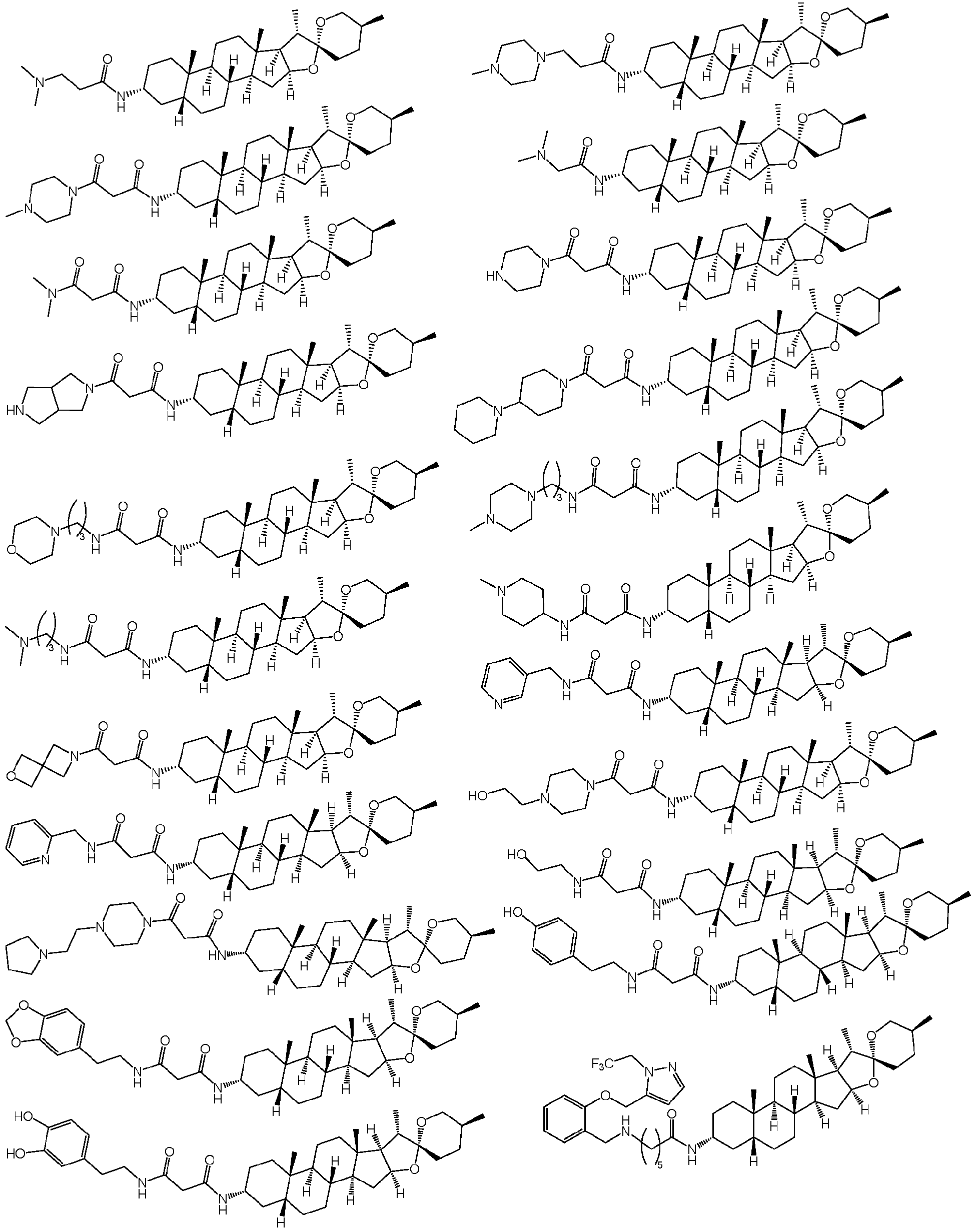 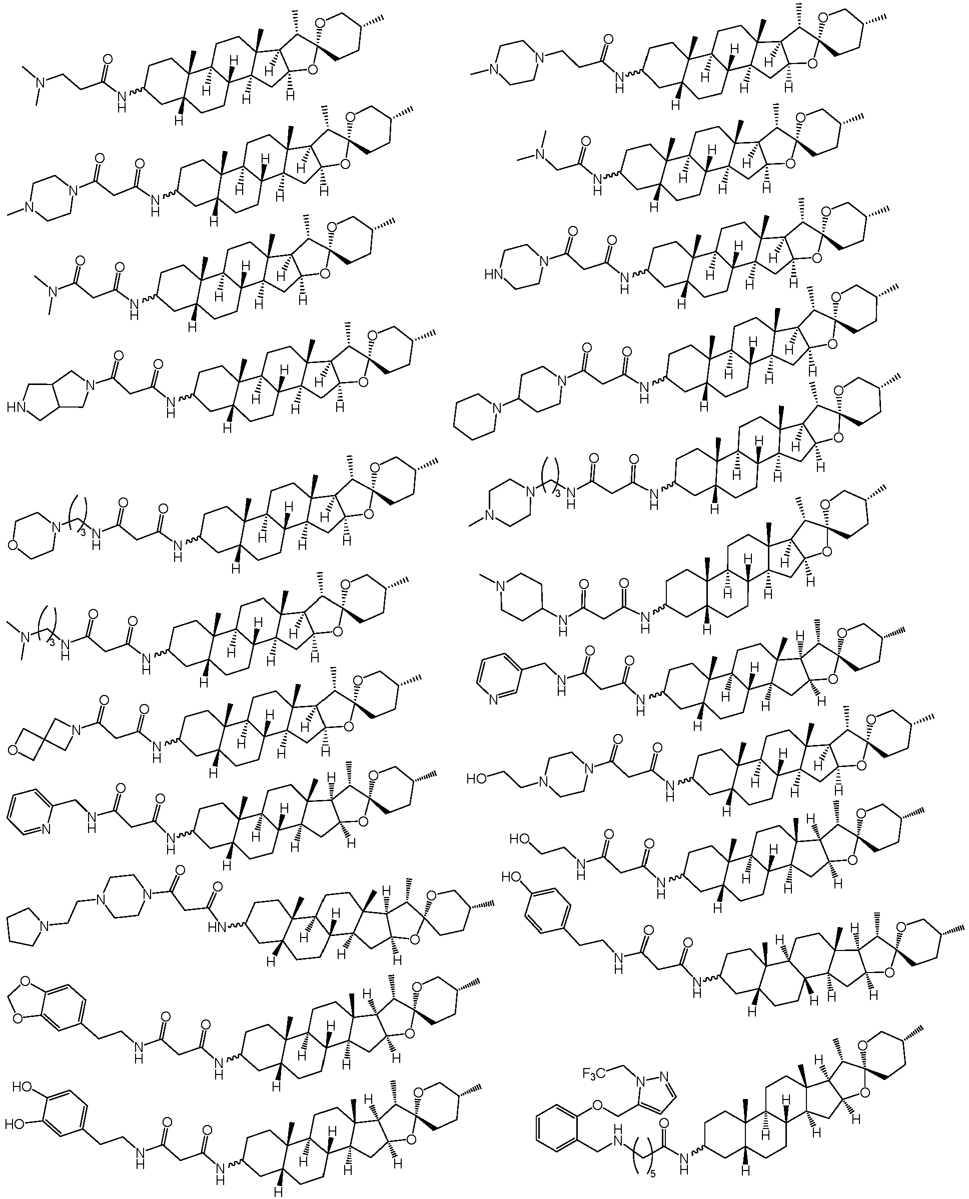 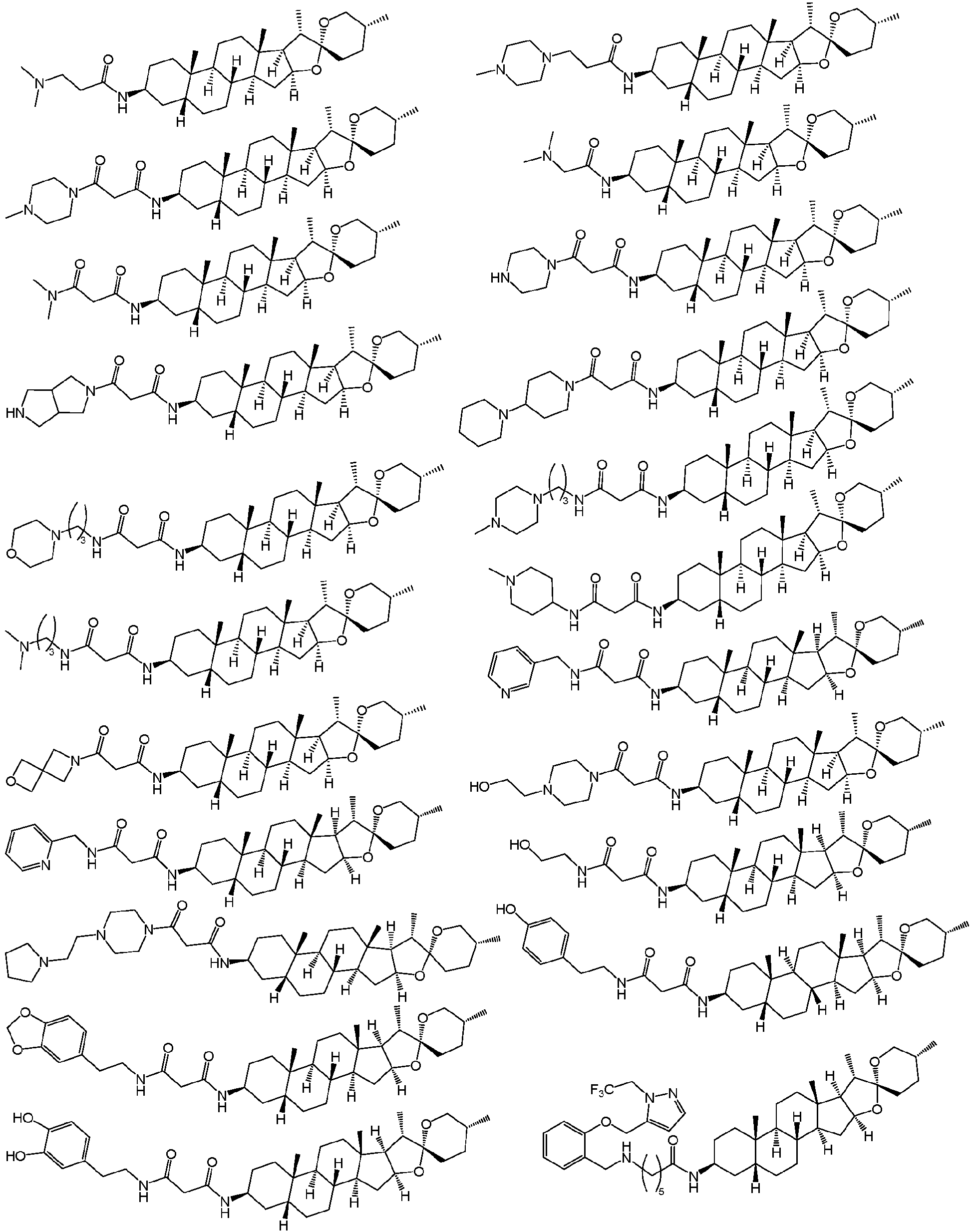 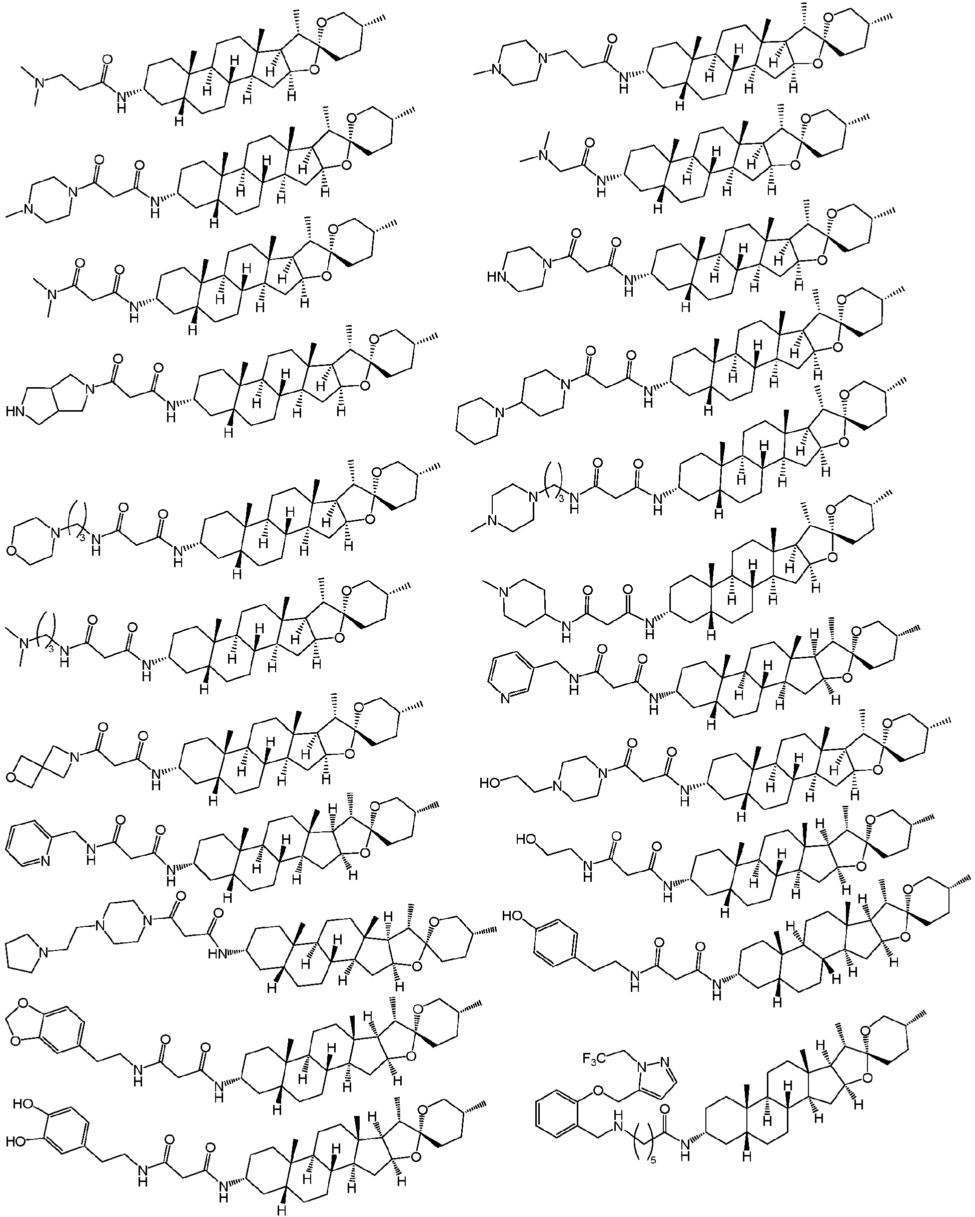 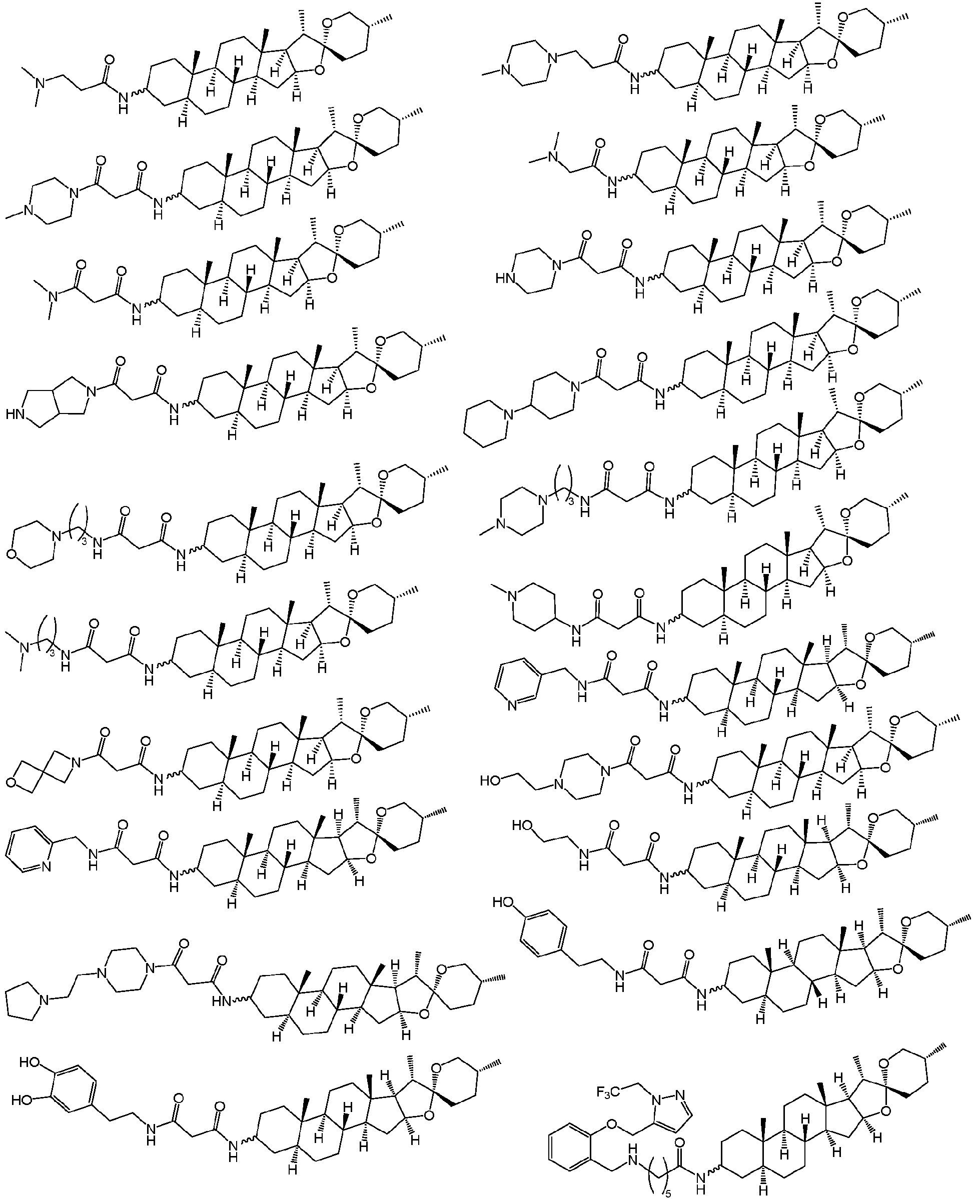 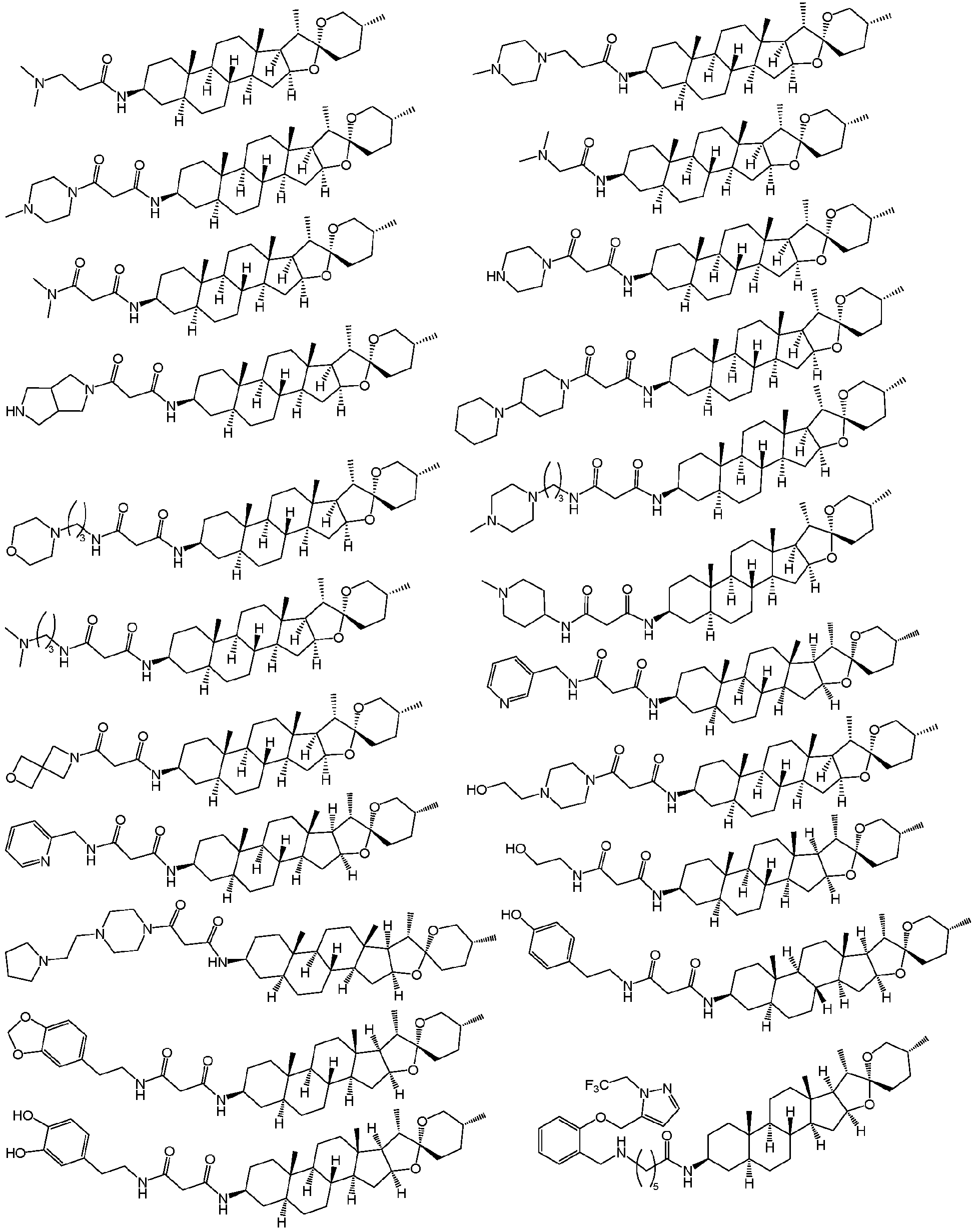 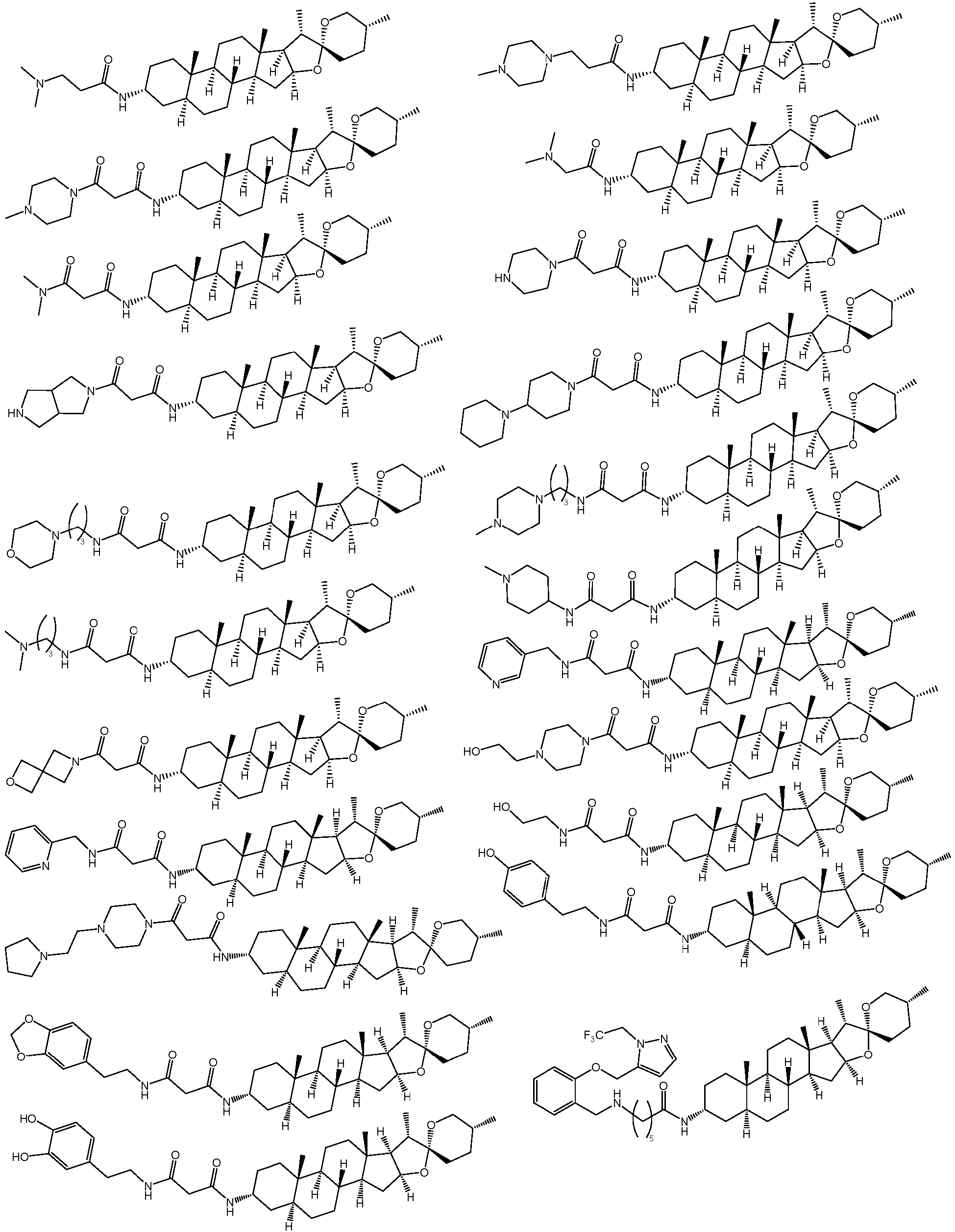 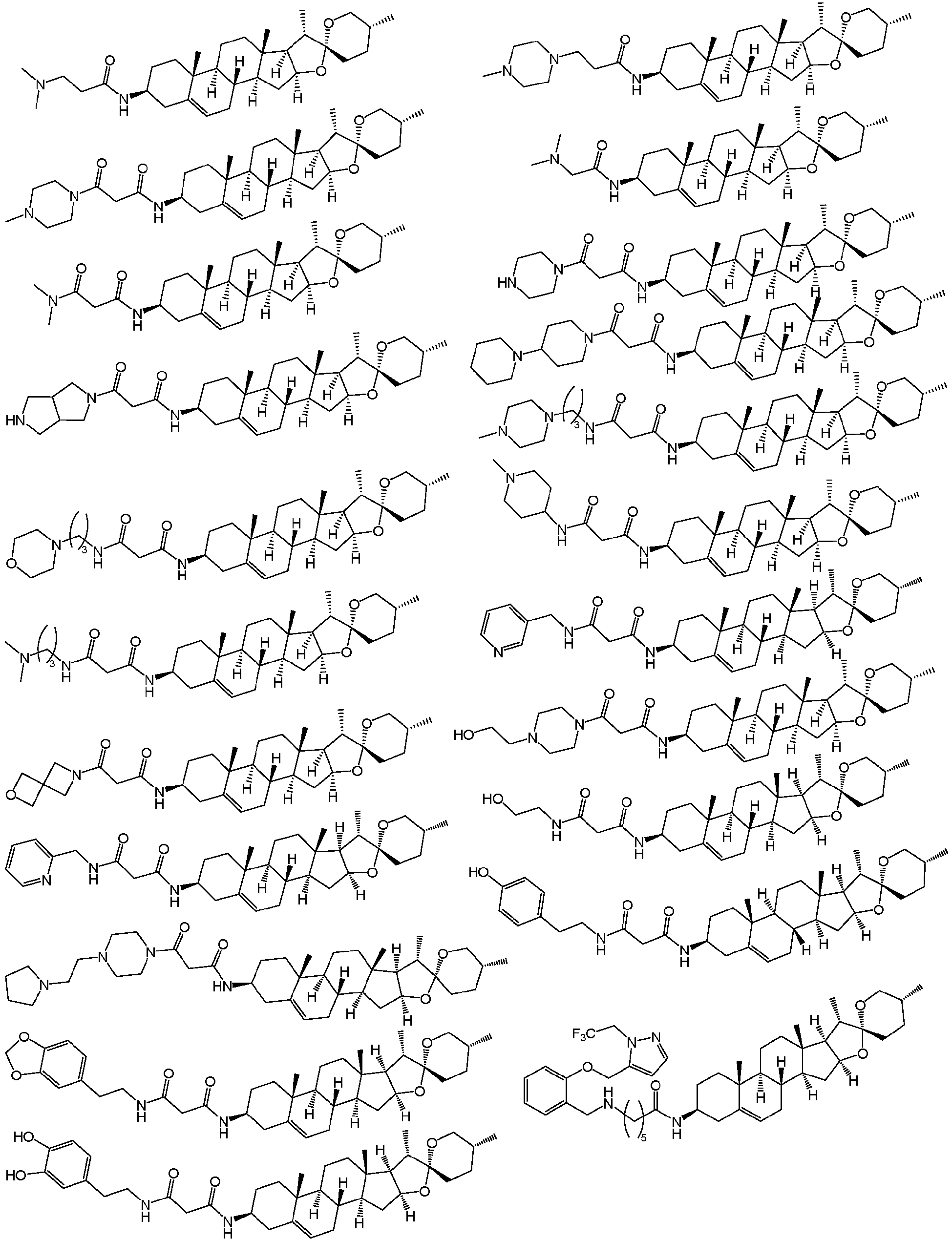 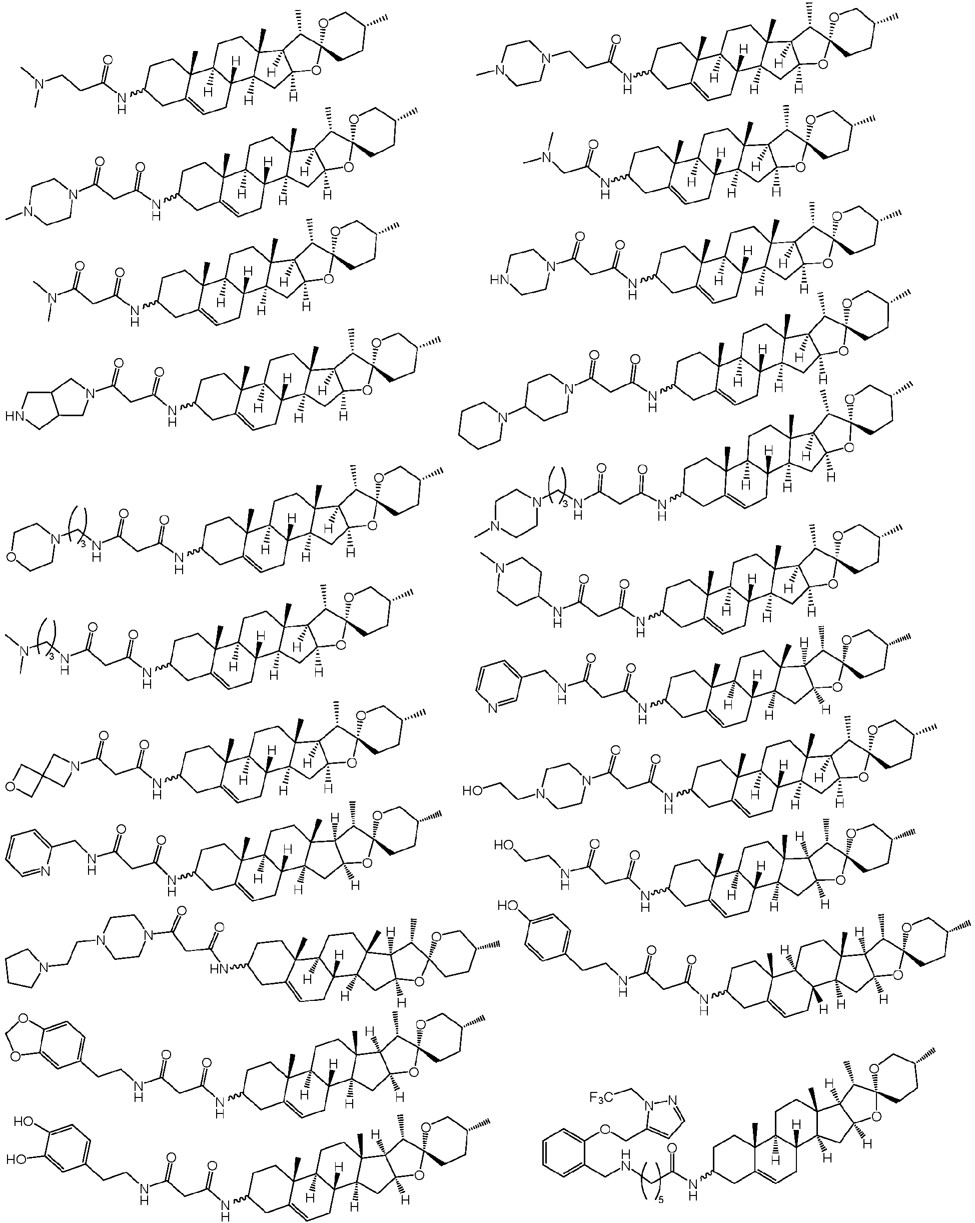 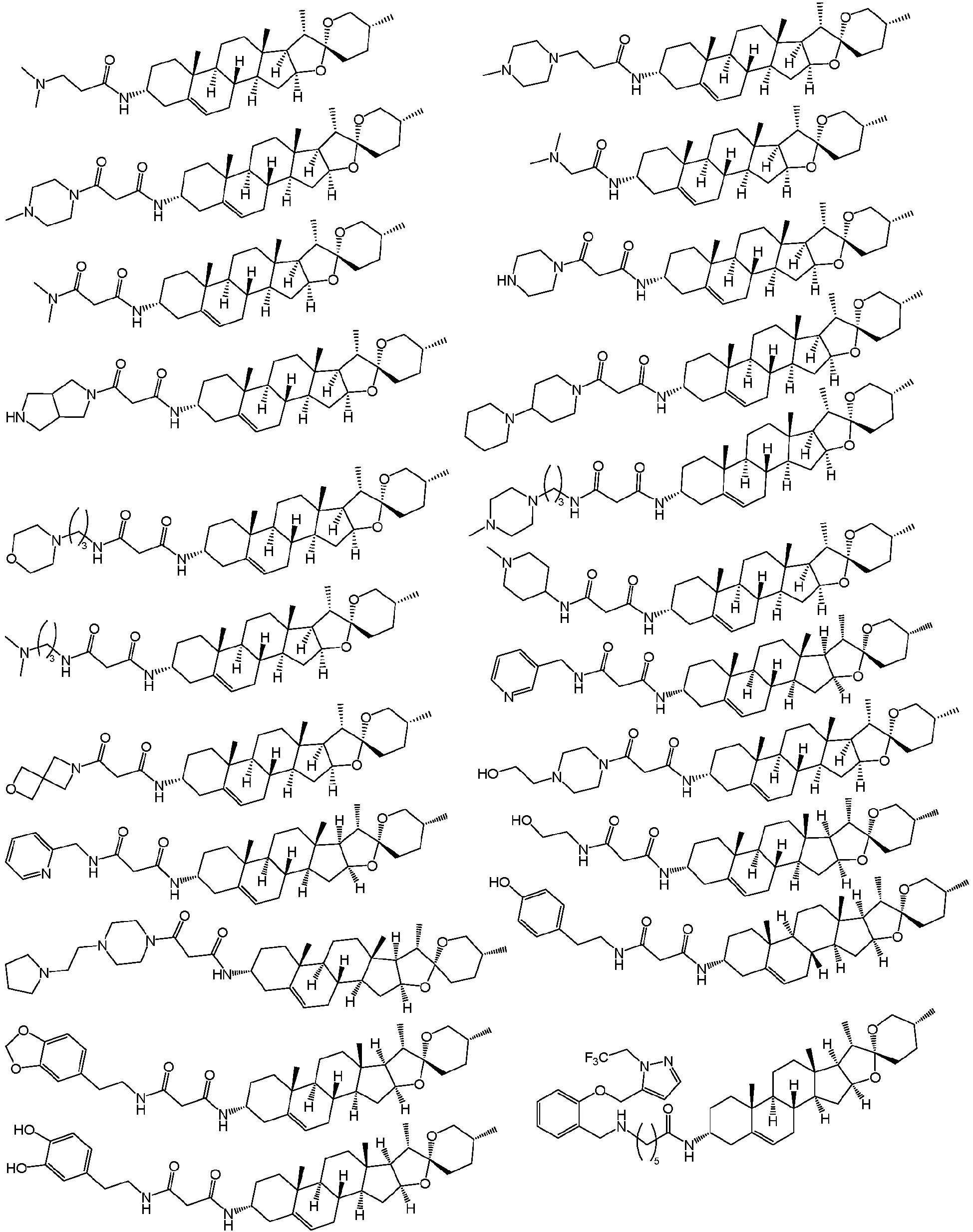 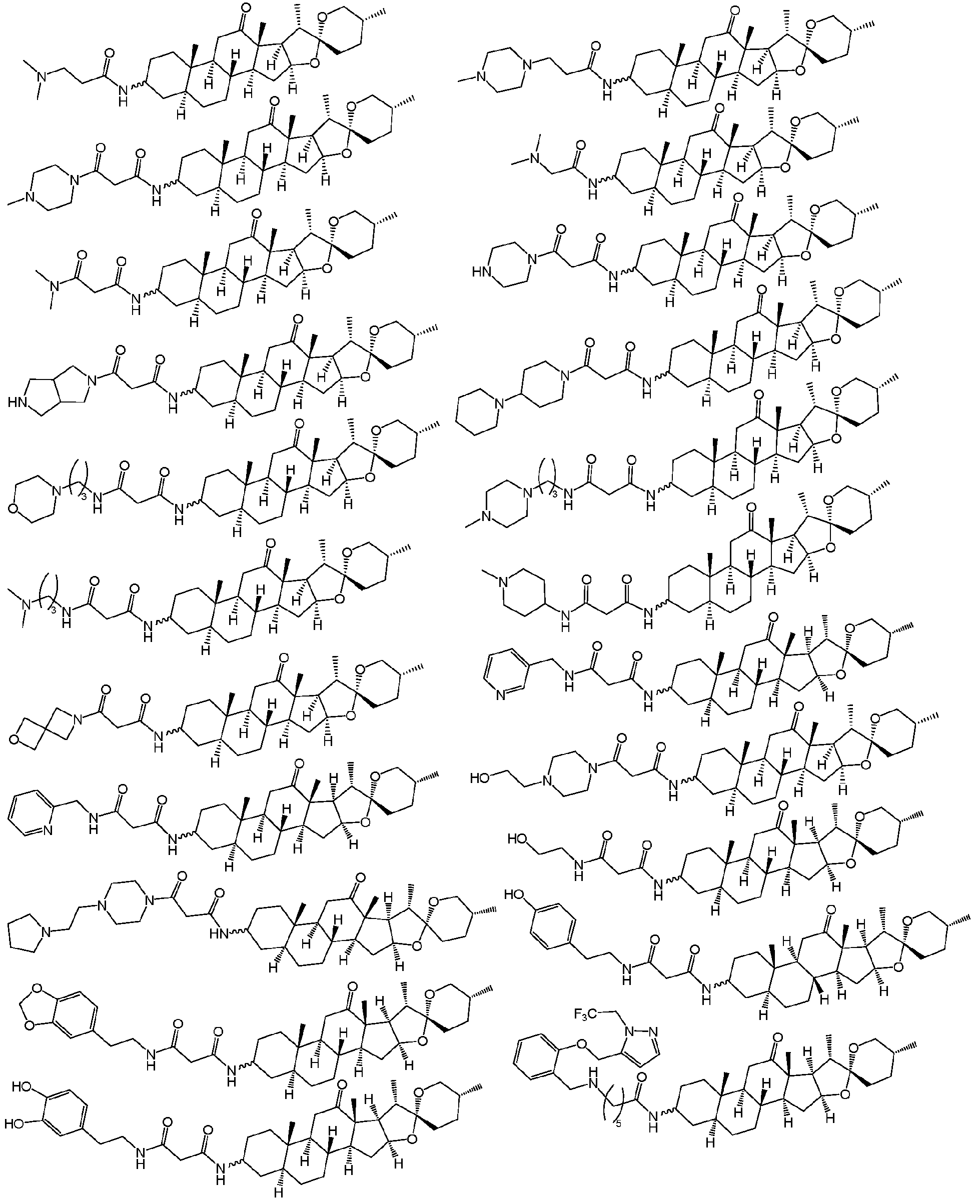 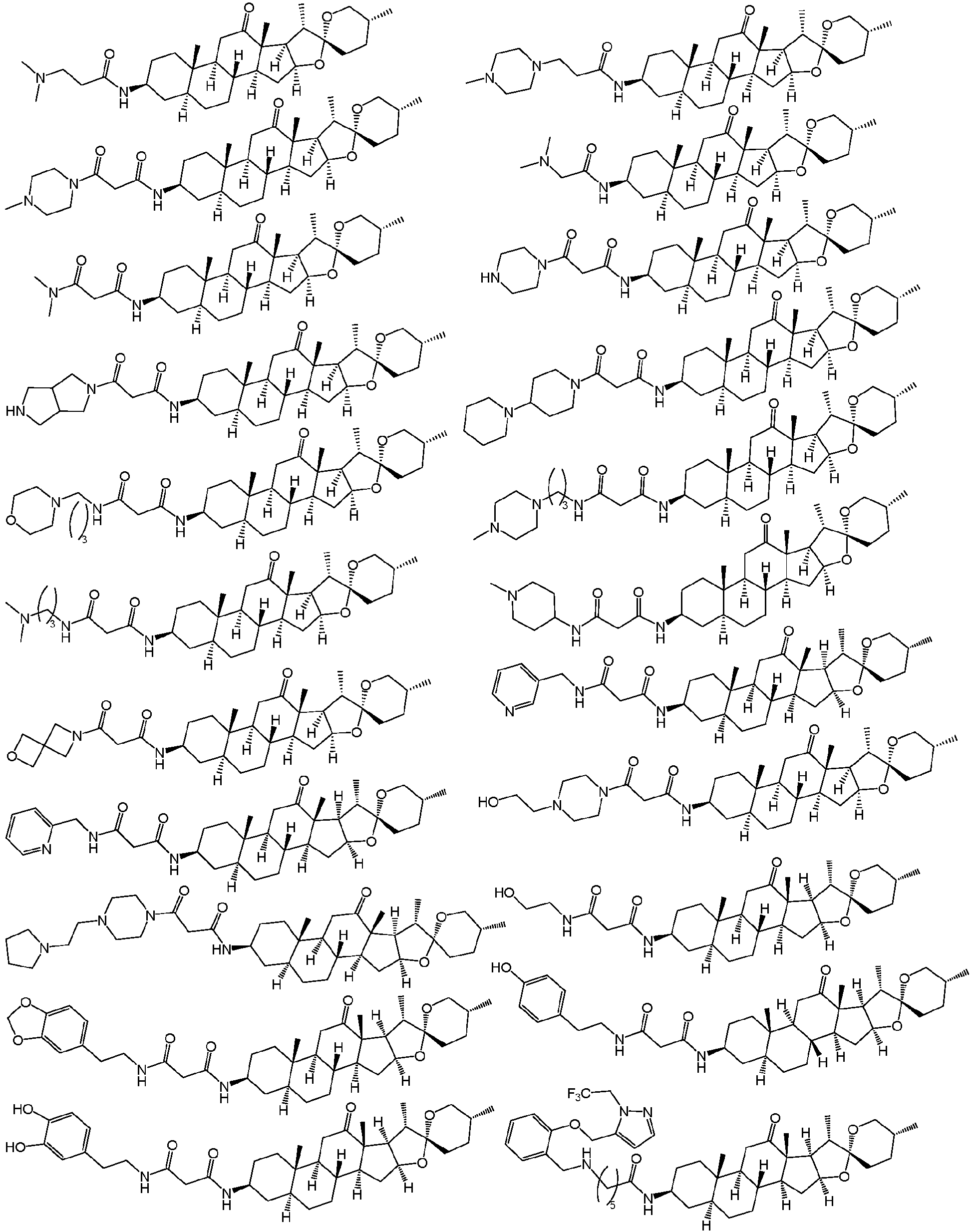 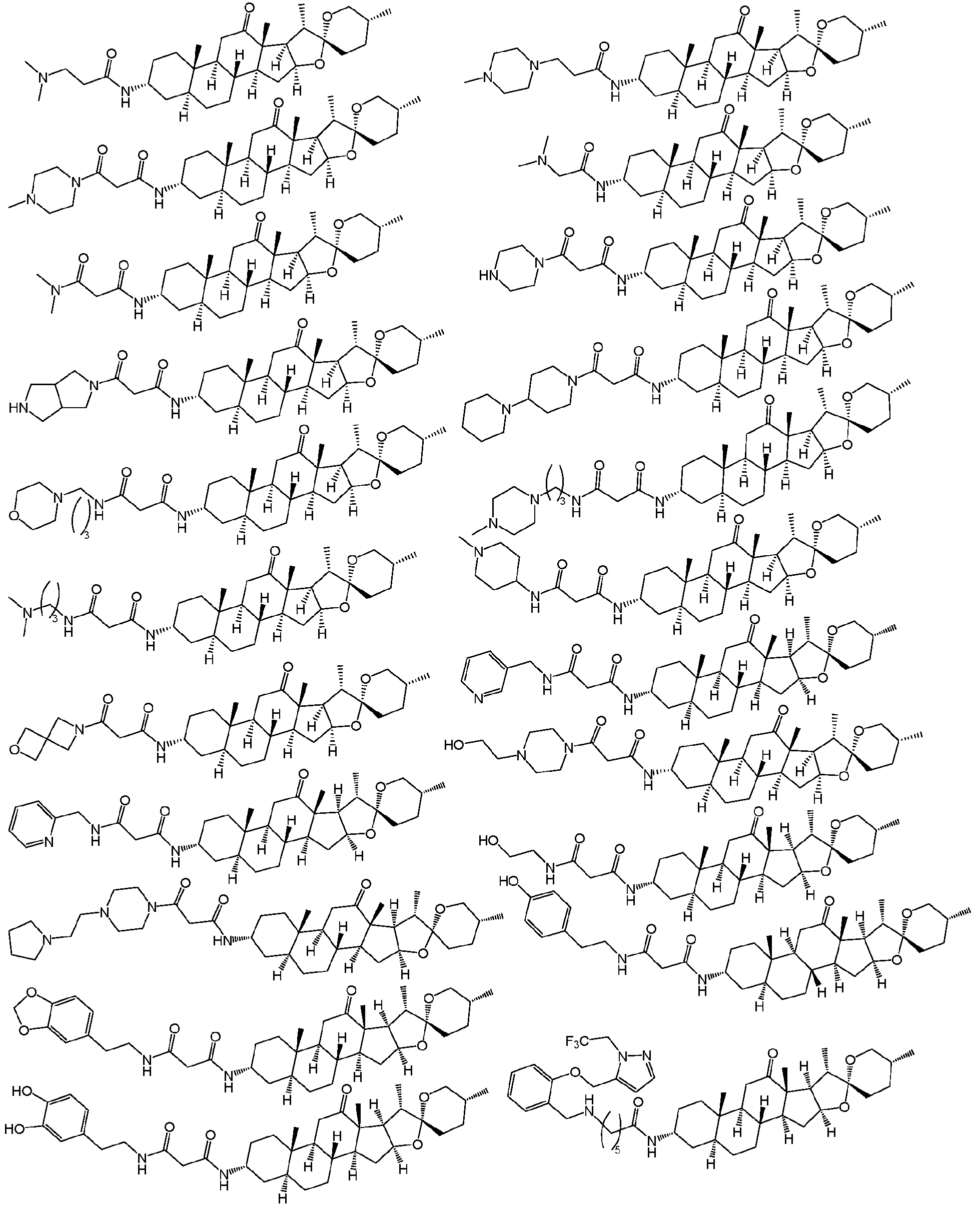 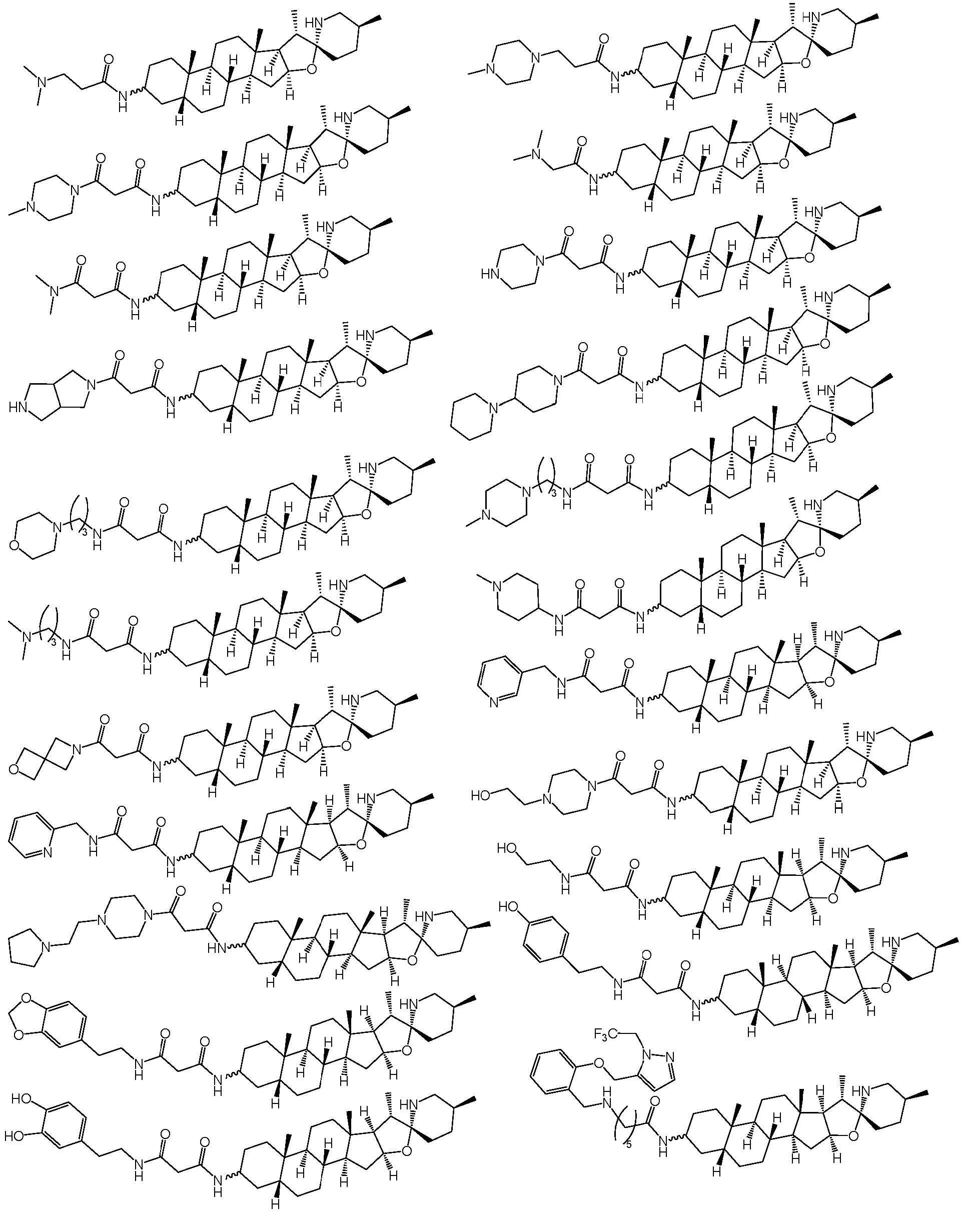 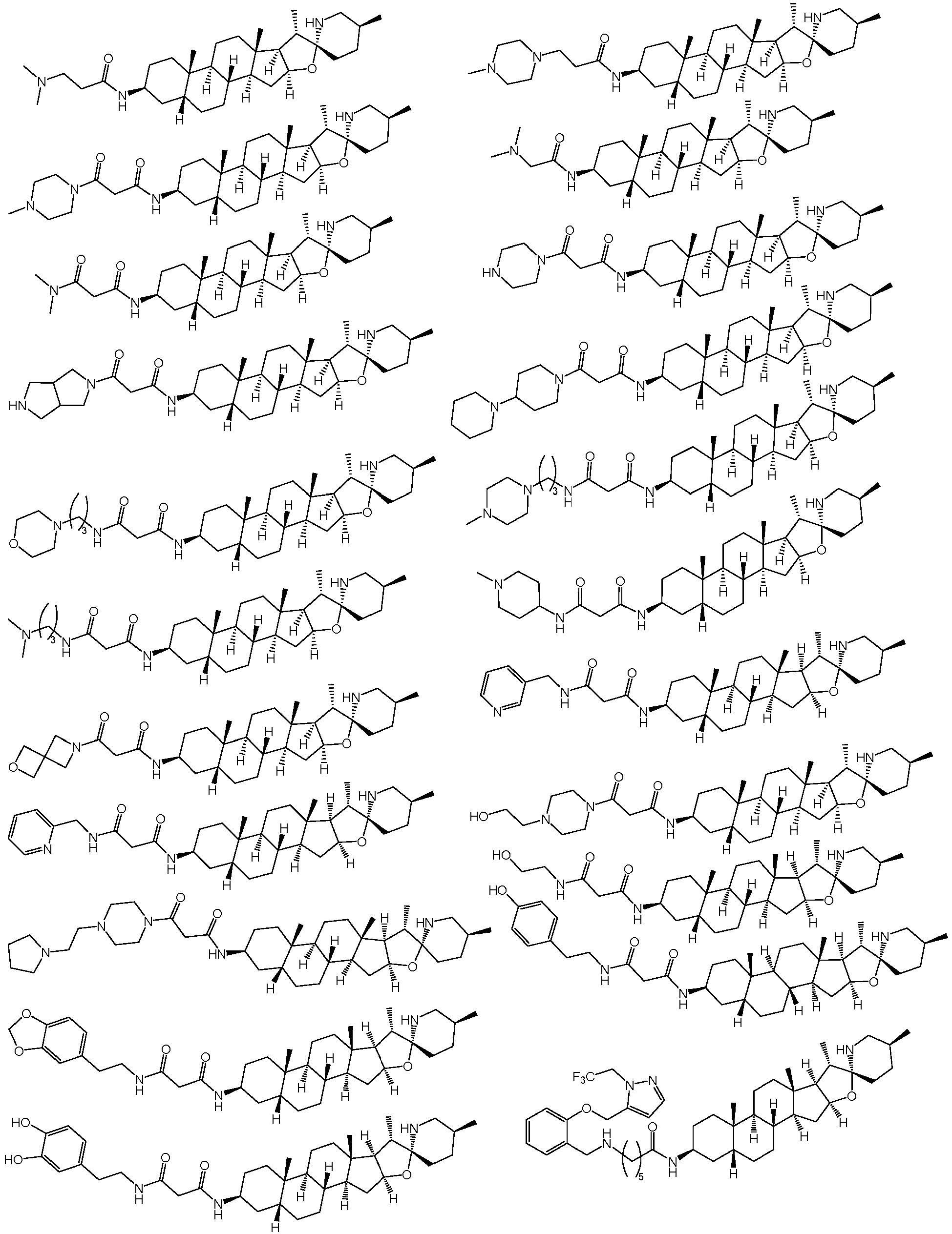 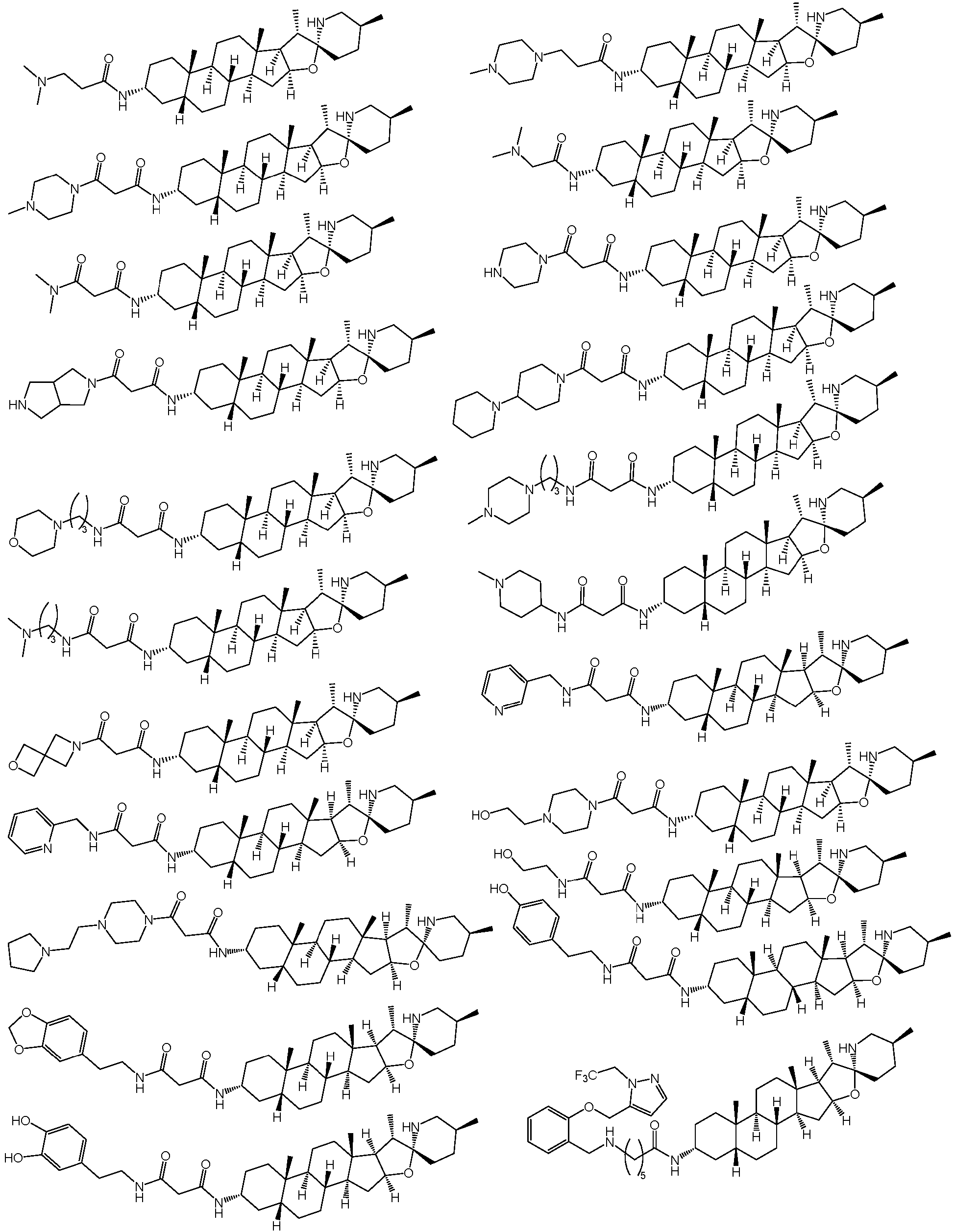 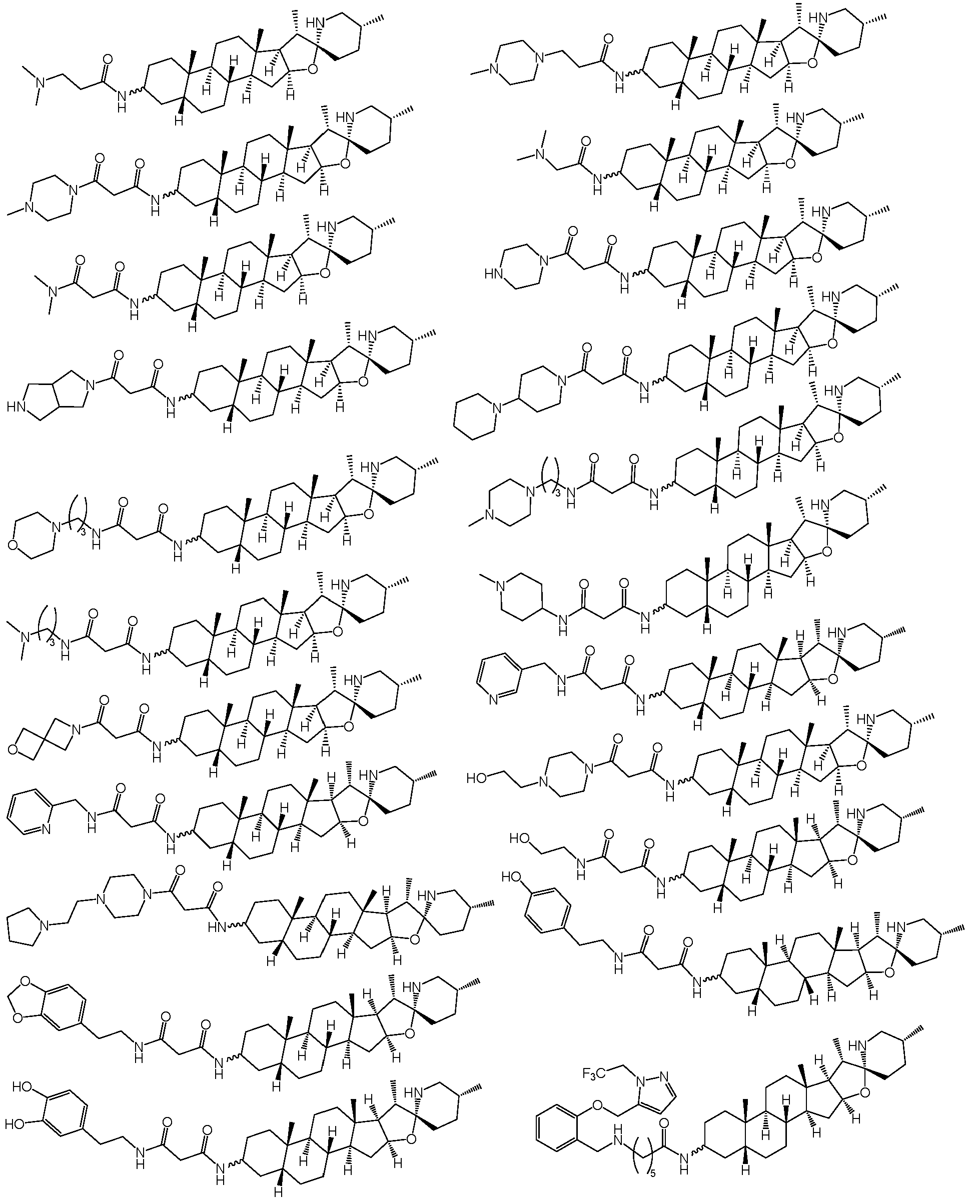 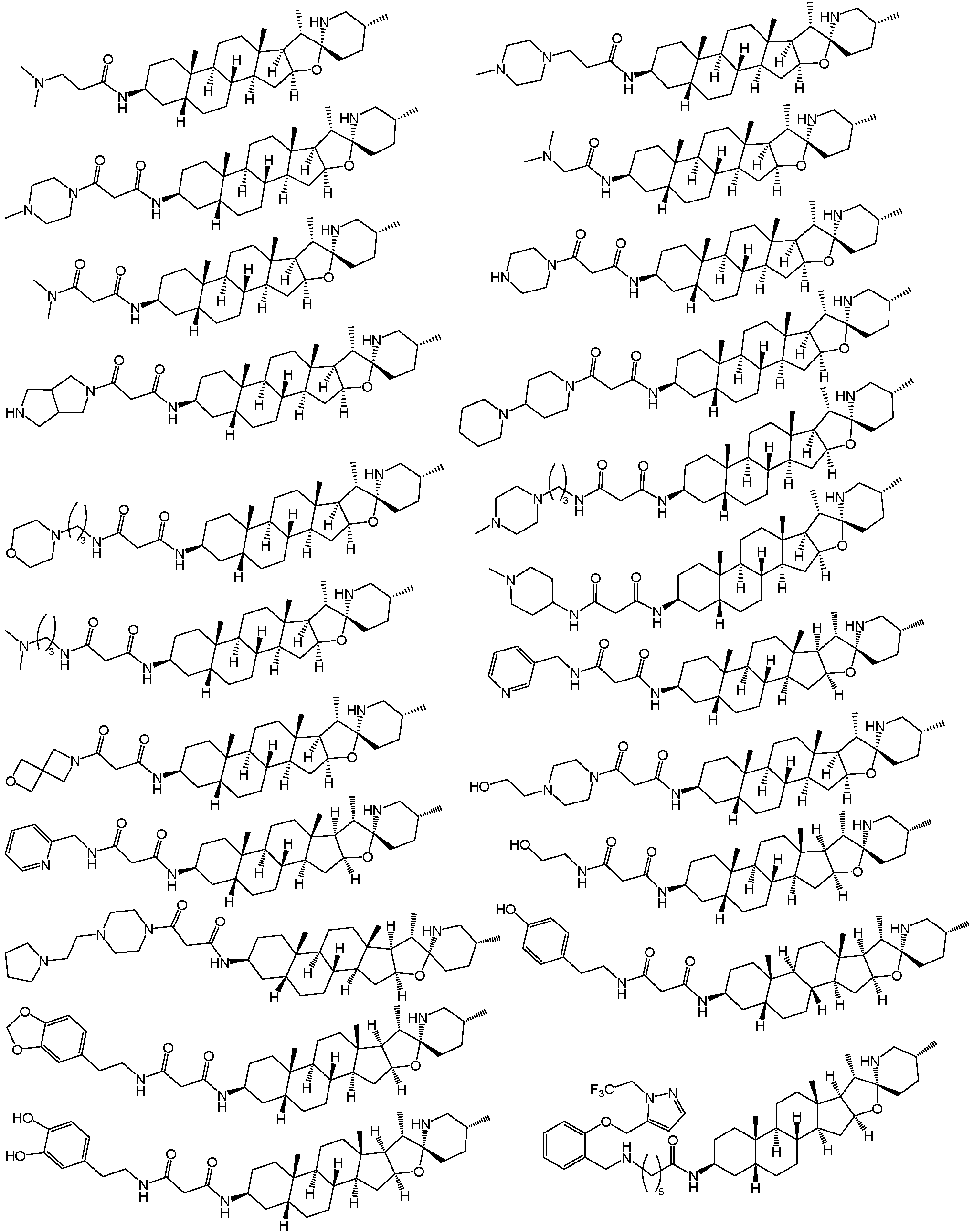 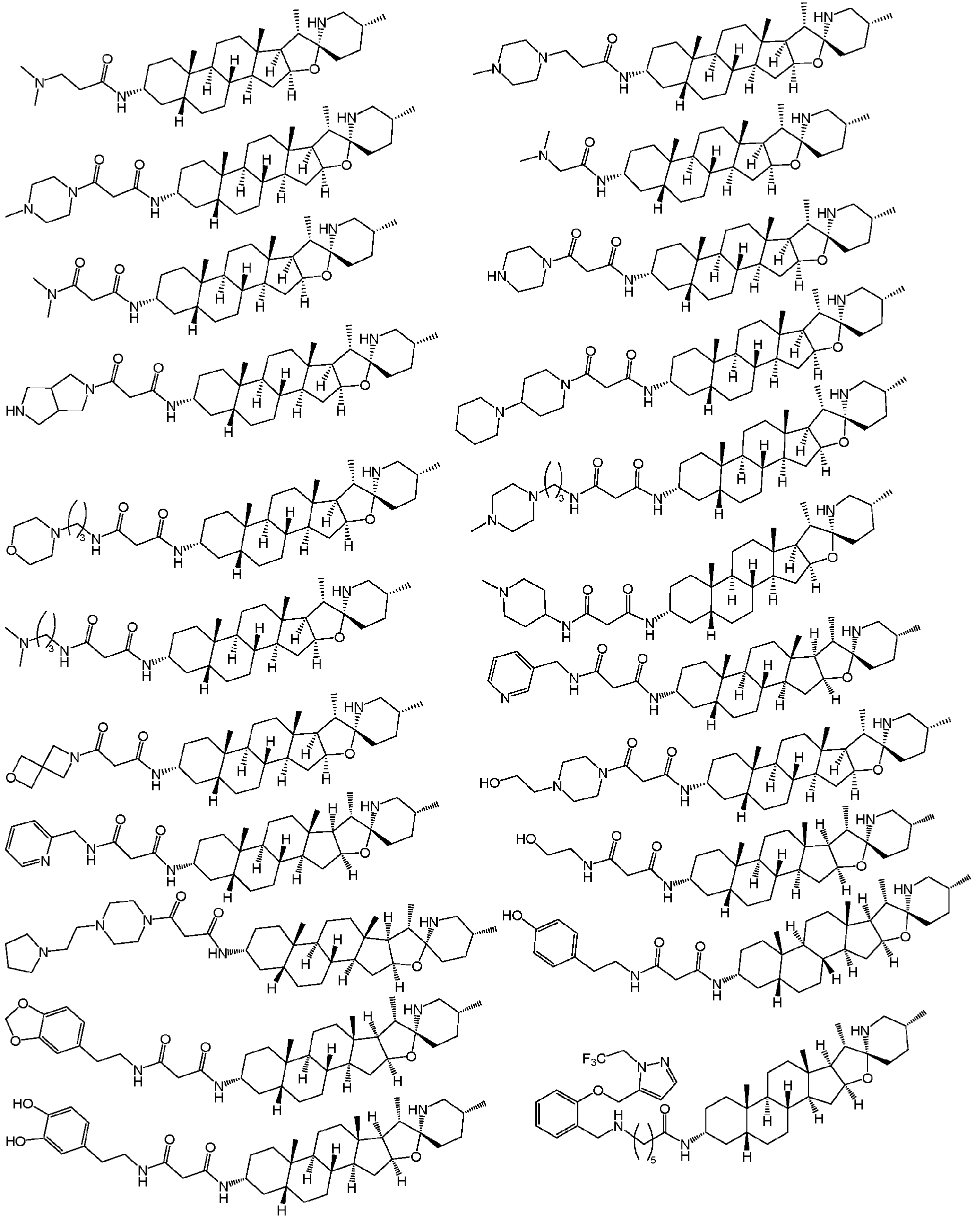 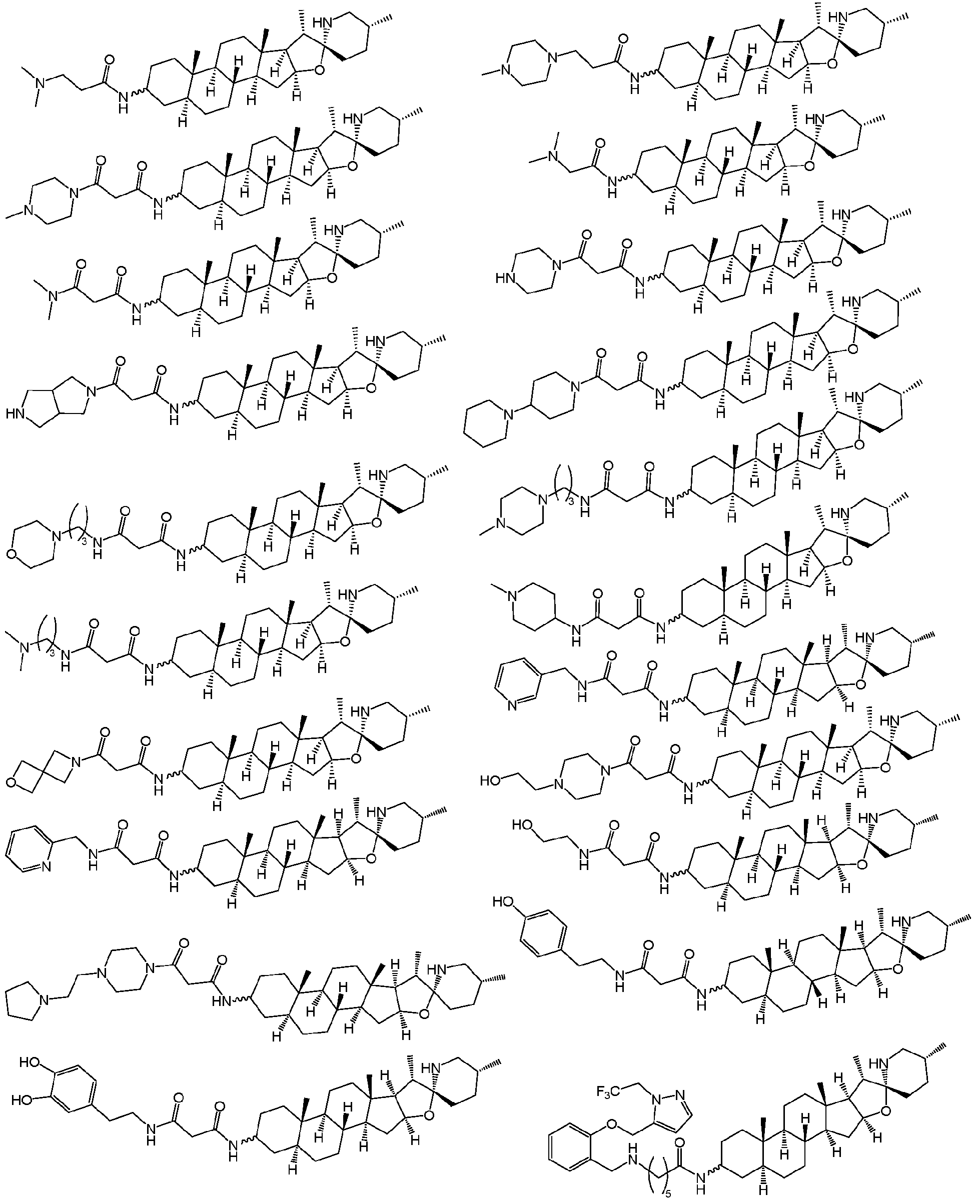 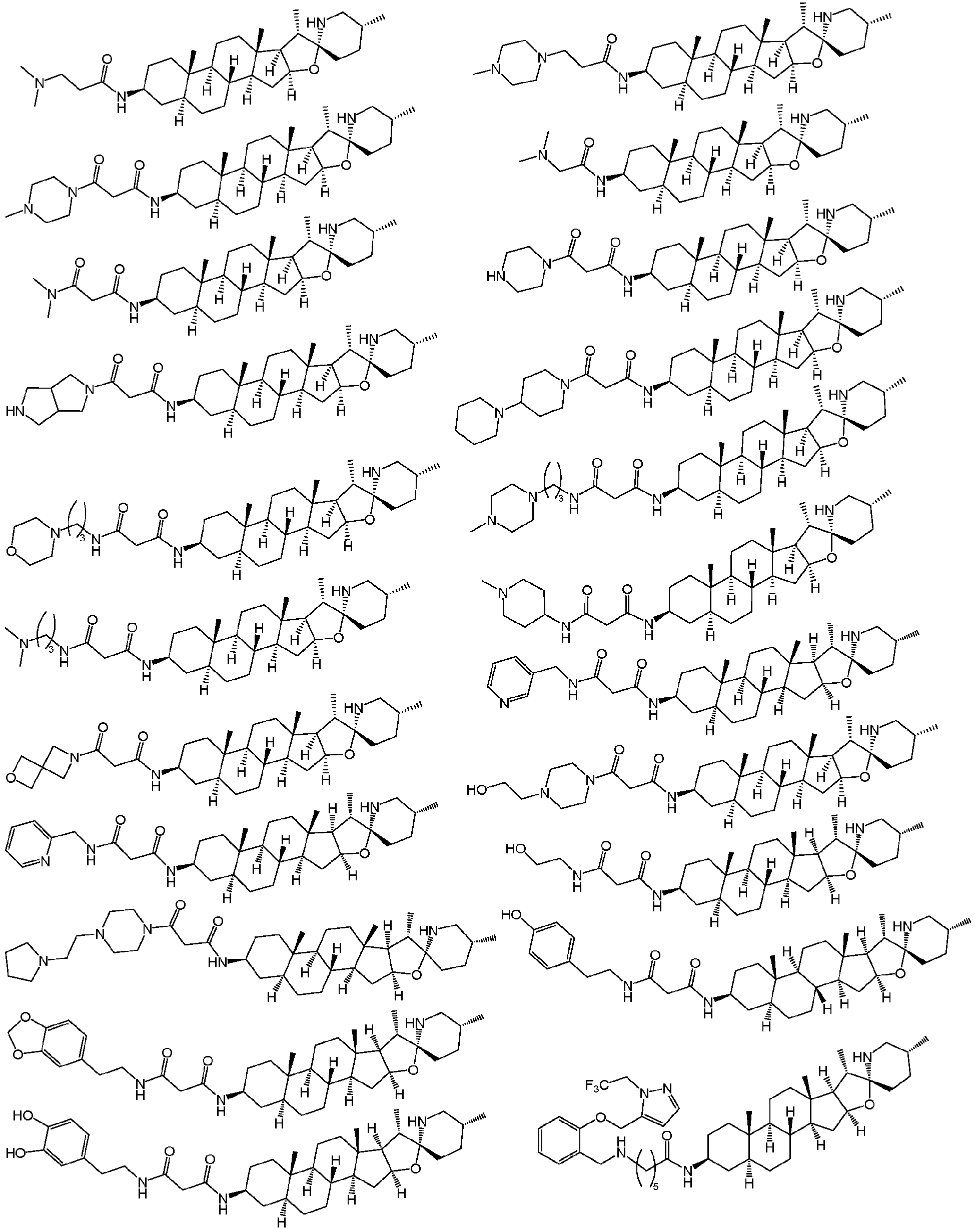 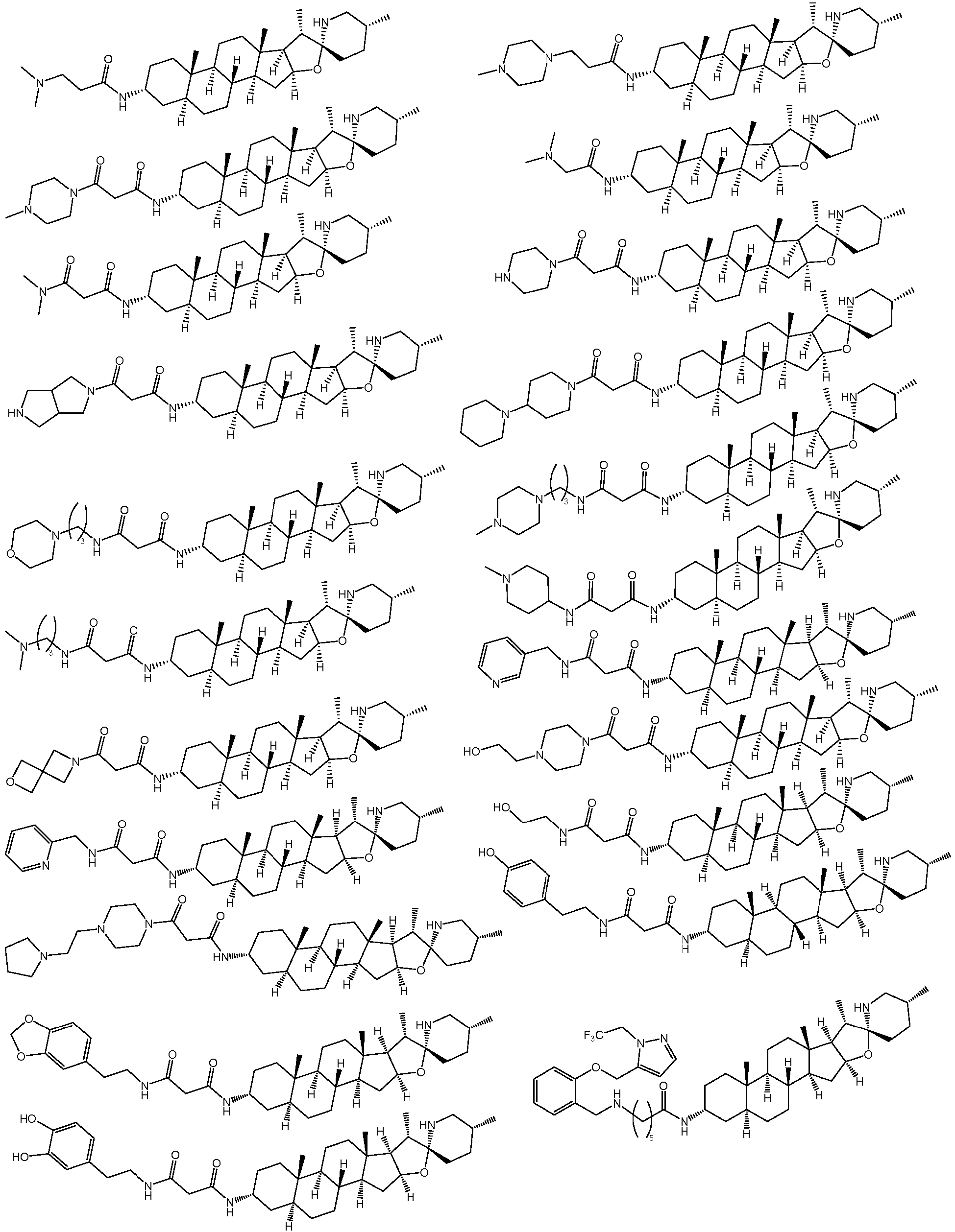 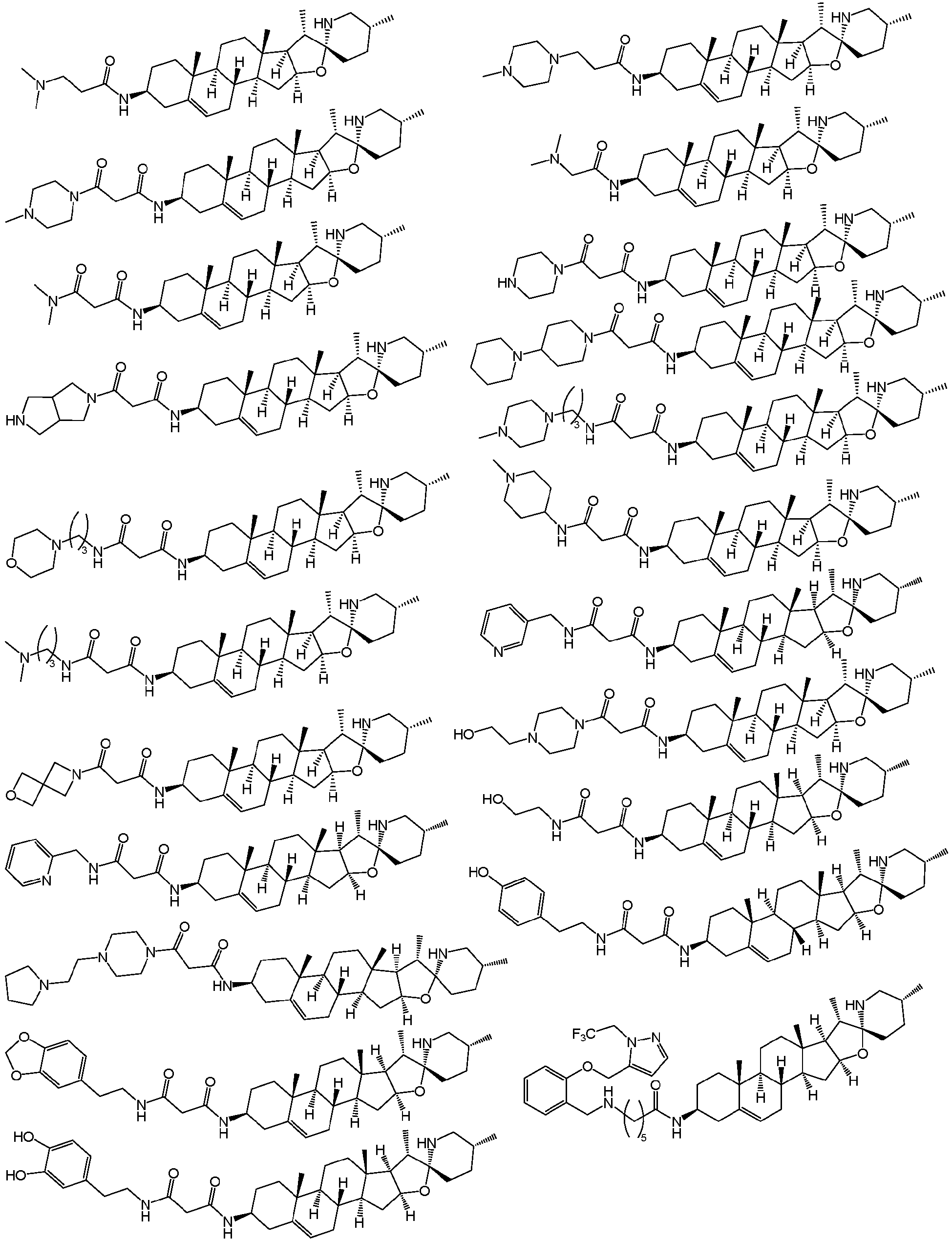 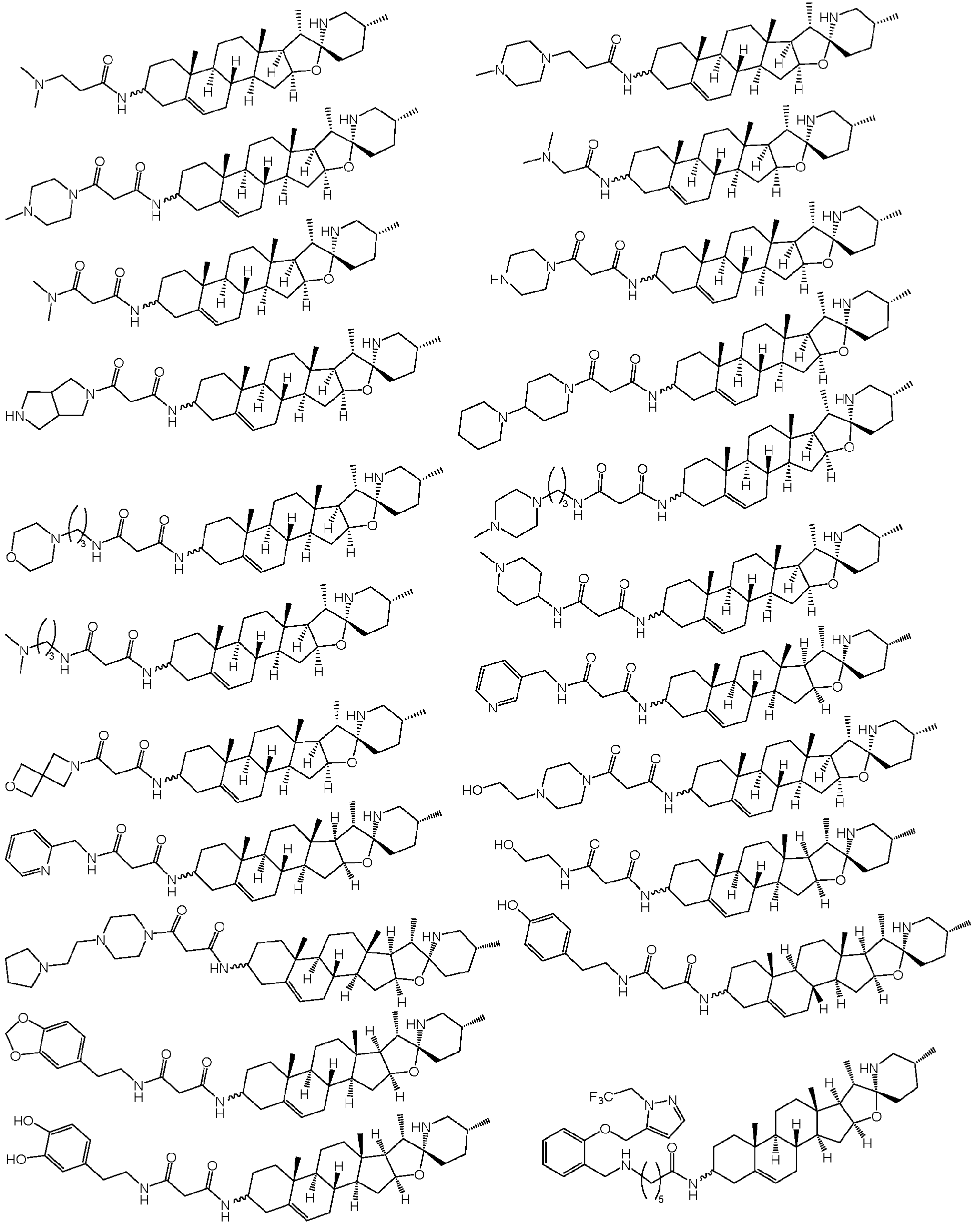 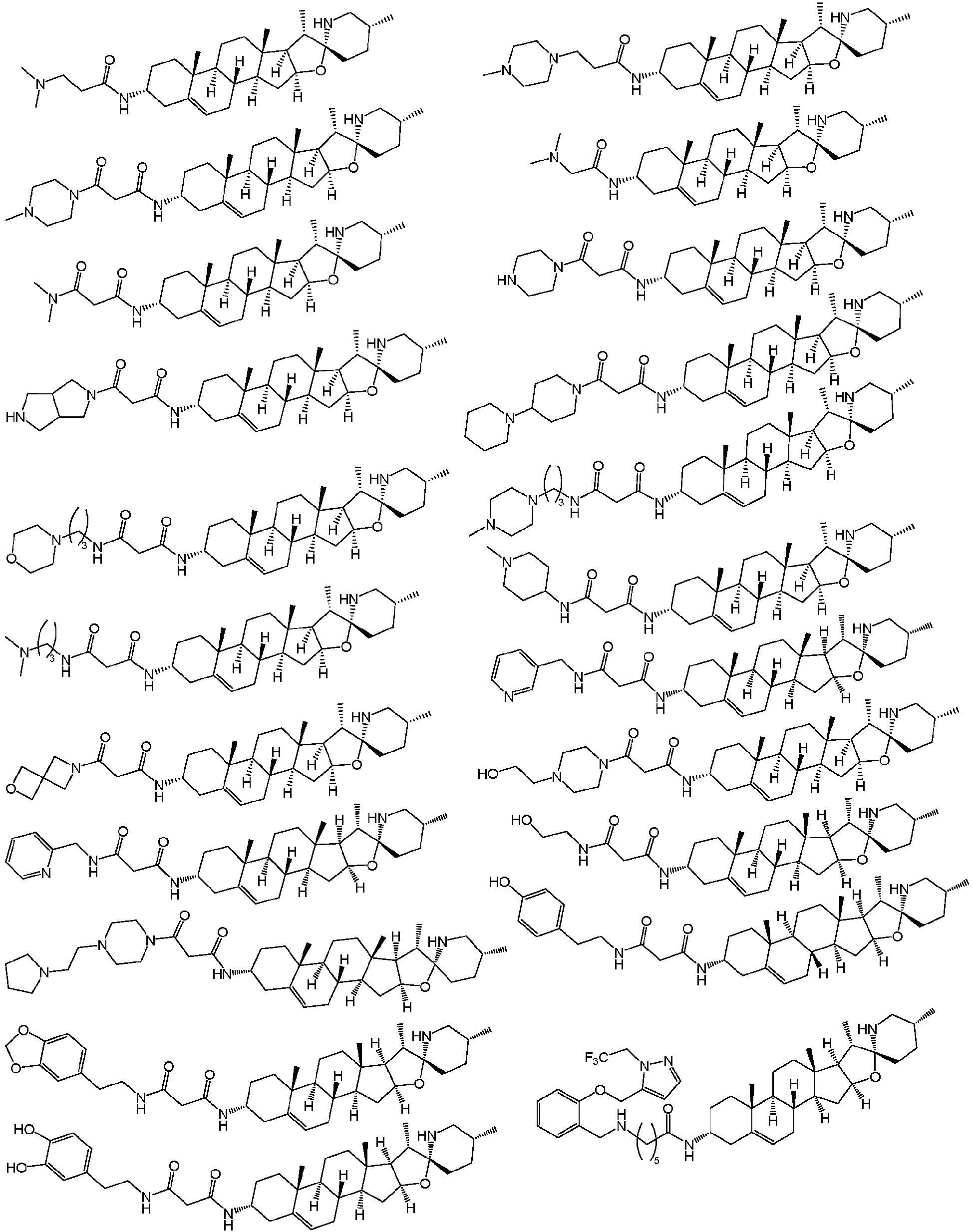 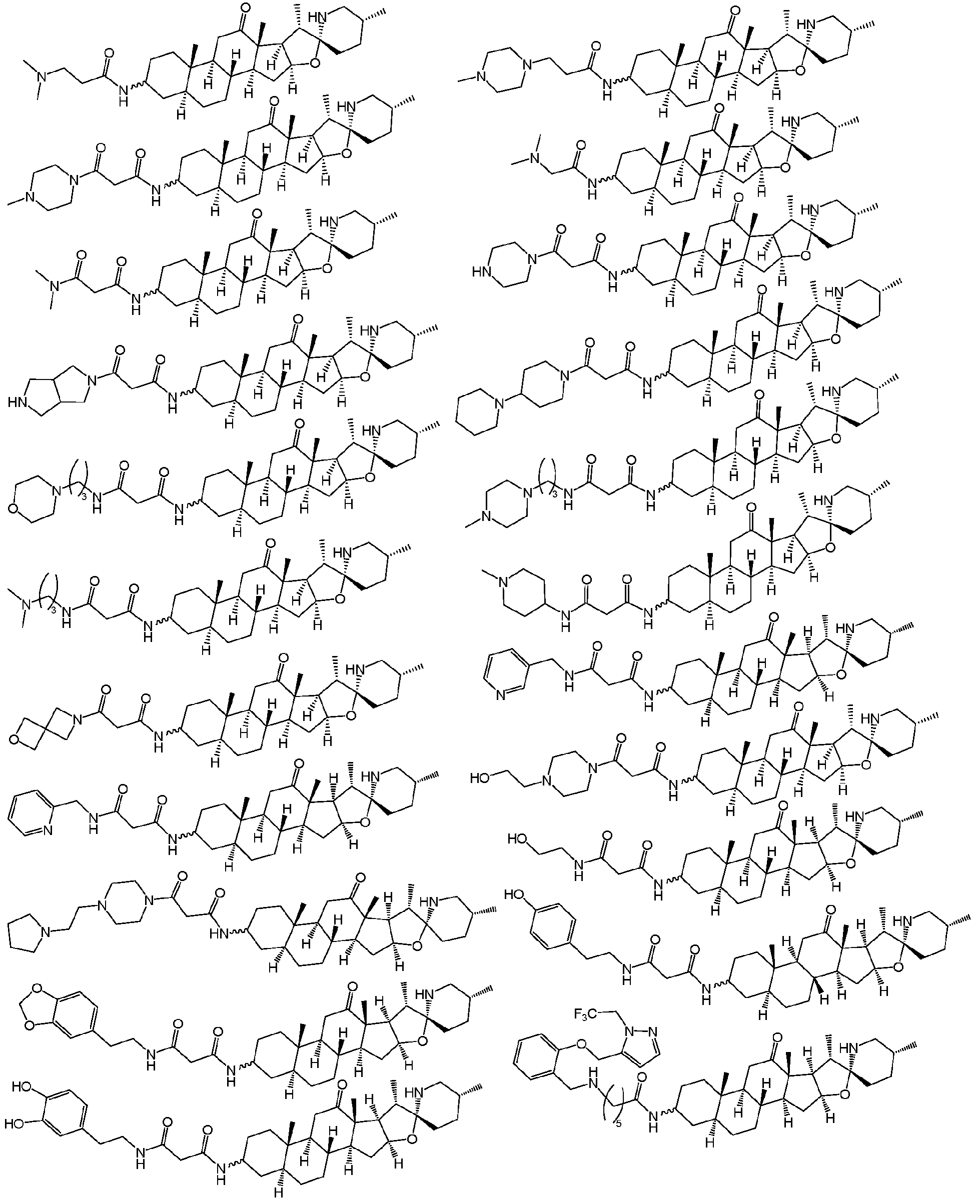 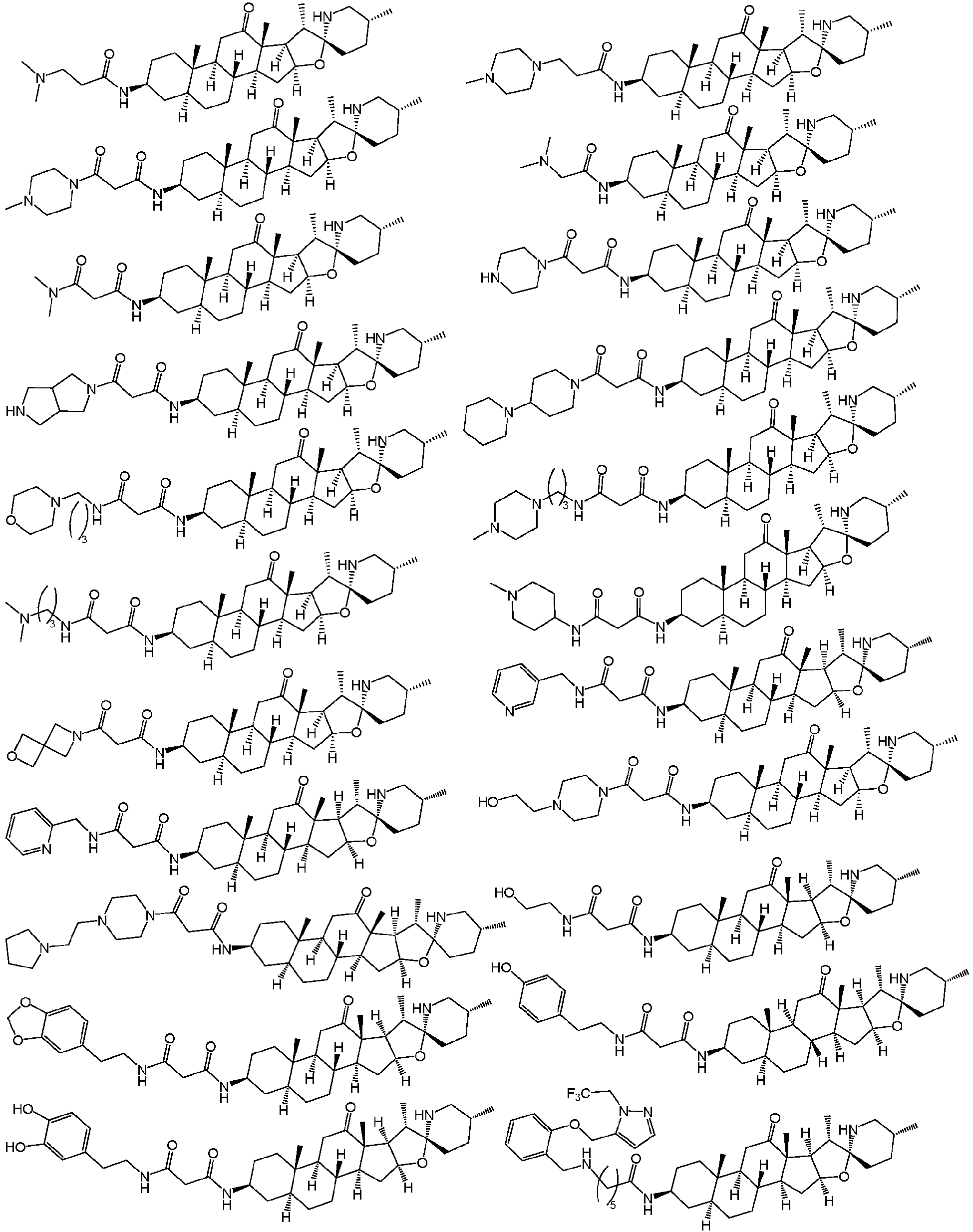 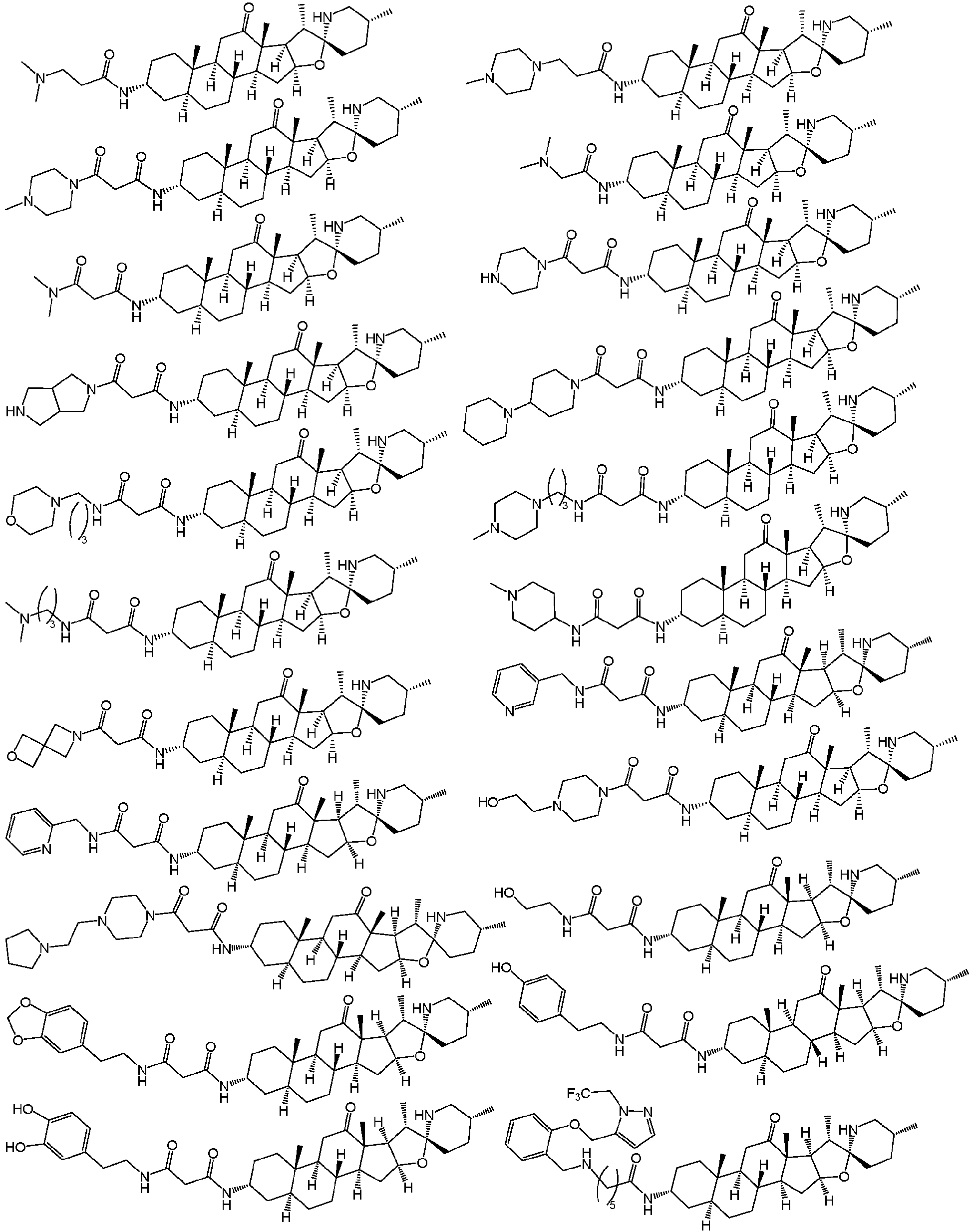 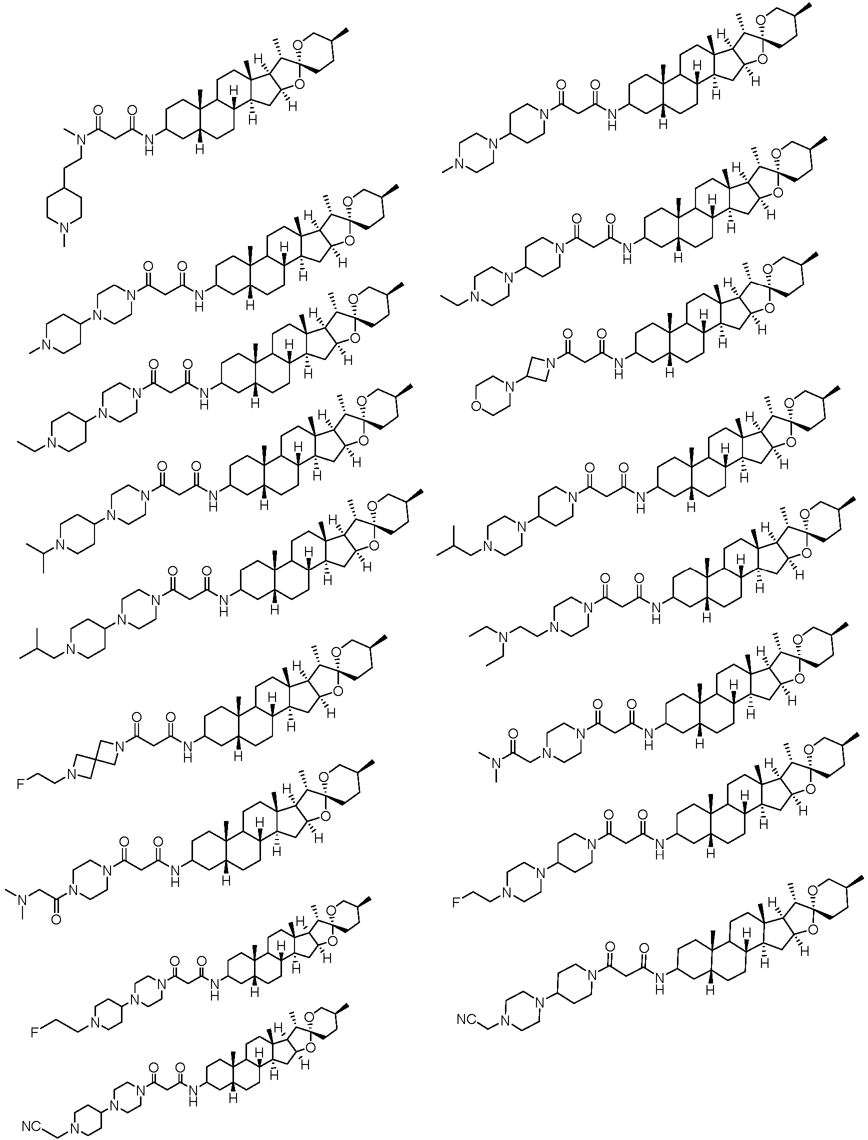 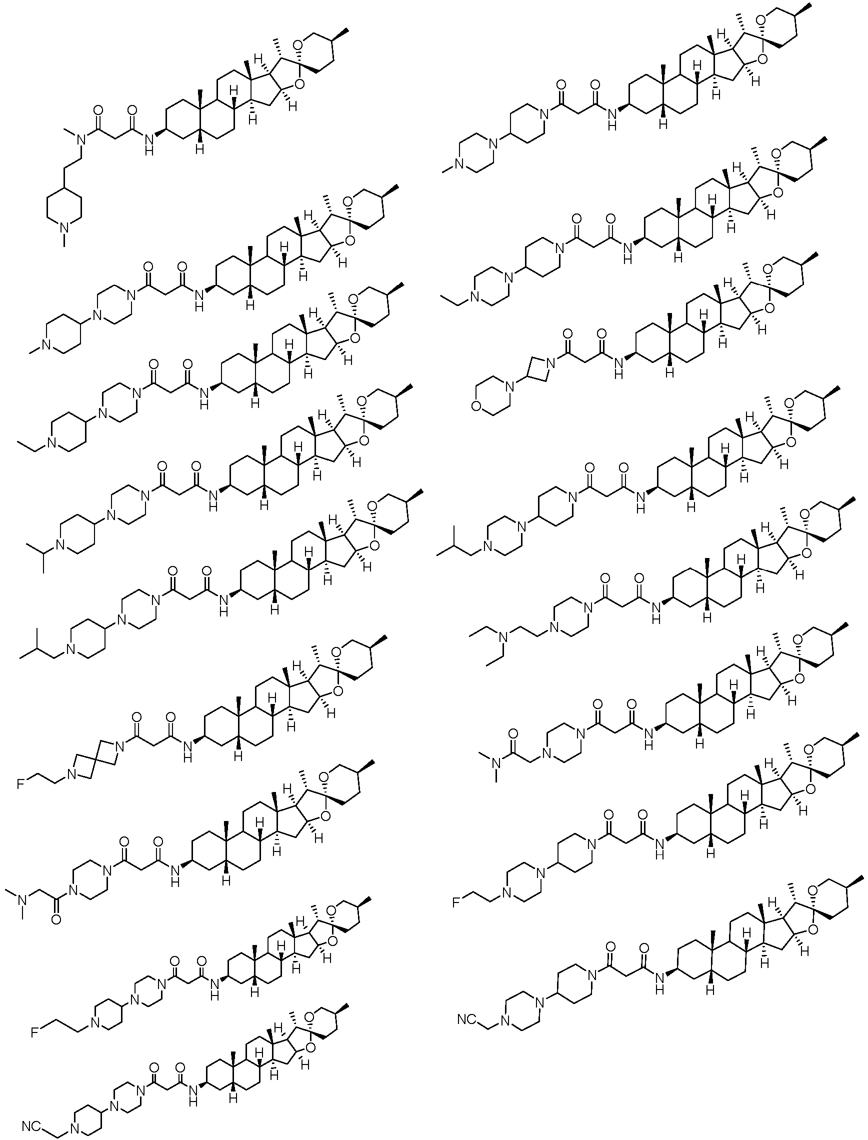 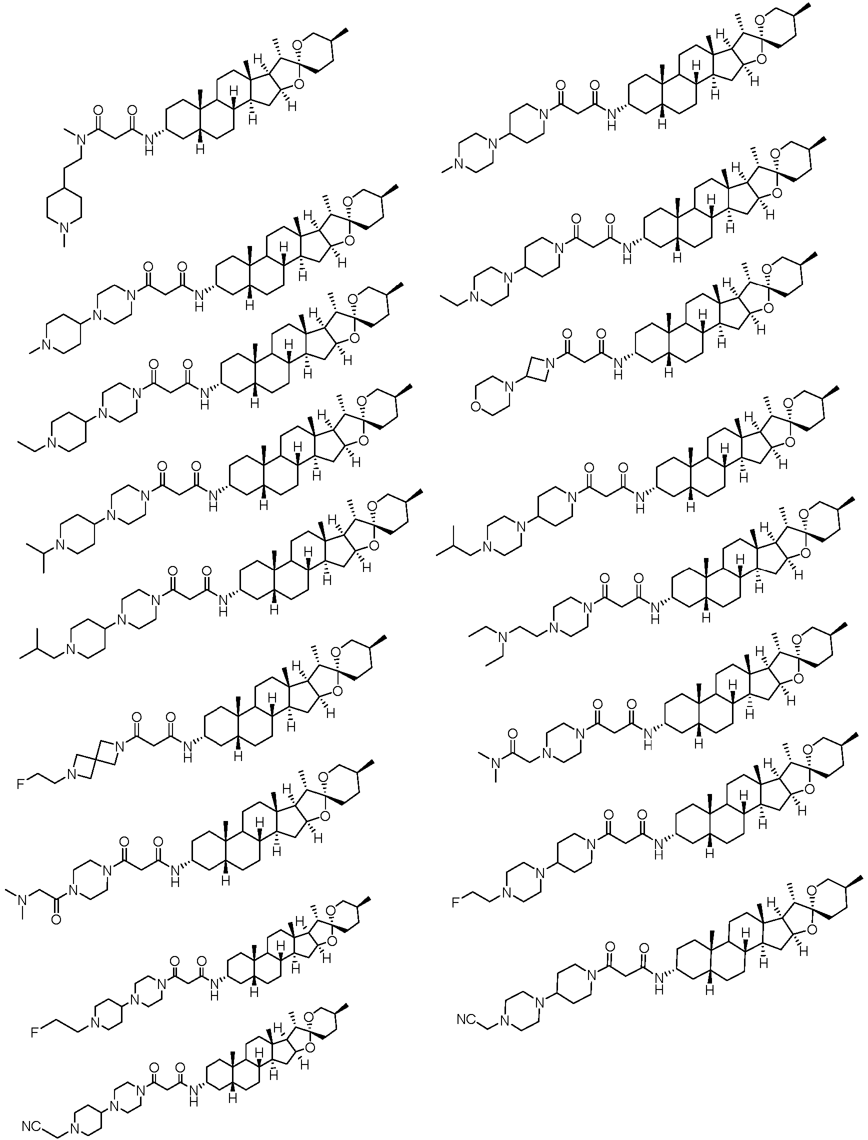 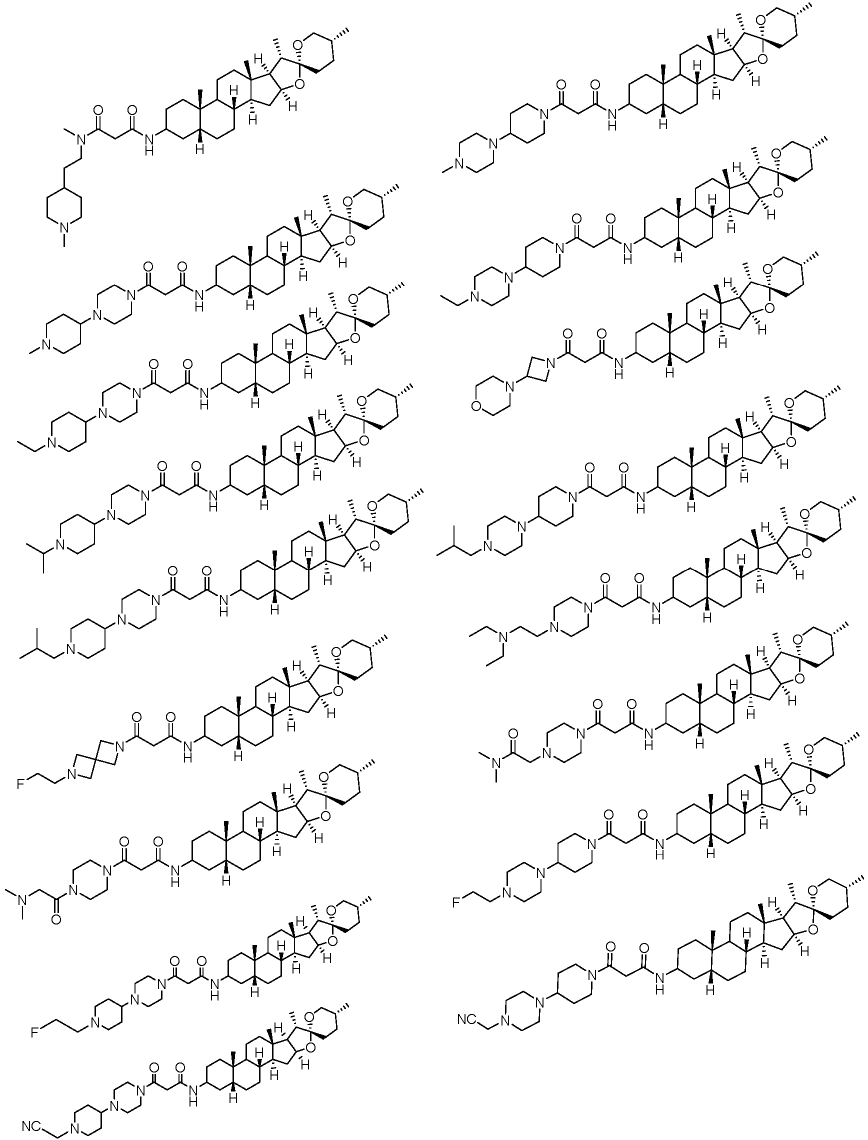 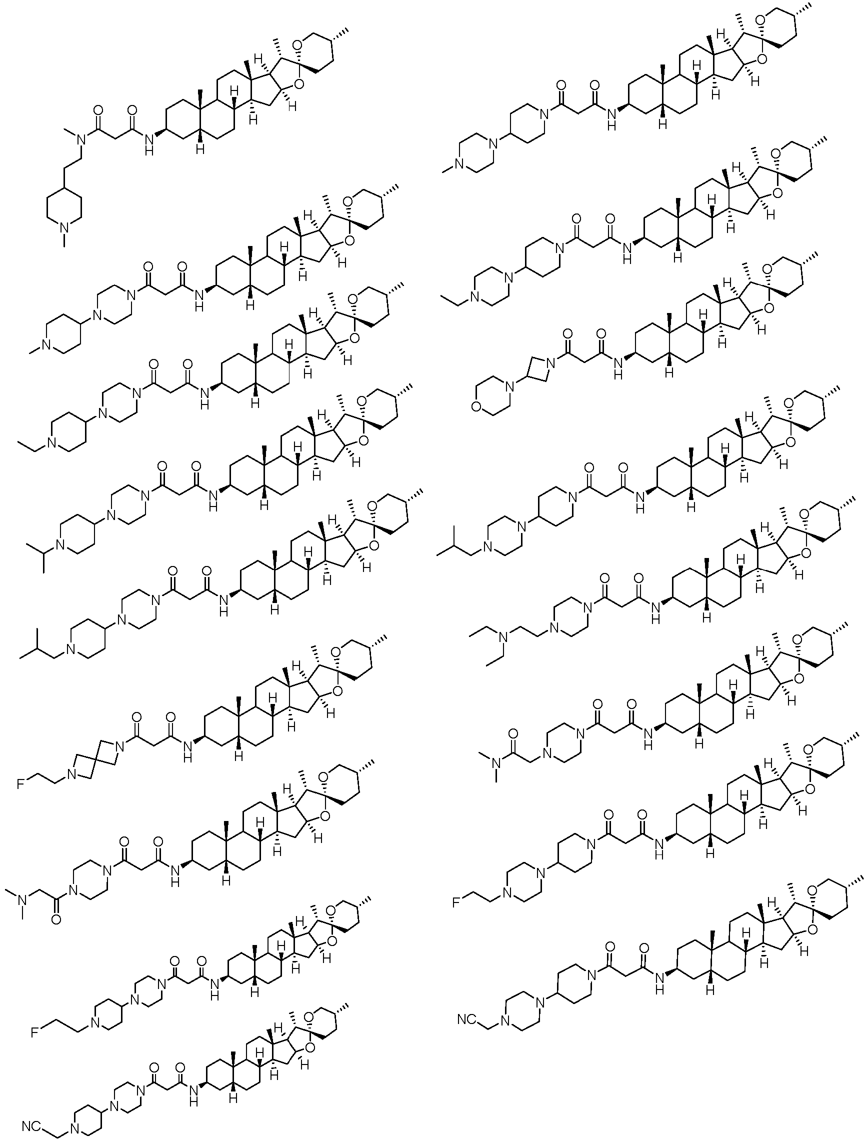 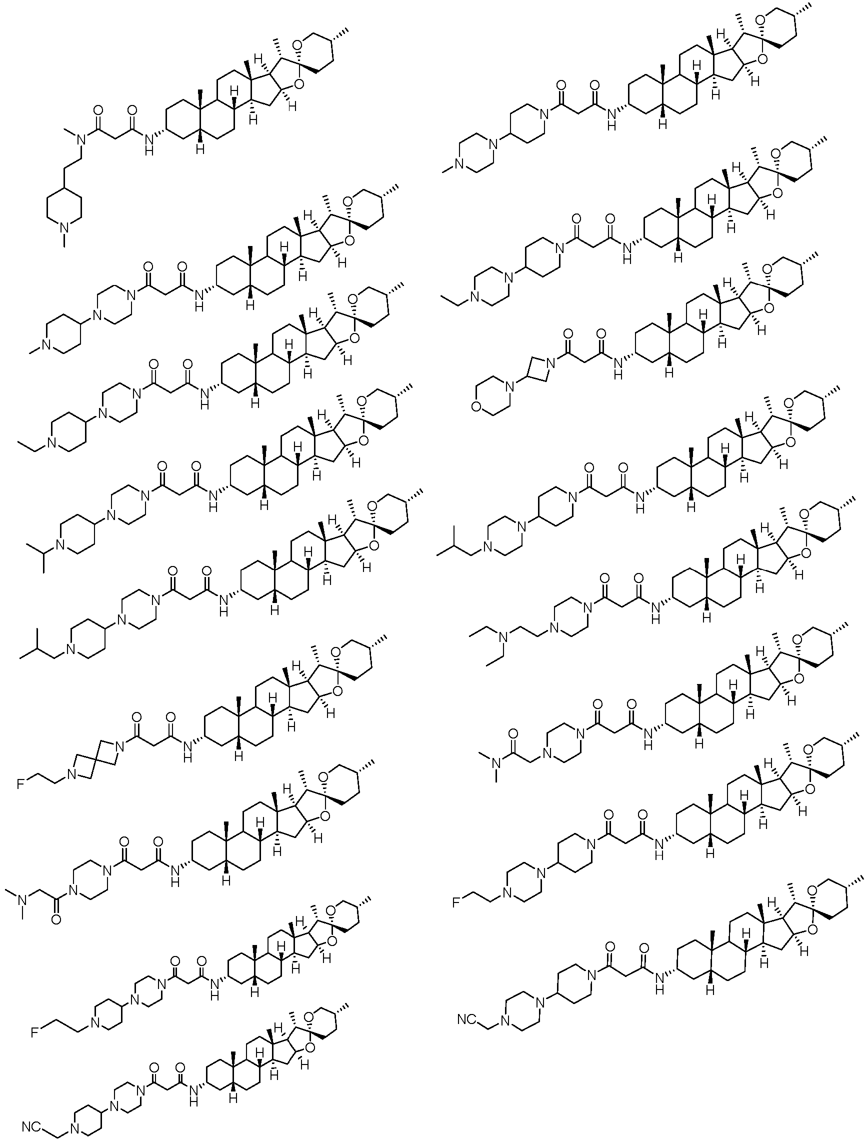 preferably, the derivatives include corresponding deuterated compounds produced by the substitution of any one or more hydrogen atoms on the derivatives by their stable isotope deuterium.

In another aspect, the present invention provides a pharmaceutical composition, the pharmaceutical composition including: the above-mentioned compound of general formula I, pharmaceutically acceptable salts, stereoisomers, tautomers, prodrugs or pharmaceutically acceptable carriers thereof.

Preferably, the pharmaceutical composition further includes additional therapeutic agents, the additional therapeutic agents include antidepressants, antimanic drugs, Parkinson's disease agents, Alzheimer's disease agents, or their combinations.

Preferably, the pharmaceutically acceptable salts are selected from the following group consisting of: hydrochloride, hydrobromide, sulfate, phosphate, methanesulfonate, trifluoromethanesulfonate, benzenesulfonate, p-toluenesulfonate (toluenesulfonate), 1-naphthalene sulfonate, 2-naphthalene sulfonate, acetate, trifluoroacetate, malate, tartrate, citrate, lactate, oxalate, succinate, fumarate, maleate, benzoate, salicylate, phenylacetate, and mandelate.

Preferably, the additional therapeutic agents are moclobemide, toloxatone, fluoxetine, paroxetine, citalopram, sertraline, venlafaxine, trimipramine, trazodone, imipramine, desipramine, clomipramine, amitriptyline, nortriptyline, doxepin, maprotiline, loxapine, amoxapine, mirtazapine, buspirone, chlormezanone, tandospirone, lithium carbonate, tacrine, huperzine a, galanthamine, donepezil, rivastigmine, memantine, pramipexole, talipexole and ropinirole, or their combinations.

The present invention further provides an application of the pharmaceutical composition in preparation of drugs for preventing, handling, treating or alleviating diseases, disorders or conditions of patients.

Preferably, the related diseases, disorders or conditions caused by mitochondrial dysfunction are specifically related diseases, disorders or conditions caused by respiratory chain abnormalities, including four categories of metabolic diseases, tumors, inflammations, and central nervous system diseases.

The metabolic diseases include: hyperglycemia, hyperlipidemia, high cholesterol, high-to-low density lipoprotein, low-to-high density lipoprotein, angiogenetic diseases, non-alcoholic fatty liver disease, cerebrovascular accident, myocardial infarction, atherosclerosis, coronary heart disease, senescence, urgent and frequent urination, type I diabetes, chronic obstructive pulmonary disease, etc.

The tumors include: prostatic hyperplasia, Wegener's granulomatosis, pulmonary sarcoidosis, leukemia, lymphoma, pancreatic cancer, neural tumors, etc.

The inflammations include: peripheral neuritis, chemotherapy-induced peripheral neuritis, autoimmune diseases, conditions associated with organ transplantation, influenza virus, coronavirus (prevention, treatment and sequelae elimination of infections), acute respiratory distress syndrome, inflammatory bowel disease, Crohn's disease, ulcerative colitis, psoriasis, retinal detachment, retinitis pigmentosa, macular degeneration, pancreatitis, atopic dermatitis, rheumatoid arthritis, spondyloarthritis, gout, systemic lupus erythematosus, Sjögren's syndrome, systemic scleroderma, antiphospholipid syndrome, vasculitis, osteoarthritis, autoimmune hepatitis, autoimmune hepatobiliary disease, primary sclerosing cholangitis, nephritis, celiac disease, autoimmune ITP, transplant rejection, ischemia-reperfusion injuries of solid organs, sepsis, periodontitis, systemic inflammatory response syndrome, myocarditis, allergic diseases, asthma, interleukin-I convertase-related fever syndrome, Behcet's disease, etc.

The central nervous system diseases include: Pick's disease, spinal cord injury repair, depression, anxiety, Parkinson's disease, Alzheimer's disease, sleep disorders, ischemic stroke, hemorrhagic stroke, amyotrophic lateral sclerosis, traumatic brain injury, brain atrophy, Huntington's disease, schizophrenia, mania, drug addiction withdrawal, multiple sclerosis, sleep disorders, muscle weakness, etc.

Using the sarsasapogenin-based compounds, the pharmaceutical compositions and the applications thereof according to the present invention, through related in-vivo and in-vitro model activity tests for sarsasapogenin-based compounds, it is accidentally found that many derivative compounds thereof have superior cytoprotective activity, especially have unexpected protection activity for a plurality of indications associated with mitochondria, more specifically, for a plurality of brain neuron cells. In addition, such compounds have excellent blood brain permeability, making them potentially applicable and valuable for treating various diseases caused by mitochondrial dysfunction, addressing the limitations of sarsasapogenin-based compounds in the prior art, and providing important scientific and commercial values.

### BRIEF DESCRIPTION OF THE DRAWINGS

Fig. 1 shows neuron cell death under the action of different small molecule compounds.
Fig. 2 shows oxidative damages of small molecule compounds to hydrogen peroxide (H₂O₂)-induced human SHSY5Y neural tumor cells.
Fig. 3 shows a proportion of dead neuron cells.
Fig. 4 shows a phenotype diagram of the effects of various experimental groups on zebrafish inflammation.
Fig. 5 shows the effects of various experimental groups on zebrafish inflammation (the number of neutrophils).
Fig. 6 shows anti-inflammatory effects of various experimental groups on zebrafish.
Fig. 7 shows the changes in cerebral blood flow in mice during surgery.
Fig. 8 shows the changes in body weights of mice before and after surgery.
Fig. 9 shows the changes in grip strength of forelimbs of mice.
Fig. 10 shows neurological deficit scores of mice.
Fig. 11 shows infract volumes of mice.
Fig. 12 shows the volumes of cerebral edema in mice.
Fig. 13 shows the immobility time of mice in FST.
Fig. 14 shows sucrose preferences of mice in SPT.
Fig. 15 shows the immobility time of mice in TST.
Fig. 16 shows the detection of ROS levels of mice in serum.
Fig. 17 shows the detection of H₂O₂ concentrations of mice in serum.
Fig. 18 shows the detection of NO concentrations of mice in serum.
Fig. 19 shows the detection of lipid peroxidation levels of mice in serum.
Fig. 20 shows the detection of ROS levels of mice in hippocampus.
Fig. 21 shows the detection of H₂O₂ concentrations of mice in hippocampus.
Fig. 22 shows the detection of NO concentrations of mice in hippocampus.
Fig. 23 shows the detection of lipid peroxidation levels of mice in hippocampus.
Fig. 24 shows the detection of IL-1β concentrations of mice in serum.
Fig. 25 shows the detection of IL-6 concentrations of mice in serum.
Fig. 26 shows the detection of II,-10 concentrations of mice in serum.
Fig. 27 shows the detection of IL-1β concentrations of mice in hippocampus.
Fig. 28 shows the detection of IL-6 concentrations of mice in hippocampus.
Fig. 29 shows the detection of II,-10 concentrations of mice in serum.
Figs. 30a to 32b show binding strengths of various small molecules to a respiratory chain complex I, respectively.
Fig. 33 is a diagram of activity results of various small molecules against SMPs.
Fig. 34 is a schematic diagram of results of various small molecules against the oxygen consumption of cells.
Figs. 35a to 35b are diagrams of results of small molecules against mitochondrial ROS and mitochondrial transmembrane potential differences, respectively.
Figs. 36a to 36d are diagrams of results of small molecules against atherosclerosis of APOE mice, respectively.
Figs. 37a to 37g are diagrams of results of small molecules against water maze test of AD rats.
Fig. 38 is a diagram of results of small molecules against T maze test of AD rats.
Figs. 39a to 39b are diagrams of results of small molecules against nesting behaviors of AD mice.
Figs. 40a to 40b are diagrams of results of small molecules against water maze test of AD mice.
Fig. 41 is a diagram of results of small molecules against light/dark box test of AD mice.
Figs. 42a to 42b are diagrams of results of small molecules against survival of TDP43^{A315T} mice.
Fig. 43 is a diagram of stride length analysis results of small molecules against SOD^{G93A} mice.
Fig. 44 is a diagram of results of small molecules against open field test of SOD^{G93A} mice.
Figs. 45a to 45b are diagrams of results of small molecules against enteritis models of DSS mice.
Figs. 46a to 46b are diagrams of results of small molecules against enteritis models of TNBS rats.
Fig. 47 is a diagram of results of small molecules against blood glucose of DB mice.
Figs. 48a to 48d are result diagrams of body weights and body fat rates of DIO mice.
Fig. 49 is a diagram of in-vivo killing effects of small molecules on hemangioma.
Fig. 50 is a result diagram of protection effects of small molecules on substantia nigra neurons.
Fig. 51 is a result diagram of protection effects of small molecules on virus infection.
Fig. 52 is a result diagram of small molecules against an acute traumatic brain injury TBI model of rats.
Fig. 53 is a result diagram of in-vitro killing effects of small molecules on pancreatic cancer cells.
Fig. 54 is a result diagram of small molecules against excessive urination of DB animals.
Figs. 55a to 55c are result diagrams of small molecules against cardiovascular inflammations of APOE mutant mice with high-fat diet.
Figs. 56a and 56b are result diagrams of efficacy experiments for the effects of small molecules on the sleep of mice under a subthreshold hypnosis dose of pentobarbital sodium.

### DETAILED DESCRIPTION OF THE PREFERRED EMBODIMENTS

In order to more clearly understand the technical content of the present invention, the specific implementation methods of the present invention will be described below.

The term "alkyl" in the present invention refers to a monovalent saturated aliphatic hydrocarbon group with 1 to 10 carbon atoms, including linear and branched hydrocarbon groups, such as methyl(CH₃-), ethyl(CH₃CH₂-), n-propyl(CH₃CH₂CH₂-), isopropyl((CH₃)₂CH-), n-butyl(CH₃CH₂CH₂CH₂-), isobutyl((CH₃)₂CHCH₂-), sec-butyl((CH₃)(CH₃CH₂)CH-), tert-butyl((CH₃)₃C-), n-amyl(CH₃CH₂CH₂CH₂CH₂-), and neo-amyl((CH₃)₃CCH₂-).

In the present invention, the term "alkyl" includes substituted or unsubstituted alkyl.

In the present invention, the term "substituted or unsubstituted" means that the group may be unsubstituted, or that H in the group is substituted by one or more (preferably 1 to 6, and more preferably 1 to 3) substituents.

In the present invention, the "substituted" means that the group has one or more (preferably 1 to 6, and more preferably 1 to 3) substituents selected from the group consisting of halogen, hydroxyl, - NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, C₂-C₈ heterocyclyl, and C₂-C₈ heteroaryl; and the heteroatoms are selected from one or more of N, O and S.

In the present invention, the term "cycloalkyl" represents substituted or unsubstituted C₃-C₁₂ cycloalkyl.

In the present invention, the term "alkoxyl" refers to -O-alkyl, where the alkyl may be saturated or unsaturated, and may be branched, linear, or cyclic. Preferably, the alkoxyl has 1 to 10 carbon atoms, preferably 1 to 6 carbon atoms. Representative examples include (but are not limited to) methoxyl, ethoxyl, and propoxyl.

In the present invention, the term "aryl" refers to a monovalent aromatic carbon ring group of 6 to 20 (preferably 6 to 14) carbon atoms, which has a single ring (e.g., phenyl) or a fused ring (e.g., naphthyl or anthryl), and if a connection point is on an aromatic carbon atom, the fused ring may be non-aromatic (e.g., 2-benzoxazolone, 2H-1,4-benzoxazine-3(4H)-one-7-yl). The preferred aryl includes phenyl and naphthyl. This term includes a substituted or unsubstituted form, where substituents are defined as above.

In the present invention, the term "alkenyl" refers to alkenyl with 2 to 10 (e.g., 2 to 6, or 2 to 4) carbon atoms, and has at least one (e.g., 1 to 2) unsaturated olefinic bond (>C = C<). Examples of such groups are vinyl, allyl, and butyl-3-enyl.

In the present invention, the term "cycloalkyl" refers to cyclic alkyl with 3 to 10 carbon atoms, and has a single ring or multiple ring (including a fused-ring system, a bridged-ring system and a spiro-ring system). In the fused-ring system, one or more rings may be cycloalkyl, heterocyclyl, aryl, or heteroaryl, as long as a connection site is a ring passing through cycloalkyl. Examples of suitable cycloalkyl include, for example, adamantyl, cyclopropyl, cyclobutyl, cyclopentyl, and cyclooctyl.

In the present invention, the term "halo" or "halogen" refers to fluorine, chlorine, bromine, and iodine.

In the present invention, the term "heteroaryl" refers to an aromatic group with 1 to 10 carbon atoms and 1 to 4 heteroatoms selected from O, N and S in a ring, and such heteroaryl may be a single ring (e.g., pyridyl or furanyl) or a fused ring (e.g., indolizinyl or benzothienyl), wherein the fused ring may be non-aromatic and/or contains one heteroatom, as long as a connection point is an atom passing through aromatic heteroaryl. In one embodiment, cyclic atom N and/or S of the heteroaryl is optionally oxidized as N-oxide (N-O), sulfinyl or sulfonyl. Preferably, the heteroaryl includes pyridyl, pyrryl, indolyl, thienyl, and furyl. This term includes substituted or unsubstituted heteroaryl.

In the present invention, the term "substituted heteroaryl" refers to heteroaryl that is substituted by 1 to 5, preferably 1 to 3, and more preferably 1 to 2 substituents, wherein the substituents are selected from the same substituents as defined by substituted aryl.

In the present invention, the term "heterocyclyl" or "heterocyclic" or "heterocycloalkyl" or "heterocyclyl" refers to a saturated, partially saturated or unsaturated group (but not aromatic) with a single ring or a fused ring (including a bridge-ring system and a spiro-ring system), which has 1 to 10 carbon atoms and 1 to 4 (e.g., 3) heteroatoms selected from N, S or O; and in a fused-ring system, one or more rings may be cycloalkyl, aryl, or heteroaryl, as long as a connection point passes through a non-aromatic ring. In one embodiment, nitrogen atoms and/or sulfur atoms in the heterocyclic group are optionally oxidized to provide N-oxide, sulfinyl and sulfonyl moieties.

In the present invention, the term "substituted heterocyclic" or "substituted heterocycloalkyl" or "substituted heterocyclyl" refers to a heterocyclic group that is substituted by 1 to 5 (e.g., 1 to 3) substituents that are same as those defined by substituted cycloalkyl.

In the present invention, the term "stereoisomer" refers to a compound that contains one or more stereocenters having different chirality. The stereoisomers include enantiomers and diastereoisomers.

In the present invention, the term "tautomer" refers to an alternative form of compounds with different proton positions, such as an enol-ketone and imine-enamine tautomer, or tautomeric forms of heteroaryl, wherein the heteroaryl contains ring atoms linked to an -NH- moiety and an N- moiety of a ring, such as pyrazole, imidazole, benzimidazole, triazole, and tetrazole.

The present invention provides a pharmaceutical composition containing active ingredients within a range of safe and effective amounts, as well as pharmaceutically acceptable carriers.

The "active ingredient" of the present invention refers to the compound of general formula I of the present invention, or pharmaceutically acceptable salts, stereoisomers, tautomers or prodrugs thereof.

The "active ingredient" and pharmaceutical compositions of the present invention may be used as a mitochondrial protective agent. In another preferred embodiment, it is used to prepare drugs for the prevention and/or treatment of neurodegenerative diseases. In another preferred embodiment, it is used to prepare drugs for the prevention and/or treatment of mitochondria-related metabolic diseases.

The "safe and effective amount" is defined as an amount of an active ingredient that is sufficient to improve a condition without serious side effects. In general cases, the pharmaceutical composition contains 1 to 2000 mg of active ingredients/agents, and preferably, 10 to 200 mg of active ingredients/agents. Preferably, the "one dose" refers to a tablet.

The "pharmaceutically acceptable carrier" refers to one or more compatible solid or liquid fillers or gel substances that are suitable for human use and must be of sufficient purity and sufficiently low toxicity. The "compatible" here means that various components in the composition can be mixed with active ingredients in the present invention or mixed with each other without significantly reducing the efficacy of the active ingredients.

The compound in the preferred embodiment of the present invention may be delivered as a single active medicament, or may be used in combination with one or more other reagents for the treatment of cancers.

In general cases, the compound in the preferred embodiment may be administered in a therapeutically effective amount, by any acceptable mode of a medicament with similar effects. The actual dosage of the compound (i.e., the active ingredients) in the preferred embodiment is determined based on a plurality of factors, e.g., the severity of a disease to be treated, the age and relative health of a patient, the potency of the used compound, route and form of administration, and other factors. This drug may be administered several times a day, preferably, once or twice a day. All of these factors are taken into account by attending physicians.

For the purpose of the preferred embodiment, the therapeutically effective dosage may typically be a total daily dosage given in one-time administration or multiple administrations to a patient, e.g., about 0.001 to about 1000 mg/kg body weight per day, preferably about 1.0 to about 30 mg/kg body weight per day. A dosage unit composition may contain a dosage factor to form a daily dosage. The choice of dosage form depends on various factors, such as a delivery mode and the bioavailability of drug substances. In general cases, the compound in the preferred embodiment may be administered as a pharmaceutical composition by any route appropriate to a condition being treated. Appropriate routes include, but are not limited to, oral, parenteral (including subcutaneous, muscular, intravenous, intraarterial, and intradermal), vaginal, intraperitoneal, intrapulmonary, and intranasal routes. It should be understood that the preferred route may vary depending on patient's conditions. The preferred way of delivery is oral administration, and a convenient daily dosage can be adjusted according to the degree of bitterness. This compound may be formulated with a pharmaceutically acceptable carrier or excipient into tablets, pills, capsules, semi-solids, powders, sustained release preparations, solutions, suspensions, elixirs, aerosols or any other appropriate compositions, etc. When the compound is formulated for a parenteral route, it may be formulated with a pharmaceutically acceptable parenteral carrier. Another preferred way to administrate the compound of the preferred embodiment is inhalation. It is an effective method of delivering therapeutics directly to the respiratory tract (see, e.g., U.S. Patent No. 5,607,915).

According to the present invention, the compound may be administrated in the form of any convenient preparation. The term "preparation" in the present invention refers to dosage forms that are conducive to drug delivery containing the compound of general formula I of the present invention, such as, but not limited to, aqueous solution injections, powder injections, pills, powders, tablets, patches, suppositories, emulsions, creams, gels, granules, capsules, aerosols, sprays, powder aerosols, sustained-release agents and controlled-release agents. These pharmaceutical adjuvants may be routinely used in various preparations, such as, but not limited to, isotonic agents, buffers, flavoring agents, excipients, fillers, adhesives, disintegrants and lubricants; or may also be used for the purpose of adapting to the substances, such as emulsifiers, solubilizers, bacteriostatic agents, analgesics and antioxidants. Such adjuvants can effectively improve the stability and solubility of the compound in the composition or change a release rate, an absorption rate, etc. of the compound, thereby improving the metabolism of the compound of the present invention in organisms, and then enhancing the delivery effect. In addition, in order to achieve specific drug delivery purposes or methods, such as sustained-release drug delivery, controlled-release drug delivery and pulse drug delivery, used adjuvants include, but are not limited to, gelatin, albumin, chitosan, polyether, as well as polyester polymer materials, for example, but not limited to, polyethylene glycol, polyurethane, polycarbonate and their copolymers. The main manifestations of the so-called "conducive to" include, but are not limited to, improving a therapeutic effect, improving the bioavailability, reducing toxic and side effects, improving the patient compliance, etc.

Suitable pharmaceutically acceptable carriers or excipients include: treatment agents, as well as drug delivery modifiers and accelerators, such as calcium phosphate, magnesium stearate, talc, monosaccharides, disaccharides, starch, gelatin, cellulose, methylcellulose, sodium carboxymethylcellulose, glucose, hydroxypropyl-β-cyclodextrin, sodium sulfobutyl-β-cyclodextrin, polyvinylpyrrolidone, low-melting-point waxes, ion exchange resins, and a combination of any two or more of the above components. The liquid and semi-solid excipients may be selected from glycerin, propylene glycol, water, ethanol and various oils, including petroleum, animal oils, vegetable oils or synthetic sources such as peanut oil, soybean oil, mineral oil, and sesame oil. Preferred liquid carriers, especially those used for injectable solutions, include water, saline, aqueous glucose solution, and ethylene glycol. Other suitable pharmacologically acceptable excipients are described in Remington's Pharmaceutical Sciences, Mack Pub. Co., New Jersey (1991) and are incorporated herein by reference.

In the present invention, the term "pharmaceutically acceptable salt" refers to a non-toxic acid or alkaline earth metal salt of the compound of general formula I. These salts may be prepared in situ during the final separation and purification of the compound of general formula I, or by reacting suitable organic or inorganic acids or alkali with alkaline or acidic functional groups, respectively. Representative salts include, but are not limited to: acetate, adipate, alginate, citrate, aspartate, benzoate, benzene sulfonate, bisulfate, butyrate, camphorate, camphorsulfonate, digluconate, cyclopentane propionate, lauryl sulfate, ethanesulfonate, glucoheptonate, glycerophosphate, hemisulfate, heptanoate, caproate, fumarate, hydrochloride, hydrobromide, hydroiodate, 2-hydroxyethanesulfonate, lactate, maleate, mesylate, nicotinate, 2-naphthalene sulfonate, oxalate, pamoate, pectate, thiocyanate, 3-phenylpropionate, picrate, neopentanoate, propionate, succinate, sulfate, tartrate, thiocyanate, salicylate, p-toluenesulfonate, and undecanoate. In addition, nitrogen-containing basic groups may be quaternized by the following reagents: alkyl halides, such as chlorides, bromides and iodides of methyl, ethyl, propyl and butyl; dialkyl sulfates, such as sulfates of dimethyl, diethyl, dibutyl and diamyl; long-chain halides, such as chlorides, bromides and iodides of decyl, lauryl, myristyl and stearyl; and arylalkyl halides, such as bromides of benzyl and phenylethyl, etc. In this way, a water-soluble or oil-soluble or dispersible product is obtained. Examples of acids that may be used to form pharmaceutically acceptable acid addition salts include inorganic acids such as hydrochloric acid, sulfuric acid and phosphoric acid, and organic acids such as oxalic acid, maleic acid, methanesulfonic acid, succinic acid, and citric acid. Alkali addition salts may be prepared in situ during the final separation and purification of the compound of general formula I, or by reacting a carboxylic acid moiety with suitable alkali (e.g., hydroxides, carbonates or bicarbonates of pharmaceutically acceptable metal cations) or ammonia, or organic primary, secondary or tertiary amines, respectively. Pharmaceutically acceptable salts include, but are not limited to, cations based on alkali metals and alkaline earth metals, such as salts of sodium, lithium, potassium, calcium, magnesium and aluminum; and non-toxic ammonium, quaternary ammonium and amine cations, including, but not limited to, ammonium, tetramethylammonium, tetraethylammonium, methylamine, dimethylamine, trimethylamine, triethylamine, ethylamine, etc. Other representative organic amines used to form alkali addition salts include diethylamine, ethylenediamine, ethanolamine, diethanolamine, piperazine, etc.

In the present invention, the term "pharmaceutically acceptable prodrug" refers to those prodrugs of the compounds in the preferred embodiments, which are rapidly transformed in vivo into parent compounds indicated in the above general formula, e.g., hydrolyzed in blood. A complete discussion is provided in "T. Higuchi and V. Stella, Pro-drugs as Novel Delivery Systems, Vol. 14 of the A.C.S. 15 Symposium Series" and "Edward B. Roche, Bioreversible Carriers in Drug Design, American Pharmaceutical Association and Pergamon Press, 1987", both of which are incorporated herein by reference.

The present invention provides a preparation method of the compound of general formula I. Using sarsasapogenin as an example, a preparation method of key intermediates is as follows:
α configuration:
   Method I: 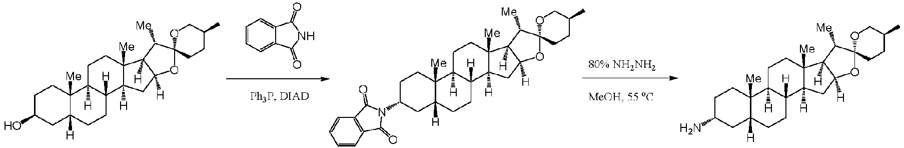
   Method II: 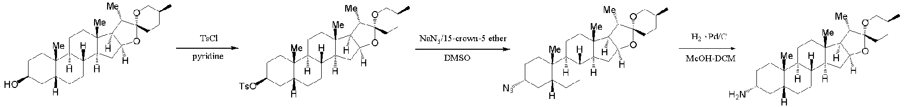
βconfiguration:
   Method I: 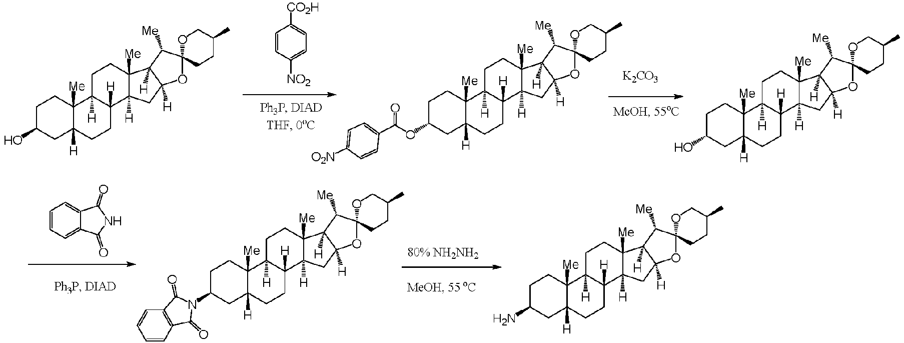
   Method II: 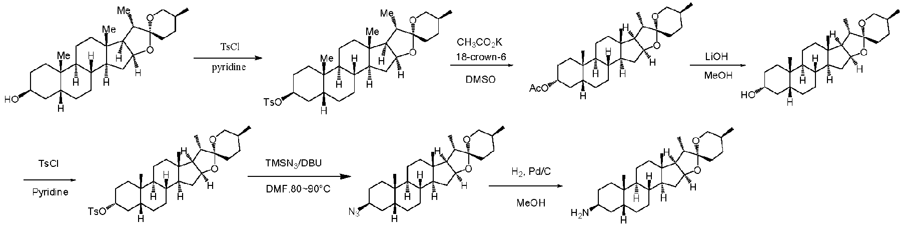
Mixed configuration:

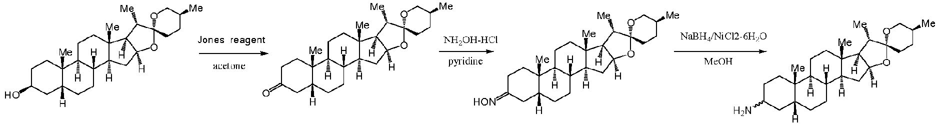

The compound of general formula I is prepared as follows: taking an α configuration of parent nuclei of sarsasapogenin as an example (other configurations or certain configuration of other parent nuclei, the preparation methods of which are consistent with the methods as provided), a synthesis route is as follows: 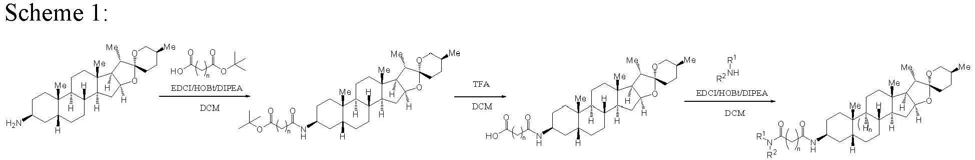 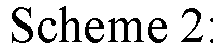 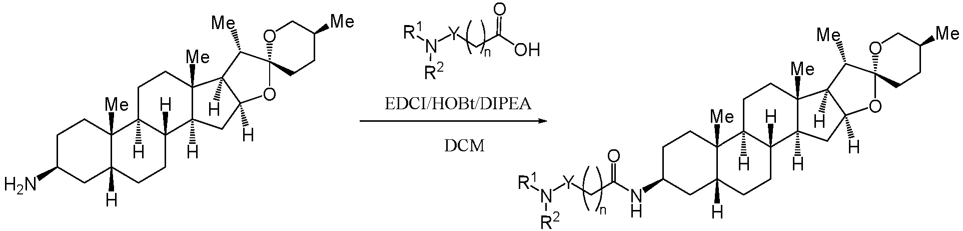 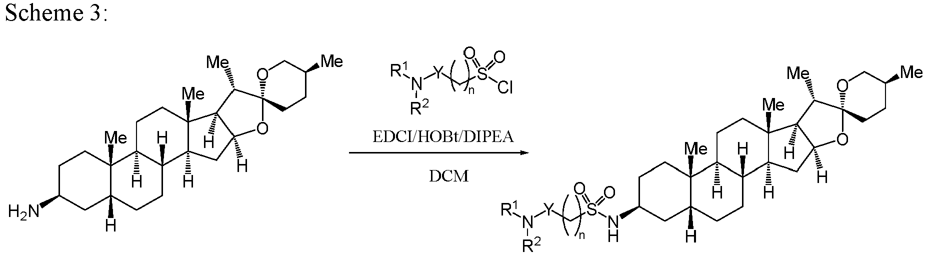 in each formula, R¹, R², Y, and n are defined as shown above.

The present invention will be further set forth below in conjunction with embodiments. It should be understood that the following embodiments are only used to illustrate the present invention, but not to limit the scope of the present invention.

The following abbreviations have meanings as below:
DBU represents 1,8-diazabicyclo[5.4.0]undeca-7-ene; DIBAL represents diisobutylaluminum hydride; DIAD represents diisopropyl azodicarboxylate; DIEA represents diisopropylyelethylamine; DMAP represents N,N-dimethylaminopyridine; DME represents 1,2-dimethoxyethane; DMF represents N,N-dimethylformamide; DMPE represents 1,2-bis(dimethylphospholano)ethane; DMSO represents dimethyl sulfoxide; DPPB represents 1,4-bis(dimethylphospholano)butane; DPPE represents 1,2-bis(dimethylphospholano)ethane; DPPF represents 1,1'-bis(dimethylphospholano)ferrocene; DPPM represents 1,1'-bis(dimethylphospholano)methane; EDC represents 1-(3-dimethylaminopropyl)-3-ethylcarbodiimide hydrochloride; HATU represents 2-(7-aza-1H-benzotriazol-1-yl)-1,1,3,3-tetramethyluronium hexafluorophosphate; HMPA represents hexamethylphosphamide; IPA represents isopropyl alcohol; LDA represents lithium diisopropylamide; LHMDS represents lithium bis(trimethylsilyl)amide; LAH represents lithium aluminum hydride; PyBOP represents benzotriazol-1-yl-oxytris(pyrrolidino)phosphonium hexafluorophosphate; TDA-I represents tris(2-(2-methoxyethoxy)ethyl)amine; DCM represents dichloromethane; TEA represents triethylamine; TFA represents trifluoroacetic acid; THF represents tetrahydrofuran; NCS represents N-chlorosuccinimide; NMM represents N-methylmorpholine; NMP represents N-methylpyrrolidone; PPh₃ represents triphenylphosphine; T₃P represents 1-propylphosphonic acid cyclic anhydride; PMA represents phosphomolybdic acid; PE represents petroleum ether; EA represents ethyl acetate; RBF represents a round-bottom flask; and r.t represents room temperature.

Unless otherwise defined, all technical and scientific terms used in the present invention have the same meaning as commonly understood by those skilled in the art. In addition, any method and material similar to or same as the described content may be applied to the methods of the present invention. The preferred implementation methods and materials described herein are for demonstration purposes only.

### Embodiment 1

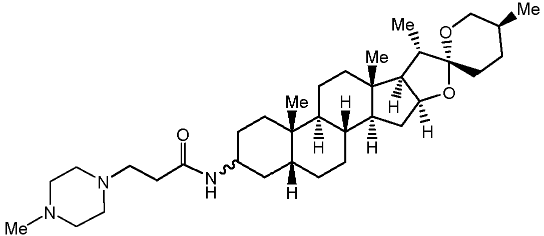

A preparation process was specifically described as follows:

### Step 1: intermediate 1

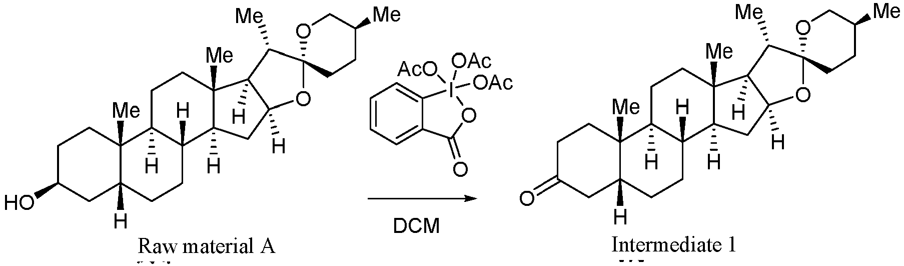

10 g of raw material A was added to a 500 mL round-bottom flask, dissolved in DCM (100 mL), added with Dess-Martin (12.8 g) at 0°C, heated to r.t. after 15 min, reacted by stirring for 1 h, and the completion of the reaction was tracked by TLC (PMA color developing). After the reaction was completed, a filter residue was removed by suction filtration; the filtrate was spin-dried, then redissolved in DCM, washed twice with water, dried, and subjected to silica-gel column chromatography to obtain 9 g of white solid product (*intermediate 1*).

### Step 2: intermediate 2

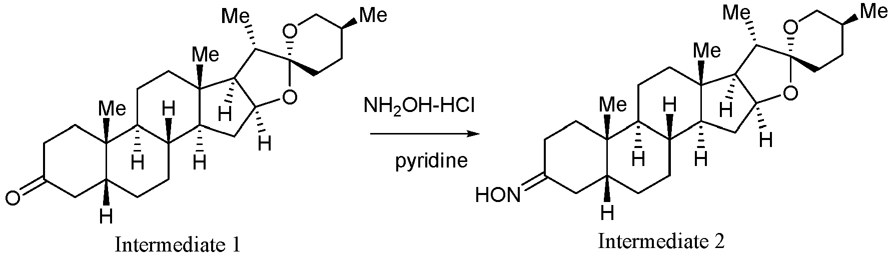

5 g of intermediate 1 and 1.7 g of NH₂OH·HCl were added to a 100 mL round-bottom flask, dissolved in 50 mL of dry Pyridine, reacted by stirring at 70°C for 1-2 h, and the completion of the reaction was tracked by TLC (PMA color developing). After the reaction was completed, the reactant was spin-dried to remove the solvent, redissolved in DCM, washed twice with 1N HCl aqueous solution, dried, and then subjected to silica gel column chromatography to obtain 5.2 g of light yellow solid crude product.

### Step 3: intermediate 3

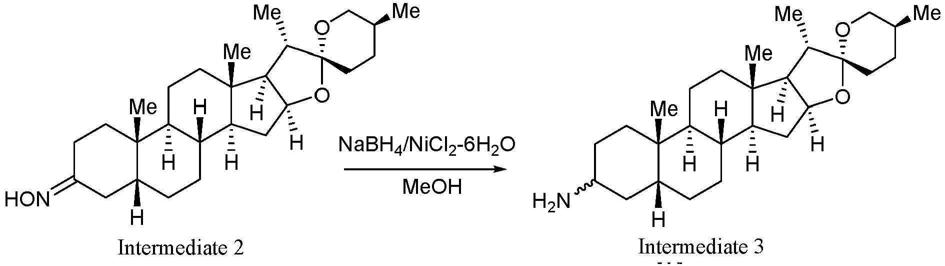

5 g of intermediate 2 was added to a 500 mL round-bottom flask, dissolved with 100 mL of dry MeOH, added with 4.2 g of NiCl₂·6H₂O at 0°C, added with 2.7 g of NaBH₄ in batches after 15 min, heated to r.t. after 30 min, reacted by stirring for 4 h, and the completion of the reaction was tracked by TLC (PMA color developing) After the reaction was completed, a filter residue was removed by suction filtration, and a filtrate was spin-dried to obtain 5 g of white solid product.

### Step 4: embodiment 1

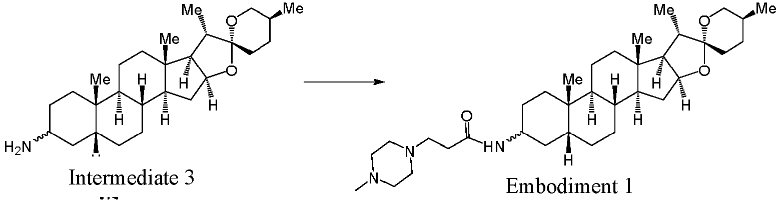

In a round-bottom flask, 0.055 g of intermediate 3, 0.041 g of 3-(4-methylpiperazine-1-yl)-propionic acid, 0.046 g of EDC, 0.084 mL of triethylamine and 0.003 g of DMAP were dissolved in 2 mL of dichloromethane, reacted at room temperature for 2 h, and the reaction was detected. After the reaction was completed, the reactant was washed twice with a saturated NH₄Cl aqueous solution, dried, and subjected to silica gel column chromatography to obtain 25 mg of product.

NMR data: ¹H NMR (400 MHz, CDCl₃) δ 0.76 (s, 3H), 0.80-2.30 (m, 36H), 2.25-2.80 (m, 11H), 3.25-4.10 (m, 2H), 4.150-4.55 (m, 2H), 8.60-8.70 (m, 5H); mass spectrum: [M+1]570.5.

### Embodiment 1A

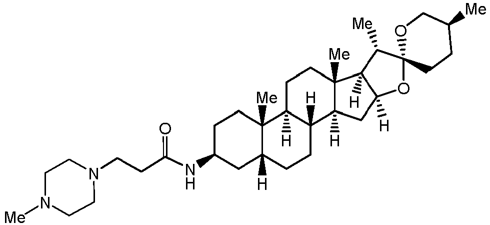

A preparation process of Embodiment 1A was specifically described as follows:

### Step 1: intermediate 4

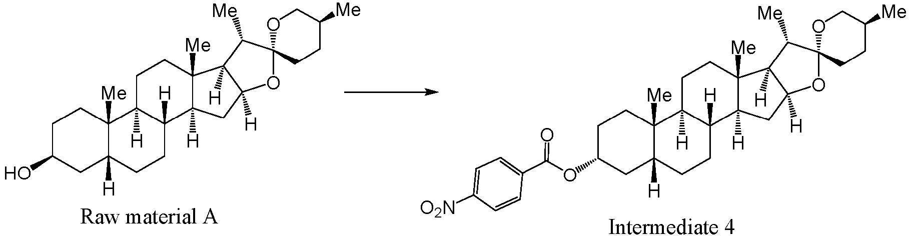

Under the protection of argon, 0.5 g of sarsasapogenin raw material A, 0.4 g of p-nitrobenzoic acid, and 0.63 g of PPh₃ were dissolved in 5 mL of dry THF, stirred in an ice water bath for 5 min, then slowly added dropwise with DIAD, stirred for 10 min, reacted at room temperature for 3 h after the ice water bath was removed, and the reaction was detected. After the reaction was completed, the reactant was spin-dried to remove the solvent, extracted with sodium bicarbonate solution/dichloromethane, purified by silica gel column: PE:EA=45:1, subjected to spot plate detection (PE:EA=15:1), and color-developed with vanillin. The yield was 55%.

### Step 2: intermediate 5

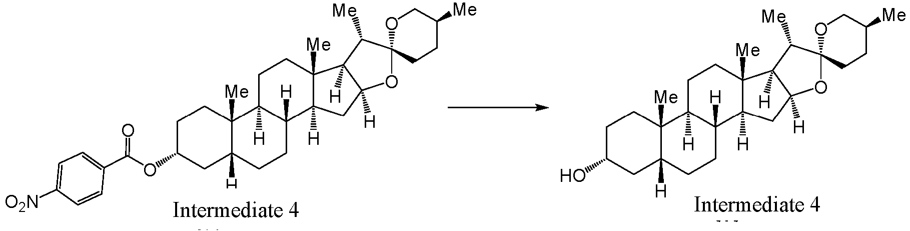

15 mL of MeOH was added to 0.36 g of intermediate 4 and 0.35 g of K₂CO₃ and stirred overnight at 55°C. The reactant was spin-dried to remove the solvent, extracted with water/dichloromethane; an organic layer was spin-dried to obtain a product, which was directly put into the next reaction and color-developed with vanillin. The yield was 80%.

### Step 3: intermediate 6

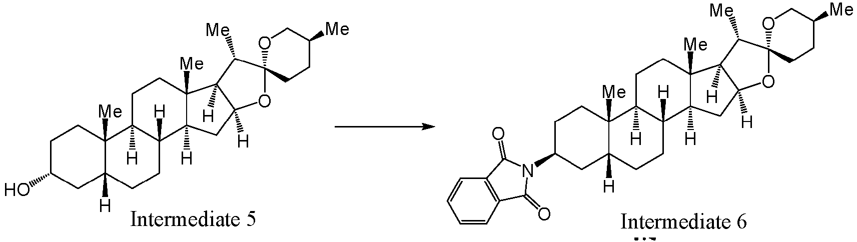

Under the protection of argon, 0.2 g of intermediate 5, 0.14 g of phthalimide, and 0.25 g of PPh₃ were dissolved in 4 mL of dry THF, stirred in an ice water bath for 5 min, then slowly added dropwise with 0.19 g of DIAD, stirred for 10 min, reacted at room temperature for 3 h after the ice water bath was removed, and the reaction was detected. The reactant was spin-dried to remove the solvent, extracted with water/dichloromethane, purified by silica gel column: PE:EA=45:1, subjected to a climbing board test (PE:EA=15:1), and color-developed with vanillin. The yield was 50%.

### Step 4: intermediate 7

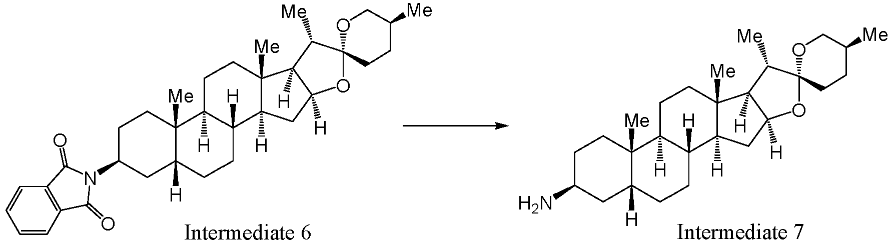

10 mL ofMeOH was added to 0.13 g of intermediate 6 and 0.072 g of N₂H₄·H₂O and stirred overnight at 55°C. The reactant was spin-dried to remove the solvent, washed with water, and extracted with dichloromethane; an organic layer was spin-dried to obtain a product, which was directly put into the next reaction and color-developed with vanillin. The yield was 90%.

### Step 5: embodiment 1A

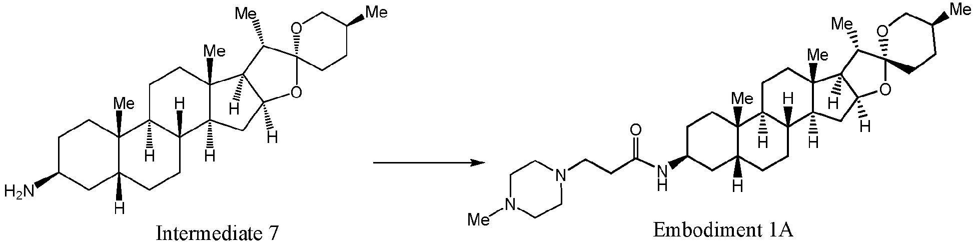

Under the protection of argon, 0.07 g of intermediate, 0.058 g of 3-(4-methylpiperazine-1-yl)-propionic acid, 0.065 g of EDC, and 0.01 of DMAP were dissolved in 4 mL of dichloromethane, reacted at room temperature for 4 h, and the reaction was detected. The reactant was extracted with sodium bicarbonate solution/dichloromethane, and passed through a peralkaline alumina column PE:EA=1:1, and color-developed with vanillin. The yield was 65%.

NMR data: ¹H NMR (400 MHz, CDCl₃) δ 8.85 (d, *J =* 6.9 Hz, 1H), 4.41 (dd, *J =* 13.0, 6.6 Hz, 1H), 4.18 (s, 1H),3.95 (d, *J=* 10.6 Hz, 1H), 3.30 (d, *J=* 11.6 Hz, 1H), 3.01-2.20(brs, 8H), 2.62 (d, *J=* 3.0 Hz, 2H), 2.39(s, 2H), 2.30 (s, 3H), 1.23-2.07 (m, 27H), 1.08 (d, *J=* 6.7 Hz, 3H), 1.05 - 0.93 (m, 6H), 0.76 (s, 3H). Mass spectrum: [M+1]570.5.

### Embodiment 1B

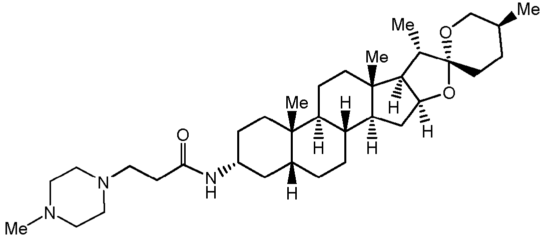

A preparation process of Embodiment 1B was specifically described as follows:

### Step 1: intermediate 8

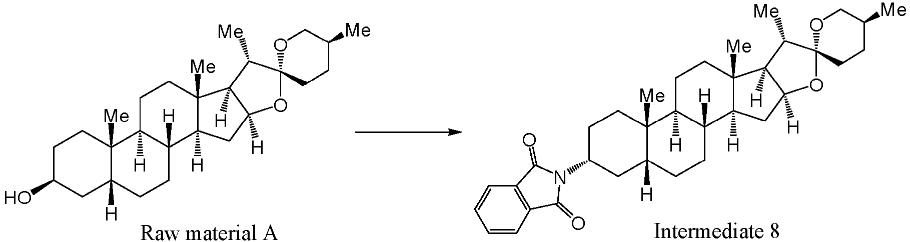

Using raw material A as a starting material, the intermediate 8 was obtained under the same conditions as the third step in Embodiment 1A. The yield was 50%.

### Step 2: intermediate 9

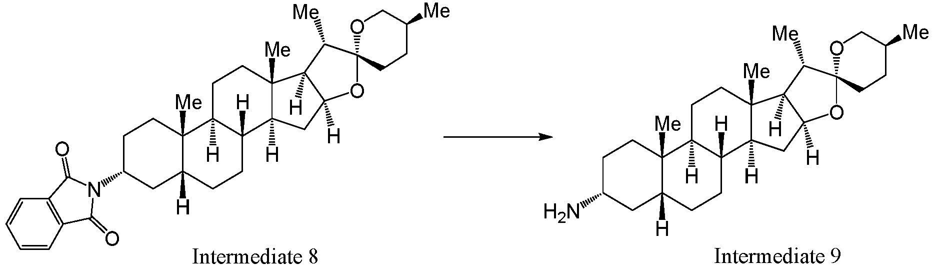

In this step, using intermediate 8 as raw material, intermediate 9 was obtained under the same conditions as the four step in Embodiment 1A. The yield was 90%.

### Step 3: embodiment 1B

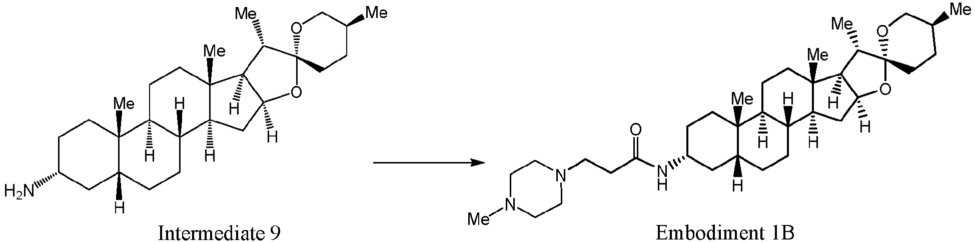

In this step, using intermediate 9 and 3-(4-methylpiperazine-1-yl)-propionic acid as raw materials, Embodiment 1B was obtained under the same conditions as the fifth step in Embodiment 1A. The yield was 65%.

NMR data: ¹H NMR (400 MHz, CDCl₃) δ 8.15 (d, *J =* 7.6 Hz, 1H), 4.33 (dd, *J=* 14.8, 7.5 Hz, 1H), 3.88 (dd, *J* =10.9, 2.4 Hz, 1H), 3.65 (m, 1H), 3.23 (d, *J=* 10.9 Hz, 1H), 2.90-2.10(brs, 8H), 2.56 (t, *J* = 6.2 Hz, 2H),2.26 (m, 2H), 2.24 (s, 3H), 1.23-2.07 (m, 27H), 1.01 (d, *J* =7. 1Hz, 3H), 0.95- 0.83 (m, 6H), 0.69 (s,3H). Mass spectrum: [M+1]570.5.

### Embodiment 2

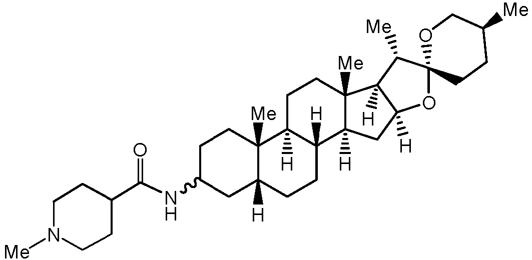

Using intermediate 3 and 1-methylpiperidin-4-formic acid as raw materials, Embodiment 2 was obtained under the same conditions as the experimental steps described in the fourth step in Embodiment 1.

NMR data: ¹H NMR(CDCl₃, 400MHz): δ 0.75 (s, 3H), 0.80-2.70 (m, 43H), 2.95-4.00 (m, 7H), 4.40-4.55 (m, 1H), 6.05 (s, 1H); mass spectrum: [M+1]541.5.

### Embodiment 3

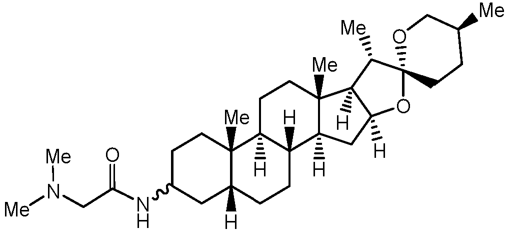

Using intermediate 3 and N,N-dimethylglycine as raw materials, Embodiment 3 was obtained under the same conditions as the experimental steps described in the fourth step in Embodiment 1.

NMR data: ¹H NMR(CDCl₃, 400MHz): δ 0.76 (s, 3H), 0.80-2.20 (m, 36H), 2.29 (s, 3H), 2.31 (s, 3H), 2.80-3.00 (m, 2H), 3.20-3.35 (s, 1H), 3.85-4.40 (m, 3H), 7.10-7.40 (m, 1H); mass spectrum: [M+1]501.3.

### Embodiment 4

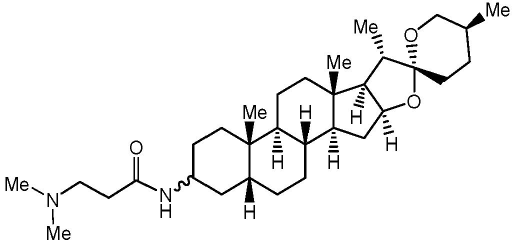

Using intermediate 3 and N,N-dimethyl-β-alanine as raw materials, Embodiment 4 was obtained under the same conditions as the experimental steps described in the fourth step in Embodiment 1.

NMR data: ¹H NMR(CDCl₃, 400MHz): δ 0.76 (s, 3H), 0.80-2.20 (m, 36H), 2.32 (s, 3H), 2.36 (s, 3H), 2.45-2.70 (m, 4H), 3.25-3.40 (m, 1H), 3.80-4.45 (m, 3H), 8.90-9.10 (m, 1H). Mass spectrum: [M+1]515.3.

### Embodiment 4A

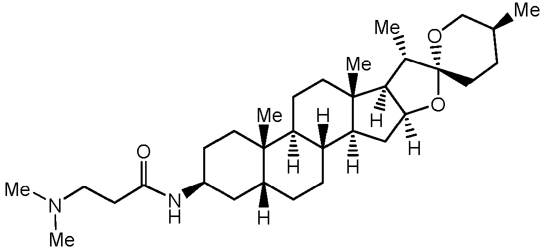

Using intermediate 7 and N,N-dimethyl-B-alanine as raw materials, Embodiment 4A was obtained under the same conditions as the experimental steps described in the fifth step in Embodiment 1A.

NMR data: ¹H NMR (400 MHz, CDCl₃) δ 9.20 (d, *J=* 7.8 Hz, 1H), 4.41 (dd, *J=* 14.1, 7.6 Hz, 1H), 4.19 (d, *J* = 7.2 Hz, 1H), 3.95 (dd, *J =* 11.0, 2.5 Hz, 1H), 3.30 (d, *J =* 10.9 Hz, 1H), 2.59 - 2.50 (m, 2H), 2.38 - 2.33 (m, 2H), 2.30 (s, 6H), 1.23-2.07 (m, 27H), 1.08 (d, *J=* 7.1 Hz, 3H), 1.02 - 0.92 (m, 6H), 0.76 (s, 3H). Mass spectrum: [M+1]515.3.

### Embodiment 4B

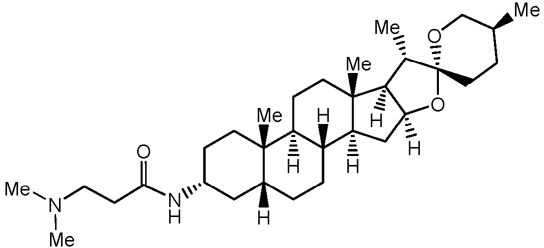

Using intermediate 9 and N,N-dimethyl-B-alanine as raw materials, Embodiment 4B was obtained under the same conditions as the experimental steps described in the fifth step in Embodiment 1A.

NMR data: ¹H NMR (400 MHz, CDCl₃) δ 7.77 (d, *J=* 7.2 Hz, 1H), 4.41 (dd, *J=* 14.1, 7.5 Hz, 1H), 3.95 (dd, *J =* 10.9, 2.3 Hz, 1H), 3.78 - 3.65 (m, 1H), 3.30 (d, *J =* 11.0 Hz, 1H), 2.53 (t, *J =* 6.2 Hz, 2H), 2.32 (t, *J=* 6.2 Hz, 2H), 2.27 (s, 6H), 1.23-2.07 (m, 27H), 1.08 (d, *J =* 7.1 Hz, 3H), 1.02 - 0.93 (m, 6H), 0.76 (s, 3H). Mass spectrum: [M+1]515.3.

### Embodiment 5

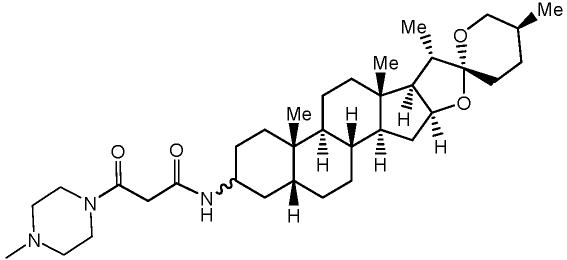

Using intermediate 3 and 3-oxo-3-(4-methylpiperazin-1-yl)propionic acid as raw materials, Embodiment 5 was obtained under the same conditions as the experimental steps described in the fourth step in Embodiment 1.

NMR data: ¹H NMR(CDCl₃, 400MHz): δ 0.76 (s, 3H), 0.80-2.20 (m, 36H), 2.40 (s, 3H), 2.55-2.70 (m, 4H), 3.25-4.20 (m, 9H), 4.45-4.55 (m, 1H); mass spectrum: [M+1]584.3.

### Embodiment 6

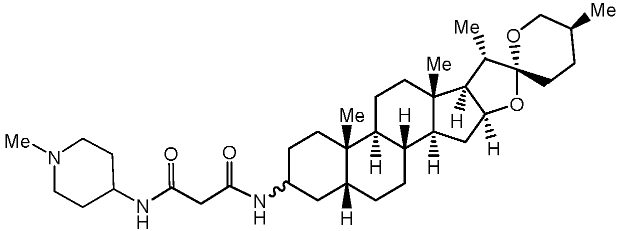

Using intermediate 3 and 3-oxo-3-(1-methylpiperazin-4-amino)propionic acid as raw materials, Embodiment 6 was obtained under the same conditions as the experimental steps described in the fourth step in Embodiment 1.

NMR data: ¹H NMR(CDCl₃, 400MHz): δ 0.76 (s, 3H), 0.80-2.20 (m, 40H), 2.75-3.70 (m, 12H), 3.25-3.70 (m, 2H), 3.84 - 4.40 (m, 3H), 7.60-7.80 (m, 5H); mass spectrum: [M+1]598.3.

### Embodiment 7

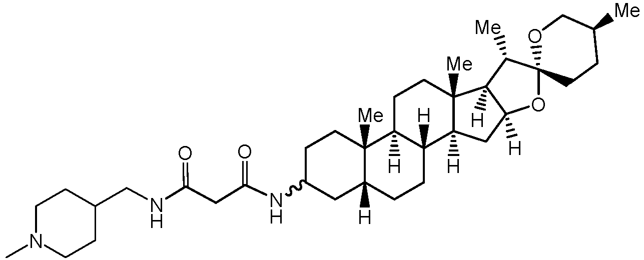

Using intermediate 3 and 3-oxo-3-(1-methylpiperidin-4-methylamino)propionic acid as raw materials, Embodiment 7 was obtained under the same conditions as the experimental steps described in the fourth step in Embodiment 1.

NMR data: ¹H NMR(CDCl₃, 400MHz): δ 0.76 (s, 3H), 0.80-2.30 (m, 41H), 2.75-3.00 (m, 4H), 3.25-3.70 (m, 11H), 3.84 - 4.40 (m, 2H), 7.60-7.70 (m, 1H); mass spectrum: [M+1]612.3.

### Embodiment 8

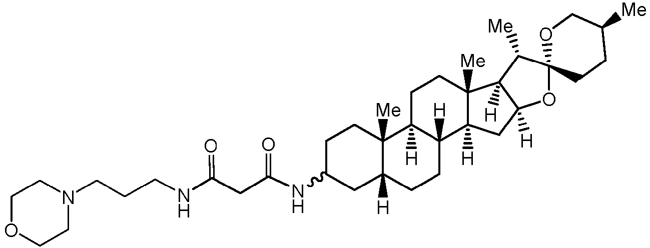

Using intermediate 3 and 3-oxo-3-(3-morpholinopropyl)aminopropionic acid as raw materials, Embodiment 8 was obtained under the same conditions as the experimental steps described in the fourth step in Embodiment 1.

NMR data: ¹H NMR(CDCl3, 400MHz): δ 0.75 (s, 3H), 0.80-2.30 (m, 38H), 2.45-2.60 (m, 4H), 3.25-4.00 (m, 13H), 4.10-4.55 (m, 2H), 7.60-7.80 (m, 1H); mass spectrum: [M+1]628.3.

### Embodiment 9

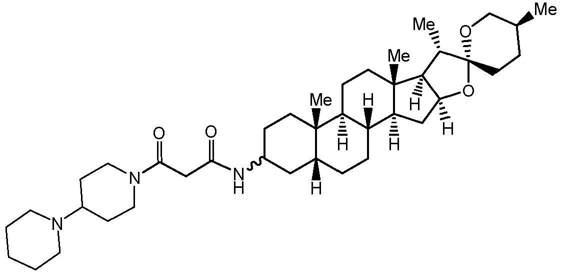

Using intermediate 3 and 3-oxo-3-(1,4-bipiperidin-1-yl)propionic acid as raw materials, Embodiment 9 was obtained under the same conditions as the experimental steps described in the fourth step in Embodiment 1.

NMR data: ¹H NMR(CDCl3, 400MHz, ppm): δ 0.76 (s, 3H), 0.80-2.30 (m, 46H), 2.55-3.60 (m, 10H), 3.75-4.55 (m, 5H), 7.20-7.40 (m, 1H); mass spectrum: [M+1]652.3.

### Embodiment 10

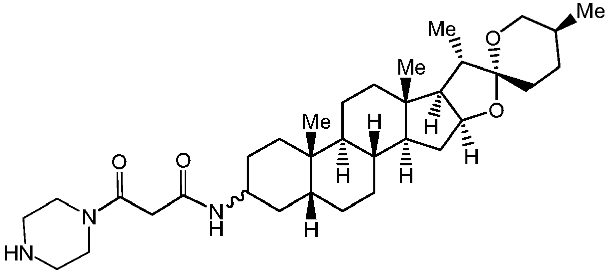

Using intermediate 3 and 3-oxo-3-(piperazin-1-yl)propionic acid as raw materials, Embodiment 10 was obtained under the same conditions as the experimental steps described in the fourth step in Embodiment 1.

NMR data: ¹H NMR(CDCl₃, 400MHz): δ 0.77 (s, 3H), 0.80-2.20 (m, 36H), 2.85-4.00 (m, 13H), 4.40-4.55 (m, 1H), 7.40-7.40 (m, 1H); mass spectrum: [M+1]570.3.

### Embodiment 11

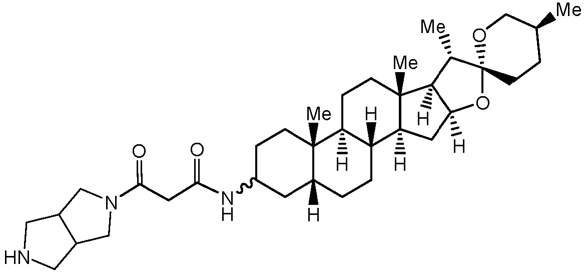

Using intermediate 3 and 3-(hexahydropyrrolo[3,4-c]pyrrol-2(1H)-yl)-3-oxopropanoic acid as raw materials, Embodiment 11 was obtained under the same conditions as the experimental steps described in the fourth step in Embodiment 1.

NMR data: ¹H NMR(CDCl3, 400MHz): δ 0.76 (s, 3H), 0.80-2.20 (m, 38H), 2.95-4.00 (m, 13H), 4.40-4.55 (m, 1H), 7.20-7.50 (m, 2H); mass spectrum: [M+1]596.4.

### Embodiment 12

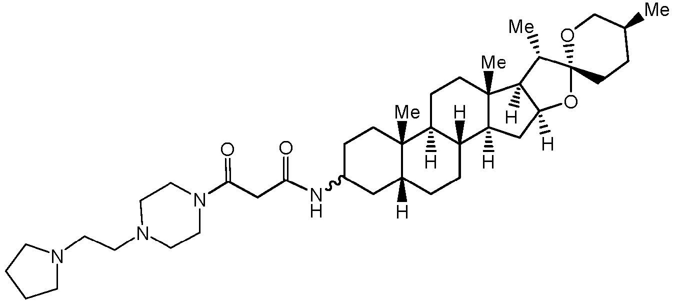

Using intermediate 3 and 3-oxo-3-(4-(2-(pyrrolidin-1-yl)ethyl)piperazin-1-yl)propanoic acid as raw materials, Embodiment 12 was obtained under the same conditions as the experimental steps described in the fourth step in Embodiment 1.

NMR data: ¹H NMR(CDCl₃, 400MHz): δ 0.74(s, 3H), 0.80-2.30 (m, 36H), 2.45-2.80 (m, 8H), 3.25-4.00 (m, 11H), 4.10-4.45 (m, 2H); mass spectrum: [M+1]667.5.

### Embodiment 13

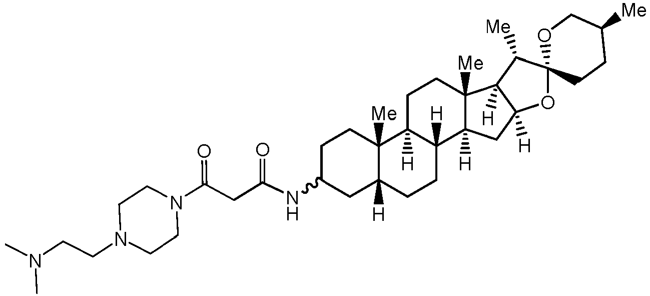

A preparation process of Embodiment 13 was specifically described as follows:

### Step 1: intermediate 10

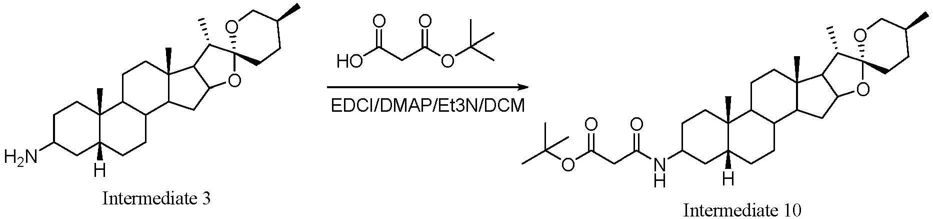

The intermediate 12 (5 g, 1 equivalent), mono-tert-butyl malonate (2 equivalents), EDC·HCl (2 equivalents), DMAP (0.1 equivalent) and Et3N (5 equivalents) were added to a 500 mL round-bottom flask, dissolved in 100 mL of dry DCM, and stirred for 2 h at room temperature, and the completion of the reaction was tracked by TLC (PMA color developing). After the reaction was completed, the reactant was washed twice with a saturated NH4Cl aqueous solution, dried, and subjected to silica gel column chromatography to obtain 4.5 g of product.

### Step 2: intermediate 11

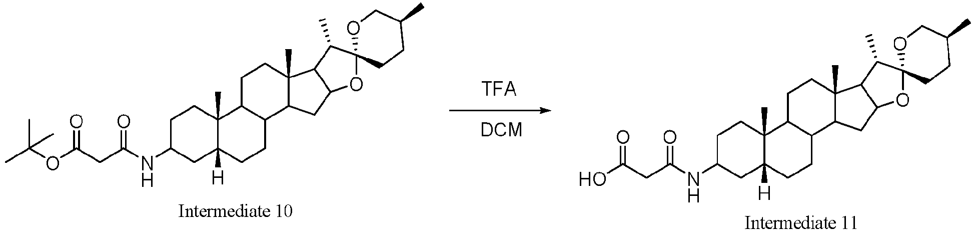

The intermediate 10 (4.5 g, 1 equivalent) was added to a 500 mL round-bottom flask, dissolved in 100 mL of dry DCM, added dropwise with TFA (10 mL, 15 equivalents) at 0°C, heated to room temperature after 15 min, reacted for 1 to 2 h, and the completion of the reaction was tracked by TLC (PMA color developing). After the reaction was completed, the reactant was spin-dried, redissolved in DCM, washed twice with a saturated NaHCO₃ aqueous solution, dried, and subjected to silica gel column chromatography to obtain 4 g of white solid product, i.e., the intermediate 11.

### Step 3: embodiment 13

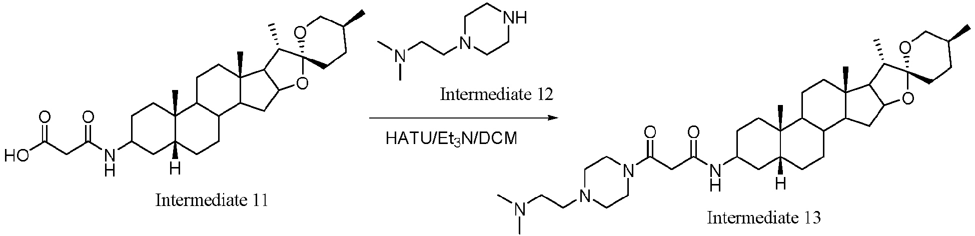

The intermediate 11 (50 mg, 1 equivalent), the intermediate 12 (2 equivalents), HATU (2 equivalents) and Et₃N (5 equivalents) were added to a 25 mL round-bottom flask, dissolved in 3 ml of dry DCM, reacted by stirring for 1 h at room temperature, and the completion of the reaction was tracked by TLC (PMA color developing). After the reaction was completed, the reactant was washed twice with a saturated NH4Cl aqueous solution, dried, and subjected to thin plate chromatography (dichloromethane:methanol = 10:1) to obtain 8 mg of white solid.

NMR data was as follows: ¹H NMR(CDCl₃, 400MHz, ppm): δ 0.76 (s, 3H), δ 0.80-2.30 (m, 36H), δ 2.55-3.00 (m, 9H), δ 3.15-4.50 (m, 14H), δ 5.25 (br s, 1H); mass spectrum: [M+1]641.5.

### Embodiment 13A

### Step 1: intermediate 13

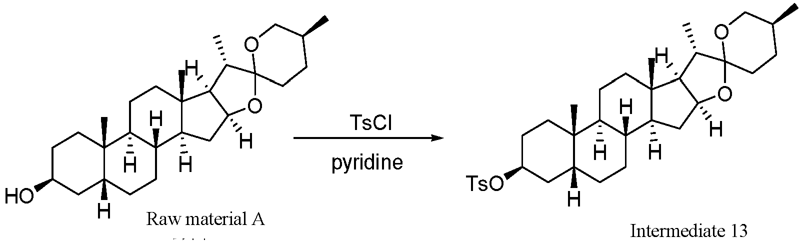

A raw material A (460 g, 1.10 mol, 1.0 eq) was dissolved in pyridine (6.9 L) and cooled to 0±5°C; TsCl (210.5 g, 1.10 mol, 1.0 eq) was dissolved in pyridine (600 mL), and slowly added to a reaction solution under the protection of N₂; the reaction solution was naturally heated to room temperature (23 to 28°C); after stirring for 4 h, 105 g of pyridine (200 mL) solution of TsCl (0.55 mol, 0.5 eq) was supplemented to the reaction solution at room temperature under the protection of N₂, reacted overnight at room temperature (23 to 28°C) after dropwise addition; and the system was burgundy, and stirred at room temperature (23 to 28°C) for 48 h. After the reaction was completed, the reaction solution was slowly added dropwise into water (65 L) at 0 to 10°C, a large number of solids were precipitated and filtered to obtain a filter cake, and the filter cake was rinsed with 500 mL of PE:EA (50:1) mixed solvent; and the obtained solid was dried under vacuum (in a water bath at 40 to 50°C) for 8 h to obtain 451 g of solid, i.e., the intermediate 13.

### Step 2: intermediate 14

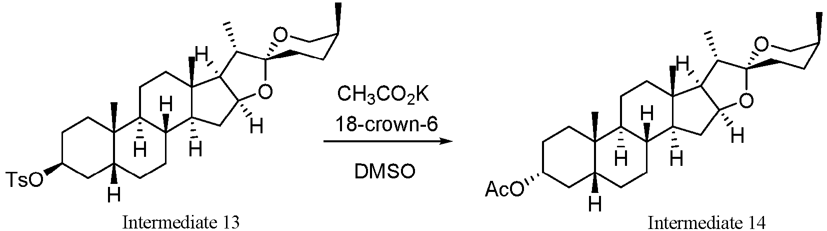

The intermediate 13 (25 g, 43.8 mmol), potassium acetate (8.60 g, 87.6 mmol), and 18-crown-6 (23.15 g, 87.6 mmol), and 300 mL of dimethyl sulfoxide solvent were added to a 500 mL flask. The reaction system was heated to 55°C, and the reaction was maintained at this temperature for 16 h. The reaction was monitored by TLC (petroleum ether:ethyl acetate = 15:1) until the reaction was completed. Then, the reaction mixture was poured into 1 L of ice water and stirred for 30 min. The reactant was filtered to obtain a filter cake, and the filter cake was washed with water to obtain 7 g of white solid.

### Step 3: intermediate 5

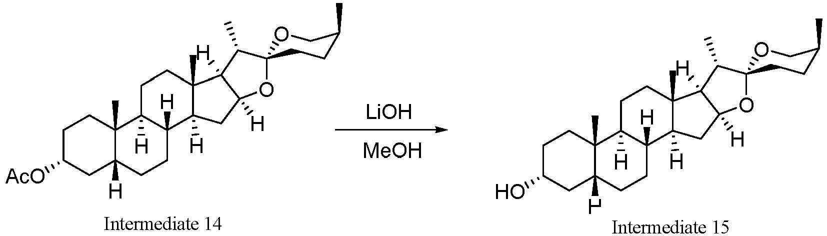

The intermediate 14 (2.0 g, 4.36 mmol), 60 mL of tetrahydrofuran, 60 mL of methanol, 30 mL of water, and 5.5 mL of 4N LiOH aqueous solution were added to a 250 mL flask. The reaction system was heated to 60°C, and the reaction was maintained at this temperature for 2 h. The reaction was monitored by a TLC plate, and a developing agent was petroleum ether: ethyl acetate = 7: 1. When the reaction was completed, most organic solvent was removed by spinning, and 50 mL of water was added. The reactant was filtered to obtain a filter cake, and the filter cake was washed with water to obtain 1.7 g of white solid.

### Step 4: intermediate 15

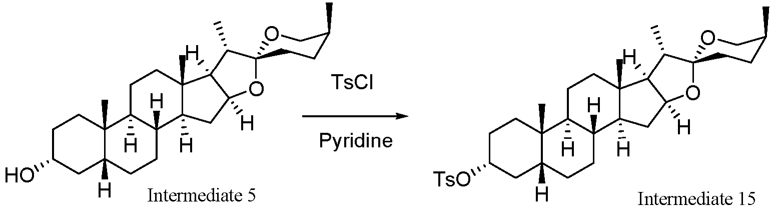

The intermediate 5 (5.0 g, 12.0 mmol), TsCl (11.4 g, 60.0 mmol), and DMAP (73 mg, 0.6 mmol) were added a 100 mL reaction flask, and dissolved with 50 mL of pyridine. The reaction system was heated to 50°C, and the reaction was maintained at this temperature for 16 h. The reaction was monitored by TLC, and a developing agent was PE:EA = 7:1. After the reaction was completed, the reaction solution was poured into 150 mL of water and stirred for 30 min. The reaction solution was filtered to obtain a filter cake, and the filter cake was washed with water to obtain 5.5 g of white solid.

### Step 5: intermediate 16

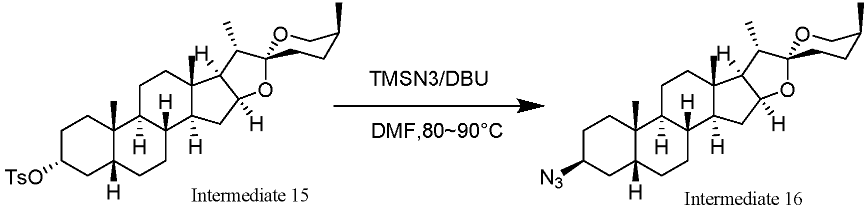

TMSN₃ (0.12 g, 1.1 eq, 2 eq) and DBU (0.32 g, 0.21 mmol, 4 eq) were added dropwise to a DMF (6 mL) suspension of intermediate 15 (0.3 g, 0.52 mmol, 1 eq) at 30 to 35°C under the protection of N₂. The obtained reaction solution was heated to 80 to 90°C for 16 to 20 h. After the intermediate 5 was detected by TLC to basically disappear (PE:EA=10:1), the reaction solution was cooled to 30 to 35°C. The reaction solution was introduced into water (20 mL) at 0 to 10°C, kept stirred at 0 to 10°C for 5 to 10 min, and then filtered. The filter cake was purified by silica gel column chromatography and eluted with PE:EA to obtain 0.13 g of solid, i.e., the intermediate 16, with a yield of 56%.

### Step 6: intermediate 7

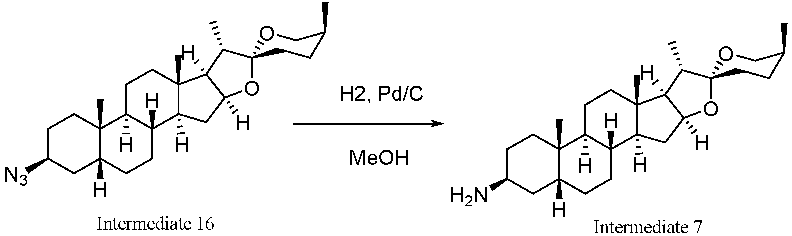

Under the protection of nitrogen, the intermediate 16 (12.0 g, 27.2 mmol) and 100 mL of dichloromethane solvent were added to a flask. 4.8 g of Pd/C and 200 mL of ethanol were then added to this flask. After nitrogen (50 psi) replacement, the reaction system was heated to 50°C, and the reaction was maintained at this temperature for 16 h. The reaction was monitored by TLC, and a developing agent was petroleum ether: ethyl acetate = 20: 1. The reaction solution was filtered to obtain a filter cake, and the filter cake passed through a column (dichloromethane:methanol = 20:1) to obtain 11 g of white solid, i.e., the intermediate 7.

### Step 7: intermediate 17

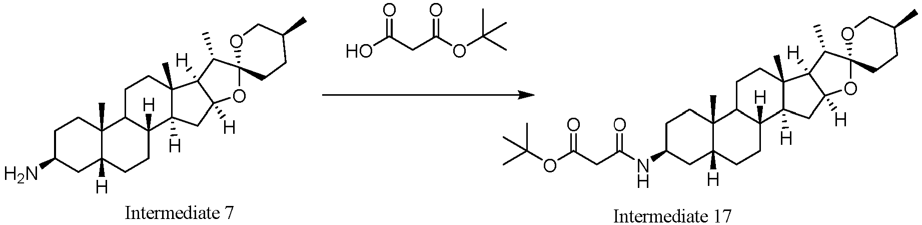

Using the intermediate 7 as raw material, a total of 40.4 g of intermediate 17 was obtained under the same conditions as described in the intermediate 10.

### Step 8: intermediate 18

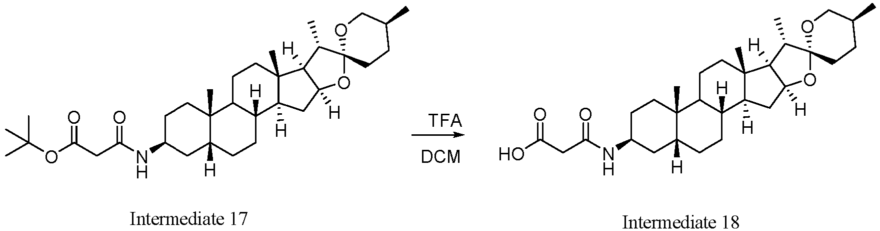

Using intermediate 17 as raw material, 35 g of intermediate 18 was obtained under the same conditions as described in the intermediate 11.

### Step 9: embodiment 13A

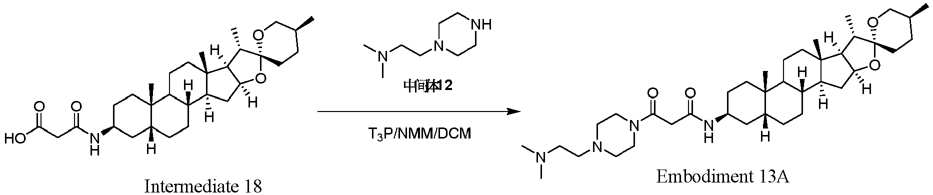

Intermediate 18 (28 g, 1.0 eq) was dissolved in DCM (300 mL), added with intermediate 12 (13.1 g, 1.5 eq), T3P (35.3 g, 2.0 eq) and NMM (11.2 g, 2.0 eq) and reacted at room temperature for 16 h. TLC spot plate detection (DCM/MeOH (5% ammonia methanol) = 20/1) was performed; after the reaction was completed, the reaction solution was washed with water (250 mL); an aqueous phase was extracted with DCM (100 mL x 2); a merged organic phase was dried with anhydrous Na₂SO₄, filtered and concentrated, and then purified by a column (DCM/MeOH (5% ammonia methanol)=45/1 to 15/1) to obtain about 17 g of crude product of Embodiment 13A; this crude product was dissolved in dichloromethane (about 120 mL), and then added with acetonitrile (200 mL) to obtain a solution; this solution was concentrated under reduced pressure to about 100 mL and stirred in an ice bath for 2 h to precipitate a large amount of white solids; the reactant was filtered to obtain a filter cake; and the filter cake was dried, then placed again in pure water (150 mL) at room temperature, slurried and stirred overnight, and then filtered and dried to obtain Embodiment 13A (12.2 g).

NMR data: ¹H NMR (400 MHz, Chloroform-d) δ 8.01 (d, J = 7.7 Hz, 1H), 4.41 (td, J = 7.8, 5.9 Hz, 1H), 4.18 (s, 1H), 3.95 (dd, J = 11.0, 2.8 Hz, 1H), 3.66 (d, J = 5.7 Hz, 2H), 3.57 (t, J = 5.1 Hz, 2H), 3.32 - 3.30 (m, 1H), 3.29 (d, J = 3.7 Hz, 2H), 2.55 (s, 4H), 2.51 - 2.44 (m, 4H), 2.37 (m, 6H), 2.10 - 1.66 (m, 12H), 1.66 - 1.45 (m, 7H), 1.33 -1.10 (m, 8H), 1.08 (m, 3H), 1.03 - 0.95 (m, 6H), 0.75 (s, 3H). Mass spectrum: [M+1]641.5.

### Embodiment 13B

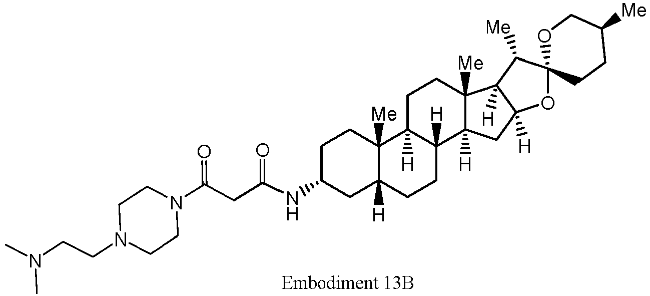

### Step 1: intermediate 19

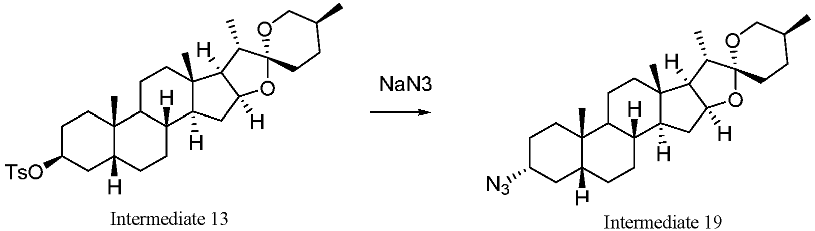

A DMSO (12 L) mixture of intermediate 13 (240 g, 0.42 mol, 1 eq) and 15-crown-5 (277.8 g, 1.2 mol, 3 eq) was stirred evenly until all dissolved, and added with NaN₃ (81.9 g, 1.26 mol, 3 eq) under the protection of N₂ at 25 to 35°C. The obtained mixture was heated to 60 to 70°C, reacted for 2.5 to 3 h, and sampled under the protection of N₂; the raw material disappeared under TLC detection (PE: EA=9:1); the reaction solution was cooled to 20 to 30°C, and added with water; a solid was precipitated, and filtered to obtain a filter cake; and the filter cake was rinsed with 2 L water and dried to obtain 177 g of white solid, i.e., the intermediate 19.

### Step 2: intermediate 23

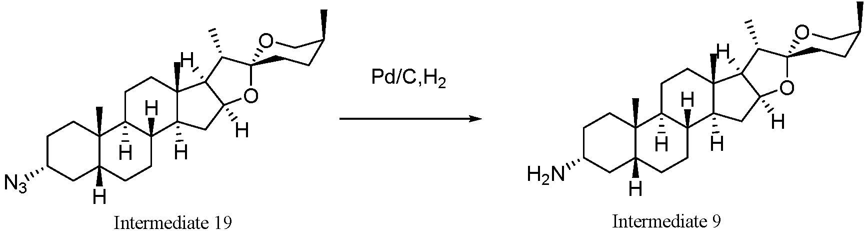

The intermediate 19 (650 g) was dissolved in 10 L of DCM and 10 L of MeOH, and the solution was transferred to an autoclave. 104 g of 10% wet Pd/C was suspended in 200 mL of MeOH and transferred to the autoclave; N₂ replacement was performed for four times in the autoclave at 28 to 35°C; and H₂ replacement was then performed four times in the autoclave at 28 to 35°C. Finally, an H₂ pressure was maintained at 2.5 to 3 MPa; the solution was then kept stirred at 28 to 35°C for 48 h, and sampled; and the raw material disappeared under TLC (PE:EA=20:1) detection. After the reaction was completed, a layer of kieselguhr was spread on a suction filter funnel, and Pd/C in the reaction solution was removed by reduced-pressure filtration to obtain a filter cake; and the filter cake was purified by column chromatography to obtain a total of 460 g of white solid, i.e., the intermediate 9.

### Step 3: intermediate 20

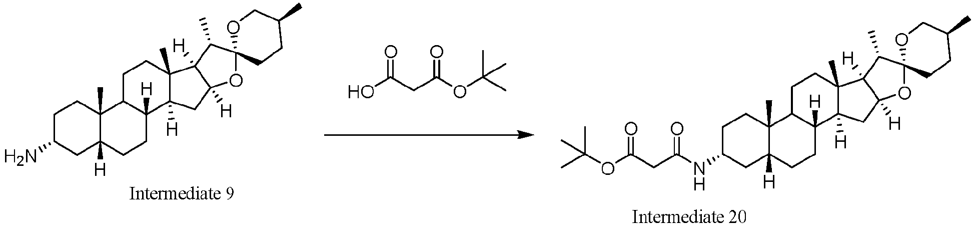

Using the intermediate 9 as raw material, a total of 40.4 g of intermediate 20 was obtained under the same conditions as described in the intermediate 10.

### Step 4: intermediate 21

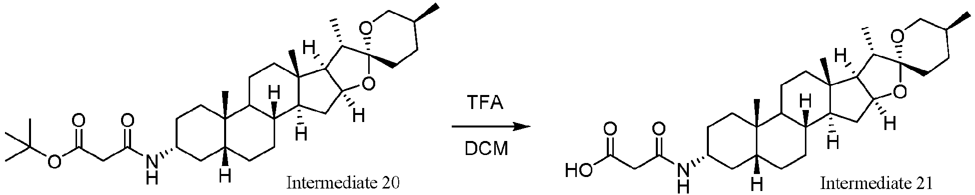

Using the intermediate 20 as raw material, 35 g of crude product of intermediate 21 was obtained under the same experimental steps as described in the intermediate 11.

### Step 5: embodiment 13B

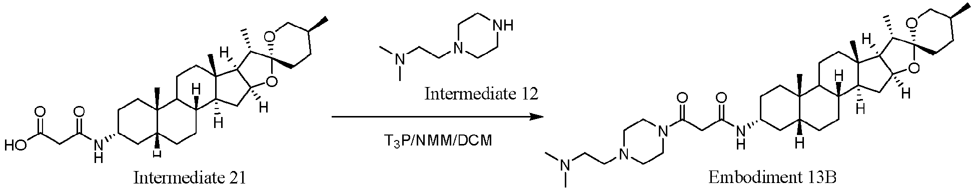

Intermediate 21 (28 g, 1.0 eq) was dissolved in DCM (300 mL), added with intermediate 12 (13.1 g, 1.5 eq), T₃P (35.3 g, 2.0 eq) and NMM (11.2 g, 2.0 eq) and reacted at room temperature for 16 h. TLC spot plate detection (DCM/MeOH (5% ammonia methanol) = 20/1) was performed; after the reaction was completed, the reactant was washed with 250 mL of water; an aqueous phase was extracted with DCM (100 mL x 2); a merged organic phase was dried with anhydrous Na₂SO₄, filtered and concentrated, and then purified by a column (DCM/MeOH (5% ammonia methanol)=45/1 to 15/1) to Embodiment 13B (12.2 g)

NMR data: ¹H NMR (400 MHz, Chloroform-d) δ 7.39 (d, J = 8.2 Hz, 1H), 4.42 (td, J = 7.6, 6.0 Hz, 1H), 3.96 (dd, J = 11.0, 2.8 Hz, 1H), 3.87 - 3.70 (m, 1H), 3.65 (t, J = 5.2 Hz, 2H), 3.56 (q, J = 5.2 Hz, 2H), 3.31 (d, J = 11.0 Hz, 1H), 3.28 (s, 2H), 2.59 - 2.36 (m, 8H), 2.28 (s, 6H), 2.13 - 1.75 (m, 11H), 1.75 - 1.49 (m, 7H), 1.49 - 1.30 (m, 7H), 1.30 - 1.11 (m, 7H), 1.08 (d, J = 7.1 Hz, 4H), 1.04 (d, J = 3.3 Hz, 1H), 1.00 (d, J = 6.5 Hz, 3H), 0.94 (s, 3H), 0.75 (s, 3H). Mass spectrum: [M+1]641.5.

### Embodiment 14

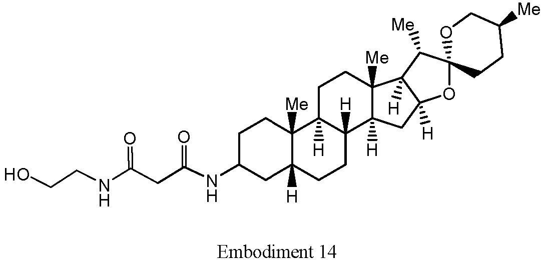

### Step 1: embodiment 14

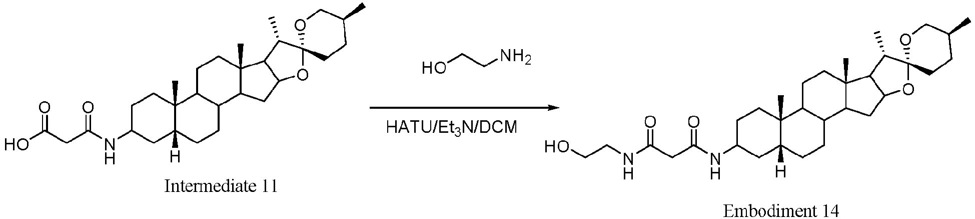

The intermediate 11 (50 mg, 1eq), aminoethanol (2 eq), HATU (2 eq) and Et₃N (5 eq) were added to a 25 mL round-bottom flask, dissolved in 3 mL of dry DCM, reacted by stirring for 1 h at room temperature, and the completion of the reaction was tracked by TLC (PMA color developing). After the reaction was completed, the reactant was washed twice with a saturated NH₄Cl aqueous solution, dried, and subjected to thin plate chromatography (dichloromethane: methanol = 10:1) to obtain 38 mg of white solid, i.e., Embodiment 14.

NMR data was as follows: ¹H NMR(CDCl3, 400MHz, ppm): 0.75 (s, 3H), 0.80-2.10 (m, 36H), 3.10-3.70 (m, 7H), 3.80-4.15 (m, 2H), 4.40-4.55 (m, 1H), 7.10-7.40 (m, 2H); mass spectrum: [M+1]545.5.

### Embodiment 14A

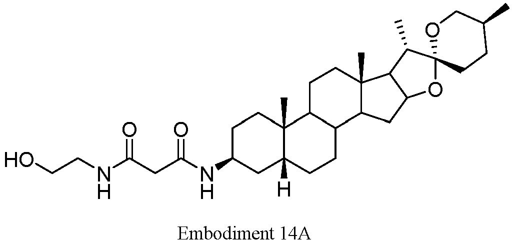

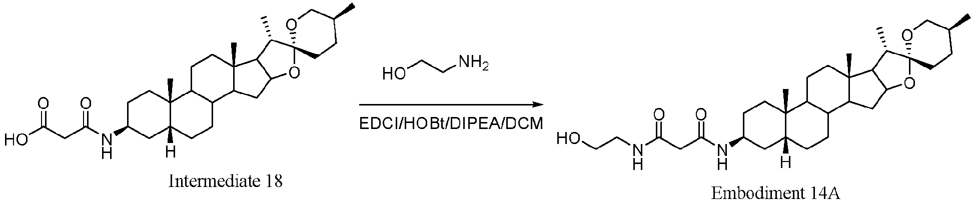

Using intermediate 18 as raw material, a white solid, i.e., Embodiment 14A, was obtained by the same synthesis method as Embodiment 14.

NMR data: ¹H NMR (400 MHz, Chloroform-d) δ 7.21 (s, 1H), 7.07 (d, J = 7.7 Hz, 1H), 4.41 (q, J = 7.5 Hz, 1H), 4.18 (s, 1H), 3.95 (dd, J = 11.0, 2.8 Hz, 1H), 3.74 (q, J = 5.1 Hz, 2H), 3.45 (td, J = 5.6, 4.5 Hz, 2H), 3.30 (d, J = 11.0 Hz, 1H), 3.18 (s, 2H), 2.54 (t, J = 5.4 Hz, 1H), 2.09-1.60 (m, 11H), 1.53-1.11 (m, 14H), 1.08 (d, J = 7.1 Hz, 4H), 1.03-0.96 (m, 5H), 0.76 (s, 3H). Mass spectrum: [M+1]545.5

### Embodiment 14B

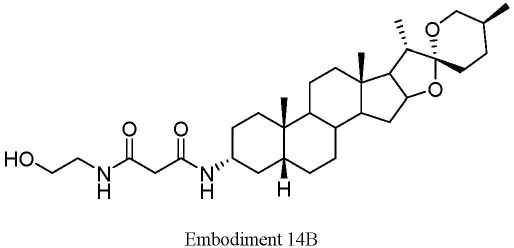

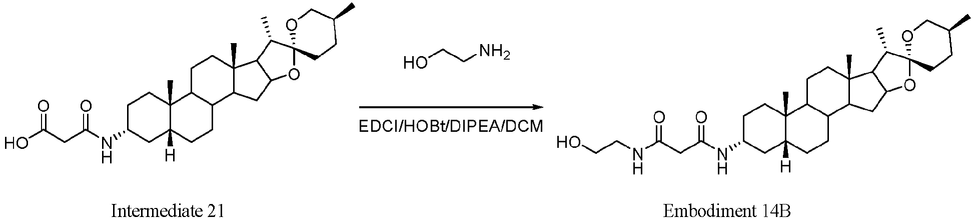

Using intermediate 21 as raw material, a white solid, i.e., Embodiment 14B, was obtained by the same synthesis method as Embodiment 14.

NMR data: ¹H NMR (400 MHz, Chloroform-d) δ 7.42 (s, 1H), 6.67 (s, 1H), 4.41 (td, J = 7.8, 6.2 Hz, 1H), 3.96 (dd, J = 11.0, 2.8 Hz, 1H), 3.84-3.66 (m, 3H), 3.44 (td, J = 5.6, 4.4 Hz, 2H), 3.35-3.22 (m, 1H), 3.16 (s, 2H), 2.81 (s, 1H), 2.00 (m, 2H), 1.92-1.75 (m, 5H), 1.75-1.50 (m, 9H), 1.50-1.31 (m, 7H), 1.31-1.13 (m, 6H), 1.08 (m, 3H), 1.00 (d, J = 6.6 Hz, 3H), 0.95 (s, 3H), 0.75 (s, 3H). Mass spectrum: [M+1]545.5

### Embodiment 15

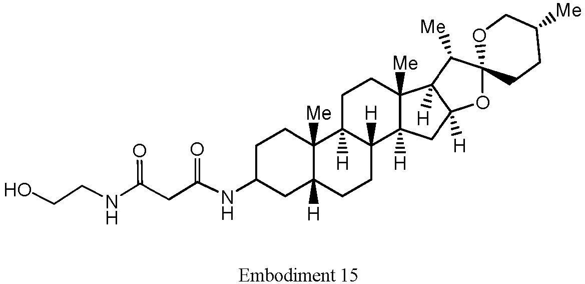

Using iso-sarsasapogenin as raw material, Embodiment 15 was obtained by the same method as Embodiment 14.

NMR data: ¹H NMR (400 MHz, Chloroform-d) δ7.48 (s, 1H), 7.40 (d, J = 7.0 Hz, 1H), 6.82 (s, 1H), 4.47-4.33 (m, 1H), 4.17 (s, 1H), 3.74 (m, 2H), 3.52-3.41 (m, 3H), 3.38 (m, 1H), 3.19 (d, J = 14.7 Hz, 2H), 3.07 (q, J = 7.4 Hz, 2H), 2.08-1.94 (m, 2H), 1.94-1.81 (m, 2H), 1.81-1.66 (m, 4H), 1.60 (dd, J = 16.2, 11.9 Hz, 5H), 1.56-1.41 (m, 5H), 1.31-1.10 (m, 6H), 1.10-1.02 (m, 2H), 1.00 (s, 1H), 0.99-0.92 (m, 4H), 0.79 (dd, J = 6.3, 1.5 Hz, 3H), 0.75 (d, J = 1.8 Hz, 3H). Mass spectrum: [M+1]545.5

### Embodiment 15A

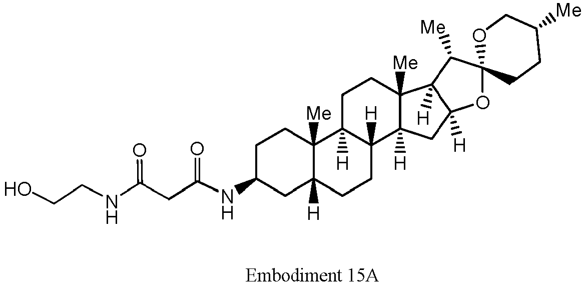

Using iso-sarsasapogenin as raw material, Embodiment 15A was obtained by the same method as Embodiment 14A.

NMR data: ¹H NMR (400 MHz, Chloroform-d) δ 7.40 (s, 1H), 7.30 (d, J = 7.6 Hz, 1H), 4.40 (td, J = 7.7, 5.9 Hz, 1H), 4.17 (s, 1H), 3.73 (d, J = 4.8 Hz, 2H), 3.41 (m, 4H), 3.20 (s, 2H), 2.92 (s, 1H), 1.43 (m, 33H), 0.78 (m, 6H). Mass spectrum: [M+1]545.5

### Embodiment 15B

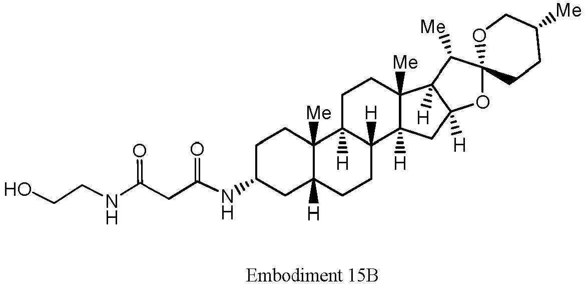

Using iso-sarsasapogenin as raw material, Embodiment 15B was obtained by the same method as Embodiment 14A.

NMR data: ¹H NMR (400 MHz, Chloroform-d) δ 7.51 (s, 1H), 6.83 (d, J = 7.9 Hz, 1H), 4.41 (q, J = 7.4 Hz, 1H), 3.74 (m, 3H), 3.42 (m, 4H), 3.17 (s, 2H), 1.38 (m, 34H), 0.78 (m, 6H). Mass spectrum: [M+1]545.5

### Embodiment 16A

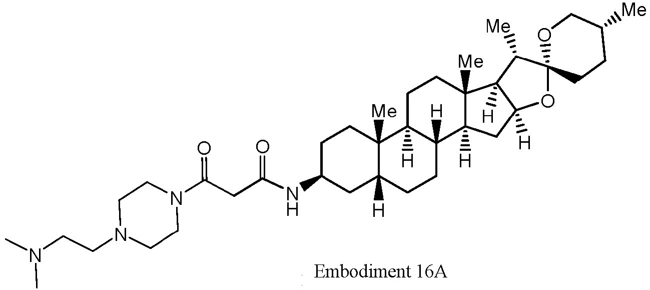

Using iso-sarsasapogenin as raw material, Embodiment 16A was obtained by the same method as Embodiment 13A.

NMR data: ¹H NMR (400 MHz, Chloroform-d) δ 8.02 (d, J = 7.7 Hz, 1H), 4.46-4.33 (m, 1H), 4.18 (d, J = 7.3 Hz, 1H), 3.66 (dq, J = 5.7, 3.1, 2.2 Hz, 2H), 3.57 (q, J = 5.2, 4.6 Hz, 2H), 3.47 (ddd, J = 10.9, 4.4, 2.0 Hz, 1H), 3.37 (t, J = 10.9 Hz, 1H), 3.30 (d, J = 3.4 Hz, 2H), 2.52 (d, J = 2.9 Hz, 4H), 2.48 (t, J = 6.1 Hz, 4H), 2.33 (d, J = 6.0 Hz, 7H), 2.07-1.28 (m, 29H), 1.23-1.02 (m, 10H), 1.00 (s, 3H), 0.96 (d, J = 6.9 Hz, 4H), 0.92-0.81 (m, 5H), 0.79 (d, J = 6.3 Hz, 3H), 0.76 (s, 3H). Mass spectrum: [M+1]641.5.

### Embodiment 16B

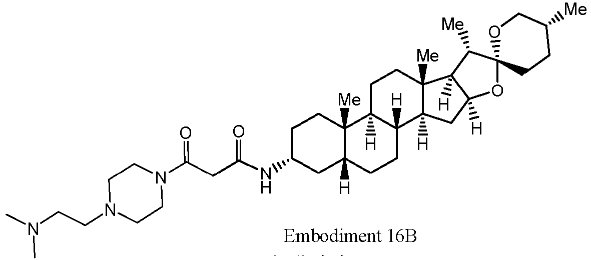

Using iso-sarsasapogenin as raw material, Embodiment 16B was obtained by the same method as Embodiment 13B.

NMR data: ¹H NMR (400 MHz, Chloroform-d) δ 7.40 (d, J = 8.1 Hz, 1H), 4.49-4.29 (m, 1H), 3.88-3.70 (m, 1H), 3.65 (t, J = 5.1 Hz, 2H), 3.60-3.53 (m, 2H), 3.53-3.45 (m, 1H), 3.38 (t, J = 10.9 Hz, 1H), 3.28 (s, 2H), 2.55-2.39 (m, 8H), 2.26 (s, 6H), 2.02-1.01 (m, 38H), 1.01-0.90 (m, 6H), 0.79 (d, J = 6.3 Hz, 3H), 0.75 (s, 3H). Mass spectrum: [M+1]641.5.

### Embodiment 17A

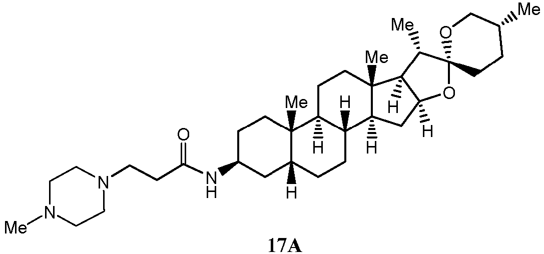

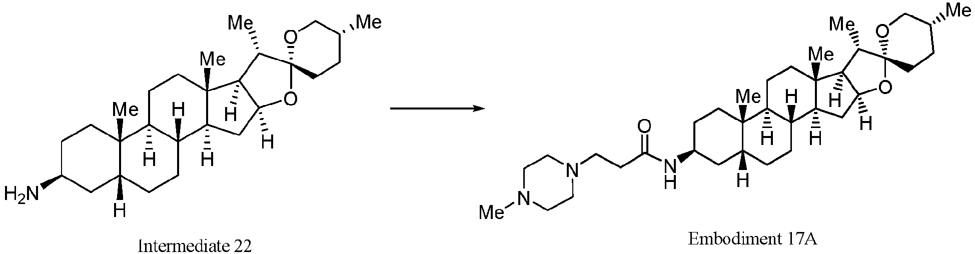

Using iso-sarsasapogenin as raw material, the intermediate 22 was obtained by the same preparation steps and the same conditions as the intermediate 7; and using intermediate 22 as raw material, Embodiment 17A was obtained by the same preparation method and conditions as described in the fifth step in Embodiment 1A. Mass spectrum: [M+1]570.

### Embodiment 17B

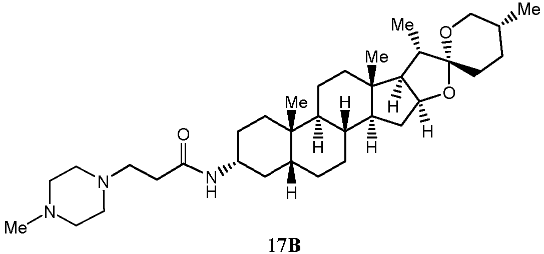

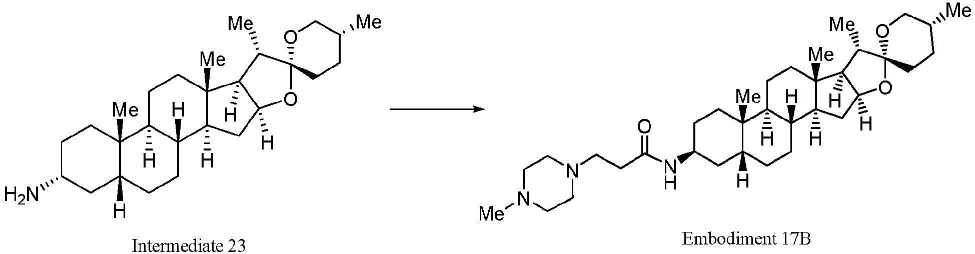

Using iso-sarsasapogenin as raw material, the intermediate 23 was obtained by the same preparation steps and the same conditions as the intermediate 9; and using intermediate 23 as raw material, Embodiment 17B was obtained by the same preparation steps and conditions as described in Embodiment 1B.

Mass spectrum: [M+1]570.

### Embodiment 18A

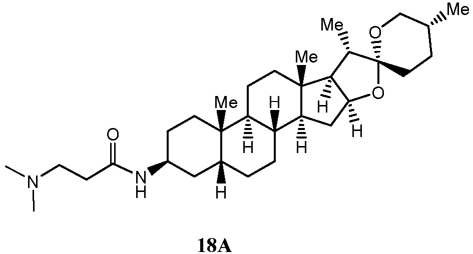

Using iso-sarsasapogenin as raw material, Embodiment 18A was obtained by the same preparation steps and conditions as described in Embodiment 4A.

NMR data: ¹H NMR (400 MHz, Chloroform-d) δ 9.14 (s, 1H), 4.45-4.34 (m, 1H), 4.19 (d, J = 8.3 Hz, 1H), 3.52-3.42 (m, 2H), 3.37 (t, J = 10.9 Hz, 1H), 2.56 (t, J = 5.9 Hz, 2H), 2.36 (dd, J = 6.5, 5.2 Hz, 2H), 2.31 (s, 6H), 2.09-1.66 (m, 10H), 1.66-1.48 (m, 8H), 1.48-1.35 (m, 7H), 1.34-1.00 (m, 14H), 0.99-0.93 (m, 7H), 0.92-0.82 (m, 4H), 0.79 (d, J = 6.3 Hz, 3H), 0.76 (s, 3H). Mass spectrum: [M+1]515.

### Embodiment 18B

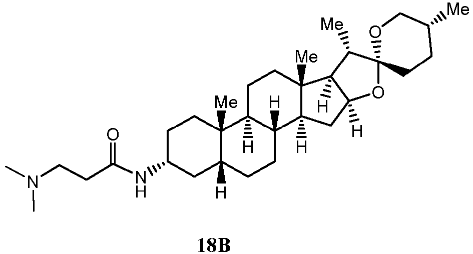

Using iso-sarsasapogenin as raw material, Embodiment 18B was obtained by the same preparation steps and conditions as described in Embodiment 4B.

NMR data: ¹H NMR (400 MHz, Chloroform-d) δ 6.46 (s, 1H), 4.46-4.34 (m, 1H), 3.81-3.60 (m, 1H), 3.47 (d, J = 4.6 Hz, 1H), 3.38 (t, J = 10.9 Hz, 1H), 3.26 (t, J = 6.6 Hz, 2H), 2.80 (t, J = 6.6 Hz, 2H), 2.72 (s, 6H), 2.05-1.53 (m, 16H), 1.46 (d, J = 4.3 Hz, 2H), 1.37-1.33 (m, 2H), 1.19-1.00 (m, 9H), 0.99-0.91 (m, 7H), 0.91-0.81 (m, 14H), 0.79 (d, J = 6.3 Hz, 3H), 0.75 (s, 3H). Mass spectrum: [M+1]515.

### Embodiment 19A

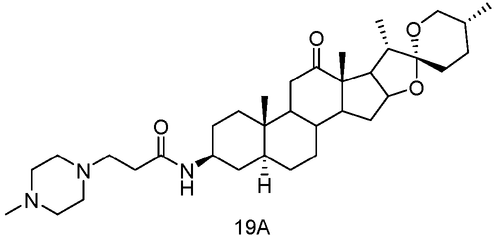

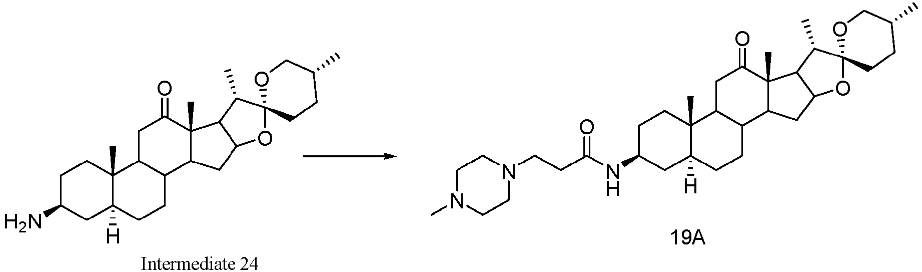

Using hecogenin as raw material, the intermediate 24 was obtained by the same preparation steps and the same conditions as the intermediate 7; and using intermediate 24 as raw material, Embodiment 19A was obtained by the same preparation steps and conditions as described in the fifth step in Embodiment 1A.

NMR data: ¹H NMR (400 MHz, Chloroform-d) δ 4.33 (t, J = 7.4 Hz, 1H), 3.79-3.61 (m, 3H), 3.50 (s, 2H), 3.35 (t, J = 10.9 Hz, 2H), 3.10 (q, J = 7.4 Hz, 2H), 2.99 (s, 7H), 2.70 (s, 1H), 2.62 -2.45 (m, 5H), 2.38 (t, J = 13.7 Hz, 2H), 2.22 (dd, J = 14.3, 5.0 Hz, 2H), 2.11 (q, J = 6.5, 6.0 Hz, 2H), 1.90 (d, J = 9.0 Hz, 3H), 1.75 (dt, J = 12.9, 6.5 Hz, 5H), 1.69-1.50 (m, 11H), 1.44 (dd, J = 11.3, 7.9 Hz, 6H), 1.40-1.09 (m, 10H), 1.09-1.01 (m, 5H), 0.97 (dd, J = 12.9, 7.9 Hz, 2H), 0.89 (s, 4H), 0.79 (d, J = 6.3 Hz, 3H). Mass spectrum: [M+1]529.4.

### Embodiment 20A

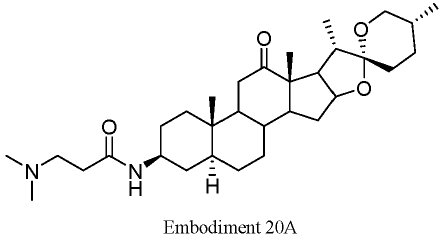

Using hecogenin as raw material, Embodiment 20A was obtained by the same preparation steps and conditions as described in Embodiment 4A.

NMR data: ¹H NMR (400 MHz, Chloroform-d) δ 6.99 (t, J = 6.4 Hz, 1H), 4.33 (dd, J = 8.5, 5.8 Hz, 1H), 3.81-3.57 (m,1H), 3.5 7-3.43 (m, 1H), 3.35 (t, J = 11.0 Hz, 1H), 3.15 (t, J = 6.5 Hz, 2H), 2.71 (t, J = 6.5 Hz, 2H), 2.66 (s, 6H), 2.51 (dd, J = 8.8, 6.7 Hz, 1H), 2.38 (t, J = 13.7 Hz, 1H), 2.21 (dd, J = 14.3, 5.0 Hz, 1H), 2.11 (t, J = 7.1 Hz, 1H), 1.89 (dd, J = 11.1, 7.3 Hz, 1H), 1.83-1.71 (m, 3H), 1.71-1.52 (m, 6H), 1.52-1.38 (m, 3H), 1.38-1.09 (m, 7H), 1.09-0.99 (m, 6H), 0.89 (s, 3H), 0.79 (d, J = 6.3 Hz, 3H). Mass spectrum: [M+1]529.4.

### Embodiment 21

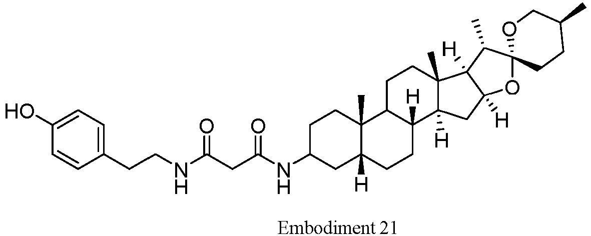

Using 4-hydroxyphenethylamine as raw material, Embodiment 21 was obtained by the same preparation steps and conditions as described in Embodiment 13.

NMR data: ¹H NMR(CDCl3, 400MHz, ppm): 0.76 (s, 3H), 0.80-2.10 (m, 36H), 2.65-2.80 (m, 2H), 3.15-3.60 (m, 5H), 3.70-4.55 (m, 4H), 6.70-7.40 (m, 5H); mass spectrum: [M+1]621.4.

### Embodiment 22

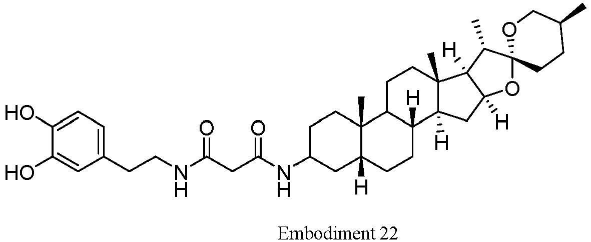

Using 3,4-dihydroxyphenethylamine as raw material, Embodiment 22 was obtained by the same preparation steps and conditions as described in Embodiment 13.

NMR data: ¹H NMR(CDCl3, 400MHz, ppm): 0.75 (s, 3H), 0.80-2.10 (m, 36H), 2.45-2.55 (m, 2H), 3.25-3.70 (m, 6H), 3.84-4.55 (m, 3H), 6.50-6.90 (m, 4H); mass spectrum: [M+1]637.4.

### Embodiment 23

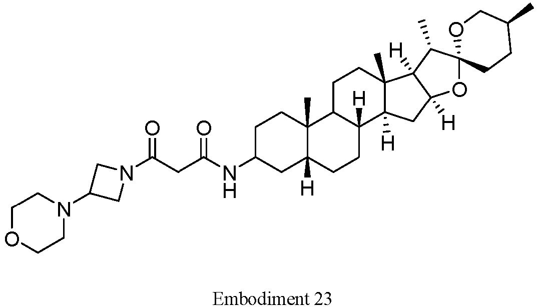

A preparation process was specifically described as follows:

### Step 1: intermediate 25

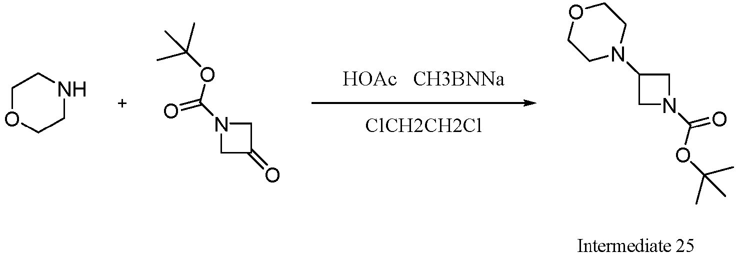

Under the protection of argon, 0.2 g of morpholine, 0.4 g of 1-Boc-3-azetidinone, 0.7 mL of acetic acid, 0.3 g of sodium cyanoborohydride were dissolved in 20 mL of dichloromethane, reacted at room temperature for 2 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 70%.

### Step 2: intermediate 26

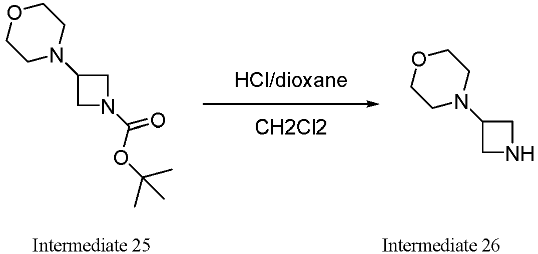

Under the protection of argon, 0.2 g of intermediate 25 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 3: synthesis of Embodiment 23

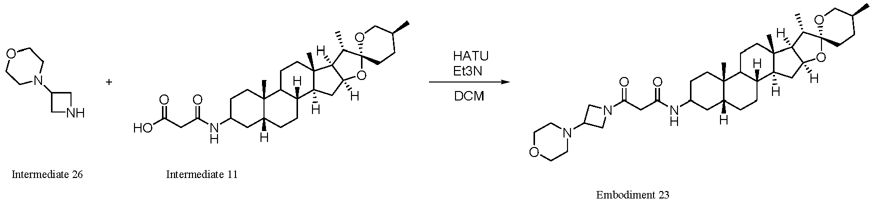

Under the protection of argon, 0.1 g of intermediate 26, 0.43 g of intermediate 11, 0.40 g of HATU, and 0.22 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, CD3OD) δ 4.5-4.65 (m, 2H), 4.26-4.45 (m, 2H), 4.05-4.25 (m, 3H), 3.85-3.99 (m, 2H), 3.72-3.85 (m, 1H), 3.60-3.70 (m, 1H)., 3.62-3.70 (m, 1H), 3.40-3.60 (m, 2H), 3.06-3.28 (m, 3H), 2.71 (s, 4H), 1.32-2.10 (m, 27H), 1.08 (dd, 3H), 0.99 (m, 6H), 0.75 (s, 3H).

Mass spectrum: [M+1]626.5.

### Embodiment 24

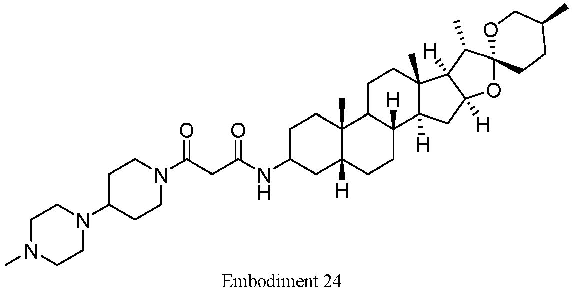

A preparation process was specifically described as follows:

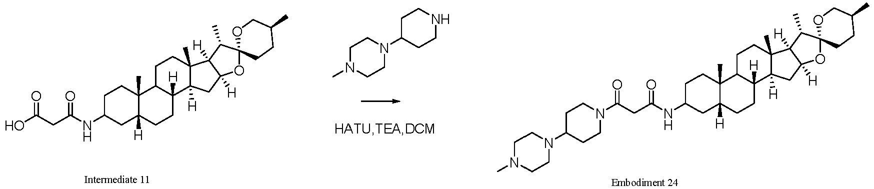

Under the protection of argon, 0.2 g of intermediate 11, 0.08 g of 1-methyl-4-(piperidin-4-yl)piperazine, 0.227 g of HATU, and 0.101 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column (DCM:MeOH=20:1), subjected to a climbing board test (DCM:EA=10:1), and color-developed with vanillin. The yield was 45%.

NMR data: ¹H NMR (400 MHz, CDCl3) δ 8.13 (t, 0.5H), 7.44 (dd, 0.5H), 4.58 (d, 1H), 4.41 (p, 1H), 3.96 (m, 2H), 3.29 (d, 3H), 3.06 (dd, 1H), 2.43 (m, 15H), 1.37 (m, 39H), 0.75 (d, 3H). Mass spectrum: [M+1]667.5.

### Embodiment 24A, Embodiments 24B

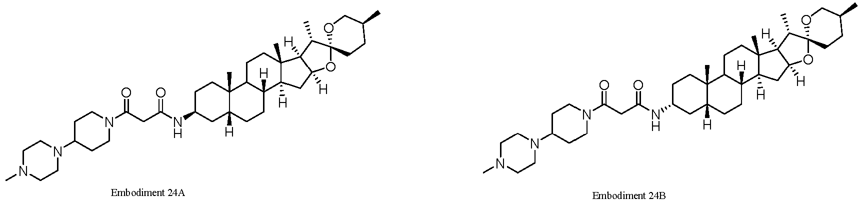

Using intermediate 18 as raw material, Embodiment 24A was obtained by the same synthesis method as Embodiment 24. Using intermediate 21 as raw material, Embodiment 24B was obtained by the same synthesis method.

NMR data of Embodiment 24A: ¹H NMR (400 MHz, CDCl3) δ 8.12 (t, 1H), 4.48 (m, 2H), 4.00 (m, 3H), 3.28 (m, 5H), 2.43 (m, 13H), 1.82 (m, 15H), 1.12 (m, 27H); mass spectrum: [M+1]667.5. NMR data of Embodiment 24B: ¹H NMR (400 MHz, CDCl3) δ 7.42 (dd, 1H), 4.50 (m, 2H), 3.94 (m, 2H), 3.31 (d, 3H), 3.06 (m, 2H), 2.56 (m, 11H), 2.31 (s, 3H), 1.40 (m, 42H); mass spectrum: [M+1]667.5.

### Embodiment 25

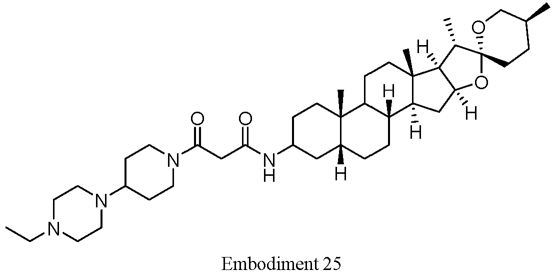

A preparation process was specifically described as follows:

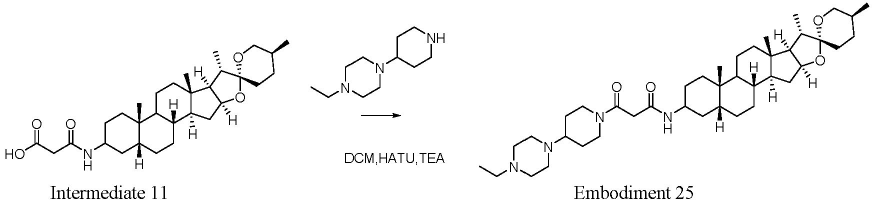

The preparation method was the same as that of Embodiment 24.

NMR data: ¹H NMR (400 MHz, CDCl3) δ 7.75 (dt, 1H), 4.46 (m, 3H), 3.94 (m, 3H), 3.39 (m, 6H), 3.06 (m, 2H), 2.65 (m, 14H), 1.28 (m, 40H). Mass spectrum: [M+1]681.6.

### Embodiment 25A, Embodiments 25B

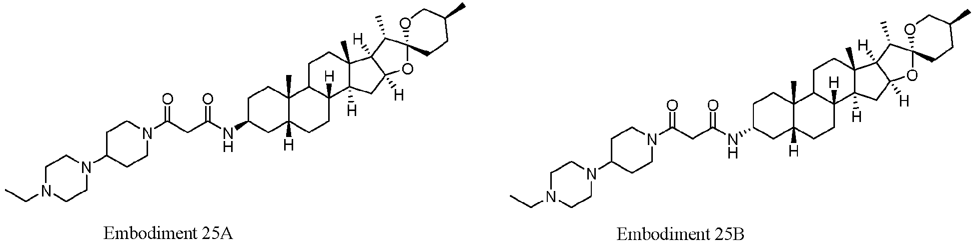

Using intermediate 18 and 1-methyl-4-(piperidin-4-yl) piperazine as raw materials, Embodiment 25A may be obtained by the same synthesis method as Embodiment 24. Using intermediate 21 and 1-methyl-4-(piperidin-4-yl) piperazine as raw materials, Embodiment 25B was obtained by the same method.

NMR data of Embodiment 25A: ¹H NMR (400 MHz, CDCl3) δ 8.15 (t, 1H), 4.61 (d, 1H), 4.41 (m, 1H), 4.19 (s, 1H), 3.96 (m, 2H), 3.31 (d, 3H), 3.06 (t, 1H), 2.51 (m, 10H), 1.34 (m, 48H); mass spectrum: [M+1]681.6.

NMR data of Embodiment 25B: ¹H NMR (400 MHz, CDCl3) δ 7.38 (d, 1H), 4.42 (q, 1H), 3.96 (dd, 1H), 3.62 (m, 5H), 3.31 (d, 3H), 2.90 (m, 3H), 2.54 (m, 4H), 2.37 (s, 3H), 1.38 (m, 45H). Mass spectrum: [M+1]681.6.

### Embodiment 26

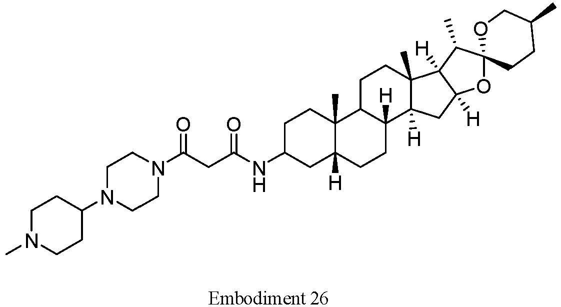

A preparation process was specifically described as follows:

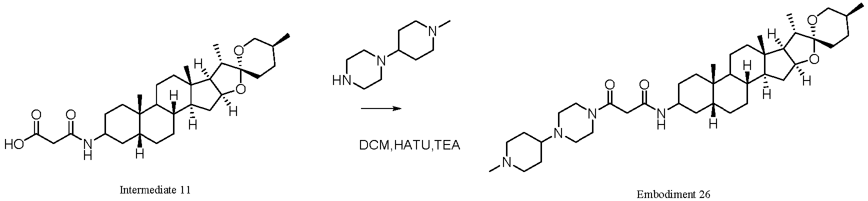

The preparation method was the same as that of Embodiment 24.

NMR data: ¹H NMR (400 MHz, CDCl3) δ 7.74 (dd, 1H), 4.41 (p, 1H), 3.96 (dt, 1H), 3.59 (m, 6H), 3.29 (d, 2H), 2.91 (m, 2H), 2.54 (q, 4H), 2.26 (s, 4H), 1.41 (m, 45H). Mass spectrum: [M+1]667.6.

### Embodiment 26A, Embodiments 26B

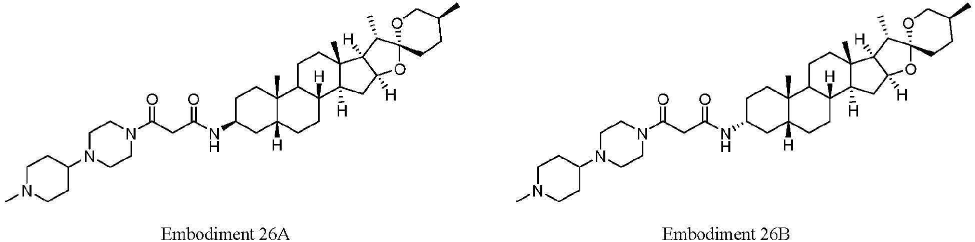

Using intermediate 18 and 1-(1-methyl-4-piperidinyl)piperazine as raw materials, Embodiment 26A may be obtained by the same synthesis method as Embodiment 24. Using intermediate 21 and 1-(1-methyl-4-piperidinyl)piperazine as raw materials, Embodiment 26B was obtained by the same method.

NMR data of Embodiment 26A: ¹H NMR (400 MHz, CDCl3) δ 8.09 (d, 1H), 4.40 (q, 1H), 4.16 (d, 1H), 3.95 (dd, 1H), 3.64 (t, 2H), 3.49 (s, 5H), 3.29 (s, 2H), 2.91 (d, 2H), 2.54 (q, 4H), 2.27 (s, 3H), 1.42 (m, 44H). Mass spectrum: [M+1]667.6.

NMR data of Embodiment 26B: ¹H NMR (400 MHz, CDCl3) δ 7.38 (d, 1H), 4.42 (q, 1H), 3.96 (dd, 1H), 3.62 (m, 5H), 3.31 (d, 3H), 2.90 (m, 3H), 2.54 (m, 4H), 2.37 (s, 3H), 1.38 (m, 45H). Mass spectrum: [M+1]667.6.

### Embodiment 27

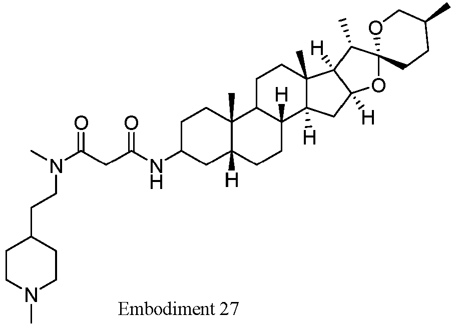

A preparation process was specifically described as follows:

### Step 1: intermediate 27

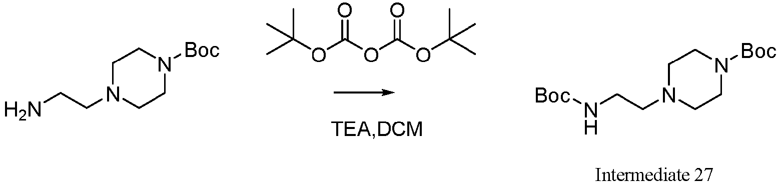

0.5 g of tert-butyl 4-(2-aminoethyl)piperazine-1-carboxylate and 0.714 g of tert-butyl dicarbonate were dissolved in 20 mL of dry DCM, reacted at room temperature for 12 h, and the reaction was detected. After the reaction was completed, the reactant was spin-dried to remove the solvent, extracted with water/dichloromethane, purified by silica gel column (DCM:EA=30:1), subjected to spot plate detection (DCM:MeOH=10:1), and color-developed with vanillin. The yield was 65%.

### Step 2: intermediate 28

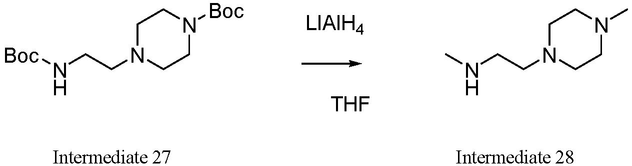

15 mL of THF was added to 0.2 g of intermediate 27 and 0.07 g of LiAlH₄, heated to a reflux state, and reacted for 3 h. The reaction was detected. After the reaction was completed, the reactant was cooled to room temperature, and spin-dried to remove the solvent and obtain a product, which was directly put into the next reaction and color-developed with vanillin. The yield was 56%.

### Step 3: synthesis of Embodiment 27

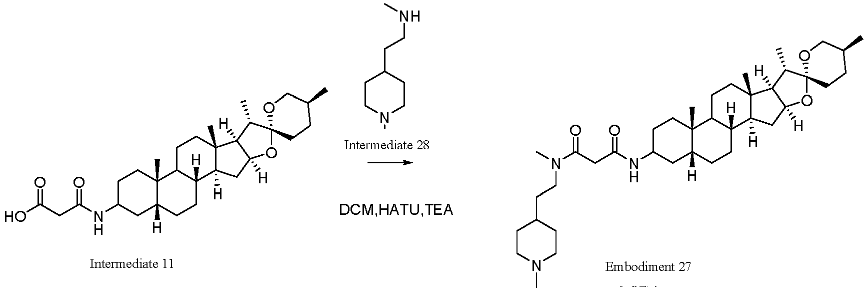

Under the protection of argon, 0.037 g of intermediate 28, 0.12 g of intermediate 11, 0.136 g of HATU, and 0.061 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column (DCM:MeOH=20:1), subjected to a climbing board test (DCM:EA=10:1), and color-developed with vanillin. The yield was 55%.

NMR data: ¹H NMR (400 MHz, CDCl3) δ 7.97 (m, 1H), 4.11 (m, 3H), 3.48 (dt, 2H), 3.30 (m, 3H), 3.02 (d, 3H), 2.44 (m, 13H), 1.23 (m, 40H). Mass spectrum: [M+1]641.6.

### Embodiment 27A, Embodiments 27B

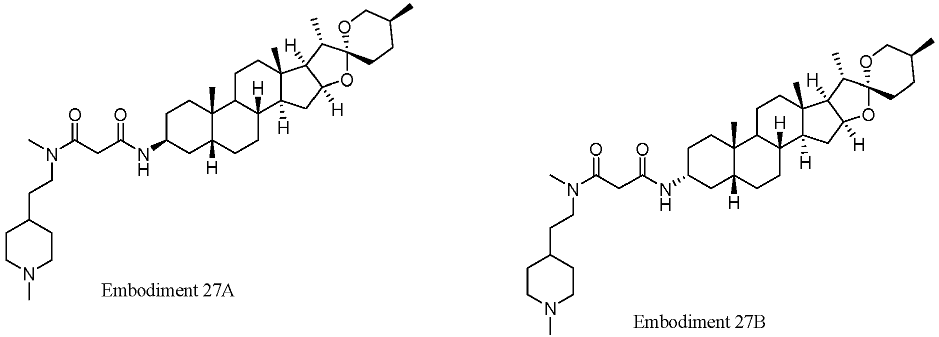

Their synthesis was the same as in Embodiment 27, to be specific:
Using intermediate 18 and N-methyl-2-(1-methylpiperidin-4-yl)ethane-1-amine as raw materials, Embodiment 27A may be obtained by the same synthesis method as Embodiment 27. Using intermediate 21 and N-methyl-2-(1-methylpiperidin-4-yl)ethane-1-amine as raw materials, Embodiment 27B was obtained by the same method.

NMR data of Embodiment 27A: ¹H NMR (400 MHz, CDCl3) δ 8.45 (dd, 1H), 4.41-4.45 (m, 1H), 3.94-3.98 (m, 1H), 3.75-3.78 (m, 1H), 3.51 (t, 1H), 3.45 (t, 1H), 3.28-3.32 (m, 3H), 3.10 (m, 3H), 2.51 (m, 10H), 2.26 (m, 3H), 1.32-2.10 (m, 27H), 1.08 (m, 3H), 0.99 (m, 6H), 0.75 (s, 3H). Mass spectrum: [M+1]641.6.

NMR data of Embodiment 27B: ¹H NMR (400 MHz, CDCl3) δ 8.7.78 (dd, 1H), 4.41-4.45 (m, 1H), 3.94-3.98 (m, 1H), 3.75-3.78 (m, 1H), 3.51 (t, 1H), 3.45 (t, 1H), 3.28-3.32 (m, 3H), 3.10 (m, 3H), 2.51 (m, 10H), 2.26 (m, 3H), 1.32-2.10 (m, 27H), 1.08 (m, 3H), 0.99 (m, 6H), 0.75 (s, 3H). Mass spectrum: [M+1]641.6.

### Embodiment 28

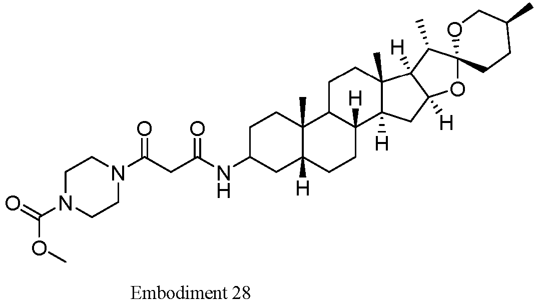

A preparation process was specifically described as follows:

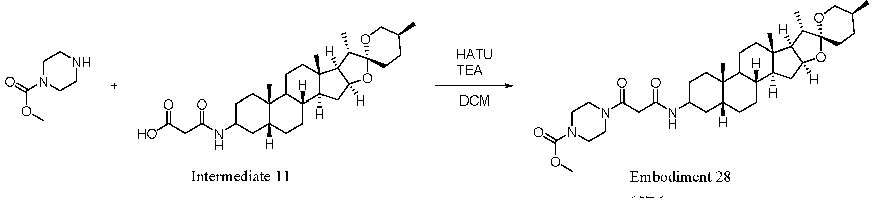

Under the protection of argon, 0.1 g of methyl piperazine-1-carboxylate, 0.42 g of intermediate 11, 0.40 g of HATU, and 0.21 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 65%.

NMR data: ¹H NMR (CDCl3-d6, 400 MHz): 7.12 (d, 1H), 4.35-4.46 (m, 1H), 3.90-4.01 (m, 1H), 3.70-3.85 (m, 1H), 3.73 (s, 3H), 3.60-3.68 (m, 2H), 3.45-3.58 (m, 6H), 3.28-3.35 (m, 1H), 3.29 (s, 2H), 1.58-2.10 (m, 11H), 1.15-1.50 (m, 16H), 1.08 (d, 3H), 0.99 (d, 3H), 0.95 (s, 3H), 0.75 (s, 3H). Mass spectrum: [M+1]628.5.

### Embodiment 29

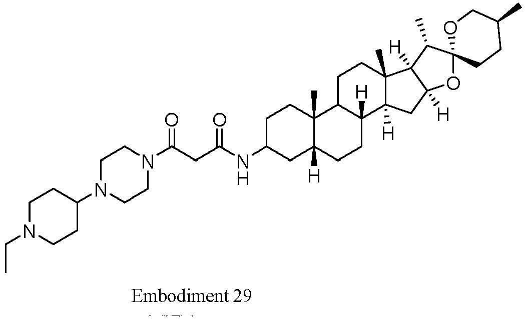

A preparation process was specifically described as follows:

### Step 1: intermediate 29

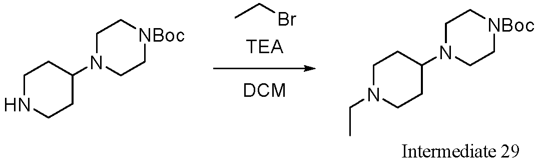

Under the protection of argon, 0.1 g of 1-Boc-4-(piperidin-4-yl)-piperazine, 0.05 g of bromoethane, and 0.08 g of TEA were dissolved in 30 mL of dichloromethane, reacted at room temperature for 12 h, and the reaction was detected. The reactant was spin-dried to remove the solvent, then directly put into the next reaction, and color-developed with phosphomolybdic acid. The yield was 80%.

### Step 2: intermediate 30

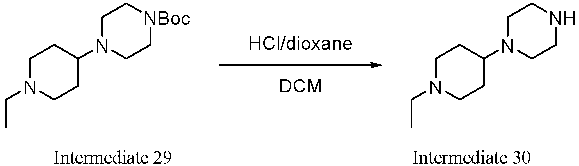

Under the protection of argon, 0.1 g of intermediate 29 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 3: synthesis of Embodiment 29

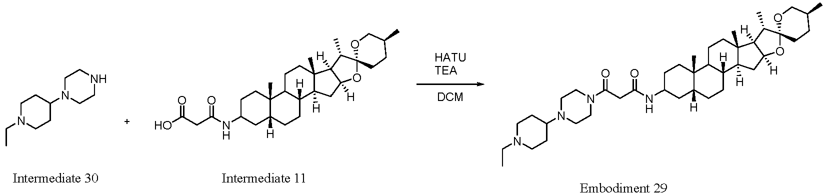

Under the protection of argon, 0.05 g of intermediate 30, 0.15 g of intermediate 11, 0.15 g of HATU, and 0.08 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, CDCl3) δ 8.7.38 (dd, 1H), 4.41-4.45 (m, 1H), 3.94-3.98 (m, 1H), 3.75-3.78 (m, 1H), 3.65 (t, 2H), 3.56 (t, 2H). 3.28-3.32 (m, 3H), 3.15 (m, 2H), 2.51 (m, 6H), 2.30 (m, 1H), 2.10 (m, 6H), 1.32-2.10 (m, 27H), 1.08 (m, 3H), 0.99 (m, 9H), 0.75 (s, 3H). Mass spectrum: [M+1]681.5.

### Embodiment 29A, Embodiments 29B

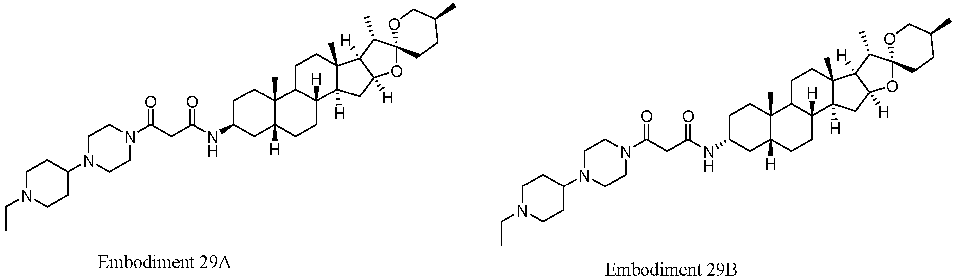

The synthesis process was the same as in Embodiment 29; using intermediate 18 and 1-(1-ethylpiperidin-4-yl)piperazine as raw materials, Embodiment 29A may be obtained by the same synthesis method as Embodiment 29. Using intermediate 21 and 1-(1-ethylpiperidin-4-yl)piperazine as raw material, Embodiment 29B was obtained by the same method.

NMR data of Embodiment 29A: ¹H NMR (400 MHz, CDCl3) δ 8.8.05 (dd, 1H), 4.41-4.45 (m, 1H), 3.94-3.98 (m, 1H), 3.75-3.78 (m, 1H), 3.65 (t, 2H), 3.56 (t, 2H). 3.28-3.32 (m, 3H), 3.15 (m, 2H), 2.51 (m, 6H), 2.30 (m, 1H), 2.10 (m, 6H), 1.32-2.10 (m, 27H), 1.08 (m, 3H), 0.99 (m, 9H), 0.75 (s, 3H). Mass spectrum: [M+1]681.6.

NMR data of Embodiment 29B: ¹H NMR (400 MHz, CDCl3) δ 8.7.38 (dd, 1H), 4.41-4.45 (m, 1H), 3.94-3.98 (m, 1H), 3.75-3.78 (m, 1H), 3.65 (t, 2H), 3.56 (t, 2H). 3.28-3.32 (m, 3H), 3.15 (m, 2H), 2.51 (m, 6H), 2.30 (m, 1H), 2.10 (m, 6H), 1.32-2.10 (m, 27H), 1.08 (m, 3H), 0.99 (m, 9H), 0.75 (s, 3H). Mass spectrum: [M+1]681.5.

### Embodiment 30

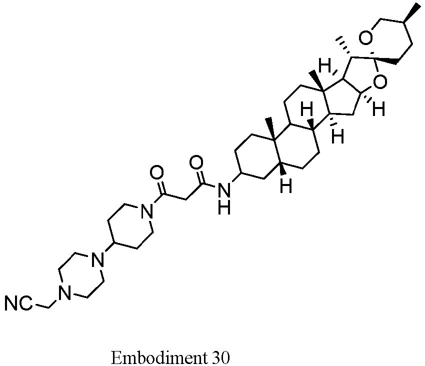

A preparation process was specifically described as follows:

### Step 1: intermediate 31

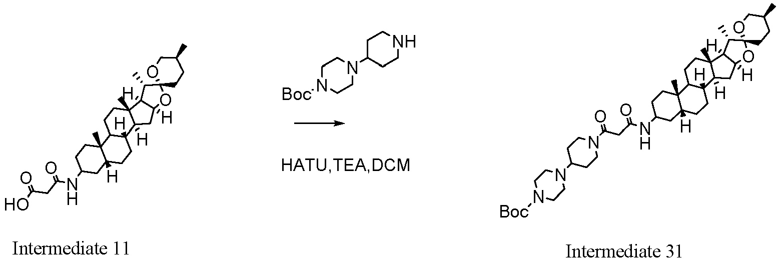

Under the protection of argon, 0.4 g of intermediate 11, 0.258 g of tert-butyl 4-(piperidin-4-yl)piperidine-1-carboxylate, 0.455 g of HATU, and 0.202 of TEA were dissolved in 15 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column (DCM:MeOH=20:1), subjected to a climbing board test (DCM:EA=10:1), and color-developed with vanillin. The yield was 75%.

### Step 2: intermediate 32

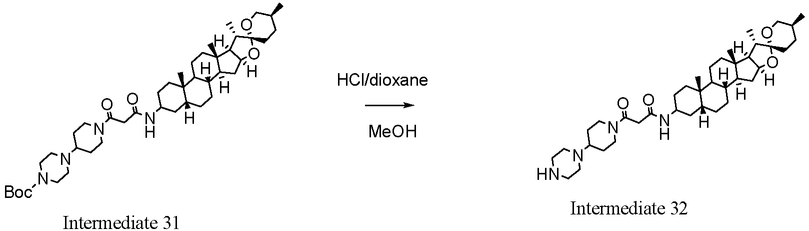

10 mL of MeOH was added to 0.2 g of intermediate 31, then added with 10 mL of 4M HCl/dioxane solution and reacted at room temperature for 3 h. The reactant was spin-dried to remove the solvent, then directly put into the next reaction, and color-developed with vanillin. The yield was 80%.

### Step 3: synthesis of Embodiment 30

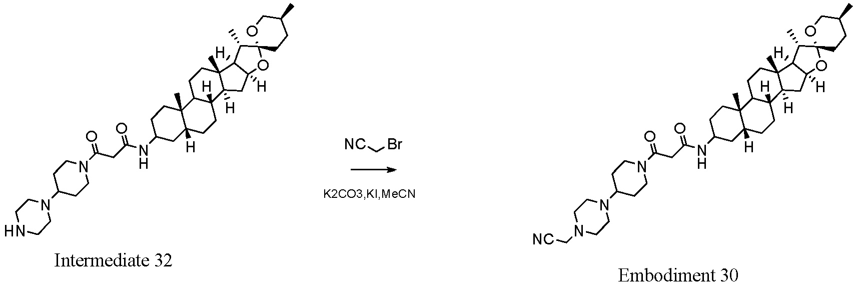

Under the protection of argon, 0.08 g of intermediate 32, 0.03 g bromoacetonitrile, 0.034 g of potassium carbonate and 0.02 g potassium iodide were dissolved in 10 mL of dichloromethane, heated to 50°C, reacted for 12 h, and the reaction was detected. The reactant was extracted with water/dichloromethane, washed with a saturated saline solution, dried with anhydrous sodium sulfate, spin-dried to remove the solvent, purified by silica gel column (DCM:MeOH=20:1), subjected to a climbing board test (DCM:EA=10:1), and color-developed with vanillin. The yield was 65%.

NMR data: ¹H NMR (400 MHz, CDCl3) δ 7.37 (m, 1H), 4.58 (d, 1H), 4.42 (q, 1H), 3.98 (d, 1H), 3.77 (m, 1H), 3.50 (s, 2H), 3.30 (m, 2H), 3.07 (t, 1H), 2.62 (s, 12H), 1.12 (m, 43H). Mass spectrum: [M+1]693.6.

### Embodiment 31

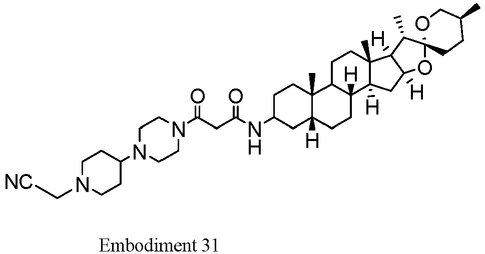

A preparation process was specifically described as follows:

### Step 1: intermediate 33

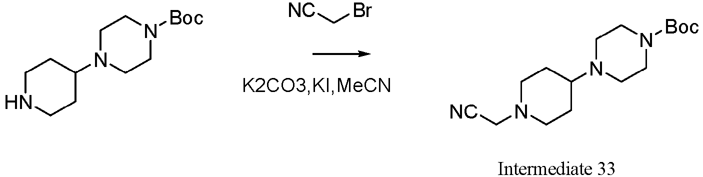

0.1 g of tert-butyl 4-(piperidin-4-yl)piperidine-1-carboxylate and 0.089 g of bromoacetonitrile were dissolved in 10 mL of dry MeCN, then added with 0.103 g of potassium carbonate and 0.062 g of potassium iodide, heated to 50°C, reacted for 12 h, and the reaction was detected. After the reaction was completed, the reactant was extracted with water/dichloromethane, purified by silica gel column (DCM:MeOH=30:1), subjected to spot plate detection (DCM:MeOH=10:1), and color-developed with vanillin. The yield was 55%.

### Step 2: intermediate 34

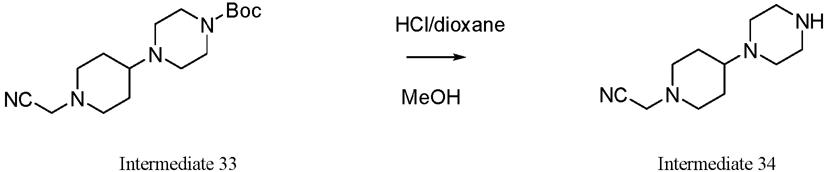

10 mL of MeOH was added to 0.2 g of intermediate 33, then added with 10 mL of 4M HCl/dioxane solution and reacted at room temperature for 3 h. The reactant was spin-dried to remove the solvent, then directly put into the next reaction, and color-developed with vanillin. The yield was 80%.

### Step 3: synthesis of Embodiment 31

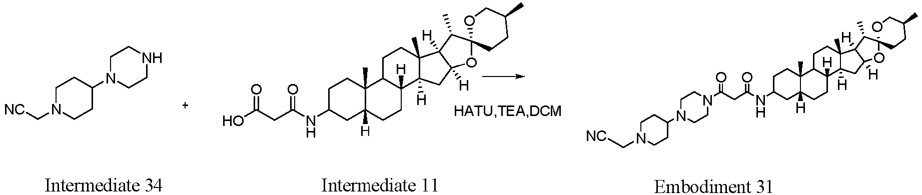

Under the protection of argon, 0.046 g of intermediate 34, 0.1 g of intermediate 11, 0.114 g of HATU, and 0.061 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column (DCM:MeOH=20:1), subjected to a climbing board test (DCM:EA=10:1), and color-developed with vanillin. The yield was 40%.

NMR data: ¹H NMR (400 MHz, CDCl3) δ 7.35 (m, 1H), 4.42 (m, 1H), 3.95 (m, 1H), 3.41 (m, 10H), 2.59 (m, 6H), 2.33 (ddd, 3H), 1.29 (m, 43H). Mass spectrum: [M+1]692.6.

### Embodiment 32

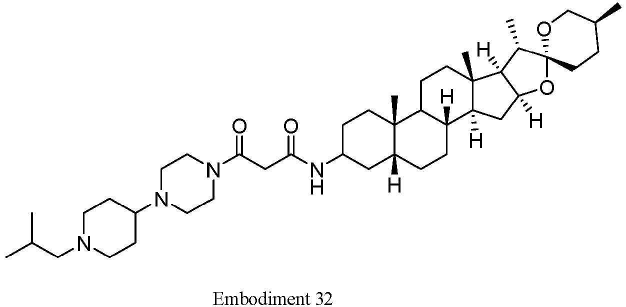

A preparation process was specifically described as follows:

### Step 1: intermediate 35

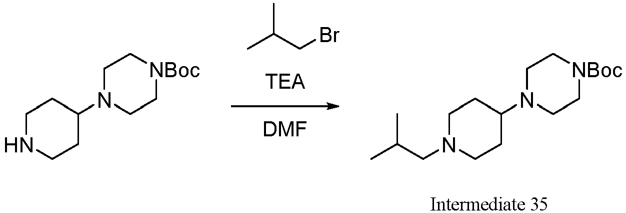

Under the protection of argon, 0.1 g of 1-Boc-4-(piperidin-4-yl)-piperazine, 0.06 g of bromoisobutane, and 0.08 g of TEA were dissolved in 30 mL of N,N-dimethylformamide, heated to 50°C, reacted at room temperature for 12 h, and the reaction was detected. The reactant was spin-dried to remove the solvent, then directly put into the next reaction, and color-developed with phosphomolybdic acid. The yield was 70%.

### Step 2: intermediate 36

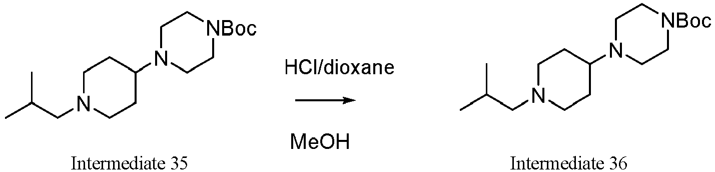

Under the protection of argon, 0.1 g of intermediate 35 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 3: synthesis of Embodiment 36

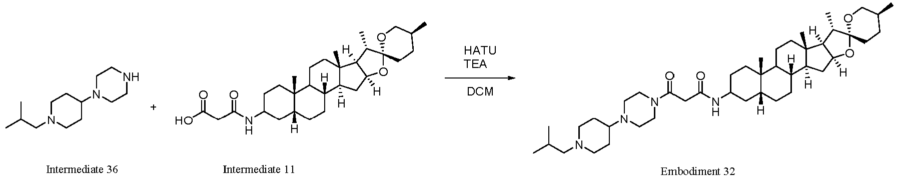

Under the protection of argon, 0.05 g of intermediate 36, 0.11 g of intermediate 11, 0.13 g of HATU, and 0.07 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, CDCl3) δ 7.32 (dd, 1H), 4.41-4.45 (m, 1H), 3.94-3.98 (m, 1H), 3.75-3.78 (m, 1H), 3.65 (t, 2H), 3.56 (t, 2H)., 3.28-3.32 (m, 3H), 2.5 (m, 5H), 2.3 (m, 2H), 1.32-2.10 (m, 27H), 1.19 (m, 9H), 1.08 (dd, 3H), 0.99 (m, 12H), 0.75 (s, 3H). Mass spectrum: [M+1]709.7.

### Embodiment 32A, Embodiments 32B

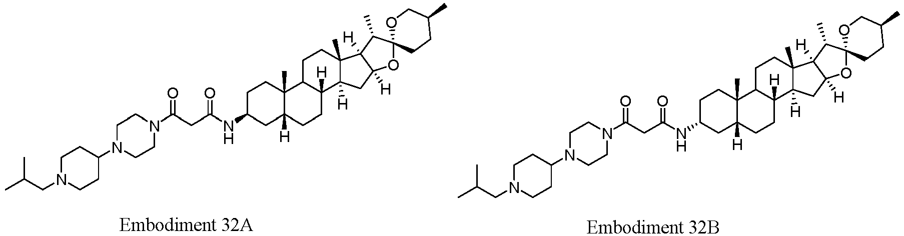

The synthesis method was the same as Embodiment 32.

Using intermediate 18 and 1-(1-isobutylpiperidin-4-yl)piperazine as raw materials, Embodiment 32A may be obtained by the same synthesis method as Embodiment 32. Using intermediate 21 and 1-(1-isobutylpiperidin-4-yl)piperazine as raw materials, Embodiment 32B was obtained by the same method.

NMR data of Embodiment 32A: ¹H NMR (400 MHz, CDCl3) δ 7.32 (dd, 1H), 4.41-4.45 (m, 1H), 3.94-3.98 (m, 1H), 3.75-3.77 (m, 1H), 3.65 (t, 2H), 3.56 (t, 2H)., 3.28-3.32 (m, 3H), 2.52 (m, 5H), 2.32 (m, 2H), 1.32-2.09 (m, 27H), 1.19 (m, 9H), 1.09 (dd, 3H), 0.99 (m, 12H), 0.75 (s, 3H). Mass spectrum: [M+1]709.7.

NMR data of Embodiment 32B: ¹H NMR (400 MHz, CDCl3) δ 7.32 (dd, 1H), 4.41-4.45 (m, 1H), 3.94-3.98 (m, 1H), 3.75-3.78 (m, 1H), 3.65 (t, 2H), 3.56 (t, 2H)., 3.28-3.32 (m, 3H), 2.51 (m, 5H), 2.31 (m, 2H), 1.32-2.10 (m, 27H), 1.19 (m, 9H), 1.08 (dd, 3H), 0.99 (m, 12H), 0.76 (s, 3H). Mass spectrum: [M+1]709.7.

### Embodiment 33

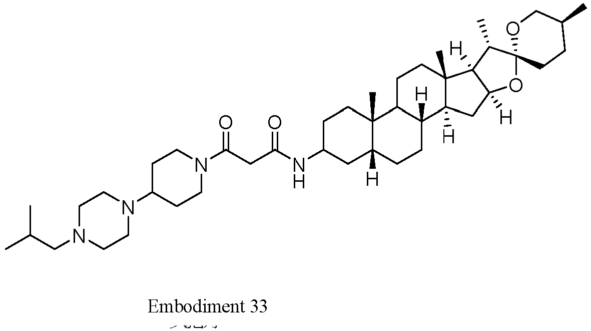

A preparation process was specifically described as follows:

### Step 1: intermediate 37

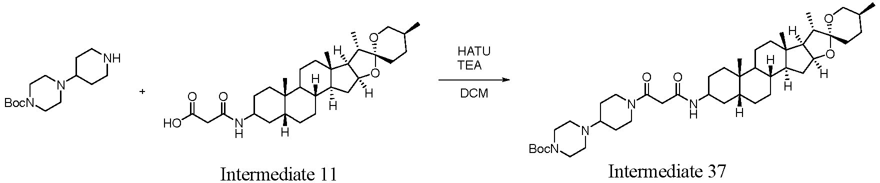

Under the protection of argon, 0.1 g of 1-Boc-4-(piperidin-4-yl)-piperazine, 0.22 g of intermediate 11, 0.21 g of HATU, and 0.11 g of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

### Step 2: intermediate 38

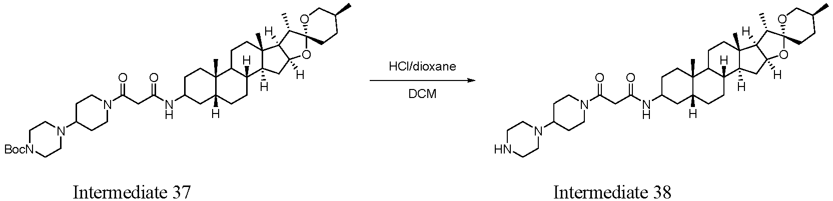

Under the protection of argon, 0.1 g of intermediate 37 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 3: synthesis of Embodiment 33

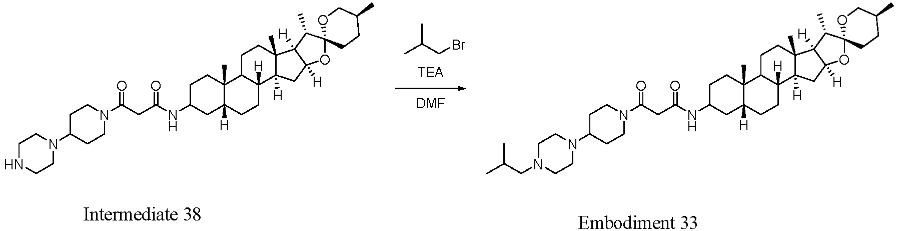

Under the protection of argon, 0.1 g of intermediate 38, 0.03 g of bromoisobutane, and 0.03 g of TEA were dissolved in 15 mL of N,N-dimethylformamide, heated to 50°C, reacted for 12 h, and the reaction was detected. The reactant was spin-dried to remove the solvent, purified by silica gel column (DCM:MeOH=20:1), subjected to a climbing board test (DCM:MeOH=10:1), and color-developed with phosphomolybdic acid. The yield was 60%.

NMR data: ¹H NMR (400 MHz, CDCl3) δ 7.32 (dd, 1H), 4.52 (m, 1H), 4.48 (m, 1H), 3.94-3.98 (m, 1H) 3.75-3.78 (m, 1H), 3.26 (m, 3H), 3.15 (t, 1H)., 2.25-2.75 (m, 10H), 2.2 (m, 2H), 1.32-2.10 (m, 27H), 1.19 (m, 6H), 1.08 (m, 9H), 0.75 (s, 3H). Mass spectrum: [M+1]709.6.

### Embodiment 33A, Embodiments 33B

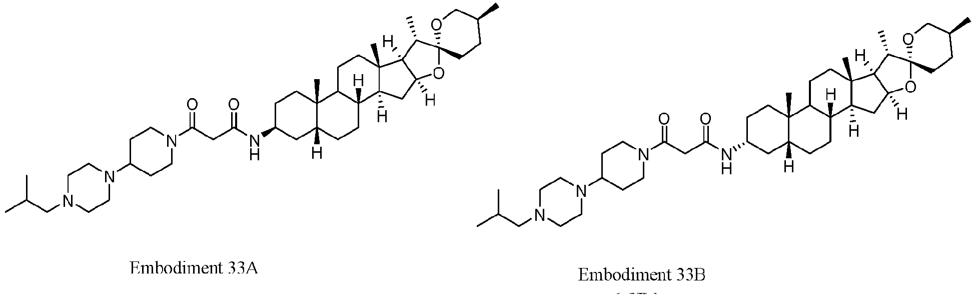

The synthesis process was as follows: using intermediate 18 and 1-bromo-2-methylpropane as raw materials, Embodiment 33A may be obtained by the same synthesis method as Embodiment 33. Using intermediate 21 and 1-bromo-2-methylpropane as raw materials, Embodiment 33B was obtained by the same method.

NMR data: ¹H NMR (400 MHz, CDCl3) δ 7.32 (dd, 1H), 4.52 (m, 1H), 4.48 (m, 1H), 3.94-3.98 (m, 1H) 3.76-3.78 (m, 1H), 3.26 (m, 3H), 3.15 (t, 1H)., 2.25-2.75 (m, 10H), 2.2 (m, 2H), 1.32-2.10 (m, 27H), 1.20 (m, 6H), 1.08 (m, 9H), 0.75 (s, 3H). Mass spectrum: [M+1]709.6.

NMR data of Embodiment 33B: ¹H NMR (400 MHz, CDCl3) δ 7.32 (dd, 1H), 4.52 (m, 1H), 4.48 (m, 1H), 3.94-3.97 (m, 1H) 3.75-3.78 (m, 1H), 3.27 (m, 3H), 3.15 (t, 1H)., 2.25-2.75 (m, 10H), 2.2 (m, 2H), 1.32-2.10 (m, 27H), 1.19 (m, 6H), 1.09 (m, 9H), 0.75 (s, 3H). Mass spectrum: [M+1]709.5.

### Embodiment 34

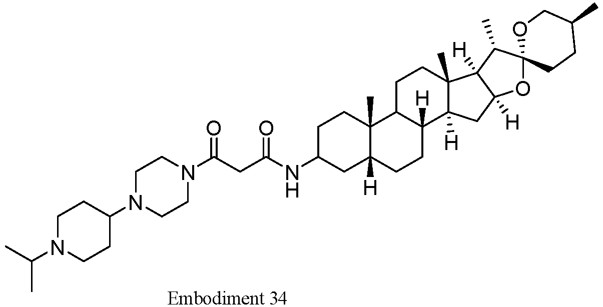

A preparation process was specifically described as follows:

### Step 1: intermediate 39

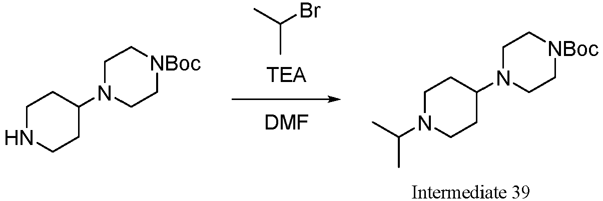

Under the protection of argon, 0.1 g of 1-Boc-4-(piperidin-4-yl)-piperazine, 0.06 g of 2-bromopropane, and 0.08 g of TEA were dissolved in 30 mL of N,N-dimethylformamide, heated to 50°C, reacted for 12 h, and the reaction was detected. The reactant was spin-dried to remove the solvent, then directly put into the next reaction, and color-developed with phosphomolybdic acid. The yield was 70%.

### Step 2: intermediate 40

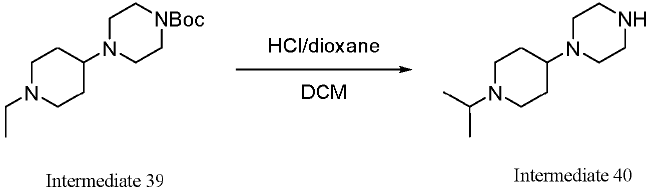

Under the protection of argon, 0.1 g of intermediate 39 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 3: synthesis of Embodiment 34

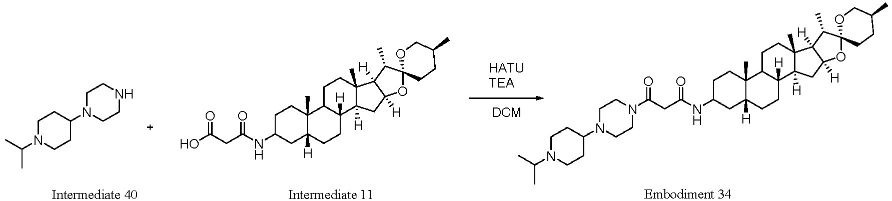

Under the protection of argon, 0.05 g of intermediate 40, 0.14 g of intermediate 11, 0.13 g of HATU, and 0.07 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, CDCl3) δ 7.32 (dd, 1H), 4.41-4.45 (m, 1H), 3.94-3.98 (m, 1H), 3.75-3.78 (m, 1H), 3.51 (m, 6H), 3.28-3.32 (m, 3H), 2.55 (m, 8H), 1.32-2.10 (m, 27H), 1.19 (m, 14H), 1.08 (m, 6H), 0.99 (m, 3H), 0.75 (s, 3H). Mass spectrum: [M+1]695.7.

### Embodiment 34A, Embodiments 34B

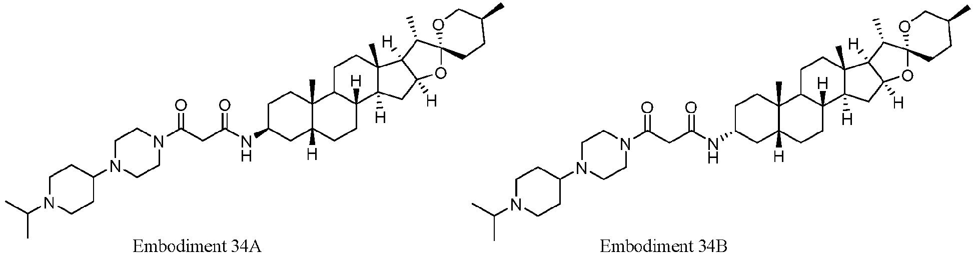

The synthesis method was as follows: using intermediate 18 and 1-(1-isobutylpiperidin-4-yl)piperazine as raw materials, Embodiment 34A may be obtained by the same synthesis method as Embodiment 34. Using intermediate 21 and 1-(1-isobutylpiperidin-4-yl)piperazine as raw materials, Embodiment 34B was obtained by the same method.

NMR data of Embodiment 34A: ¹H NMR (400 MHz, CDCl3) δ 7.33 (dd, 1H), 4.41-4.45 (m, 1H), 3.94-3.98 (m, 1H), 3.75-3.79(m, 1H), 3.51 (m, 6H), 3.28-3.32 (m, 3H), 2.55 (m, 8H), 1.32-2.11(m, 27H), 1.19 (m, 14H), 1.08 (m, 6H), 0.99 (m, 3H), 0.75 (s, 3H). Mass spectrum: [M+1]695.7.

NMR data of Embodiment 34B: ¹H NMR (400 MHz, CDCl3) δ 7.32 (dd, 1H), 4.41-4.45 (m, 1H), 3.94-3.98 (m, 1H), 3.75-3.78 (m, 1H), 3.52 (m, 6H), 3.28-3.32 (m, 3H), 2.55 (m, 8H), 1.32-2.11 (m, 27H), 201.19 (m, 14H), 1.08 (m, 6H), 0.99 (m, 3H), 0.75 (s, 3H). Mass spectrum: [M+1]695.6.

### Embodiment 35

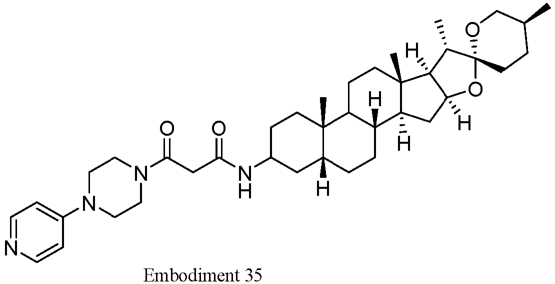

A preparation process was specifically described as follows:

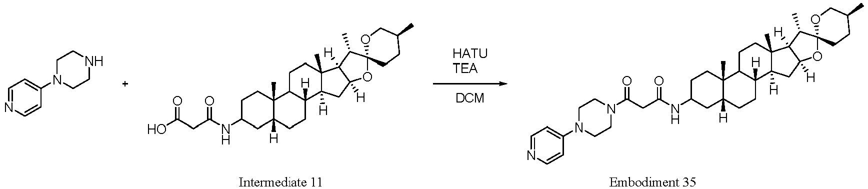

Using intermediate 11 and 1-(pyridyl-4-yl)piperazine as raw materials, Embodiment 35 may be obtained by the same synthesis method as Embodiment 28.

NMR data: ¹H NMR (400 MHz, CDCl3) δ 8.25 (m, 2H), 7.15 (dd, 1H), 6.65 (m, 2H), 4.41-4.45 (m, 1H), 3.94-3.98 (m, 1H), 3.76 (m, 5H), 3.25-3.50 (m, 7H), 1.32-2.10 (m, 27H), 1.08 (m, 6H), 0.99 (m, 3H), 0.75 (s, 3H). Mass spectrum: [M+1]647.5.

### Embodiment 35A, Embodiments 35B

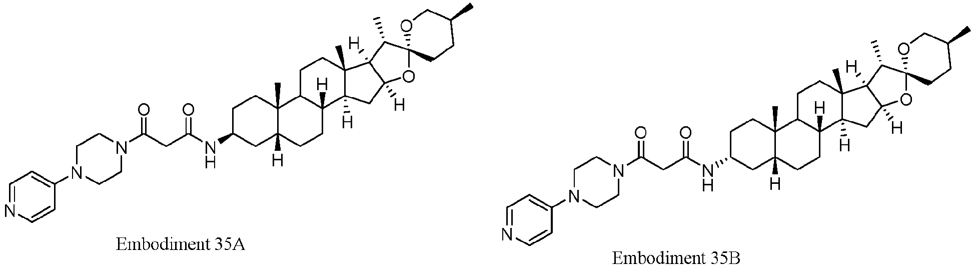

The synthesis method was as follows: using intermediate 18 and 1-(pyridyl-4-yl)piperazine as raw materials, Embodiment 35A may be obtained by the same synthesis method as Embodiment 28. Using intermediate 21 and 1-(pyridyl-4-yl)piperazine as raw materials, Embodiment 35B was obtained by the same method.

NMR data of Embodiment 35A: ¹H NMR (400 MHz, CDCl3) δ 8.24 (m, 2H), 7.14 (dd, 1H), 6.65 (m, 2H), 4.41-4.44 (m, 1H), 3.94-3.98 (m, 1H), 3.75 (m, 5H), 3.25-3.50 (m, 7H), 1.32-2.10 (m, 27H), 1.07 (m, 6H), 0.99 (m, 3H), 0.75 (s, 3H). Mass spectrum: [M+1]647.6.

NMR data of Embodiment 35B: ¹H NMR (400 MHz, CDCl3) δ 8.26 (m, 2H), 7.16 (dd, 1H), 6.66 (m, 2H), 4.41-4.45 (m, 1H), 3.94-3.99(m, 1H), 3.77(m, 5H), 3.25-3.50 (m, 7H), 1.32-2.11 (m, 27H), 1.08 (m, 6H), 0.99 (m, 3H), 0.75 (s, 3H). Mass spectrum: [M+1]647.5.

### Embodiment 36

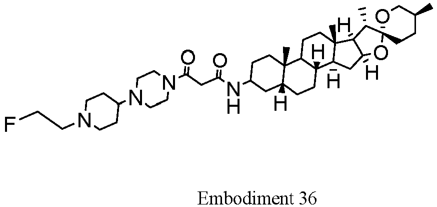

A preparation process was specifically described as follows:

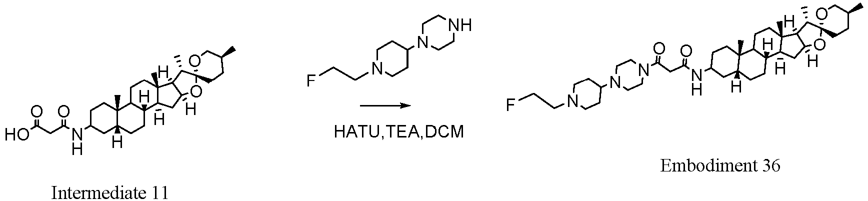

Using intermediate 11 and 1-(1-(2-fluoroethyl)piperidin-4-yl)piperazine as raw materials, Embodiment 36 may be obtained by the same synthesis method as Embodiment 31.

NMR data: ¹H NMR (400 MHz, CDCl3) δ 8.09 (d, 1H), 4.62 (td, 1H), 4.50 (td, 1H), 4.41 (h, 1H), 4.19 (d, 1H), 3.95 (dt, 1H), 3.54 (m, 7H), 3.29 (d, 3H), 3.02 (dt, 3H), 2.61 (m, 8H), 2.30 (m, 1H), 1.34 (m, 39H). Mass spectrum: [M+1]699.6.

### Embodiment 37

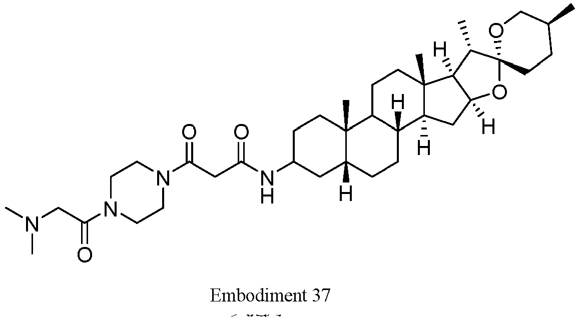

A preparation process was specifically described as follows:

### Step 1: intermediate 41

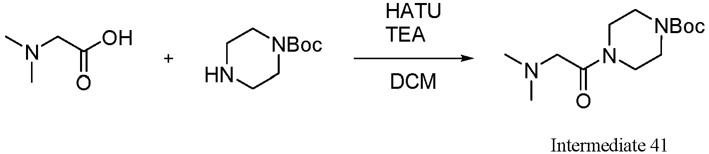

Under the protection of argon, 0.2 g of N,N-dimethylglycine, 0.36 g of tert-butyl 1-piperazinecarboxylate, 1.1 g of HATU, and 0.6 of TEA were dissolved in 60 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 80%.

### Step 2: intermediate 42

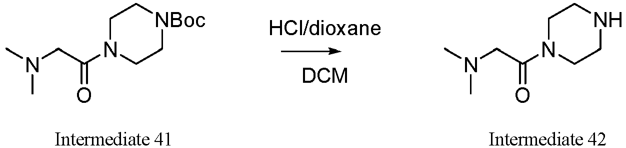

Under the protection of argon, 0.2 g of intermediate 41 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 3: synthesis of Embodiment 37

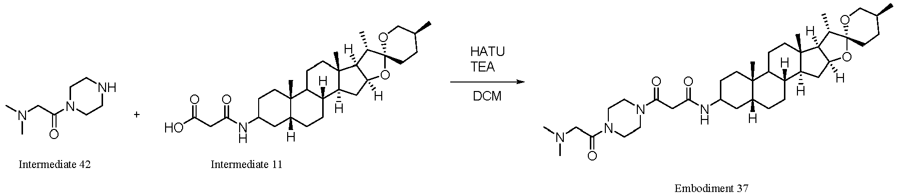

Under the protection of argon, 0.05 g of intermediate 42, 0.18 g of intermediate 11, 0.17 g of HATU, and 0.1 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, CDCl3) δ 7.76 (dd, 1H), 4.40 (m, 1H), 4.18 (s, 1H), 3.95 (dt, 1H), 3.64 (t, 8H), 3.32 (m, 3H), 3.14 (d, 2H), 2.28 (d, 6H), 1.66 (m, 27H), 1.08 (dd, 3H), 0.99 (m, 6H), 0.75 (d, 3H). Mass spectrum: [M+1]655.6.

### Embodiment 38

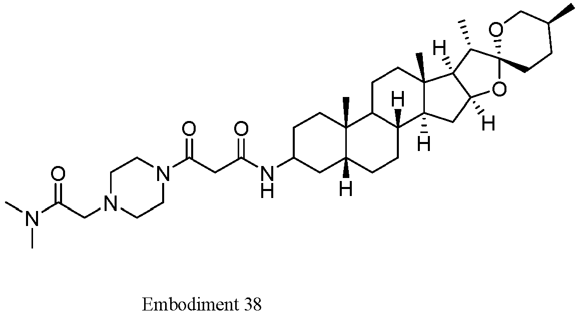

A preparation process was specifically described as follows:

### Step 1: intermediate 43

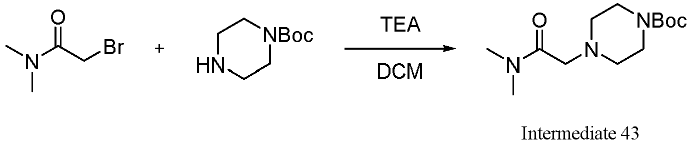

Under the protection of argon, 0.1 g of 1-Boc-4-(piperidin-4-yl)-piperazine, 0.1 g of 2-bromo-N,N-dimethylacetamide, and 0.08 g of TEA were dissolved in 30 mL of dichloromethane, reacted at room temperature for 12 h, and the reaction was detected. The reactant was spin-dried to remove the solvent, then directly put into the next reaction, and color-developed with phosphomolybdic acid. The yield was 80%.

### Step 2: intermediate 44

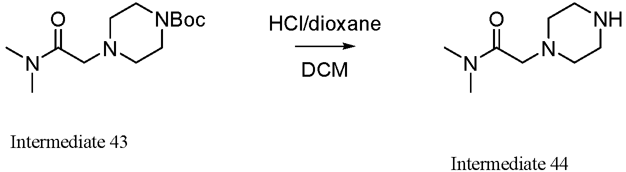

Under the protection of argon, 0.1 g of intermediate 43 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 3: synthesis of Embodiment 38

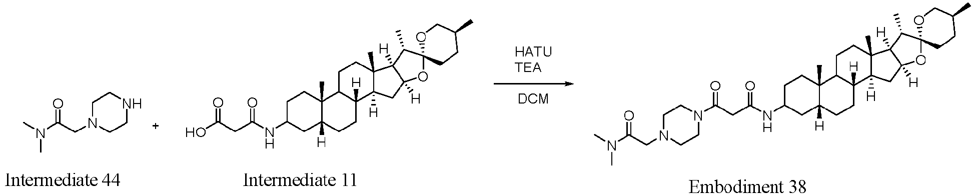

Under the protection of argon, 0.05 g of intermediate 44, 0.18 g of intermediate 11, 0.17 g of HATU, and 0.09 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, DMSO) δ 8.18 (d, 1H), 8.12 (d, 1H), 7.58 (d, 1H), 7.43 (ddd, 2H), 7.20 (m, 3H), 6.96 (pd, 2H), 4.76 (d, 2H), 4.42 (q, 2H), 3.56 (s, 3H), 2.50 (p, 2H), 1.28 (t, 3H). Mass spectrum: [M+1]655.6.

### Embodiment 39

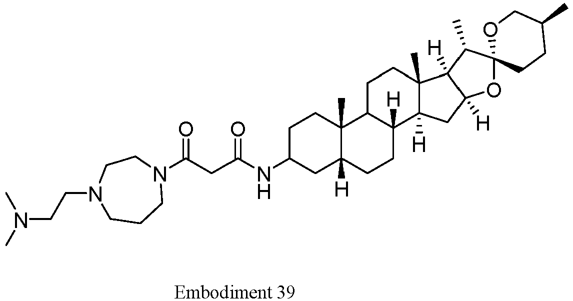

A preparation process was specifically described as follows:

### Step 1: intermediate 45

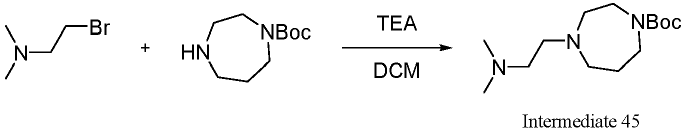

Under the protection of argon, 0.1 g of 2-bromo-N,N-dimethylethanamine hydrobromide, 0.08 g of tert-butyl 1,4-diazepane-1-carboxylate, and 0.13 of TEA were dissolved in 30 mL of dichloromethane, reacted at room temperature for 12 h, and the reaction was detected. The reactant was spin-dried to remove the solvent, then directly put into the next reaction, and color-developed with phosphomolybdic acid. The yield was 80%.

### Step 2: intermediate 46

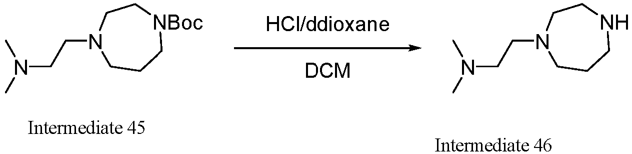

Under the protection of argon, 0.1 g of intermediate 45 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 3: synthesis of Embodiment 39

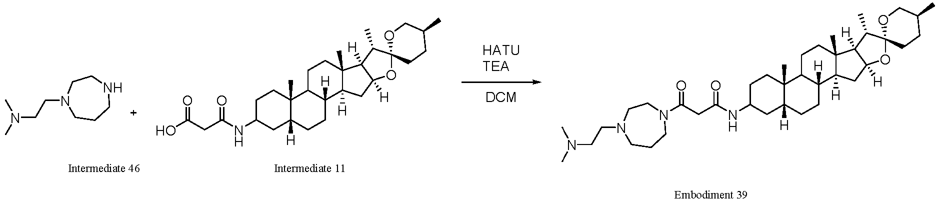

Under the protection of argon, 0.05 g of intermediate 46, 0.18 g of intermediate 11, 0.17 g of HATU, and 0.09 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, CDCl3) δ 7.90 (d, 1H), 4.40 (q, 1H), 4.18 (m, 1H), 3.95 (dd, 1H), 3.7 (m, 5H), 3.32 (t, 3H), 3.10 (m, 4H), 2.85 (d, 6H), 2.35 (m, 5H), 1.15-2.10 (m, 27H), 1.08 (dd, 3H), 0.97 (m, 6H), 0.75 (d, 3H).0.75 (s, 3H). Mass spectru [M+1]655.6.

### Embodiment 40

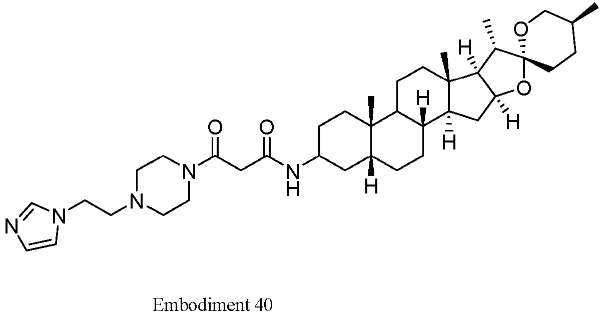

A preparation process was specifically described as follows:

### Step 1: intermediate 47

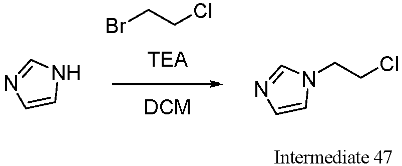

Under the protection of argon, 0.2 g of imidazole, 0.5 g of 1-bromo-2-chloroethane, 0.6 g of TEA were dissolved in 30 mL of dichloromethane, reacted at room temperature for 12 h, and the reaction was detected. The reactant was spin-dried to remove the solvent, then directly put into the next reaction, and color-developed with phosphomolybdic acid. The yield was 80%.

### Step 2: intermediate 48

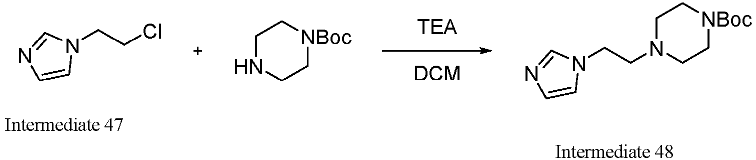

Under the protection of argon, 0.1 g of 1-Boc-4-(piperidin-4-yl)-piperazine, 0.08 g of intermediate 47, and 0.08 g of TEA were dissolved in 30 mL of dichloromethane, reacted at room temperature for 12 h, and the reaction was detected. The reactant was spin-dried to remove the solvent, then directly put into the next reaction, and color-developed with phosphomolybdic acid. The yield was 70%.

### Step 3: intermediate 49

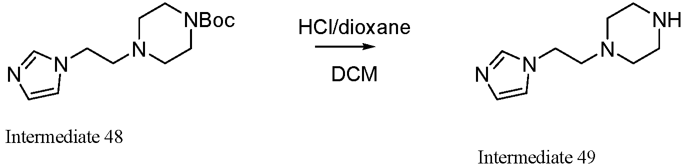

Under the protection of argon, 0.1 g of intermediate 48 was dissolved in 10 mL of dichloromethane, added with 5 mL of dioxane hydrochloride solution, reacted at room temperature for 2 h, and the reaction was detected. The reactant was spin-dried to remove the solvent, then directly put into the next reaction, and color-developed with phosphomolybdic acid. The yield was 90%.

### Step 4: synthesis of Embodiment 40

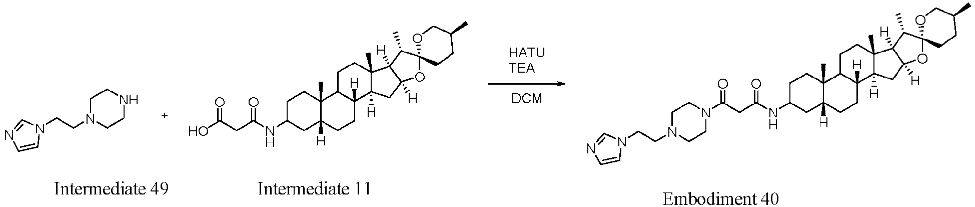

Under the protection of argon, 0.05 g of intermediate 49, 0.17 g of intermediate 11, 0.16 g of HATU, and 0.08 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, CDCl3) δ 7.90 (d, 1H), 7.62 (s, 1H), 7.07 (s, 1H), 6.98 (d, 1H), 4.40 (m, 1H), 4.05 (t, 2H), 4.18 (m, 1H), 3.95 (dt, 1H), 3.64 (q, 2H), 3.54 (t, 2H), 3.29 (d, 3H), 2.71 (td, 2H), 2.46 (q, 4H), 1.15-2.10 (m, 27H), 1.08 (dd, 3H), 0.97 (m, 6H), 0.75 (d, 3H). Mass spectrum: [M+1]664.6.

### Embodiment 41

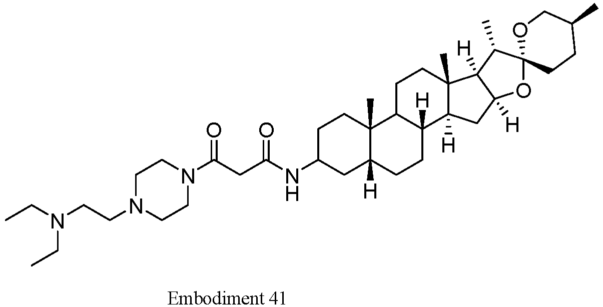

A preparation process was specifically described as follows:

### Step 1: intermediate 50

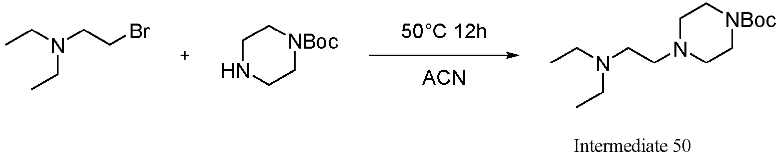

Under the protection of argon, 0.2 g of 2-diethylamino-1-bromoethane hydrobromide and 0.15 g of tert-butyl 1-piperazinecarboxylate were dissolved in 40 mL of acetonitrile, reacted at 50°C for 12 h, and the reaction was detected. The reactant was spin-dried to remove the solvent, then directly put into the next reaction, and color-developed with phosphomolybdic acid. The yield was 80%.

### Step 2: intermediate 51

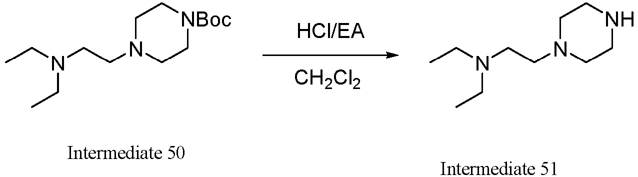

Under the protection of argon, 0.1 g of intermediate 50 was dissolved in 10 mL of dichloromethane, added with 5 mL of ethyl acetate hydrochloride solution, reacted at room temperature for 12 h, and the reaction was detected. The reactant was spin-dried to remove the solvent, then directly put into the next reaction, and color-developed with phosphomolybdic acid. The yield was 90%.

### Step 3: synthesis of Embodiment 41

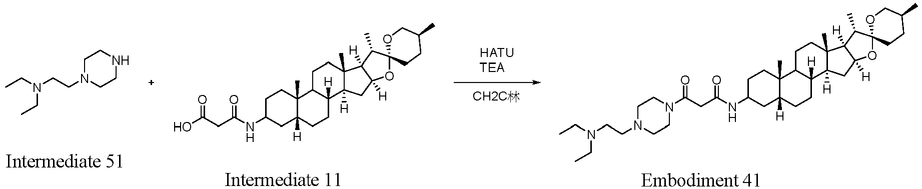

Under the protection of argon, 0.05 g of intermediate 51, 0.14 g of intermediate 11, 0.15 g of HATU, and 0.08 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column: DCM:MeOH=20:1, subjected to a climbing board test (DCM:EA=10:1), and color-developed with phosphomolybdic acid. The yield was 60%.

NMR data: ¹H NMR (400 MHz, CDCl3) δ 7.32 (dd, 1H), 4.41-4.45 (m, 1H), 3.94-3.98 (m, 1H), 3.75-3.78 (m, 1H), 3.65 (t, 2H), 3.56 (t, 2H)., 3.28-3.32 (m, 3H), 2.63-2.80 (m, 8H), 2.47-2.52 (m, 4H), 1.32-2.10 (m, 27H), 1.08 (dd, 3H), 0.99 (m, 6H), 0.75 (s, 3H). Mass spectrum: [M+1]669.7.

### Embodiment 41A, Embodiments 41B

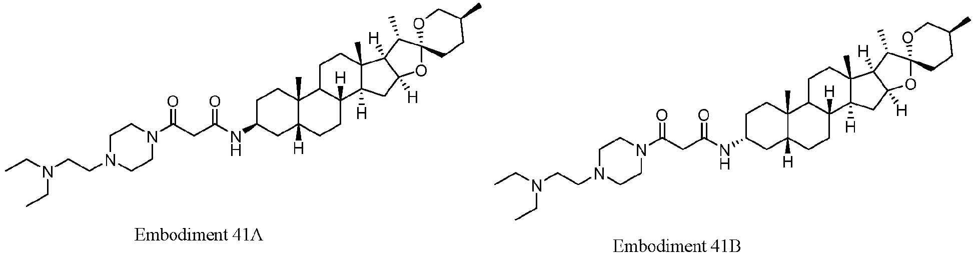

The synthesis process of Embodiment 41A was the same as Embodiment 41, wherein the intermediate 11 was replaced by intermediate 18, obtaining NMR data: ¹H NMR (CDCl3-d6, 400 MHz): 7.98 (d, 1H), 4.42 (q, 1H), 4.18 (s, 1H), 3.95 (dd, 1H), 3.66 (t, 2H), 3.57 (t, 2H), 3.31 (d, 1H), 3.29 (s, 2H), 2.67-2.82 (m, 8H), 2.47-2.52 (m, 4H), 1.58-2.10 (m, 11H), 1.15-1.50 (m, 16H), 1.09-1.10 (m, 6H), 1.08 (d, 3H), 0.99 (d, 3H), 0.95 (s, 3H), 0.75 (s, 3H). Mass spectrum: [M+1]669.7.

The synthesis process of Embodiment 41B was the same as Embodiment 41, wherein the intermediate 11 was replaced by intermediate 21, obtaining NMR data: ¹H NMR (CDCl3-d6, 400 MHz): 7.35 (d, 1H), 4.42 (q, 1H), 3.98 (dd, 1H), 3.73-3.78 (m, 1H), 3.63 (t, 2H), 3.56 (t, 2H), 3.32 (d, 1H), 3.28 (s, 2H), 2.63-2.76 (m, 8H), 2.47-2.52 (m, 4H), 1.58-2.10 (m, 11H), 1.15-1.50 (m, 16H), 1.09-1.10 (m, 6H), 1.08 (d, 3H), 0.99 (d, 3H), 0.95 (s, 3H), 0.75 (s, 3H). Mass spectrum: [M+1]669.6.

### Embodiment 42

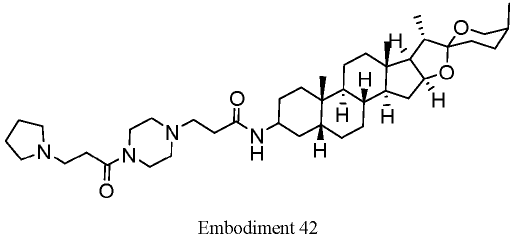

A preparation process was specifically described as follows:

### Step 1: intermediate 52

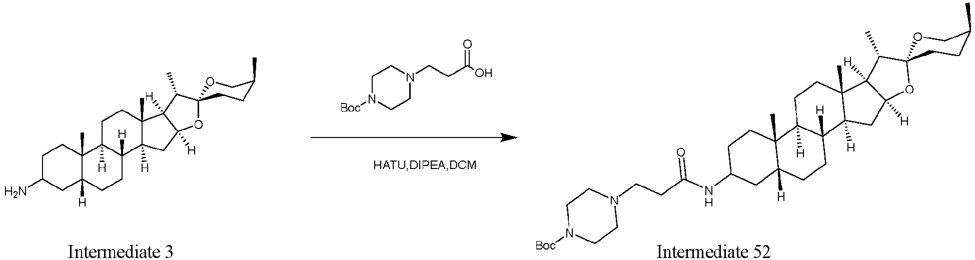

Under the protection of argon, 1 g of intermediate 3, 0.933 g of 3-(1-tert-butoxycarbonylpiperazin-4-yl)propionic acid, 1.373 g of HATU, and 0.788 of DIPEA were dissolved in 20 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column (DCM:MeOH=20:1), subjected to a climbing board test (DCM:EA=10:1), and color-developed with vanillin. The yield was 65%.

### Step 2: intermediate 53

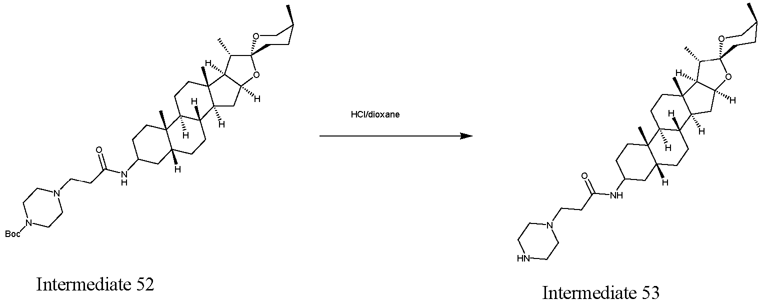

1 g of intermediate 52 was dissolved in 10 mL of MeOH, added with 4 M HCl/dioxane and reacted for 3 h. The reaction was detected. After the reaction was completed, the reactant was spin-dried to remove the solvent and obtain a product, which was directly put into the next reaction and color-developed with vanillin. The yield was 80%.

### Step 3: synthesis of Embodiment 42

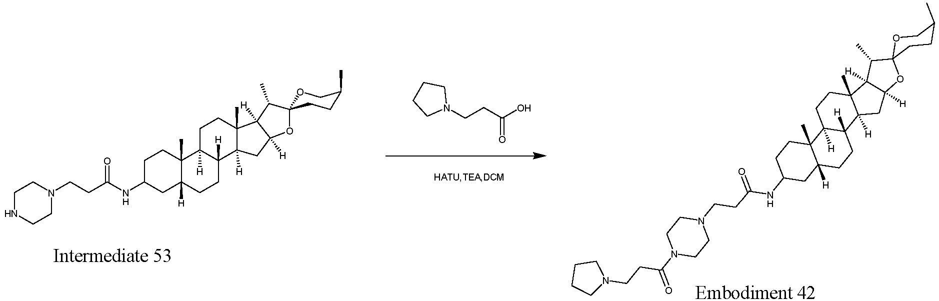

Under the protection of argon, 0.2 g of intermediate 53, 0.077 g of 3-(pyrrolidin-1-yl)propanoic acid, 0.205 g of HATU, and 0.091 of TEA were dissolved in 10 mL of dichloromethane, reacted at room temperature for 6 h, and the reaction was detected. The reactant was extracted with water/dichloromethane, washed with a saturated saline solution, dried with anhydrous sodium sulfate, spin-dried to remove the solvent, purified by silica gel column (DCM:MeOH=20:1), and color-developed with vanillin. The yield was 60%.

NMR data: ¹H NMR (400 MHz, CDCl3) δ 7.13 (d, 1H), 4.42 (q, 1H), 3.62 (m, 9H), 2.89 (d, 2H), 2.51 (m, 17H), 1.31 (m, 38H). Mass spectrum: [M+1]682.6.

### Embodiment 42A

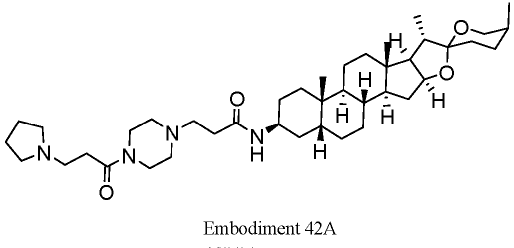

The preparation process was the same as that of Embodiment 42, and Embodiment 42A was obtained by replacing intermediate 3 with intermediate 7. NMR data: ¹H NMR (400 MHz, CDCl3) δ 8.29 (d, 1H), 4.41 (m, 1H), 4.20 (s, 1H), 3.95 (dd, 1H), 3.55 (m, 6H), 2.83 (ddd, 2H), 2.56 (m, 16H), 1.34 (m, 40H). Mass spectrum: [M+1]682.7.

### Embodiment 43 Activity testing of compounds

### Experiment 1. Detection of protective effects of small molecule compounds on the injuries of primary cultured neurons in rats induced by oxygen glucose deprivation model (OGD).

Experimental and analytical method: rats, 15 days pregnant, were ordered, brains of fetal rats (E15-16) were dissected, and neurons in the cerebral cortex were subjected to primary culture in a neuronal medium for 14 days (DIV 14) using 48-well plates, followed by an OGD experiment. During the OGD experiment, a neuronal culture solution was replaced by a glucose- and oxygen-free culture solution (95% N₂/5% CO₂ balance), and the neurons were subjected to OGD treatment in an OGD chamber. Then, the oxygen- and glucose-free culture medium was replaced by a normal neuronal culture solution, which was then cultured in a 95% Air/5% CO₂ incubator for 24 h for neuronal activity analysis. By staining with Hoechst 33342 and Propidium iodide, a high-content fluorescence microscope was used for photographing, wherein 20 random non-overlapping visual fields were photographed per duplicate well, and the total number of neurons and the number of dead neurons were counted by software (Thermo Scientific^{™}, HCS Studio Cell Analysis Software). A standard deviation and a standard error were calculated using the obtained data, and the P value was calculated by T-test analysis to determine whether there was a significant difference.

Compound treatment: 13 small molecule compounds in Embodiments 1 to 20 were each diluted with DMSO to a 0.5 mM mother solution; 1 µL of mother solution was respectively added to 0.5 ml of culture medium per well in a 48-well plate to a final concentration of 1 µM; and 1 µL of DPQ (10 mM mother solution) as a positive control compound and 1 µL of DMSO as a negative control were added to a culture solution 24 h before the OGD experiment, till the glucose- and oxygen-free culture medium has the same concentration as the subsequent normal neuronal culture medium.

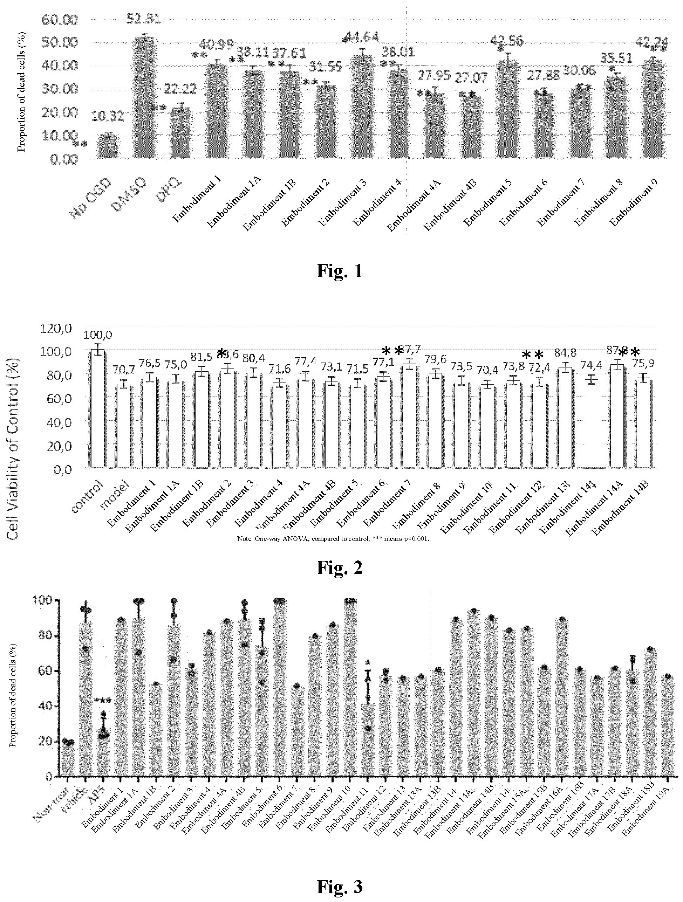

Results: as shown in Fig. 1, in the results of this OGD experiment, the proportion of dead neuron cells without OGD was 10.32%, the proportion of dead cells in the negative control with DMSO was 52.31%, and a damage rate was about 42%. The proportion of dead cells in the positive control with DPQ was 22.22%, which had a significant protective effect on neuronal damages.

### Experiment 2. Detection of protective effects of oxidative damages of small molecule compounds in Embodiment 1 to Embodiment 14B on hydrogen peroxide (H₂O₂)-induced human SHSY5Y neural tumor cells

**Table 1. Experimental conditions and methods**

| Cell line | SHSY5Y |
|---|---|
| State of plated cells | The cells were in good condition |
| Density of plated cells | 3 × 10⁵ cells/ml |
| Final drug concentration | 1µM |
| Drug pre-protection time | 24 h |
| Detection method | MTT: 490nm |

**Table 2. Experimental process**

| Day 1 | Day 2 | Day 3 | Day 4 |
|---|---|---|---|
| Plating | A compound was added to an original medium to a working concentration of 1 µM. | A serum-based medium was replaced with a serum-free medium containing the compound having a concentration of 1 µM, and added with 250 µM H₂O₂. | An MTT result was measured after 24 h. |

Results: as shown in Fig. 2, Embodiment 2, Embodiment 7, Embodiment 13 and Embodiment 14A significantly protected H₂O₂-induced neural tumor cell damages, wherein undifferentiated SH-SY5Y cells were pre-protected for 24 h till a final concentration was 1 µM, treated with H₂O₂ for 24 h, and then detected.

### Experiment 3. Detection of protective effects of small molecule compounds on L-cysteine-induced neuronal excitotoxicity

Experimental and analytical method: before the experiment, a 24-well cell culture plate was pretreated with Poly-D-lysine and placed in a cell incubator at 37°C and 5% CO₂ overnight. SD rats, 18 days pregnant, were ordered, brains of fetal rats (E18-19) were dissected, and hippocampal neurons in the brains were subjected to primary culture in a neuronal medium using a 24-well plate, and then cultured by a serum-free medium. The hippocampal neurons were cultured in a cell incubator at 37°C, 5% CO₂ for 19 days (DIV19), then added with a compound to be tested (a final concentration of 1 µM) and a positive control AP5 [(2R)-amino-5-phosphonopentanoic acid(phosphonatopentanoate)] (a final concentration of 100 µM), cultured for 24 h, and then added with L-Cystine (400 µM) and NaHCO₃ (10 mM). After 18 h, Hoechst3342 (2.5 µg/ml) was added for cell staining. The cells were placed in the incubator and cultured for 15 min, and photographed using the high-content fluorescence microscope, wherein 6 random non-overlapping fields were photographed for each duplicate well, and the total number of neurons and the number of dead neurons were counted with software Image J. The obtained data was used to determine whether there was a difference between a drug treatment group and a control group.

Compound treatment: 32 small molecule compounds in Embodiment 1 to Embodiment 19A were diluted with DMSO to a 1 mM mother solution; and 1 µL of mother solution was added to 1 ml of culture solution per well in a 24-well plate to a final concentration of 1 µM, wherein a positive control compound was 100 µl of AP5 (100 mM mother solution), and a negative control was 1 µl of DMSO.

Results: as shown in Fig. 3, the proportion of dead neuron cells without L-Cystine (400 µM) and NaHCO₃ (10 mM) was 20.12%, the proportion of dead cells in the negative control with DMSO was 87.62%, and a damage rate was about 67.5%. The proportion of dead cells in the positive control with AP5 was 27.58%, which had a very significant protective effect on the neuronal excitotoxicity. The results of experiments where the above four small molecule compounds were added showed that the compounds, i.e., Embodiment 1, Embodiment 1A, Embodiment 2, Embodiment 4B, Embodiment 6, Embodiment 10, Embodiment 13, Embodiment 13B, etc., had protective effects on L-Cystine (400 µM)-induced neuronal excitotoxicity.

### Experiment 4. Evaluation of anti-inflammatory effects of "Embodiment 5" and "Embodiment 6" on zebrafish

### Experimental animals

Transgenic neutrophil fluorescent zebrafishes were matched and propagated naturally in pairs. The age was 3 days after fertilization (3 dpf), and there was a total of 810 zebrafishes, with 30 zebrafishes per experimental group. The purposes were to determine maximum tolerated concentrations (MTC) of "Embodiment 5" and "Embodiment 6" in an LPS-induced inflammation experiment, and evaluate anti-inflammatory effects of "Embodiment 5" and "Embodiment 6" on LPS-induced inflammations.

Zebrafishes were fed in fish-farming water at 28°C (water quality: 200 mg of instant sea salt was added to every 1 L of reverse osmosis water, wherein the conductivity was 480 to 510 µS/cm, pH was 6.9 to 7.2, and the hardness was 53.7 to 71.6 mg/L CaCO₃), and provided by a fish-farming center in our company, wherein the experimental animals had a use license number: SYXK (Zhejiang) 2012-0171. Feeding management met the requirements of the international AAALAC certification.

### Experimental drugs

"Embodiment 5", a white powder provided by Shenzhen Qingbo Huineng Pharmaceutical Technology Co., Ltd, was dried and stored at 4°C, and sampled on October 18, 2019. A 20 mM mother solution was prepared from DMSO before an experiment and stored at -20°C.

"Embodiment 6", a white powder provided by Shenzhen Qingbo Huineng Pharmaceutical Technology Co., Ltd, was dried and stored at 4°C, and sampled on October 18, 2019. A 20 mM mother solution was prepared from DMSO before an experiment and stored at -20°C.

Indomethacin, batch number 1108939, produced by Shanghai Jingchun Biochemical Technology Co.,Ltd., was provided by Hangzhou Hunter Biotechnology Co.,Ltd. A 80 mM mother solution was prepared from a DMSO solution before an experiment and stored at -20°C.

### Instruments and reagents

Dissecting microscope (SZX7, OLYMPUS, Japan); camera (VertA1) connected to a microscope; precision electronic balance (CP214, OHAUS, AmericaCP214, OHAUS); fluorescence microscope (AZ100, Nikon, Japan); methylcellulose (Sigma, USA); dimethyl sulfoxide (Sigma, France); and 6-well plate (Nest Biotech).

### Concentration groups

1. Determination of the maximum tolerated concentrations (MTC) of "Embodiment 5" and "Embodiment 6"
   Experimental group 1 Normal control group (without any treatment)
   Experimental group 2 Model control group
   Experimental group 3 0.625 µM of "Embodiment 5"
   Experimental group 4 1.25 µM of "Embodiment 5"
   Experimental group 5 2.5 µM of "Embodiment 5"
   Experimental group 6 5 µM of "Embodiment 5"
   Experimental group 7 10 µM of "Embodiment 5"
   Experimental group 8 50 µM of "Embodiment 5"
   Experimental group 9 100 µM of "Embodiment 5"
   Experimental group 10 200 µM of "Embodiment 5"
   Experimental group 11 0.625 µM of "Embodiment 6"
   Experimental group 12 1.25 µM of "Embodiment 6"
   Experimental group 13 2.5 µM of "Embodiment 6"
   Experimental group 14 5 µM of "Embodiment 6"
   Experimental group 15 10 µM of "Embodiment 6"
   Experimental group 16 50 µM of "Embodiment 6"
   Experimental group 17 100 µM of "Embodiment 6"
   Experimental group 18 200 µM of "Embodiment 6"
   2. Evaluation of anti-inflammatory effects of "Embodiment 5" and "Embodiment 6"
   Experimental group 1 Normal control group (without any treatment)
   Experimental group 2 Model control group
   Experimental group 3 80 µM of positive control drug indomethacin
   Experimental group 4 0.28 µM of "Embodiment 5"
   Experimental group 5 0.83 µM of "Embodiment 5"
   Experimental group 6 2.5 µM of "Embodiment 5"
   Experimental group 7 0.28 µM of "Embodiment 6"
   Experimental group 8 0.83 µM of "Embodiment 6"
   Experimental group 9 2.5 µM of "Embodiment 6"
   Basis for concentration determination

After communicating with customers according to the results of concentration exploration, it was determined that the maximum tolerated concentrations of "Embodiment 5" and "Embodiment 6" in the evaluation of anti-inflammatory effects were both 2.5 µM.

### Model making

LPS was used to treat normal 3 dpf transgenic neutrophil fluorescent zebrafishes in a yolk sac injection, and a zebrafish inflammation model was established.

### Experimental Method

### 1. Determination of the maximum tolerated concentrations (MTC) of "Embodiment 5" and "Embodiment 6"

Transgenic neutrophil fluorescent zebrafishes (3 dpf), well developed and consistent in stage on 3 days after fertilization, were picked under a microscope and placed in a six-well plate, with 30 zebrafishes being randomly picked for each well at a capacity of 3 mL in each well. A zebrafish inflammation model was established by administrating LPS in a yolk sac injection. "Embodiment 5" and "Embodiment 6" were respectively dissolved with water, wherein the concentrations were 0.625 µM, 1.25 µM, 2.5 µM, 5 µM, 10 µM, 50 µM, 100 µM and 200 µM, respectively. Meanwhile, a normal control group (i.e., zebrafishes were treated with fish-farming water) and a model control group were set, and cultured in a 28°C incubator for 3 h, and the death of zebrafishes was then observed and recorded. The number of dead zebrafishes in each experimental group was counted, and the maximum tolerated concentrations (MTCs) of zebrafishes in "Embodiment 5" and "Embodiment 6" were determined.

### 2. Evaluation of anti-inflammatory effects of "Embodiment 5" and "Embodiment 6"

Transgenic neutrophil fluorescent zebrafishes (3 dpf), well developed and consistent in stage on 3 days after fertilization, were picked under a microscope and placed in a six-well plate, with 30 zebrafishes being randomly picked for each well at a capacity of 3 mL in each well. A zebrafish inflammation model was established by administrating LPS in a yolk sac injection. "Embodiment 5" and "Embodiment 6" were respectively dissolved with water, wherein the concentrations were 0.28 µM, 0.83 µM and 2.5 µM, respectively. The concentration of indomethacin was 80 µM. Meanwhile, a normal control group (i.e., zebrafishes were treated with fish-farming water) and a model control group were set, and incubated in a 28°C incubator for 3 h. 10 zebrafishes were randomly selected from each group, and observed and photographed under a fluorescence microscope, and the photos were saved. Nikon NIS-Elements D 3.10 advanced image processing software was used for image analysis, the number (N) of inflammatory neutrophils in zebrafish was calculated, and the statistical processing results were expressed as mean ± SE. The statistical analysis results of the number of inflammatory neutrophils in zebrafish were used to evaluate whether "Embodiment 5" and "Embodiment 6" had significant anti-inflammatory effects on LPS-induced inflammatory zebrafish, respectively. A formula for calculating the anti-inflammatory effects of "Embodiment 5" and "Embodiment 6" was as follows: Inflammation resolution (%)=((N(model control group) - N(test group))/N(model control group)) * 100%

ANOVA and Dunnett's T-test were used for statistical analysis, and p < 0.05 showed a significant difference.

### Experimental results

### 1. MTC

The maximum solubility of DMSO in "Embodiment 5" was 20 mM, and a maximum concentration of DMSO that zebrafishes could tolerate was 1%, so the maximum tolerated concentration of "Embodiment 5" in the evaluation of the anti-inflammatory effect was 200 µM. 30 zebrafishes all died when the concentrations of "Embodiment 5" were 200 µM, 100 µM, 50 µM and 10 µM, with a mortality of 100%. At a concentration of 5 µM, 3 zebrafishes died, with a mortality rate of 10%. At a concentration of 2.5 µM, zebrafishes were in a normal state and the drug was not precipitated, so the maximum tolerated concentration of "Embodiment 5" in the evaluation of the anti-inflammatory effect was 2.5 µM.

The maximum solubility of DMSO in "Embodiment 6" was 20 mM, and a maximum concentration of DMSO that zebrafishes could tolerate was 1%, so a maximum administration concentration of "Embodiment 6" in the evaluation of the anti-inflammatory effect was 200 µM. 30 zebrafish all died when the concentrations of "Embodiment 6" were 200 µM, 100 µM, and 50 µM, with a mortality of 100%. At a concentration of 10 µM and 5 µM, 4 zebrafishes died, with a mortality rate of 13.33%. At a concentration of 2.5 µM, zebrafishes were in a normal state and the drug was not precipitated, so the maximum tolerated concentration of "Embodiment 6" in the evaluation of the anti-inflammatory effect was 2.5 µM.

The maximum tolerated concentrations of "Embodiment 5" and "Embodiment 6" were implemented according to concentration exploration results, i.e., the maximum tolerated concentrations of "Embodiment 5" and "Embodiment 6" in the evaluation of the anti-inflammatory effects were both 2.5 µM. Details were shown in Table 3.

**Table 3. Statistics on the number of dead zebrafishes under tolerated concentrations of "Embodiment 5" and "Embodiment 6" (n = 30)**

| Group | Concentratio n (µg/mL) | Death count | Death rate (%) | Toxicity | If precipitatio n occurred? |
|---|---|---|---|---|---|
| Normal control group | - | 0 | 0 | - | - |
| Model control group | - | 0 | 0 | - | - |
| "Embodime nt 5" | 0.625 | 0 | 0 | No obvious toxicity | No |
| | 1.25 | 0 | 0 | No obvious toxicity | No |
| | 2.5 | 0 | 0 | No obvious toxicity | No |
| | 5 | 3 | 10 | - | No |
| | 10 | 30 | 100 | - | No |
| | 50 | 30 | 100 | - | No |
| | 100 | 30 | 100 | - | No |
| | 200 | 30 | 100 | - | No |
| "Embodime nt 6" | 0.625 | 0 | 0 | No obvious toxicity | No |
| | 1.25 | 0 | 0 | No obvious toxicity | No |
| | 2.5 | 0 | 0 | No obvious toxicity | No |
| | 5 | 4 | 13.33 | - | No |
| | 10 | 4 | 13.33 | - | No |
| | 50 | 30 | 100 | - | No |
| | 100 | 30 | 100 | - | No |
| | 200 | 30 | 100 | - | No |

### 2. Anti-inflammatory effects

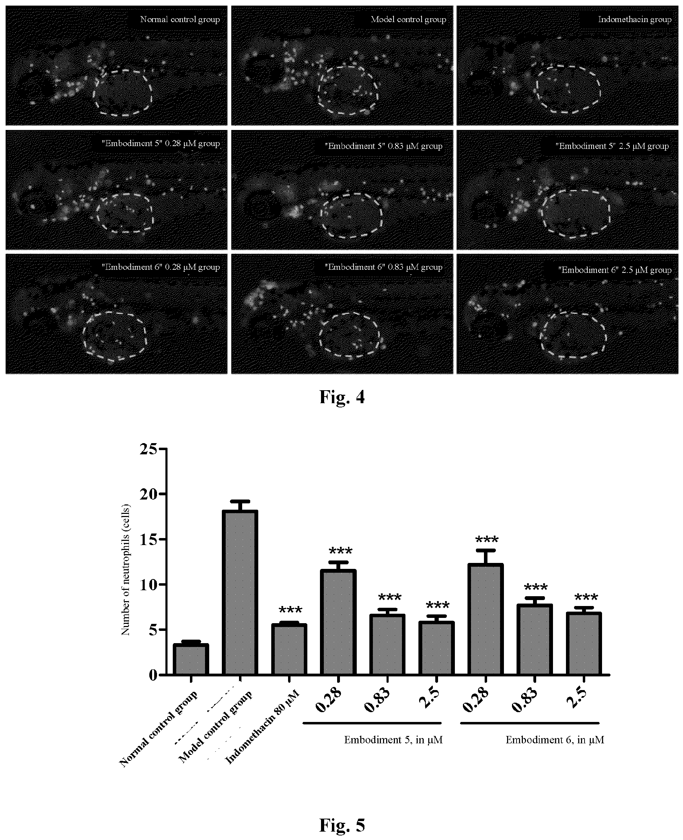

As shown in Table 4, Fig. 4, Fig. 5 and Fig. 6, a dotted line area in Fig. 4 presented neutrophil at an inflammation site. The number (18) of neutrophils at an inflammation site of each zebrafish in a model control group was compared with the number (3) of neutrophils at an inflammation site of each zebrafish in a normal control group, where P < 0.001, indicating that an LPS-induced transgenic neutrophil fluorescent zebrafish inflammation model was successfully established. The number (6) of neutrophils at an inflammation site of each zebrafish in an 80 µM indomethacin group was compared with that in a model control group, where P < 0.001, and an anti-inflammatory effect was 67%, indicating that indomethacin had an obvious anti-inflammatory effect on inflammatory zebrafish.

When the concentrations of "Embodiment 5" were 0.28 µM, 0.83 µM and 2.5 µM, the numbers of neutrophils at the inflammation site of each zebrafish were respectively 12, 7 and 6, and the anti-inflammatory effects on zebrafish were respectively 33%, 61% and 67%. Compared with the model control group (18), P< 0.001 was shown for 0.28 µM, 0.83 µM and 2.5 µM concentration groups, indicating that "compound 3" had obvious anti-inflammatory effects on inflammatory zebrafish at the concentration of 0.28 to 2.5 µM.

When the concentrations of "Embodiment 6" were 0.28 µM, 0.83 µM and 2.5 µM, the numbers of neutrophils at the inflammation site of each zebrafish were respectively 12, 8 and 7, and the anti-inflammatory effects on zebrafish were respectively 33%, 56% and 61%. Compared with the model control group (18), P< 0.001 was shown for 0.28 µM, 0.83 µM and 2.5 µM concentration groups, indicating that "compound 4" had obvious anti-inflammatory effects on inflammatory zebrafish at the concentration of 0.28 to 2.5 µM.

**Table 4. Quantitative results of the effects of various experimental groups on zebrafish inflammation (n=10)**

| Group | Concentration µM | Number of neutrophils (mean ± SE) | Inflammation resolution (%) |
|---|---|---|---|
| Normal control group | - | 3 ± 0.40 | - |
| Model control group | - | 18 ± 1.08 | - |
| Indomethacin | 80 | 6 ± 0.31*** | 67*** |
| "Embodiment 5" | 0.28 | 12 ± 0.96*** | 33*** |
| | 0.83 | 7 ± 0.64*** | 61*** |
| | 2.5 | 6 ± 0.71*** | 67*** |
| "Embodiment 6" | 0.28 | 12 ± 1.58*** | 33*** |
| | 0.83 | 8 ± 0.82*** | 56*** |
| | 2.5 | 7 ± 0.65*** | 61*** |

### Experimental conclusion

Under the concentration condition of this experiment, both Embodiment 5 and Embodiment 6 had obvious anti-inflammatory effects on inflammatory zebrafish.

### Experiment 5 Pharmacodynamic study for evaluating the effects of the compounds on mice middle cerebral artery occlusion (MCAO) damage model

Neurological function scores and cerebral infarction areas were used to evaluate the effects of the compounds on mice middle cerebral artery occlusion (MCAO) damage model. The results showed that test compounds had a protective effect on mice MCAO damages.

### Experimental drug

**Table 5. Compound to be tested**

| No. | Provided by | Batch | Appearance | Mass | Storage condition |
|---|---|---|---|---|---|
| NO.1 | MedChemExpress inc. | Edaravone | Yellow solid powder | 50mg | -20°C (solid) |
| NO.2 | Shenzhen Qingbo Huineng Pharmaceutical Technology Co., Ltd | Embodiment 10 | White solid powder | 230mg | 4°C |
| NO.3 | Shenzhen Qingbo Huineng Pharmaceutical Technology Co., Ltd | Embodiment 11 | White solid powder | 230mg | 4°C |
| NO.4 | Shenzhen Qingbo Huineng Pharmaceutical Technology Co., Ltd | Embodiment 14A | White solid powder | 230mg | 4°C |
| NO. 5 | Shenzhen Qingbo Huineng Pharmaceutical Technology Co., Ltd | Embodiment 14B | White solid powder | 230mg | 4°C |
| NO.6 | Shenzhen Qingbo | Embodiment | White solid | 230mg | 4°C |
| | Huineng Pharmaceutical Technology Co., Ltd | 19A | powder | | |

Normal saline Name: homemade normal saline.

Hydroxypropyl-β-cyclodextrin Name: hydroxypropyl-β-cyclodextrin; provided by: Sun Chemical Technology (Shanghai) Co., Ltd.; batch: FG310174; appearance: white solid powder; mass: 30 g; storage condition: room temperature.

### Experimental method Experimental animal and feeding

Experimental animal strain: C57BL/6 mouse; weeks of age: 6-8; sex: male; weight of ordered animals: 16-20 g; body weight of used animals: 20-23 g; number: 40; experimental animal provider: Zhejiang Vital River Laboratory Animal Technology Co., Ltd.; production license number: SCKX (Zhejiang) 2019-0001; and quality certificate number: No2005130052, No2004280010.

### Animal feeding

Quarantine: the quarantine period was 7 days, the routine health examination was completed by veterinarians, and the animals exhibiting abnormal behavior were excluded before the experiment.

Animal feeding condition: experimental animals were fed in an SPF-grade constant-temperature and constant-humidity laminar flow cleaning room in the Animal Center (AAALAC certified unit), one cage for every 3 mice. A feeding room had a temperature of 22 ± 3°C, and a humidity of 40 -70%, with light alternated with darkness for 12 h. Cage: made of polycarbonate. Padding was made of autoclaved and cleaned soft corn cobs, replaced twice a week. Feed and drinking water: clean-grade mouse diet, purchased from Beijing Keao Xieli Feed Co.,Ltd. The drinking water was autoclaved and the food was irradiated with cobalt-60 rays. Animals were free to eat sterile food and drink water.

Animal serial number: each cage had a cage label indicating the number of animals, sex, strain, time of receipt, group, and start time of the experiment. Animal serial number: each animal was labeled with a separate animal number on the tail.

Animal grouping and treatment: according to body weight on Day 1, the animals were randomly divided into a total of 8 groups (N=5), one group was treated every day, and 3 animals were selected in each group for measuring cerebral blood flows. Specific grouping and treatments were shown in Table 6.

**Table 6 Animal grouping and drug treatment**

| Group | If MCAO occurred ? | Therapeutic drug | Delivery dosage (mg/kg) | Delivery volume (mL/kg) | Delivery route | Delivery cycle and frequency |
|---|---|---|---|---|---|---|
| 1 | No | Vehicle | - | 10 | PO | Once 1 day before surgery and once 2 h before MCAO, a total of 2 times |
| 2 | Yes | Vehicle | - | 10 | PO | |
| 3 | Yes | NO.1 | 10 | 10 | IP | Once before MCAO |
| 4 | Yes | NO.2 | 60 | 10 | PO | Once 1 day before surgery and once 2 h before MCAO, a total of 2 times |
| 5 | Yes | NO.3 | 80 | 10 | PO | |
| 6 | Yes | NO.4 | 80 | 10 | PO | |
| 7 | Yes | NO.5 | 80 | 10 | PO | |
| 8 | Yes | NO.6 | 80 | 10 | PO | |

### MCAO model making

Mice were fasted overnight before surgery, but did not abstain from water. Mice were preanesthetized in an induction box of an isoflurane gas anesthesia machine at an isoflurane concentration of 2.5%. If the hind paws of the mice had no response when clamped with dissecting forceps, they were transferred to an anesthesia mask, and the isoflurane concentration would be increased to 1.5%. A thermostat and an anal temperature probe were used to maintain intraoperative body temperatures of the mice near 37°C. The neck hair of each mouse in a supine position was shaved, the skin was disinfected with iodophor and alcohol, and an incision was made in the middle of the neck. The tissue was bluntly dissected to expose the common carotid artery (CCA) under a stereomicroscope, and a proximal end of the CCA was ligated with a 6-0 braided thread. The external carotid artery (ECA) and the internal carotid artery (ICA) were dissected upward, a distal end of the ECA was permanently ligated, a distal end of the ECA was temporarily ligated, and the carotid sinus was loosely ligated. The ECA blood vessel wall here was cut off and inserted with a monofilament (Beijing Cinontech Co.Ltd., A5-162020), and the monofilament was fixed by tensioning the loose ligation. The ECA was fused off with an electrocoagulation pen, the ECA ligation was released, and the monofilament was inserted into the ICA until the cerebral blood flow stopped while reaching about 10% of baseline. The middle cerebral artery (MCA) was blocked by the monofilament for 30 min, and the monofilament was withdrawn after 30 min. The vascular stump was cauterized, and the CCA ligation was released. The neck skin was sutured. The mice were placed in an intensive care cage, and the postoperative body temperatures of the mice were maintained at 37°C until materials were drawn.

Measurement of cerebral blood flow: the head of each mouse was fixed under a brain stereotaxic instrument, the head hair of the mouse was shaved, and an incision was made in the middle. The periosteum on the skull was removed. Optical fibers of a laser Doppler flow meter were fixed with glue at coordinates of Bregma AP 1.0 mm and ML 5.0 mm, and the blood flow changes during MCA surgery were recorded. Successful modeling was defined as a decrease in cerebral blood flow to 80% to 90% of baseline.

### Preparation of drug

Vehicle: normal saline
Vehicle: 20% hydroxypropyl-β-cyclodextrin

Preparation of PK sample: firstly, a drug dosage m2 required for one day was calculated according to the total weight of the mice in each group and a delivery dosage. P2 having a dosage slightly more than m2 was weighed and placed in a flask, and a vehicle volume v2 was calculated according to a delivery volume of 10 mL/kg. A 20% hydroxypropyl-β-cyclodextrin solution (v2) was added to the flask, vortexed for one minute, sonicated for 15 min, and then vortexed for another one minute until the solution was clear.
Vehicle: 20% hydroxypropyl-β-cyclodextrin

Preparation of PK sample: firstly, a drug dosage m3 required for one day was calculated according to the total weight of the mice in each group and a delivery dosage. P3 having a dosage slightly more than m3 was weighed and placed in a mortar, and a vehicle volume v3 was calculated according to a delivery volume of 10 mL/kg. A vehicle of v2 was measured with a syringe. Two to three drops of vehicle were added to the mortar, ground for 5 min, added with another two to three drops of vehicle after the solvent was ground to dryness, and then ground for another 5 min. This process was repeated for three times. Then, the compound in the mortar was washed several times with vehicle, then added to a suitable sample bottle using a clean glass dropper, sonicated for 15 min, and then vortexed for 5 min until being homogeneous without larger particles.

### Detection indicators and methods

### Body weight: the body weights of the mice on the day before surgery and 24 h after surgery were recorded.

Neurological function scoring: Longa behavioral scoring was performed at 2 h and 24 h after surgery.

Longa behavior scoring criteria:
Score 0: no nerve damage symptoms;
Score 1: inability to fully extend contralateral forepaws;
Score 2: turn in a circle to the opposite side;
Score 3: incline to the opposite side; and
Score 4: inability to walk spontaneously, loss of consciousness.

### Forelimb grip test

Each mouse was tested for forelimb grip 24 h before surgery and 24 h after surgery. The mouse was placed on a grip meter, and the mouse actively grabbed the grip induction rod. The grip peaks were recorded, repeatedly measured for three times, and averaged.

### Infarct size

The mice were anesthetized with isoflurane 24 h after surgery, then decapitated and sacrificed. The whole brain was peeled off, washed twice with normal saline, and placed under a coronal brain mold. 1 mm of olfactory bulb in the front and 4 mm of cerebellum in the back were removed. The brain was cut into 2 mm slices, a total of 4 slices. The brain slices were incubated with a 1% TTC solution at 37°C in the dark and strained for 20 min, then transferred to 4% PFA, and stored at 4°C in the dark for 48 h before photographing. The volumes of cerebral infarction and edema were measured by software ImageJ. Cerebral edema volume = (injured hemisphere volume - contralateral hemisphere volume)/ contralateral hemisphere volume * 100% Corrected cerebral infarction volume = (contralateral hemisphere volume - (injured hemisphere volume - white infarct area volume))/contralateral hemisphere volume * 100%

### Statistics

Graghpad Prism 7.0 was used for data statistics in each group, and the experimental results were expressed as "mean ± standard deviation". A statistical method used One-way ANOVA to compare the statistical difference between the groups, and P<0.05 had statistical difference.

### Experimental results

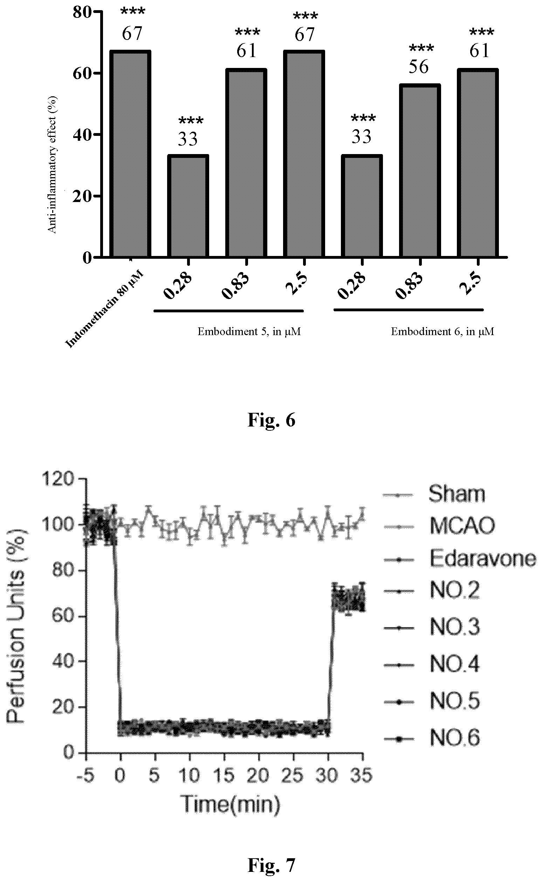

As shown in Fig. 7, in MCAO surgery, the cerebral blood flow in each group (n=3) decreased to about 10% of baseline, indicating that the model was successful and could carry out drug efficacy evaluation.

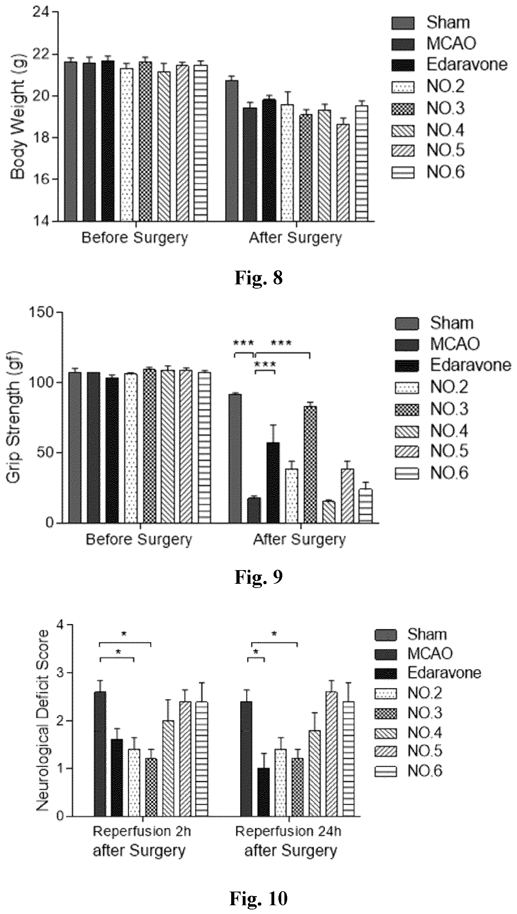

As shown in Fig. 8, the body weight of each mouse in each group decreased after surgery, but there was no significant difference in body weights of the mice in various groups after MCAO surgery.

As shown in Fig. 9, after surgery, the forelimb grip strength of each mouse in various groups decreased significantly, wherein there was a significant difference (P < 0.001) between a postoperative sham surgery group and an MCAO model group, there was a significant difference (P < 0.001) between the MCAO model group and a positive control drug Edaravone group, and there was a significant difference (P < 0.001) between the MCAO model group and an No. 3 administration group.

As shown in Fig. 10, on 2 h after surgery, there were significant differences (P < 0.05) in neurological deficit scores between the MCAO model group and No. 2 and No. 3 administration groups; and on 24 h after surgery, there were significant differences (P < 0.05) in neurological deficit scores between the MCAO model group and the positive control drug Edaravone group as well as the No. 3 administration group.

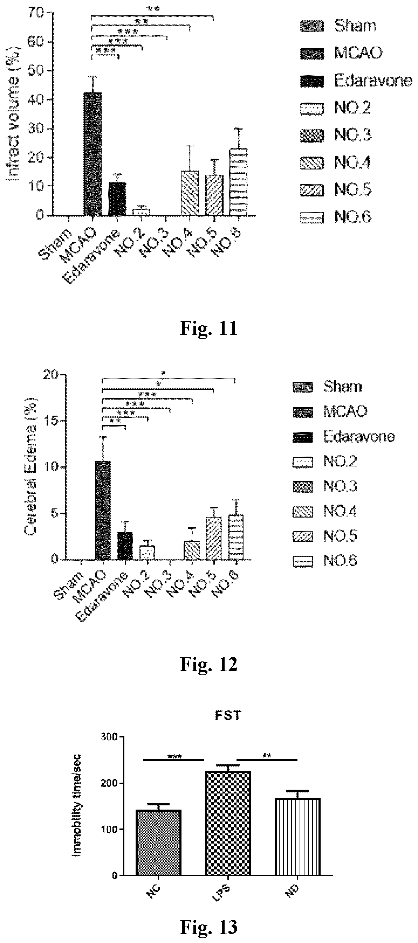

As shown in Fig. 11, the TTC staining results showed that the volume of cerebral infarction in the MCAO model group had significant difference from that of the positive control drug Edaravone group (P < 0.001), the No. 2 administration group (P < 0.001), the No. 3 administration group (P < 0.001), a No. 4 administration group (P < 0.01) and a No. 5 administration group (P < 0.01), respectively.

As shown in Fig. 12, the TTC staining results showed that the volume of encephaledema in the MCAO model group had significant difference from that of the positive control drug Edaravone group (P < 0.001), the No. 2 administration group (P < 0.001), the No. 3 administration group (P < 0.001), a No. 4 administration group (P < 0.01), the No. 5 administration group (P < 0.05) and a No. 6 administration group (P < 0.05), respectively.

### Experiment 6 Evaluation of the effects of Embodiment 6 in an LPS-induced depression-like mouse model

LPS was used to induce the mice to produce peripheral and central inflammations, and then produce depression-like symptoms in the mice, and the changes of depression-related behavioral indicators in the mice were detected after medication. The results showed that Embodiment 6 (ND) had an anti-depression effect on the mice.

**Table 7 Compound to be tested**

| No. | Compound name | Provided by | Appearance | Storage condition |
|---|---|---|---|---|
| 1 | Embodiment 6 (ND) | Shenzhen Qingbo Huineng Pharmaceutical Technology Co., Ltd | White solid powder | 4°C |

Vehicle: sodium carboxymethyl cellulose (NC); name: sodium carboxymethylcellulose; provided by: Meryer; batch: 69881020; appearance: white solid powder; number: 200 g; storage condition: room temperature

Peripheral and central inflammation inducer (LPS) name: lipopolysaccharide; produced by: Sigma; batch: L2880-100MG; appearance: white powder; purity: >97%; specification: 100 mg; storage condition: 2-8°C, in the dark

### Experimental methods Experimental animals and feeding management

### Experimental animal strain: C57BL/ 6J mice; weeks of age: 6-8; sex: male; body weights of animals: 18-20 g; number: 24; experimental animal provider: Guangdong Medical Laboratory Animal Center

### Animal feeding

Quarantine: the quarantine period was 5 days, and the animals exhibiting abnormal behavior were excluded before the experiment.

Animal feeding condition: the experimental animals were fed in a laminar flow cleaning room of the Animal Center of Shenzhen Graduate School of Peking University, with 4-5 animals per cage. A feeding room had a temperature of 22 ± 3°C, and a humidity of 40 -70%, with light alternated with darkness for 12 h. Feed and drinking water: clean-grade mouse diets, purchased from Beijing Keao Xieli Feed Co.,Ltd. The drinking water was autoclaved. Animals were free to eat sterile food and drink water.

Animal serial number: each cage had a cage label indicating the number of animals, sex, strain, time of receipt, group, and start time of the experiment. Animal serial number: each animal was labeled with a separate animal number on the tail.

Animal grouping and treatment: the animals were randomly divided into 3 groups, and the specific grouping and treatments were shown in Table 8.

**Table 8 Animal grouping and drug treatment**

| Group | Number of animals | LPS | Therapeuti c drug | Delivery dosage (mg/kg) | Delivery volume (mL/kg) | Delivery time (days) | Delivery route |
|---|---|---|---|---|---|---|---|
| 1 | 8 | No | Vehicle | - | 10 | QD*3 | PO |
| 2 | 8 | Yes | Vehicle | - | 10 | QD*3 | PO |
| 3 | 8 | Yes | ND | 30 | 10 | QD*3 | PO |

### Establishment of LPS-induced depression-like model

Modeling method: LPS (2 mg/kg, i.p., QD) was injected on Day 1, Day 2 and Day 3 to induce peripheral and central inflammations of mice. An ND drug test group was subj ected to administration pre-protection (ND 30 mg/kg, p.o., QD) 2 h before LPS injection. An experimental group D4 was subjected to forced swimming, tail suspension and sucrose preference experiments.

### Preparation of test drug and model inducer

Vehicle (0.5% CMC-Na solution): 0.5 g of CMC-Na white solid powder was taken, added with 100 mL of double distilled water, and vortexed until the solution was clear. The prepared solution should be stored airtightly in a 4°C freezer. This solution may be stored for one month, but if mold was found, it was forbidden to be used in experiments and needed to be prepared again.

During the test, the compound to be tested and the model inducer were freshly prepared, and the specific preparation method was shown in Table 9:

**Table 9: Preparation of vehicle and test drug**

| Name | Preparation method | Concentrati on (mg/mL) | Storage condition |
|---|---|---|---|
| ND (30 mg/kg) | 18 mg of ND (purity: 99%) was accurately weighed and added with 6 mL of vehicle. The mixture was sonicated and mixed gently by inversion until a homogeneous solution was obtained. | 3 | 4°C |
| LPS (2 mg/kg) | 5 mg of LPS was weighed accurately, added with 25 mL of 0.9% NaCl solution, and shaken well until dissolved. | 0.2 | -20°C |

### Detection indicators and methods

General state observation: living states of animals were observed and recorded.

### Forced swim test (FST)

The mice were placed separately into a clear glass cylinder (diameter: 23 cm; height: 31 cm) which was filled with water to reach a depth of 15 cm, and the temperature was maintained at 24±1°C. The FST lasted for 6 min, during which it was photographed with a high-definition camera. The immobility time of the mice during the test was calculated with professional testing software. The mice were immediately put back into the cages after the test, and kept warm.

### Sucrose preference test (SPT)

The mice were trained prior to the test, i.e., two bottles of 1% (W/V) sucrose solution were placed in each cage, and one of the bottles was replaced with pure water after 24 h. After the acclimatization, the test was carried out after fasting for solids and liquids for 10-24 h. Two pre-weighed water bottles were placed in each cage, one bottle contained a 1% (W/V) sucrose solution, and the other bottle contained pure water. The two water bottles were weighed again after 12 h, and the sucrose consumption and pure water consumption of each mouse were recorded. Sucrose preference index % = sucrose consumption/(sucrose consumption + pure water consumption) × 100%.

### Tail suspension test (TST)

After adapting to an environment, the tail of each mouse was pasted on a suspension rod, and the head of the mouse was about 20-25 cm away from the ground and kept for about 6 min. A high-definition camera was used to take a video, and a behavioral analysis software was used to identify and count the immobility time of the mice. After the experiment, the mice were put back to their cages.

### Statistics

Experimental results were expressed as "mean ± standard deviation". One-way ANOVA was used to compare the data of each group to determine whether there was a statistical difference between the groups.

### Experimental results

### FST

As shown in Fig. 13, the results of the immobility time of the mice in each group during FST showed that the cumulative immobility time in Embodiment 6 (ND) was significantly lower than that in an LPS group. ** , P<0.01; ***, P<0.001,

### SPT

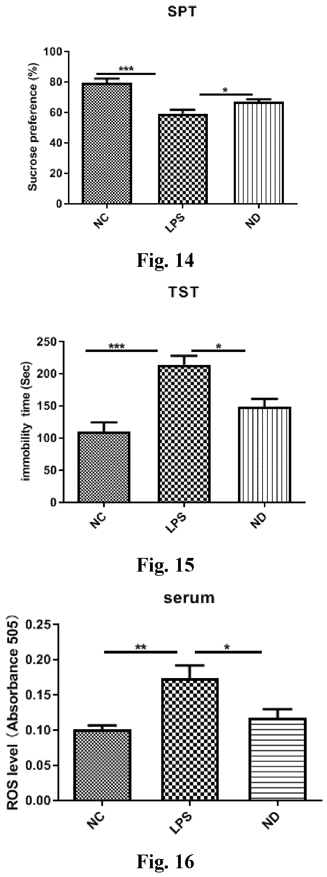

As shown in Fig. 14, the results of sucrose preference rates of the mice in each group during SPT showed that Embodiment 6 (ND) was not significantly improved compared with the LPS group. * , P<0.05; ***, P<0.001

### TST

As shown in Fig. 15, the results of the immobility time of the mice in each group during TST showed that the cumulative immobility time in Embodiment 6 (ND) was significantly lower than that in the LPS group. * , P<0.05; ***, P<0.001

Conclusion: compound Embodiment 6 (ND) exhibited good anti-depression effects in FST and TST experiments.

### Experiment 7 Evaluation of the effects of Embodiment 20A on LPS-induced peripheral and central inflammation mouse model

LPS was used to induce the mice to produce peripheral and central inflammations, and the changes of inflammation and oxidative stress levels in serum and hippocampal tissues of the mice were detected after medication. The results showed that Embodiment 20A (ND) had an obvious anti-inflammatory effect.

### Test product

**Table 10 Compound to be tested**

| No. | Compound name | Provided by | Appearance | Storage condition |
|---|---|---|---|---|
| 1 | Embodiment 20A (ND) | Shenzhen Qingbo Huineng Pharmaceutical Technology Co., Ltd | White solid powder | 4°C |

### Vehicle

Sodium carboxymethyl cellulose (NC); name: sodium carboxymethylcellulose; provided by: Meryer; batch: 69881020; appearance: white solid powder; number: 200 g; storage condition: room temperature.

Peripheral and central inflammation inducer (LPS), name: lipopolysaccharide; provided by: Sigma; batch: L2880-100MG; appearance: white powder; purity: >97%; specification: 100 mg; storage condition: 2-8°C, in the dark.

### Test method

### Experimental animals and feeding management

Experimental animal strain: C57BL/ 6J mice; weeks of age: 6-8; sex: male; body weights of animals: 18-20 g; number: 24. Experimental animal provider: Guangdong Medical Laboratory Animal Center Animal feeding

Quarantine: the quarantine period was 5 days, and the animals exhibiting abnormal behavior were excluded before the experiment.

Animal feeding condition: the experimental animals were fed in a laminar flow cleaning room of the Animal Center of Shenzhen Graduate School of Peking University, with 4-5 animals per cage. A feeding room had a temperature of 22 ± 3°C, and a humidity of 40 -70%, with light alternated with darkness for 12 h. Feed and drinking water: clean-grade mouse diets, purchased from Beijing Keao Xieli Feed Co.,Ltd. The drinking water was autoclaved. Animals were free to eat sterile food and drink water.

Animal serial number: each cage had a cage label indicating the number of animals, sex, strain, time of receipt, group, and start time of the experiment. Animal serial number: each animal was labeled with a separate animal number on the tail.

### Animal grouping and treatment

The animals were randomly divided into three groups, and the specific grouping and treatments were shown in Table 11.

**Table 11 Animal grouping and drug treatment**

| Group | Number of animals | LPS | Therapeuti c drug | Delivery dosage (mg/kg) | Delivery volume (mL/kg) | Delivery time (days) | Delivery route |
|---|---|---|---|---|---|---|---|
| 1 | 8 | No | Vehicle | - | 10 | QD*3 | PO |
| 2 | 8 | Yes | Vehicle | - | 10 | QD*3 | PO |
| 3 | 8 | Yes | ND | 30 | 10 | QD*3 | PO |

### Establishment of LPS inflammation model

Modeling method: LPS (2 mg/kg, i.p., QD) was injected on Day 1, Day 2 and Day 3 to induce peripheral and central inflammations of mice. An ND drug test group was subj ected to administration pre-protection (ND 30 mg/kg, p.o., QD) 2 h before LPS injection. After the experiment, plasma and brain tissues of the mice were collected for inflammatory factors, peroxidation factors and other indicators.

### Preparation of test drug and model inducer

Vehicle (0.5% CMC-Na solution): 0.5 g of CMC-Na white solid powder was taken, added with 100 mL of double distilled water, and vortexed until the solution was clear. The prepared solution should be stored airtightly in a 4°C freezer. This solution may be stored for one month, but if mold was found, it was forbidden to be used in experiments and needed to be prepared again.

During the test, the compound to be tested and the model inducer were freshly prepared, and the specific preparation method was shown in Table 12:

**Table 12: Preparation of vehicle and test drug**

| Name | Preparation method | Concentrati on (mg/mL) | Storage condition |
|---|---|---|---|
| ND (30 mg/kg) | 18 mg of ND (purity: 99%) was accurately weighed and added with 6 mL of vehicle. The mixture was sonicated and mixed gently by inversion until a homogeneous solution was obtained. | 3 | 4°C |
| LPS (2 mg/kg) | 5 mg of LPS was weighed accurately, added with 25 mL of 0.9% NaCl solution, and shaken well until dissolved. | 0.2 | -20°C |

### Detection indicators and methods

General state observation: living states of animals were observed and recorded.

### Animal materials

Serum: after the mice were anesthetized with 1% sodium pentobarbital, the eyeballs were harvested, the blood was taken and placed in an EP tube, and then centrifuged at low temperature after standing at 4°C for 1 h, and a supernatant was taken, and frozen quickly in liquid nitrogen.

Hippocampal tissues: the mice were anesthetized with 1% sodium pentobarbital and then subjected to cardiac perfusion with cold PBS, brains were harvested, and tissues were taken on ice and frozen quickly in an EP tube with liquid nitrogen.

### Determination of oxidative stress-related indicators

Cryopreserved plasma was taken, and an reactive oxygen detection kit, a hydrogen peroxide detection kit and a nitric oxide detection kit were used to detect various indicators. The specific operation processes were the same as the instruction of each kit.

The cryopreserved hippocampal tissues were taken, added with a RIPA tissue lysate containing a protease inhibitor, fully homogenized and cleaved. A supernatant was taken after low-temperature centrifugation. The protein quantification was performed by a BCA protein quantification kit. Tissue samples were adjusted to a unified protein concentration. An reactive oxygen detection kit, a hydrogen peroxide detection kit and a nitric oxide detection kit were used to detect various indicators. The specific operation processes were the same as the instruction of each kit.

### Cytokine assays

Cryopreserved plasma was taken, and IL-1β, IL-6 and IL-10 kits were used to detect various indicators. The specific operation processes were the same as the instruction of each kit.

A hippocampal tissue supernatant was taken, and IL-1β, IL-6 and IL-10 kits were used to detect various indicators. The specific operation processes were the same as the instruction of each kit.

### Statistics

Experimental results were expressed as "mean ± standard deviation". One-way ANOVA was used to compare the data of each group to determine whether there was a statistical difference between the groups.

### Experimental results

### Oxidative stress-related indicators

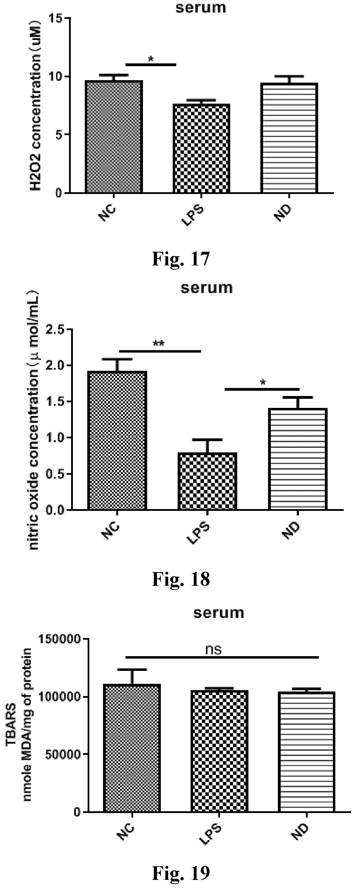

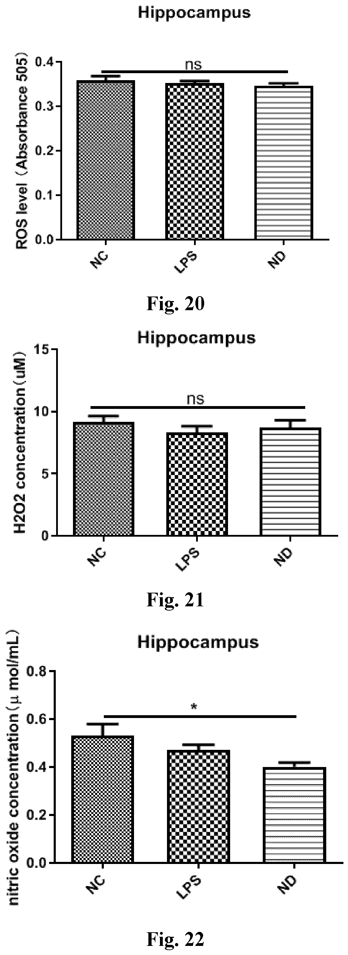

As shown in Figs. 16-23, the detection results of important indicators of oxidative stress in the serum and hippocampus tissues of the mice in each group showed that the ROS level, H₂O₂ concentration and NO concentration of Embodiment 20A (ND) in the serum were significantly improved compared with the LPS group, indicating that Embodiment 20A (ND) had a good antioxidant effect. * , P<0.05;

### ***, P<0.01.

### Inflammatory cytokine indicators

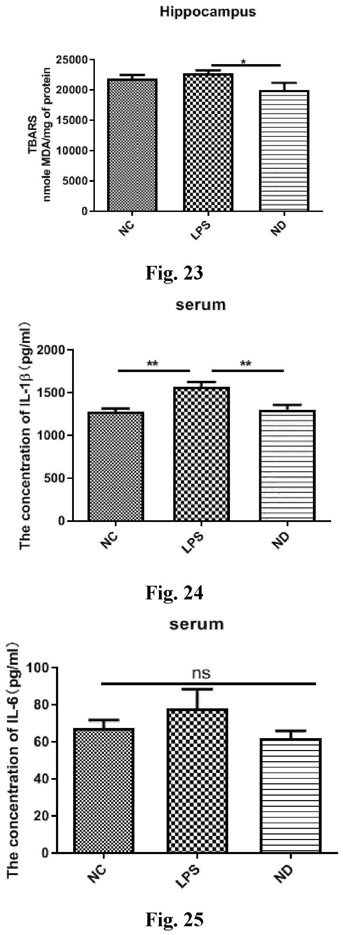

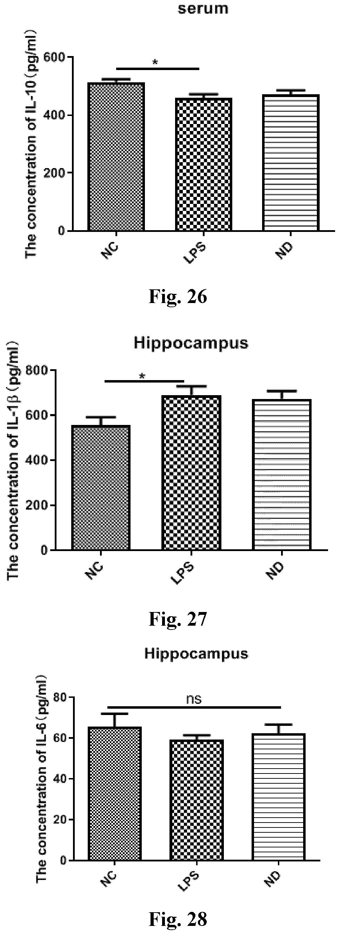

As shown in Figs. 24-29, the detection results of important indicators related to the serum and hippocampus tissues of the mice in each group showed that Embodiment 20A only affected IL-1β in serum and IL-10 in hippocampus, indicating that Embodiment 20A (ND) had a good anti-inflammatory effect. * , P<0.05; ***, P<0.01.

### Experiment 8: Detection of the binding strength of small molecule compounds to mitochondrial respiratory chain supercomplexes

Experimental and analytical method: HEK293 cells were cultured at 5% CO₂, and purified to obtain a mitochondrial respiratory chain supercomplex I1III2IV1. After the respiratory chain supercomplex was chemically covalently coupled to the surface of a metal chip, small molecule compound solutions of different concentrations flowed through the metal chip; the changes in reflection coefficients of a metal surface were monitored by an SPR technology on a Biacore 8K plus analyzer; and then a corresponding response curve graph of small molecule binding was plotted. The binding strength (KD value) of the small molecules to the human mitochondrial respiratory chain supercomplex I1III2IV1 was calculated by built-in fitting software of the Biacore 8K plus analyzer.

Compound treatment: the small molecules as shown were dissolved with ddH₂O and prepared into a 10 mM stock solution. An Embodiment 13B stock solution was then gradiently diluted to 1.5625 µM, 0.78125 µM, 0.390625 µM, and 0.1953125 µM with a 10 mM HEPES (pH=7.4) solution. Similarly, an Embodiment 1A stock solution was then gradiently diluted to 6.25 µM, 3.125 µM, 1.5625 µM, 0.78125 µM, 0.390625 µM and 0.1953125 µM with the 10 mM HEPES (pH=7.4) solution. During determination, 200 µL of each of small molecule solutions of various concentrations was taken and passed through the surface of the metal chip.

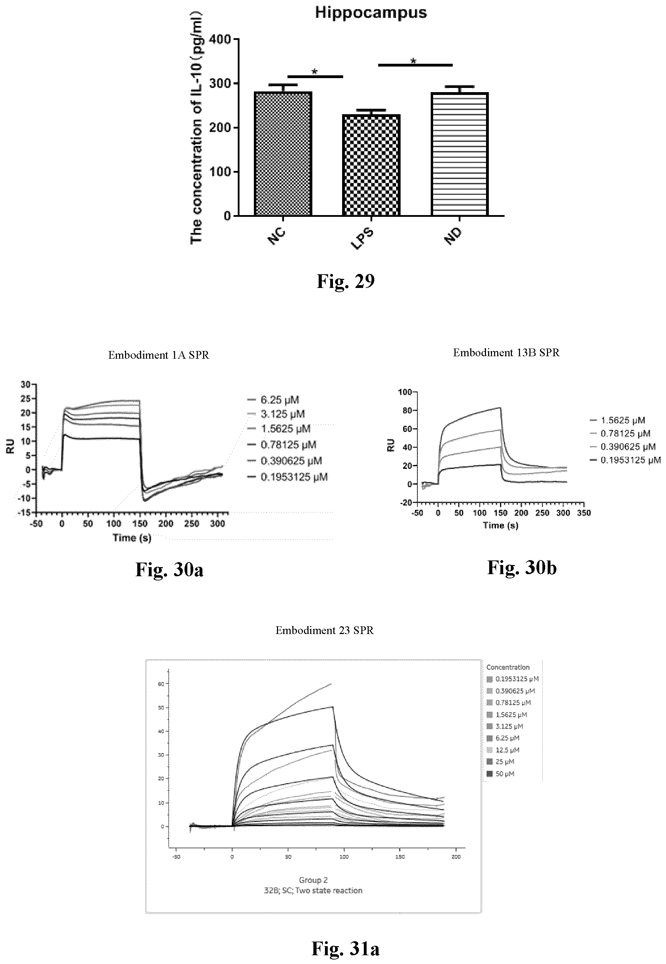

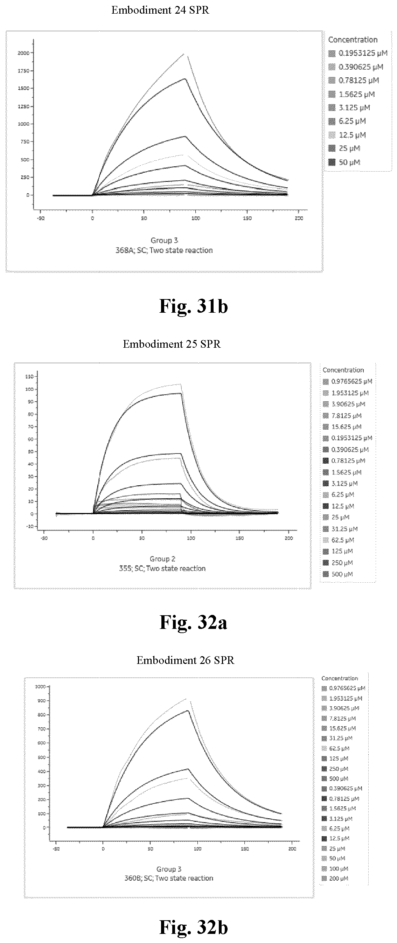

The results were shown in Figs. 30a to 32b. The binding strength of the small molecules to a respiratory chain complex I was achieved at a very high level.

### Experiment 9. Detection of small molecule compounds that facilitated the catalytic activity of SMPs (submitochondrial particles)

Experimental and analytical method: HEK293 cells were cultured at 5% CO₂, and purified to obtain mitocondria, which were sonicated to obtain SMPs (submitochondrial granules). SMPs were incubated with different concentrations of small molecule compounds or a control drug (Rotenone, 2 µM) for 10 min, and then added with 500 µM of NADH. The absorbance of NADH was detected by an Enspire multi-mode microplate reader, an enzyme activity curve with the decrease of NADH concentration catalyzed by SMPs was plotted, and a maximum reaction rate of SMPs was calculated by linear fitting. The addition of 1 mM FeCN to a normal NADH catalytic system of SMPs could cause an electronic pathway from NADH to coenzyme Q to be short-circuited. In a system where FeCN and Rotenone were present, a NADH concentration reduction curve was also determined, and a maximum reaction rate of SMP flavin sites was calculated by linear fitting.

Compound treatment: the small molecules as shown were dissolved with ddH₂O to prepare a 10 mM stock solution, and then the small molecules were gradiently diluted to 0.075 µM, 0.1 µM, 0.133 µM, 0.178 µM, 0.237 µM, 0.316 µM, 0.422 µM, 0.563 µM, 0.75 µM, and 1 µM with a 10 mM Tris buffer solution for an SMP catalysis experiment.

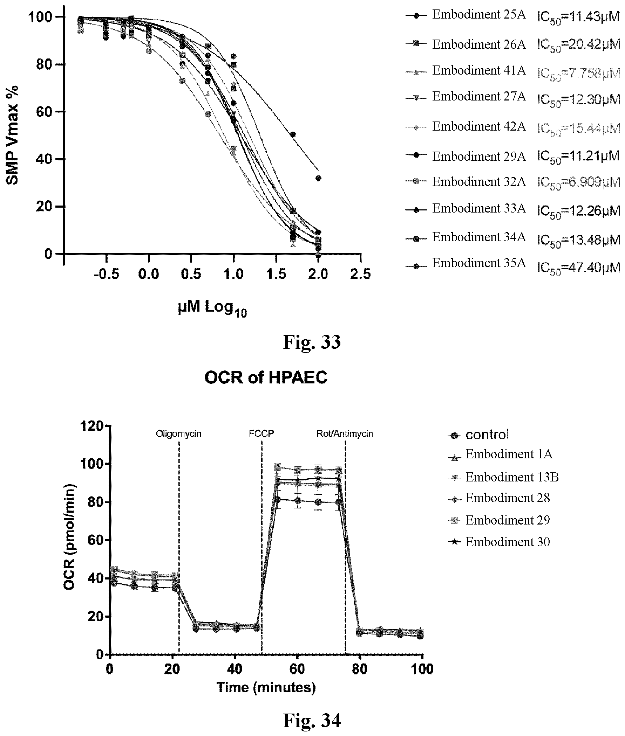

The results were shown in Fig. 33. In a catalysis experiment system, a control group had catalytic activity, Rotenone completely blocked the dehydrogenation reaction of NADH, and the reaction system was effective. The small molecules as shown within 1 µM had no significant effect on the activity of SMPs. The high-concentration small molecules as shown had a greater effect on the activity of SMPs.

### Experiment 10. Detection of small molecule compounds that facilitated mitochondrial stress in HPAEC cells.

Experimental and analytical method: HPAEC cells (human pulmonary artery endothelial cells) were purchased from ATCC and transferred to a Seahorse-specific cell plate after four passages under 5% CO₂ culture conditions, with approximately 20,000 cells per well. After the cells were adherent, the cell oxygen consumption rate (OCR) per well was detected using a Seahorse XFe96 analyzer after 0.5 µM and 1 µM Embodiment 13B were incubated with HPAEC cells for 48 h, and an OCR curve was plotted. After the determination, the total protein content in each cell well was determined by BCA, and an OCR curve was corrected.

The results were shown in Fig. 34. In a resting state (time: 0-20 min),
the five compounds had little effects on cell oxygen consumption. With the addition of an inhibitor oligomycin of complex V, a proton gradient was not depleted, and a high proton gradient inhibited the activity of the complex I, resulting in a decrease in an oxygen consumption rate, but there was little difference in decreasing amplitudes between different small molecules and the control group. With the addition of an uncoupling agent FCCP, the proton gradient dropped sharply, and the oxygen consumption increased significantly. All small molecules had increases in the oxygen consumption rate, which reflected that these small molecules could increase the sensitivity of the complex I in response to the sharp drop of the proton gradient, and an electron transport rate of the complex I could be increased greatly. Finally, Rotenone (a mitochondrial electron transport chain complex Iinhibitor) and Antimycin (a mitochondrial electron transport chain complex III inhibitor) were added to completely block electron transport, such that the oxygen consumption rate was reduced to the lowest value, but the small molecules had no effects on the blocking of electron transport.

### Experiment 11. Detection of small molecule compounds to protect HPAEC cells from COVID-19 Spike-induced ROS production.

Experimental and analytical method: HPAEC cells (human pulmonary artery endothelial cells) were purchased from ATCC and transferred to a MatTek cell imaging dish after four passages under 5% CO₂ culture conditions, with approximately 50,000 cells per bath. HPAEC cells were incubated with Spike (COVID-19 surface spike protein, 8 µg/mL) for 24 h or pre-incubated with Embodiment 13B (0.25, 0.5, 1 µM) for 6 h, and then incubated with Spike (8 µg/mL) for 24 h. Subsequently, mitochondrial staining was performed using MitoSox Red, PKMDR, and Mitotracker Green mitochondrial fluorescent probes, and fluorescence confocal photographs of cells were taken by a Leica stimulated emission depletion super-resolution confocal fluorescence microscope (STED). Subsequently, the fluorescence intensities of cells in different groups were measured with ImageJ. A standard deviation and a standard error were calculated using the obtained data. The P value was calculated by T-test analysis to determine whether there was a significant difference.

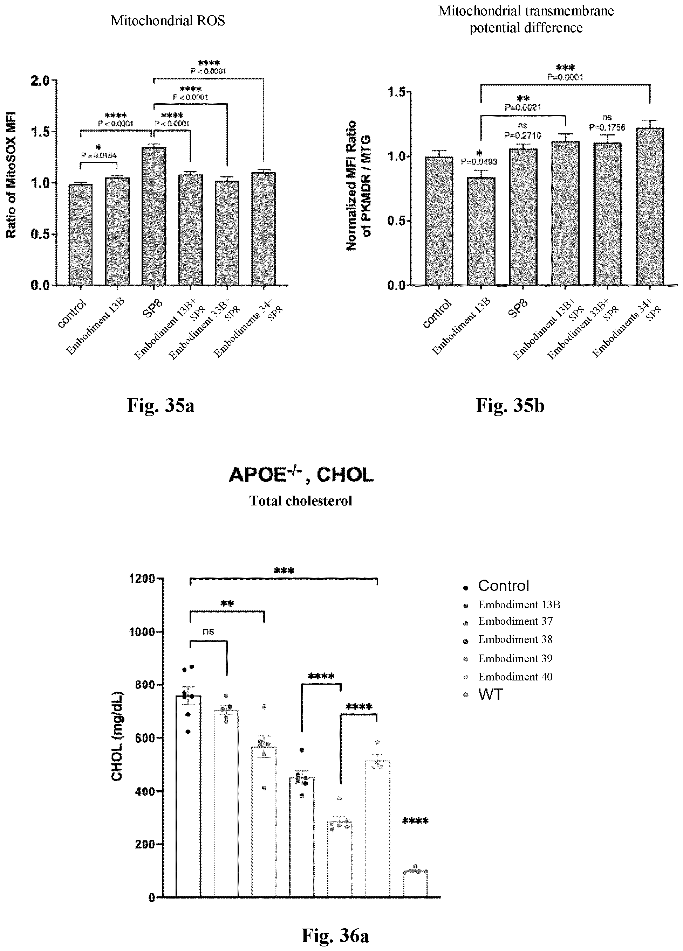

Results: with the addition of Spike to HPAEC cells, the mitochondrial ROS was significantly increased (red fluorescence was brighter) compared with the control group. As shown in Figs. 35a to 35b, the mitochondrial ROS decreased significantly with the addition of Spike after the small molecules as shown protected the mitochondria. A value of PKMDR/MTG was used as an indicator to measure a mitochondrial transmembrane potential difference per unit mass, and the small molecules as shown had protective effects on the mitochondrial transmembrane potential difference.

### Experiment 12. Detection of small molecule compounds to improvement atherosclerosis of APOE mice

Experimental and analytical method: 6-week-old male APOE-/- mutant B6J mice and WT B6J mice were purchased and acclimatized in a feeding room for 2 weeks. When the mice were 8 weeks old, except for a Vehicle group and a WT group, the rest of the groups were given high-fat diets (Research Diets, D12492). When the mice were 9 weeks old, the Control group was given a 10% cyclodextrin solution, while the rest of the administration groups began to be grouped and administrated with different concentrations of small molecule compounds by oral gavage, at a frequency of gavage once every 2 days. When the administration was completed for 1 month, blood was collected from the orbital veins of the APOE mice at a blood collection volume of about 500 µL, and a supernatant plasma was pipetted after centrifugation and inspected for the total cholesterol (CHOL), high-density lipoprotein (HDL) and low-density lipoprotein (LDL) levels in the plasma. After being fed with high-fat diets for 16 weeks, the APOE mice were sacrificed and aortic arches were taken and dissected. The aortic arches of the mice were fixed with 4% paraformaldehyde and then stained with oil red for arterial plaques inside the aortic arches, and the stained arterial arches were imaged by a Zeiss stereomicroscope. An overall area of a longitudinal section of each arterial arch and an area of an orange-red plaque were analyzed by Imaged from captured photos, a proportion of the plaque area was calculated, and the P value was calculated by T-test analysis of the data to determine whether there was a significant difference.

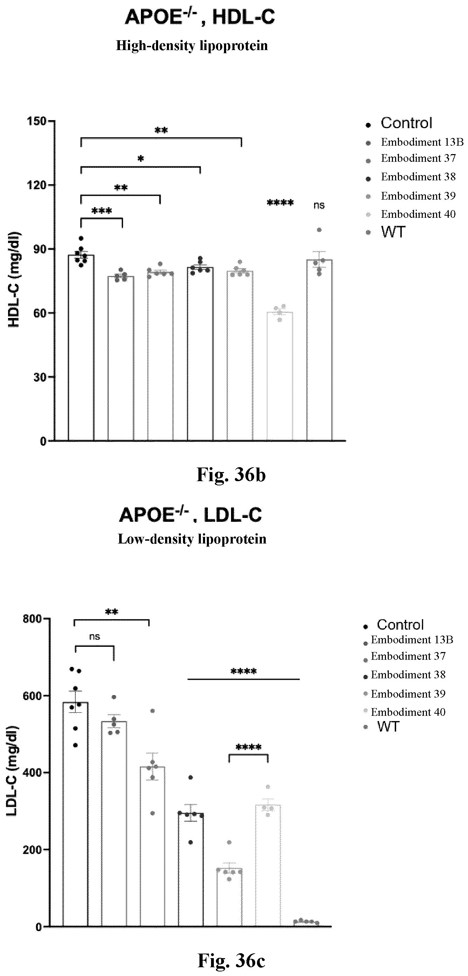

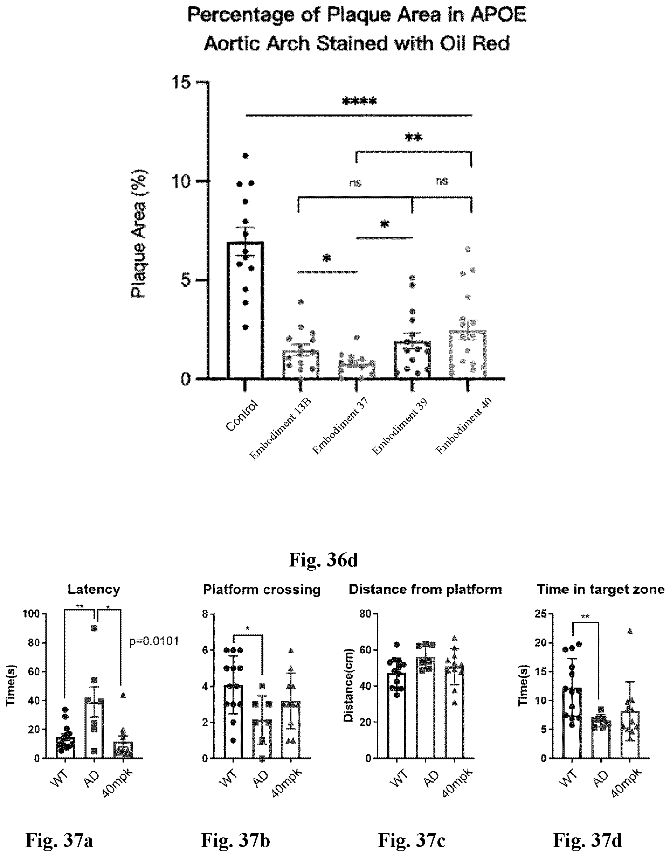

Compound treatment: the small molecules as shown (Figs. 36a to 36d) were dissolved in a 10% cyclodextrin solution to a final concentration of 12 mg/mL, then gradiently diluted with a 10% cyclodextrin solution to 6 mg/mL, 3 mg/mL, and 1.5 mg/mL.

The results were shown in Figs. 36a to 36d: the total cholesterol level of serum in each APOE mouse in the Control group was close to 800 mg/dL after being fed with high-fat diets, while the total cholesterol level of serum in each WT-genotype mouse was only about 100 mg/dL. The total cholesterol levels of the APOE mice administrated with the small molecules as shown were reduced. Meanwhile, the small molecules as shown basically did not affect the HDL of the APOE mice, while the trend of LDL was almost the same as that of the total cholesterol, indicating that the small molecules as shown mainly reduced bad LDL and could maintain beneficial HDL. It was obvious through staining with oil red for the APOE arterial arches that orange-red plaque areas in the administration group were much smaller than that in the Control group, which also had statistically significant difference. The above results showed that the small molecules as shown could reduce serum LDL levels and thus inhibit the occurrence of atherosclerosis.

### Experiment 13. Water maze test of AD rats

Experimental and analytical method: CRISPR Cas9 technology was used to introduce a human APP mutant protein into embryos in SD rats, and a transgenic AD rat model was obtained. Rats of this strain began to exhibit amyloid protein deposition in the brains at 2 months of age and began to exhibit certain behavioral disorders at 5 months of age. In order to verify the therapeutic effect, drugs were administered from 7 months of age to 13 months of age, and the water maze test was done to verify the improvement of spatial memory. The process of the water maze test was divided into three stages: the first stage lasted for 1 day; and in order to adapt to this stage, there was an escape platform having a diameter of 15 cm in a large pool having a diameter of 1.8 m, and there were four marks of circle, triangle, box and fork in four directions of east, west, north and south on the pool wall as hints of spatial positions. The rats were sequentially placed into transparent water along the pool wall, with the water surface 1 cm below the escape platform. The rats would easily find the escape platform, and were then rescued from the water maze after climbing onto the platform, thereby learning basic rules of the water maze. The second stage lasted for 4 days and was a training stage. The settings for the tool remained unchanged, the water surface was raised to 1 cm above the escape platform, and ink was poured to strain the water black, so that the escape platform was not visible. Every day, the rats were put into the pool from the four directions of the east, south, west and north, and rescued after successfully climbing onto the escape platform. If the platform was not found within 90 s, the rats were manually guided to climb onto the platform and rescued after standing on the platform for 15 s. The third stage lasted for 1 day and was an examination stage. The escape platform was taken out, and the rats were still placed once along the pool wall. The rats with strong spatial memory would repeatedly shuttle around the position of the original escape platform. A latency period of the first arrival at the platform, a number of platform shuttles, an average distance from the platform, dwell time in a target quadrant and other indicators were recorded.

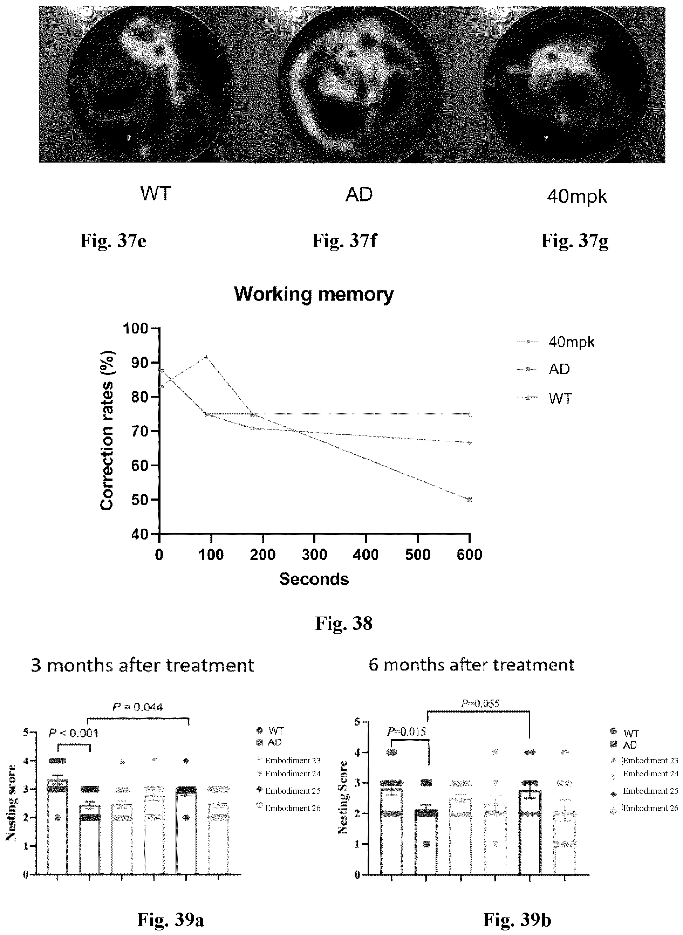

Results: there were three groups of rats, 13 rats in a WT group, 7 rats in a model group (AD group), and 11 mice in an administration group (Embodiment 13B group); and in terms of the latency period of the first arrival at the platform (as shown in Fig. 37a), the number of platform shuttles (as shown in Fig. 37b), the average distance from the platform (as shown in Fig. 37c), the dwell time in the target quadrant (as shown in Fig. 37d) and other indicators, the rats in the administration group showed improved behavioral performances compared to the model group. Figs. 37e to 37g showed heat maps of movement trails of rats in various groups in the pool.

### Experiment 14. T-maze test of AD rats

Experimental and analytical method: CRISPR Cas9 technology was used to introduce a human APP mutant protein into embryos in SD rats, and a transgenic AD rat model was obtained. Rats of this strain began to exhibit amyloid protein deposition in the brains at 2 months of age and began to exhibit certain behavioral disorders at 5 months of age. In order to verify the therapeutic effect, drugs were administered from 7 months of age to 15 months of age, and the T-maze test was done to verify the improvement of spatial memory. The T-maze test had four stages: I. in an acclimatization stage, food was placed in both arms, and valves on both arms were opened. Each rat was trained twice. All rats entered the next stage after they had eaten the food on both sides within 5 min twice. II. In a forced training stage, a valve on one side was closed first, and the rats were allowed to eat the food on the opposite side. A valve on the opposite side was closed again, and the rats were allowed to eat the food on the other side. This was a training process. Each animal was trained 4 times a day for 3 days. III. In an optional training stage, the valve on one side was closed in half first, and the rats were allowed to eat the food on the opposite side. All the valves were then closed. If the rats went to the other side, they could finish eating the food there. If the rats went to the same side, there was no food here, and they were closed on this side for 30 s. This was a training process. Each animal was trained 4 times a day until the accuracy rate exceeded 75% for 2 consecutive days and proceeded to the next stage. IV. In a delayed optional test stage, it was basically the same as the operation of the III stage, except that interval time was added after the forced training stage and before the optional training stage. The interval time was set to 1.5 min, 3 min, and 10 min. Test was performed for 4 times at each time.

Results: there were three groups of mice, 3 mice in a WT group, 2 mice in a model group (AD group), and 6 mice in an administration group (Embodiment 13B group). As shown in Fig. 38, at 1.5 min and 3 min, there was no significant difference between the model group and the administration group, but at 10 min, the working memory of the model group was significantly weakened, while the working memory of the administration group also persisted.

### Experiment 15. Nesting behaviors of AD mice

Experimental and analytical method: in this experiment, transgenic mice from APP/P Embodiment 13B were used, and background mice were C57BL/6J. A phenotype of each model mouse reflected cognitive behavioral changes at 3 months of age, age spots at 5 months of age, and a large number of age spots at 12 months of age. One symptom of AD patients was the inability to take care of themselves, while nesting behaviors reflected the self-care level of the mice. At the beginning of the experiment, 10 pieces of rectangular paper were placed neatly in mouse cages in order. Normal mice would tear up pieces of paper and build nests, while mice with severe AD symptoms would not tear up pieces of paper and build nests. Scoring was based on the tear-up degree of the paper pieces and the integrity of nesting, a higher score indicating a better nesting behavior and a higher level of self-care ability.

Results: drugs were administrated in this experiment from 2 months of age, and a nesting behavior score test was performed 3 months after administration and 6 months after administration. Four small molecules, i.e., Embodiment 23, Embodiment 24, Embodiment 25 and Embodiment 26, were tested. The results were shown in Figs. 39a and 39b, and the nesting behaviors of the model group were significantly weakened compared with a WT group, while the improvement of the nesting behaviors was the most obvious in Embodiment 25.

### Experiment 16. Water maze test of AD mice

Experimental and analytical method: in this experiment, transgenic mice from APP/P Embodiment 13B were used, and background mice were C57BL/6J. A phenotype of each model mouse reflected cognitive behavioral changes at 3 months of age, age spots at 5 months of age, and a large number of age spots at 12 months of age. The process of the water maze test was divided into three stages: the first stage lasted for 1 day; and in order to adapt to this stage, there was an escape platform having a diameter of 8 cm in a large pool having a diameter of 1.2 m, and there were four marks of circle, triangle, box and fork in four directions of east, west, north and south on the pool wall as hints of spatial positions. The rats were sequentially placed into transparent water along the pool wall, with the water surface 1 cm below the escape platform. The mice would easily find the escape platform, and was then rescued from the water maze after climbing onto the platform, thereby learning basic rules of the water maze. The second stage lasted for 4 days and was a training stage. The settings for the tool remained unchanged, the water surface was raised to 1 cm above the escape platform, and ink was poured to strain the water black, so that the escape platform was not visible. Every day, the mice were put into the pool from the four directions of the east, south, west and north, and rescued after successfully climbing onto the escape platform. If the platform was not found within 90 s, the rats were manually guided to climb onto the platform and rescued after standing on the platform for 15 s. The third stage lasted for 1 day and was an examination stage. The escape platform was taken out, and the mice were still placed once along the pool wall. The rats with strong spatial memory would repeatedly shuttle around the position of the original escape platform. The number of platform shuttles and the dwell time in a target quadrant are recorded.

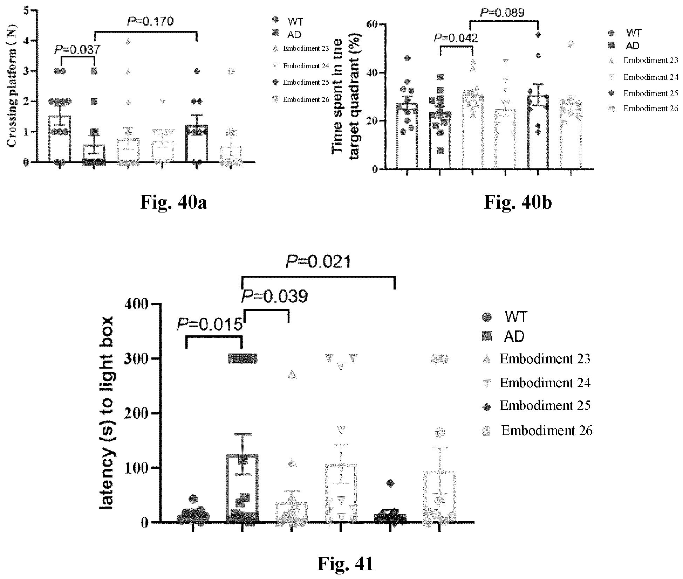

Results: drugs were administrated in this experiment from 2 months of age, and a water maze test was done 6 months after administration to evaluate the spatial memory. Four small molecules, i.e., Embodiment 23, Embodiment 24, Embodiment 25 and Embodiment 26, were tested. The results were shown in Figs. 40a and 40b, the spatial memory capability of the model group was weakened significantly compared to that of the WT group, while the improvement of Embodiment 25 on the spatial memory capability was the most obvious in terms of the number of platform shuttles, the dwell time in a target quadrant and other indicators.

### Experiment 17. Light/dark box test of AD mice

Experimental and analytical method: in this experiment, transgenic mice from APP/P Embodiment 13B were used, and background mice were C57BL/6J. A phenotype of each model mouse reflected cognitive behavioral changes at 3 months of age, age spots at 5 months of age, and a large number of age spots at 12 months of age. The principle of the light/dark box test is: the mice have a natural tendency to darkness; and after the mice was put into a light box, the mice would spontaneously enter a connected dark box. However, in this experiment, the dark box was equipped with an electrical stimulation mechanism, and the mice would be shocked when they entered the dark box. As a result, normal mice would no longer enter the dark box after being shocked several times, while AD mice would continue to enter the dark box because they did not remember to be shocked after entering the dark box. Therefore, after the mice were placed in the light box, a latency period before the mice spontaneously entered the dark box reflected the memory strength of the mice about the electric shock, and the later they entered the dark box, the stronger the memory about the electric shock.

Results: drugs were administrated in this experiment from 2 months of age, and a water maze test was done 6 months after administration to evaluate the spatial memory. Four small molecules, i.e., Embodiment 23, Embodiment 24, Embodiment 25 and Embodiment 26, were tested. Results were shown in Fig. 41. Compared with a WT group, the memory of a model group on electric shock was significantly weakened, and two small molecules (Embodiment 23 and Embodiment 25) could effectively enhance the memory of model mice on electric shock.

### Experiment 18. Survival time of TDP43^{A315T} mice

Experimental and analytical method: this experiment used TDP43^{A315T} transgenic mice, and the mice of this strain would simulate the pathogenesis of amyotrophic lateral sclerosis (ALS). The mice of this strain generally began to die at Day 90 and reached a death median at around Day 120. Our experiments were divided into two groups, i.e., a model group and an administration group. The model group was not administered with drugs, and the administration group was administered with drugs from Day 60, and small molecule Embodiment 13B was tested at a dosage of 40 mpk. There were 20 mice in each group, the date of death and body weight of each mouse were recorded in this experiment, and a body weight curve and a survival curve were plotted.

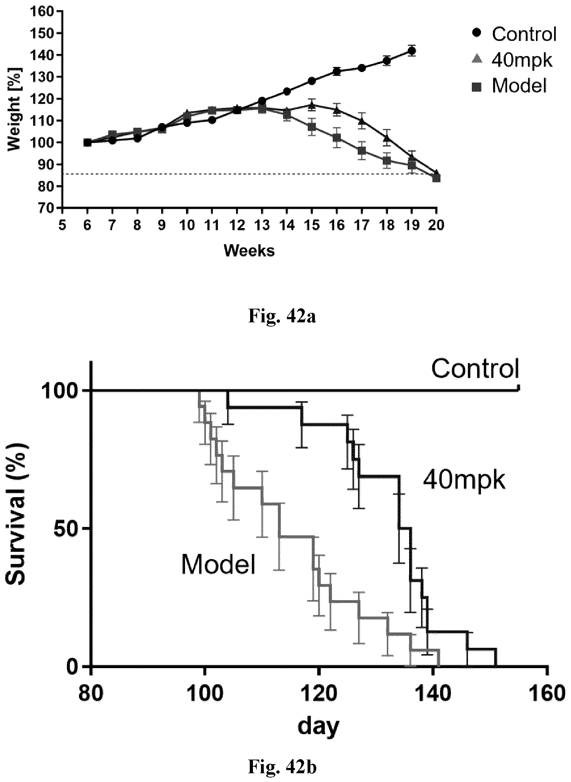

Results: body weight was an important indicator reflecting an ALS course. As shown in Fig. 42a, the body weight of each mouse in the model group began to decrease significantly at Week 13, while the body weight of each mouse in the administration group began to decrease significantly from Week 16. As shown in the survival curve of Fig. 42b, a large number of mice in the model group died from Day 90, reached a death median at about Day 120 and all died at about Day 140. However, the mice in the administration group did not die until Day 100 later, died in a large number after Day 120, reached a death median at Day 135 and all died at Day 150. The results showed that the small molecule Embodiment 13B could effectively prolong the survival time of TDP43^{A315T} transgenic mice, indicating that this small molecule might be effective in prolonging the survival time of ALS patients.

### Experiment 19. Gait analysis of SOD^{G93A} mice

Experimental and analytical method: this experiment used SOD^{G93A} transgenic mice, and the mice of this strain would simulate the pathogenesis of amyotrophic lateral sclerosis (ALS); and with the increase in week age, significant motor dysfunction would occur. Gait analysis was a behavioral analysis method that tested motor dysfunction. The mice with motor dysfunction would have a significant reduction in stride length. Our experiments were divided into two groups, i.e., a model group and an administration group. The model group was not administered with drugs, and the administration group was administered with drugs from Day 60, and small molecule Embodiment 13B was tested at a dosage of 40 mpk. There were 10 mice per group, which were tested at Week 19 for the stride length of each mouse.

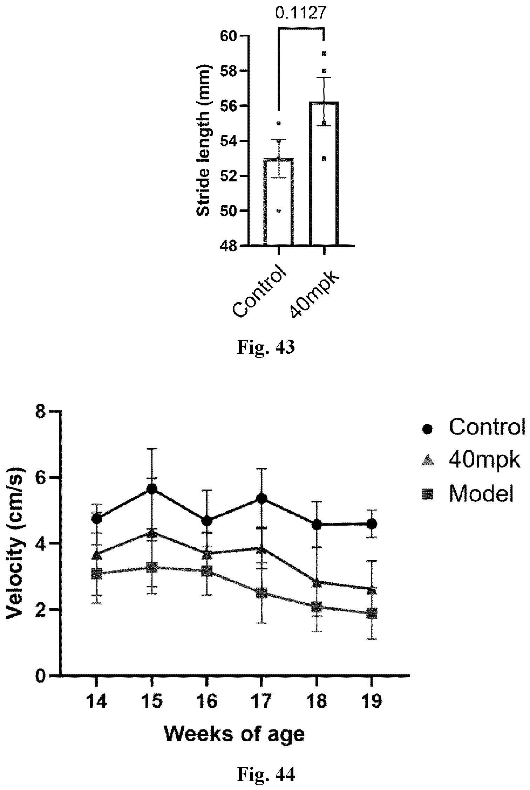

Results: as shown in Fig. 43, the stride length of each mouse in the administration group was much larger than that of each mouse in a model group, indicating that the small molecule Embodiment 13B significantly improved the motor ability of the SOD^{G93A} mice.

### Experiment 20. Open field test of SOD^{G93A} mice

Experimental and analytical method: this experiment used SOD^{G93A} transgenic mice, and the mice of this strain would simulate the pathogenesis of amyotrophic lateral sclerosis (ALS); and with the increase in week age, significant motor dysfunction would occur. A mean moving speed was calculated in an open field, which is a behavioral analysis method to test motor dysfunction. The mice with motor dysfunction would have a significant reduction in mean moving speed. Our experiments were divided into three groups, i.e., a WT group, a model group and an administration group. The WT group was normal mice; the model group was not administered with drugs; the administration group was administered with drugs from Day 60; and the small molecule Embodiment 13B was tested at a dosage of 40 mpk. There were 10 mice per group, which were tested at Week 14 for a mean moving speed of each mouse in an open field on time each week until Week 19.

Results: as shown in Fig. 44, a mean moving rate of each mouse in the administration group was much larger than that in the model group within 6 weeks in test, indicating that the small molecule Embodiment 13B significantly improved the motor ability of the SOD^{G93A} mice.

### Experiment 21. DSS mouse enteritis model

Experimental and analytical method: the purpose of this experiment was to investigate whether test products (Embodiment 13B, Embodiment 33, and Embodiment 34) were administered by oral gavage for multiple times and had a therapeutic effect on ulcerative colitis in mice induced by 3.0% dextran sodium sulfate (DSS). 60 mice in good health were divided into 6 groups according to body weight, with 10 mice in each group: a normal control group, a model group, a positive drug group, and groups of different test products (Embodiment 13B, Embodiment 33, and Embodiment 34, at a dosage of 80 mpk each). In addition to the normal control group, the mice in other groups were administrated with a 3.0% DSS solution to drink freely, and consecutively modeled for 8 days. The normal control group was administrated with autoclaved filtered water to drink freely. The drug was administered on the day of modeling, and a test product was administered by oral gavage according to a designed dosage, once a day for 9 consecutive days. During the administration period, the body weight of each mouse was monitored daily, the feces of mice were observed for shape change and bleeding. On the next day after the last administration, after deep anesthesia with CO2, cardiac blood and plasma were collected, and cryopreserved at -80°C. Subsequently, the abdominal cavity was dissected, the liver was removed and cryopreserved at -80°C. Finally, the entire colon tissue was removed, the colon length was measured, photographs were taken, and the colon content was washed with normal saline after dissection along the mesenteric side and weighed. Some of diseased tissues were fixed with 10% formalin, and subjected to histopathological observation by HE staining; and the rest of the colon tissues were cryopreserved at -80°C.

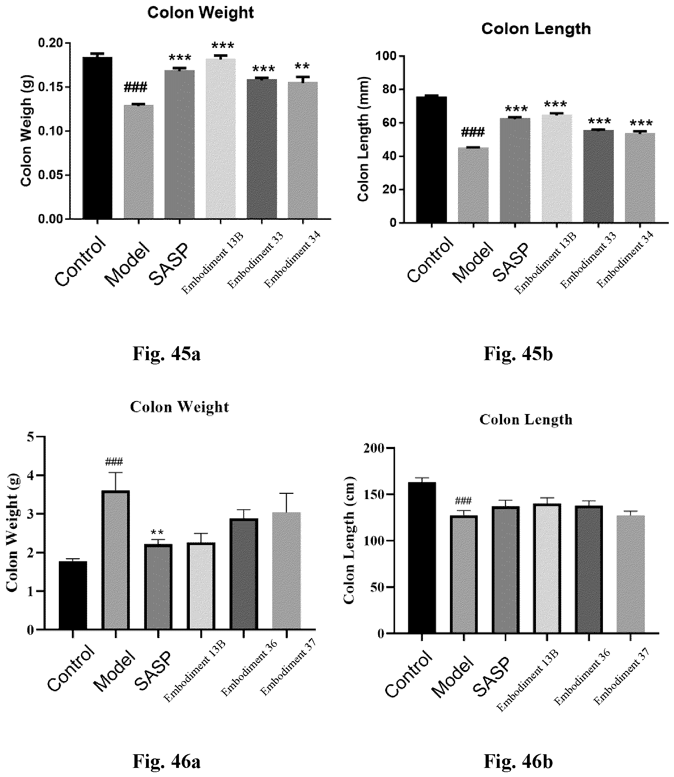

Results: (1) the effects on general conditions of DSS colitis mice were shown in Fig. 45a: after 4 days of modeling with 3.0% DSS, modeled animals successively showed abnormal conditions such as loose stool, bloody stool, and weight loss, and then gradual aggravation; and the mice in the model group had symptoms of reduced diet, tiredness, emaciation, erect hair, and dull hair. A 720 mg/kg positive drug SASP and test products (Embodiment 13B, Embodiment 33, and Embodiment 34) had certain improvement effects, but there were still abnormal conditions such as weight loss, loose stool and bloody stool to the end point of the experiment. (2) The effects on colon weight and length of mice with DSS colitis were shown in Fig. 45b: the colons of mice atrophied after drinking a 3.0% DSS solution freely, manifested as colon length shortening and weight loss; both the positive drug and the test products could inhibit colon atrophy in the mice; and the test products (Embodiment 13B, Embodiment 33, and Embodiment 34) could increase the weights and lengths of the colons of DSS mice, and inhibit colon atrophy in the mice.

### Experiment 22.TNBS rats enteritis model

Experimental and analytical method: the purpose of this experiment was to investigate whether test products (Embodiment 13B, Embodiment 36, and Embodiment 37, at a dosage of 80 mpk each) which were administered by oral gavage for multiple times had therapeutic effects on ulcerative colitis induced by 2,4,6-trinitrobenzenesulfonic acid (TNBS) in rats. All rats except the normal control group were fasted for solids (not liquids) for 24 h. After being anesthetized with isoflurane, the rats were transrectally infused with a TNBS ethanol solution (18 mg TNBS/mouse) at 0.5 mL/mouse through a latex hose, the length of the hose into the rectum was about 8 cm, an anesthesia state was continued after the hose was withdrawn, and the rats were kept in an inclined state for 15 min. The rats in the normal control group were not stimulated with rectal cannula. After the animals were awake, they were randomly divided into groups, which then began to be administrated with drugs (marked as D1). The blank group and the model group were administrated with the same amount of vehicle by oral gavage once a day for 7 consecutive days (D1-D7). The animal's status and feces (loose stools or bloody stools) were observed every day. The body weights were monitored. On the next day after the last administration (D8), the animals were deeply anesthetized with CO2, sacrificed and dissected. The entire colon tissues were harvested. The colon content was washed with normal saline after dissection along the mesenteric side. The body weights were measured and the colon lengths were measured. A ulcer surface was measured by Image J software. The general conditions of the colons were observed and photographed. Some of diseased tissues were fixed with formalin, and subjected to histopathological observation by HE staining.

Results: (1) the effects on general conditions of TNBS colitis mice were shown in Fig. 46a: the rats had loose stool on the next day of TNBS modeling; and the modeled mice had symptoms of reduced diet, tiredness, emaciation, erect hair, and dull hair. The feces of some rats were improved in the later stage of administration, but some rats still maintained loose stool until the end of the experiment, and some rats had obvious abdominal distension in the later stage of the experiment. (2) The effects on colon weights and lengths of TNBS-induced colitis rats were shown in Fig. 46b: it can be seen by summarizing the results of colon weight (colon weight/colon length) and colon ulcer area of each rat per unit length that test products (Embodiment 13B, Embodiment 36, and Embodiment 37) could reduce the colon weight and colon ulcer area of each rat per unit length, wherein the effects of Embodiment 13B were more significant.

### Experiment 23. Reduction in blood glucose of DB mice

Experimental and analytical method: leptin receptor gene (OB-R) was also known as diabetes gene (db), leptin receptor (Lepr) is closely related to obesity, hypertension, diabetes, lipid metabolism disorders, etc. The courses of these diseases are greatly influenced by the genetic background. Topical insulin can not control the increase in blood glucose and gluconeogenesis levels in the liver. Gene editing technologies and embryo injection technologies were used to construct Lepr gene mutant mice. Blood glucose monitoring of homozygotes of this strain showed significant differences in blood glucose levels compared to a wild control, which could be used for researches of type II diabetes. The blood glucose in each mouse of this strain began to rise sharply at 8 weeks of age, reaching 30 mmol/L, while the blood glucose of the normal background was only about 6 mmol/L. We started drug delivery from 8 weeks of age. Test small molecules were Embodiment 28, Embodiment 29 and Embodiment 30, all of which were at a dosage of 80 mpk, and delivered by gavage once a day. 20 mice in each group, starting at 8 weeks of age, were monitored weekly for blood glucose until they were sacrificed at 20 weeks of age.

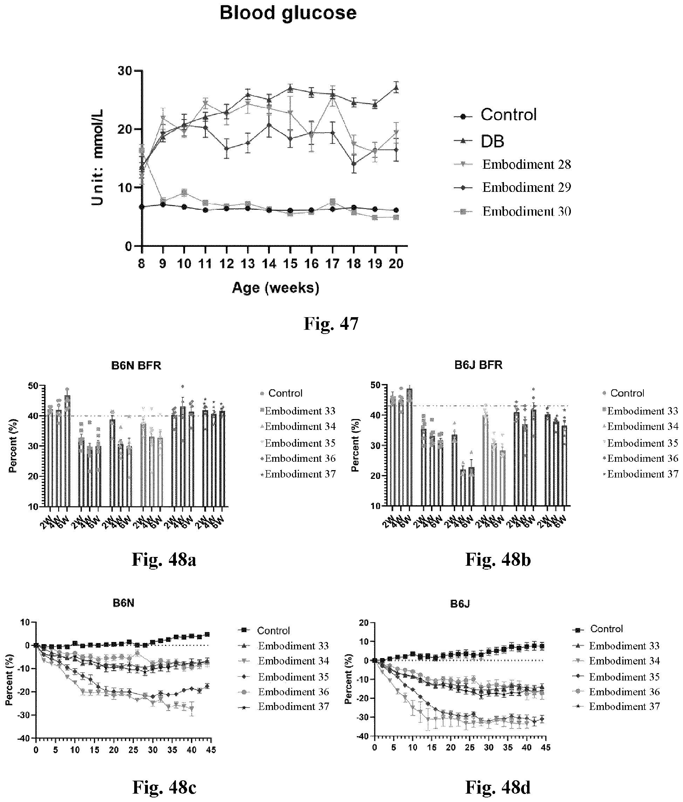

Results: the results were shown in Fig. 47 in which both Embodiment 28 and Embodiment 29 had certain blood glucose-reducing effects, but Embodiment 30 had the best blood glucose-reducing effect and could reach the level of normal background mice.

### Experiment 24. Decreases in body weights and body fat rates of DIO mice

Experimental and analytical method: the DIO model was a model of obesity caused by high-fat diets. In this experiment, two strains of mice, C57BL/6J and C57BL/6N, were fed with high-fat diets containing 60% of fat. At the age of 7 weeks, the mice were modeled with high-fat diets, and continued to be fed with high-fat diets for 5 months, till the body weight of each mouse reached more than 50 g. The mice begun to be administrated with the drug, and the body weights were recorded weekly. A body fat rate was determined every two weeks using an Echo MRI small animal body composition analyzer. We tested weight loss effects of 5 small molecules, i.e., Embodiment 33, Embodiment 34, Embodiment 35, Embodiment 36, and Embodiment 37, at a dosage of 80 mpk each, which were administrated by gavage once every 2 days.

Results: as shown in Figs. 48a to 48d, the weight loss effects of Embodiment 34 and Embodiment 35 were the best, so that the body weight of an obese model mouse could be reduced from more than 50 g to about 35 g of normal mice. In addition, Embodiment 33, Embodiment 36 and Embodiment 37 also had weight loss effects, and the weights could be reduced to about 40 g. From the perspective of body fat rate, all small molecules could effectively burn the fat in the bodies of the mice and reduce the body fat rate, while Embodiment 34 and Embodiment 35 still had the best fat-burning effect, and Embodiment 33, Embodiment 36 and Embodiment 37 second.

### Experiment 25. NOD-Scid immunodeficient mice were inoculated with hematologic tumor cells Raji-Luc

Experimental and analytical method: an objective of this experiment was to test in-vivo killing effects of six small molecules on hematologic tumors. In this experiment, eighty 8-week-old female NOD-Scid immunodeficient mice were used as Raji-Luc cell inoculation mice, and in-vivo fluorescence imaging was carried out on Day 9 after inoculation, and sixty mice having the most concentrated mid-segment fluorescence intensity values were selected for follow-up experiments according to the fluorescence intensity. After first in-vivo imaging, administration was started once a day by gavage at 100 mpk. The tested small molecules were Embodiment 25A, Embodiment 25B, Embodiment 28, Embodiment 38, Embodiment 39, and Embodiment 40, 10 mice in each group. In-vivo imaging was performed twice weekly after initiation of administration to observe the development of whole blood tumors in mice.

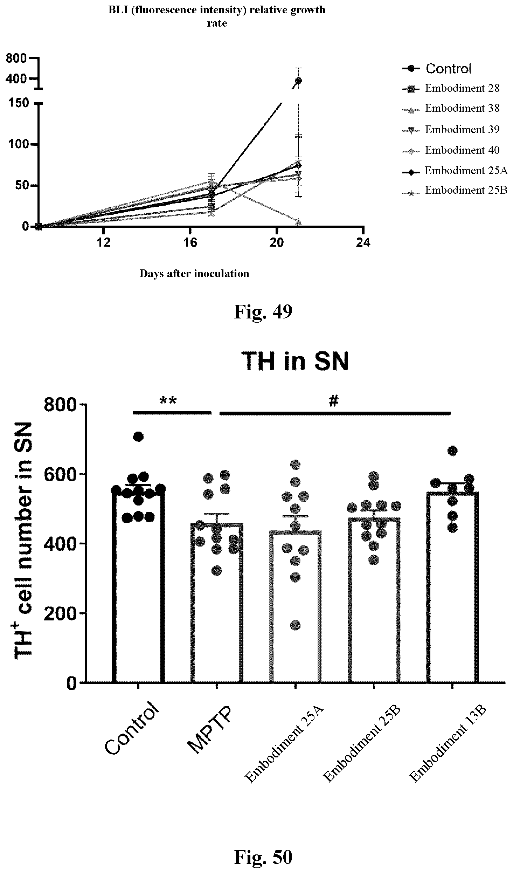

Results: as shown in Fig. 49, the fluorescence intensity was very high in the control group and significantly decreased in the administration group. The two groups of Embodiment 28 and Embodiment 38 had excessive toxicity and all died on Day 21, and there was no fluorescence value. The other Embodiment 25A, Embodiment 25B, Embodiment 39 and Embodiment 40 had relatively good tumor killing effects.

### Experiment 26. Cell proliferation assays for small molecule compounds

Experimental and analytical method: cells were inoculated into a 96-well cell culture plate with a lid at a maximum concentration of 10 µM for a test compound, and diluted in three folds to a series of test concentrations, and cells were incubated at 37°C, 5% CO2 for 72 h. The concentration of DMSO was 0.5%. 100 µl of medium was taken from each well. 100 µl of reagent (Celltitute Glo Assay Kit) was added to each well, and a flat plate was shaken on a plate shaker (protected from light) for 2 min. A culture plate was incubated (protected from light) at room temperature for 30 min. Chemiluminescence values were recorded by a multi-plate plate reader, and the experimental results were as follows:

**Table 13 Inhibition of embodiment compounds on the growth of lymphoma cells in vitro**

| **Compound** | **U937 (IC50, uM)** | | **OCI-LY3** | **U2932** | **HT** |
|---|---|---|---|---|---|
| | **Low sugar** | **Normal sugar** | **(IC50, uM)** | **(IC50, uM)** | **(IC50, uM)** |
| **Embodiment 1A** | 1.7997 | 2.1344 | 1.9 | 0.62 | 1.645 |
| **Embodiment 2A** | 1.4849 | 2.0239 | - | - | - |
| **Embodiment 13** | 2.2765 | 4.0439 | 4.4 | 1.19 | - |
| **Embodiment 13A** | - | - | 1.78 | 0.43 | 1.5063 |
| **Embodiment 13B** | - | - | 4.39 | 0.72 | - |

U937 (histiocytic lymphoma cells), OCI-LY3 (human diffuse large B-cell lymphoma cells), U2932 (human diffuse large B-cell lymphoma cells), and HT cells (human mixed lymphoma cells) were analyzed in the table for experimental analysis of cell proliferation. The table showed that the compounds of the present invention had inhibitory effects on lymphoma cell proliferation. Embodiment 2A had the best inhibitory activity against U937, and Embodiment 13A had the best inhibitory effect on the proliferation of OCI-LY3 cell line and HT cells. In the table, " " indicated that there was no measured data.

### Experiment 27. PD mouse model

Experimental and analytical method: mice of C57BL/6 strain were used in this experiment, which were divided into 5 groups, including a normal control group (no injection of MPTP), a model group (modeling with MPTP), and treatment groups of three small molecules (Embodiment 13B, Embodiment 25A, and Embodiment 25B). The drug was administered at the same time as modeling was made. The modeling lasted for 5 days, followed by 30 mpk intraperitoneal injection till lasting for 15 days, and lasted for 15 days at 80 mpk. At the endpoint, the brain was soaked and fixed in PFA after cardiac perfusion, and transferred to sucrose dehydration after complete fixation, and OCT embedding and sectioning were used for TH staining after complete dehydration. The detection indicator was the number of TH-positive cells in the substantia nigra. The more positive cells, the better the protection effect of small molecules against MPTP damage.

Results: there were 12 mice in each group, and the statistical results were shown in Fig. 50, T-test, **: p<0.01, #: p<0.05. Compared with the model group, only the Embodiment 13B group of three small modules had statistically significant differences, indicating that Embodiment 13B had a good effect of protecting substantia nigra neurons.

### Experiment 28. SARS-CoV-2 pseudovirus coronavirus infection cell model

Experimental and analytical method: HEK293T cells were first used to culture SARS-CoV-2 pseudovirus, which only had the ability to infect but not to replicate. After the pseudovirus in a supernatant was collected, the pseudovirus infection was performed using normal HEK293T cells with ACE2 receptors on the surfaces as an infection object. After viral infection, the mitochondria in the HEK293T cells would be fragmented, which could be monitored by mitochondrial fluorescence staining (mitotracker red), indicating that the cells were destroyed by the pseudovirus, referred to as a model group. In the experimental group, 300 nM Embodiment 13B was used to incubate with cells for 1 h in advance before pseudovirus infection, in order to test a protective effect of the small molecule Embodiment13B against virus infection.

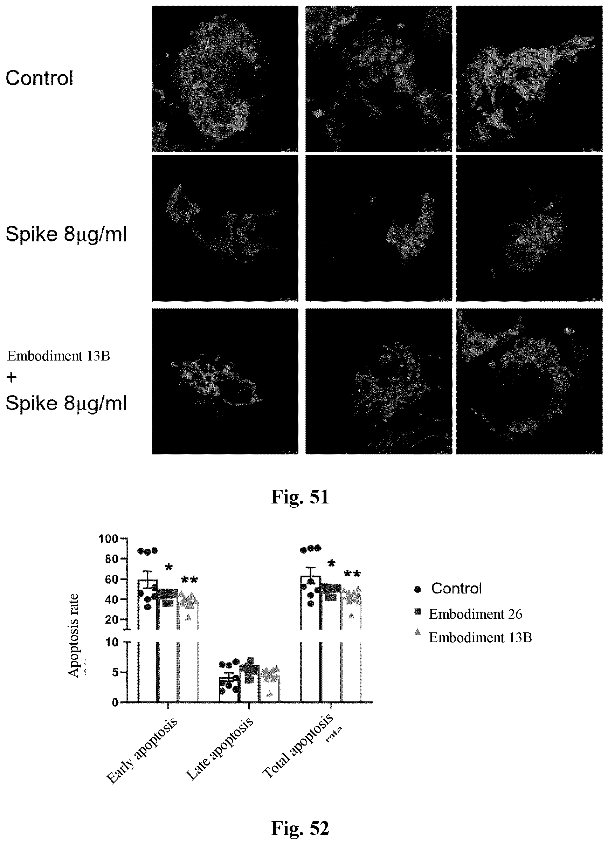

Results: the results were shown in Fig. 51; compared with the normal control group (no pseudovirus infection), the mitochondria in the cells of the model group were fragmented obviously, while the mitochondria in the cells of the experimental group were not fragmented, indicating that the small molecule Embodiment 13B had a good protective effect against the infection of SARS-CoV-2 pseudovirus.

### Experiment 29. Acute traumatic brain injury (TBI) rat model

Experimental and analytical method: male SD rats were used in this experiment to establish a TBI rat model, which was randomly divided into: a solvent control group (8 rats), a test product Embodiment 13B (80 mpk, 10 rats), and a test product Embodiment 26 (80 mpk, 9 rats). After TBI surgery, drugs were administered by gavage once a day for 7 consecutive days. At the endpoint of this experiment (Day 7), a brain tissue was collected to detect the percentages of apoptosis in the hippocampus and injured adjacent areas.

Results: flow cytometry results were shown in Fig. 52, and early brain apoptosis rates and total apoptosis rates of rats in the test product Embodiment 13B (80 mpk) and test product Embodiment 26 (80 mpk) groups were significantly lower than those in the solvent control group (P<0.05). Under this experiment system, multiple intravenous administrations of the test product Embodiment 13B and Embodiment 26 could significantly improve the neurological impairment and apoptosis of brain cells in TBI rats with acute TBI, and promote the recovery of motor ability and neurological functions.

### Experiment 30. In-vitro killing effects on pancreatic cancer cells

Experimental and analytical method: we used Mia paca-2 cells as a research object. This cell line was a pancreatic cancer cell line and was widely used in an antimicrobial susceptibility experiment. We performed the antimicrobial susceptibility experiment on 11 small molecules, with a maximum concentration of 10 uM and a 3-fold dilution, resulting in a concentration gradient of seven points. Cell viability assay was performed using CellTiter-Glo, and a dosage-response curve was plotted using GraphPad Prism. A resulting antimicrobial susceptibility curve was shown in the figure below.

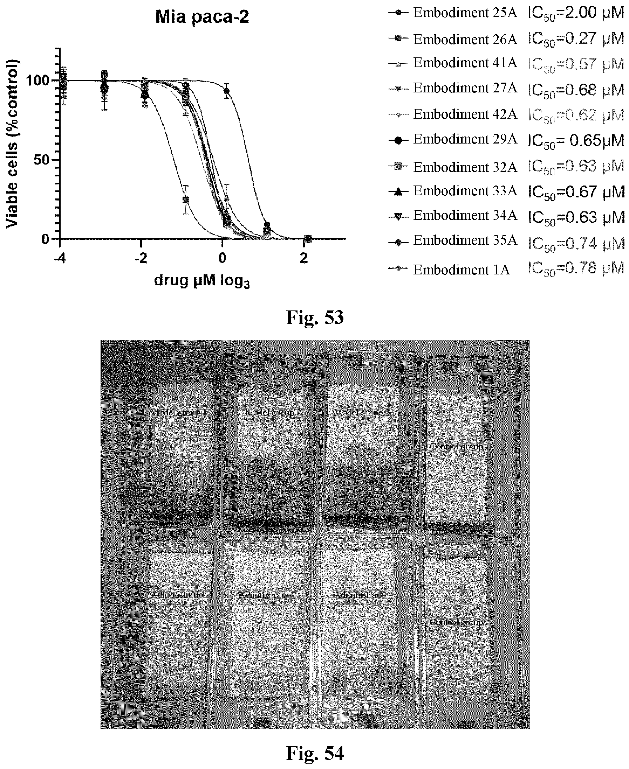

Results: the antimicrobial susceptibility curve was shown in Fig. 53, where a lower IC50 value indicates a higher sensitivity to the small molecule. It can be seen that Mia paca-2 cells had the highest sensitivity to Embodiment 26A.

### Experiment 31. Inhibition of excessive urination in DB animals

Experimental and analytical method: leptin receptor gene (OB-R) was also known as diabetes gene (db), leptin receptor (Lepr) is closely related to obesity, hypertension, diabetes, lipid metabolism disorders, etc. The courses of these diseases are greatly influenced by the genetic background. Topical insulin can not control the increase in blood glucose and gluconeogenesis levels in the liver. Gene editing technologies and embryo injection technologies were used to construct Lepr gene mutant mice. Blood glucose monitoring of homozygotes of this strain showed significant differences in blood glucose levels compared to a wild control, which could be used for researches of type II diabetes. The urine volume of each mouse of this strain was as huge as hundreds of times that of normal mice, so patients with frequent urination could be simulated. The experiment was divided into three groups, i.e., a model group (non-administrated, onset mice with high urine volume), a control group (non-administrated, non-onset normal mice), and an experimental group (administrated Embodiment 1A, 80 mpk, by gavage once every two days), with 2 mice in the control group, and 3 mice in the other two groups. The small molecule used was Embodiment 1A. We observed the urine volume in cages of mice housed in single cages starting from Week 8 and after 5 weeks of continuous drug treatment. The larger the urine wetting area in the cage, the greater the urine volume of mice in that cage.

Results: as shown in the cage photo of Fig. 54, an urine wetting area in a mouse cage of a three-cage model group was the largest, and a urine wetting area in a mouse cage of the control group was almost invisible, while the urine wetting area in a mouse cage of the administration group was significantly reduced compared with that of the model group. It was shown that Embodiment 1A had a function of inhibiting frequent urination.

### Experiment 32. Cardiovascular inflammation alleviation effect on APOE mutant mice fed with high-fat diets.

Experimental and analytical method: 6-week-old male APOE-/- mutant B6J mice and WT B6J mice were purchased and acclimatized in a feeding room for 2 weeks. When the mice were 8 weeks old, except for a Vehicle group and a WT group, the rest of the groups were given high-fat diets (Research Diets, D12492). When the mice were 9 weeks old, the Control group was given a 10% cyclodextrin solution, while the rest of the administration groups began to be grouped and administrated with different small molecule compounds (Embodiment 13B, Embodiment 33, Embodiment 36 and Embodiment 37) by oral gavage, at a frequency of once every 2 days. After being fed with high-fat diets for 16 weeks, the APOE mice were sacrificed and aortic arches were taken and dissected. MSD multi-factor detection was performed on an aortic vascular epithelial tissue at a fixed mass of 100 mg/mouse to determine the expression quantities of various inflammatory factors, including IL 10, IL-1β, and KC/GRO.

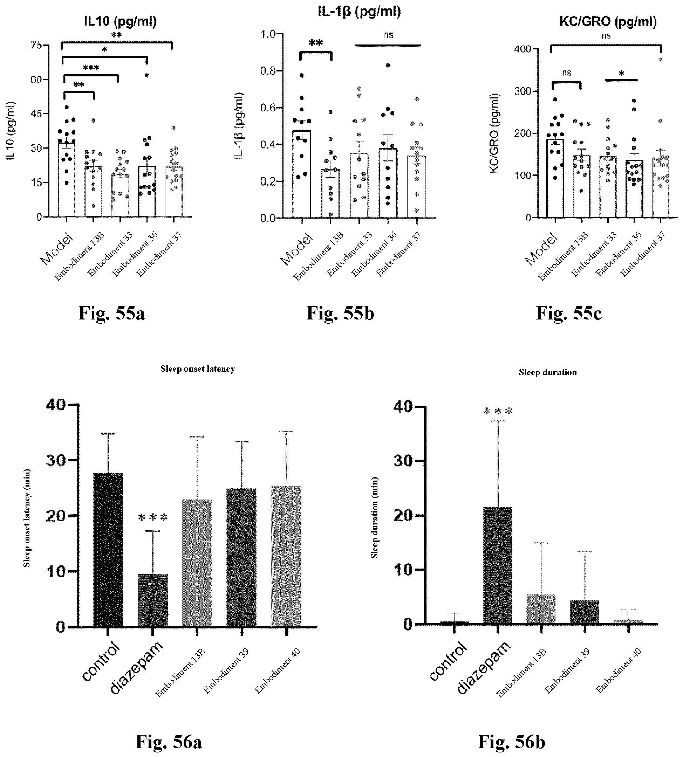

Results: as shown in Figs. 55a to 55c, three types of inflammatory factors all had a downward trend under the action of small molecule intervention, and the decrease in Embodiment 13b was the most significant, indicating that the small molecule shown had an anti-cardiovascular inflammatory effect.

### Experiment 33. Efficacy experiment for the effect of Embodiment 13B at a subthreshold hypnotic dosage of sodium pentobarbital on the sleep of mice

Experimental and analytical method: by detecting the effects of Embodiment 13B by single oral gavage administration on the sleep onset time (sleep latency) and sleep duration of ICR mice induced by subthreshold hypnotic dosage of sodium pentobarbital, whether a test product had a hypnotic effect was investigated, and the pharmacodynamic comparison was carried out between the test product and a positive control drug (Diazepam). After adaptive feeding of mice, 50 qualified animals were selected according to their body weights and divided into 5 groups by Excel complete randomization: a blank control group, a positive control group (Diazepam), and high-, medium- and low-dosage groups of test product, with 10 animals in each group. Animals in each group should be administrated with drug solutions and vehicles. After 1 h of administration, the animals in each group were intraperitoneally injected with a maximum subthreshold dosage of sodium pentobarbital (firstly, a pre-experiment was conducted to determine a maximum subthreshold hypnotic dosage of sodium pentobarbital). The sleep onset time (sleep onset latency), the number of animals falling asleep within 30 min (those with righting reflex disappearing for more than 1 min) and the sleep duration of each mouse were recorded.

Results: as shown in Figs. 56a and 56b, 1 mg/kg Diazepam significantly shortened the sleep latency of the mice. Compared with the blank control group, the small molecules as shown could shorten the sleep latency of the mice, which had a certain dosage-response relationship, but no statistically significant difference. In addition, the effect of the small molecule in shortening sleep latency of the mice was significantly weaker than that of Diazepam at 1 mg/kg. Compared with the blank control group, the positive drug group was administrated with Diazepam at 1 mg/kg, which significantly prolonged the sleep time of the mice. Compared with the blank control group, the small molecule as shown could prolong the sleep time of the mice, but there was no statistically significant difference. In addition, the effect of the small molecule in prolonging the sleep time of the mice was significantly weaker than that of Diazepam at 1 mg/kg.

In this specification, the present invention has been described with reference to its specific embodiments. However, it is obvious that various modifications and transformations can still be made without deviating from the spirit and scope of the present invention. Therefore, the specification should be considered as illustrative but not restrictive.

## Claims

1. A use of derivatives based on sarsasapogenin structure in preparation of drugs for treating related diseases caused by mitochondrial dysfunction, **characterized in that** the structural formula of the derivatives is represented by general formula I, 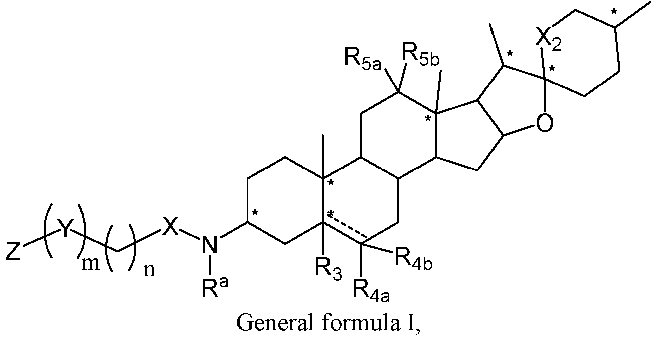 the derivatives represented by general formula I is formed by connecting the following fragment A and fragment B, 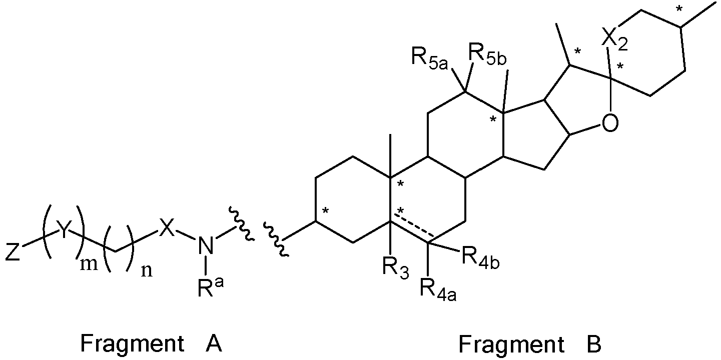
wherein, Z is NR¹R²; R¹ and R² are each independently hydrogen, substituted or unsubstituted C₁-C₁₀ alkyl, the substituents of C₁-C₁₀ alkyl are selected from halogen, hydroxyl, amino, nitro, cyano, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, C₃-C₁₄ heterocyclyl, C₃-C₁₄ heteroaryl, and the heteroatoms are selected from one or more of N, O, S; or R¹ and R² together form a three- to eight-membered ring, the three- to eight-membered ring has one or more substituents selected from C₁-C₁₀ alkyl, C₃-C₁₀ cycloalkyl, C₆-C₂₀ aryl, or C₃-C₁₄ heteroaryl, halogen, hydroxyl, amino, alkoxyl, -CF₃, -SF₅ or three- to eight-membered heterocyclyl whose heteroatoms are sulfur, oxygen, NH or NR^{a}, and the heteroatoms are selected from one or more of N, O, S;
X is C(O) or S(O)₂;
Y is C(R^{d})(R^{e}), C(O) or S(O)₂, R^{d} and R^{e} are independently hydrogen or C₁-C₁₀ alkyl, C₃-C₁₀ cycloalkyl, C₆-C₂₀ aryl, or C₃-C₁₄ heteroaryl with at least one substituent, wherein the substituents are selected from halogen, hydroxyl, amino, nitro, cyano, aldehyde, carboxyl, alkoxyl, -CF₃ or -SF₅, the heteroatoms are selected from one or more of N, O, S, or R^{d} and R^{e} together form a three- to eight-membered ring, the three- to eight-membered ring has one or more substituents selected from C₁-C₁₀ alkyl, C₃-C₁₀ cycloalkyl, C₆-C₂₀ aryl, or C₃-C₁₄ heteroaryl, halogen, hydroxyl, amino, alkoxyl, -CF₃, -SF₅ or three- to eight-membered heterocyclyl whose heteroatoms are sulfur, oxygen, NH or NR^{a}, and the heteroatoms are selected from one or more of N, O, S;
X₂ is O, S, or NH;
R^{a} is independently hydrogen or C₁-C₁₀ alkyl, C₃-C₁₀ cycloalkyl, C₆-C₂₀ aryl, or C₃-C₁₄ heteroaryl with at least one substituent, wherein the substituents are selected from halogen, hydroxyl, amino, nitro, cyano, alkoxyl, -CF₃ or -SF₅, and the heteroatoms are selected from one or more of N, O, S;
n is an integer from 0 to 10, n is not 0 and m is 1, or n is 0 and m is 1, or n is an integer from 0 to 10, n is not 0 and m is 0;
R₃, R₄ₐ, R_{4b}, R₅ₐ, and R_{5b} are each independently hydrogen or selected from halogen, substituted alkyl, hydroxyl, and amino;
" - - - - - - " represents a single bond or double bond; and
each " " independently represents a racemic, S or R configuration.

2. The use according to claim 1, **characterized in that** the structural formula of the derivatives is represented by general formula II,

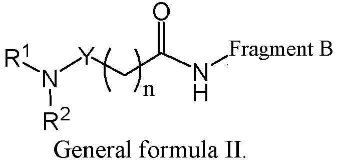

3. The use according to claim 1, **characterized in that** the structural formula of the derivatives is represented by general formula III,

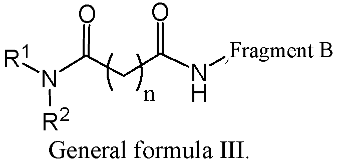

4. The use according to claim 1, **characterized in that** the structural formula of the derivatives is represented by general formula IV,

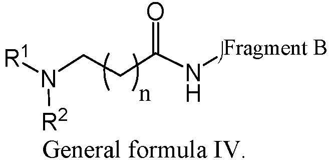

5. The use according to claim 1, **characterized in that** the structural formula of the derivatives is represented by general formula V, 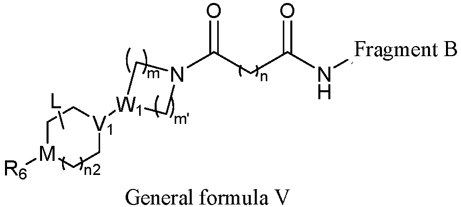
wherein, R₆ is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, pyridyl, -CO alkyl, -CO aryl, -SO₂ alkyl, -SO₂ aryl, -CO₂ alkyl, C₂-C₄ (CO) alkenyl, -CO₂ aryl, -SO₃H;
L is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl;
n is an integer from 0 to 10;
n2 is 0, 1, 2, or 3;
m, m' are each independently 1, 2, 3 or 4;
W₁ is C or NH;
V₁ is C or NH; and
Mis C, S, O or NH.

6. The use according to claim 1, **characterized in that** the structural formula of the derivatives is represented by general formula VI, 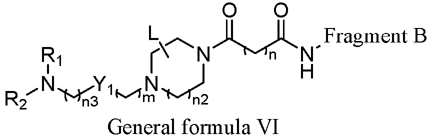
Y₁ is C(Rd)(Re), C(O) or S(O)₂, Rd and Re are independently hydrogen or C₁-C₁₀ alkyl, C₃-C₁₀ cycloalkyl, C₆-C₂₀ aryl, or C₃-C₁₄ heteroaryl with at least one substituent, wherein the substituents are selected from halogen, hydroxyl, amino, nitro, cyano, aldehyde, carboxyl, alkoxyl, -CF₃ or -SF₅, the heteroatoms are selected from one or more of N, O, S, or R^{d} and R^{e} together form a three- to eight-membered ring, the three- to eight-membered ring has one or more substituents selected from C₁-C₁₀ alkyl, C₃-C₁₀ cycloalkyl, C₆-C₂₀ aryl, or C₃-C₁₄ heteroaryl, halogen, hydroxyl, amino, alkoxyl, -CF₃, -SF₅ or three- to eight-membered heterocyclyl whose heteroatoms are sulfur, oxygen, NH or NR^{a}, and the heteroatoms are selected from one or more of N, O, S;
L is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl;
n is an integer from 0 to 10;
n2 is 0, 1, 2, or 3;
n3 is an integer from 1 to 10; and
m is an integer from 0 to 10.

7. The use according to claim 1, **characterized in that** the structural formula of the derivatives is represented by general formula VII, 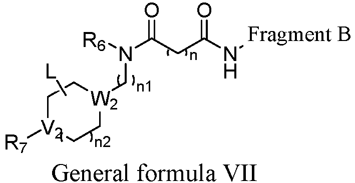
wherein, R₆ and R₇ are independently hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, pyridyl, -CO alkyl, -CO aryl, -SO₂ alkyl, -SO₂ aryl, -CO₂ alkyl, C₂-C₄ (CO) alkenyl, -CO₂ aryl, -SO₃H;
L is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl;
W₂ is C or NH;
V₂ is C, O, S, or NH;
n is an integer from 0 to 10;
n1 is an integer from 1 to 10; and
n2 is 0, 1, 2, or 3.

8. The use according to claim 1, **characterized in that** the structural formula of the derivatives is represented by general formula VIII 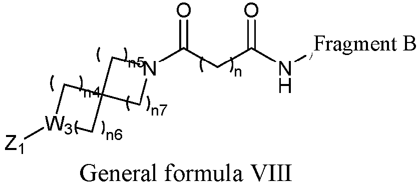
Z₁ is hydrogen, substituted or unsubstituted C₁-C₁₀ alkyl, and the substituents of C₁-C₁₀ alkyl are selected from halogen, hydroxyl, -NH₂, nitro, -CN, C₁-C₄ alkyl, C₁-C₄ haloalkyl, C₁-C₄ alkoxyl, C₃-C₆ cycloalkyl, C₂-C₄ alkenyl, C₂-C₄ alkynyl, phenyl, benzyl, pyridyl, -CO alkyl, -CO aryl, -SO₂ alkyl, -SO₂ aryl, -CO₂ alkyl, C₂-C₄ (CO) alkenyl, -CO₂ aryl, -SO₃H;
W₃ is C, O, S or NH;
n is an integer from 0 to 10; and
n4, n5, n6 and n7 are independently integers of 1 to 4.

9. The use according to Claim 1, **characterized in that** the fragment B in the structural formula of the derivatives is as follow:

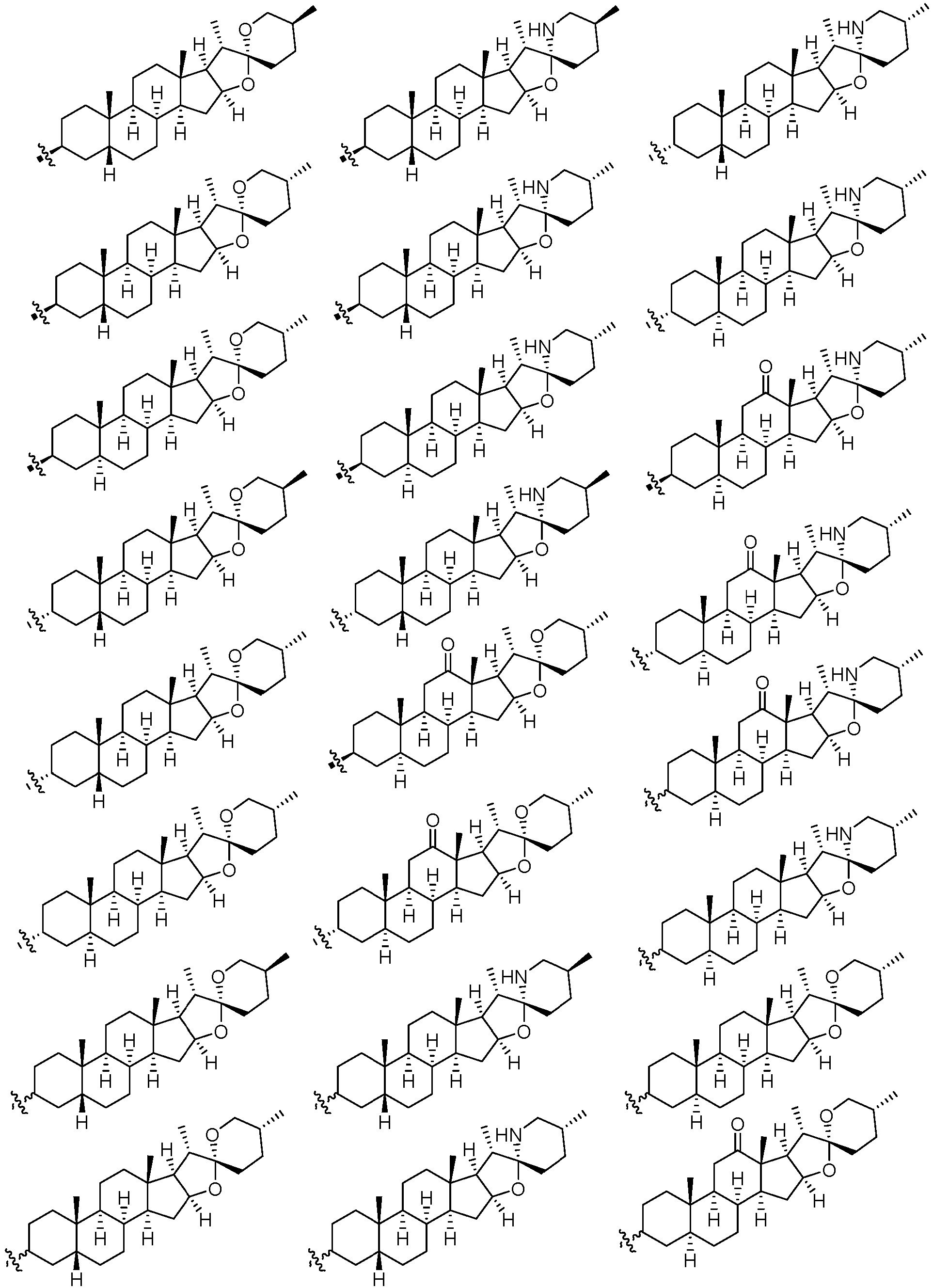

10. The use according to Claim 1, **characterized in that** the derivatives are selected from one of the following compounds, mixtures of diastereomers of the following compounds, or enantiomers of the following compounds:

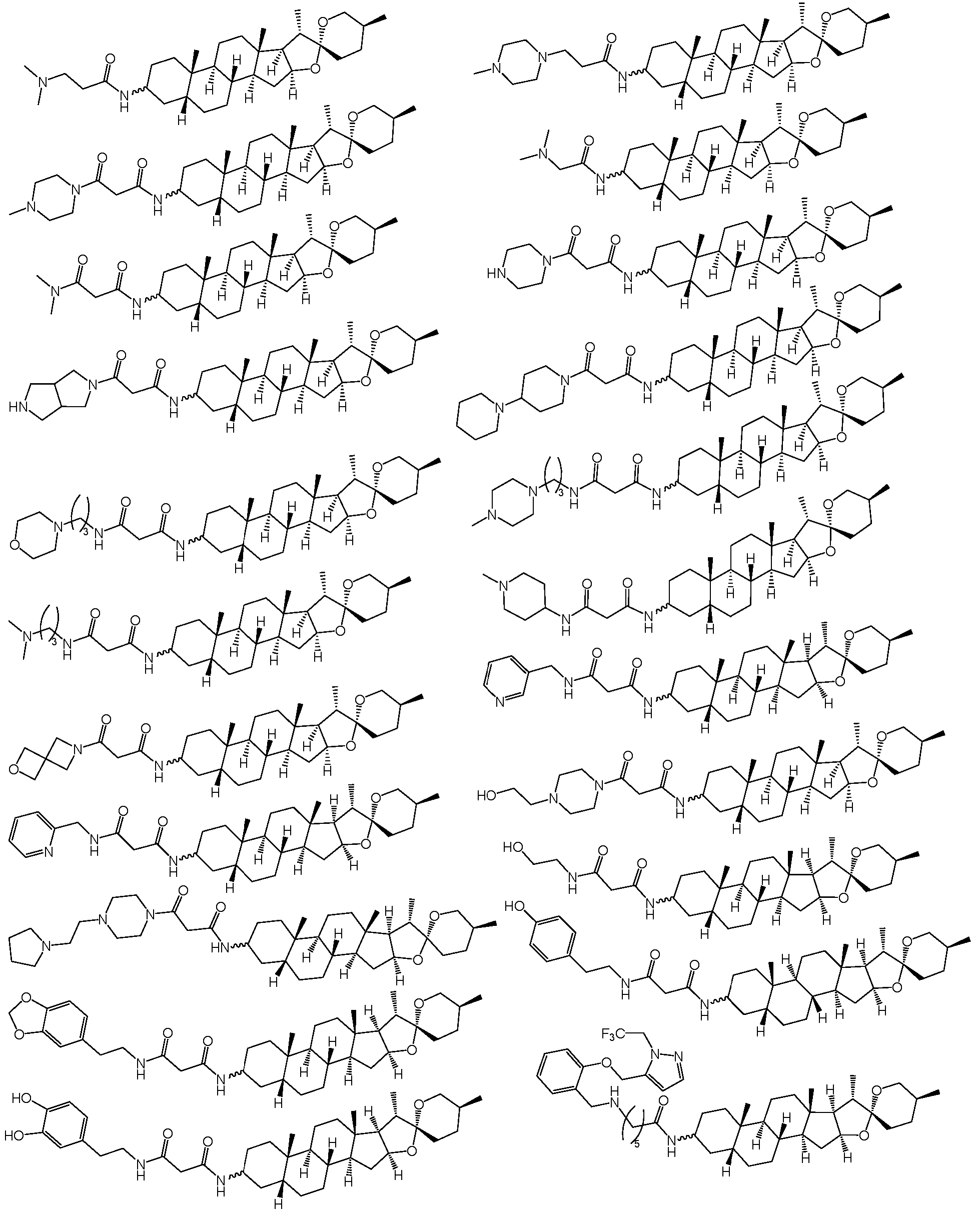

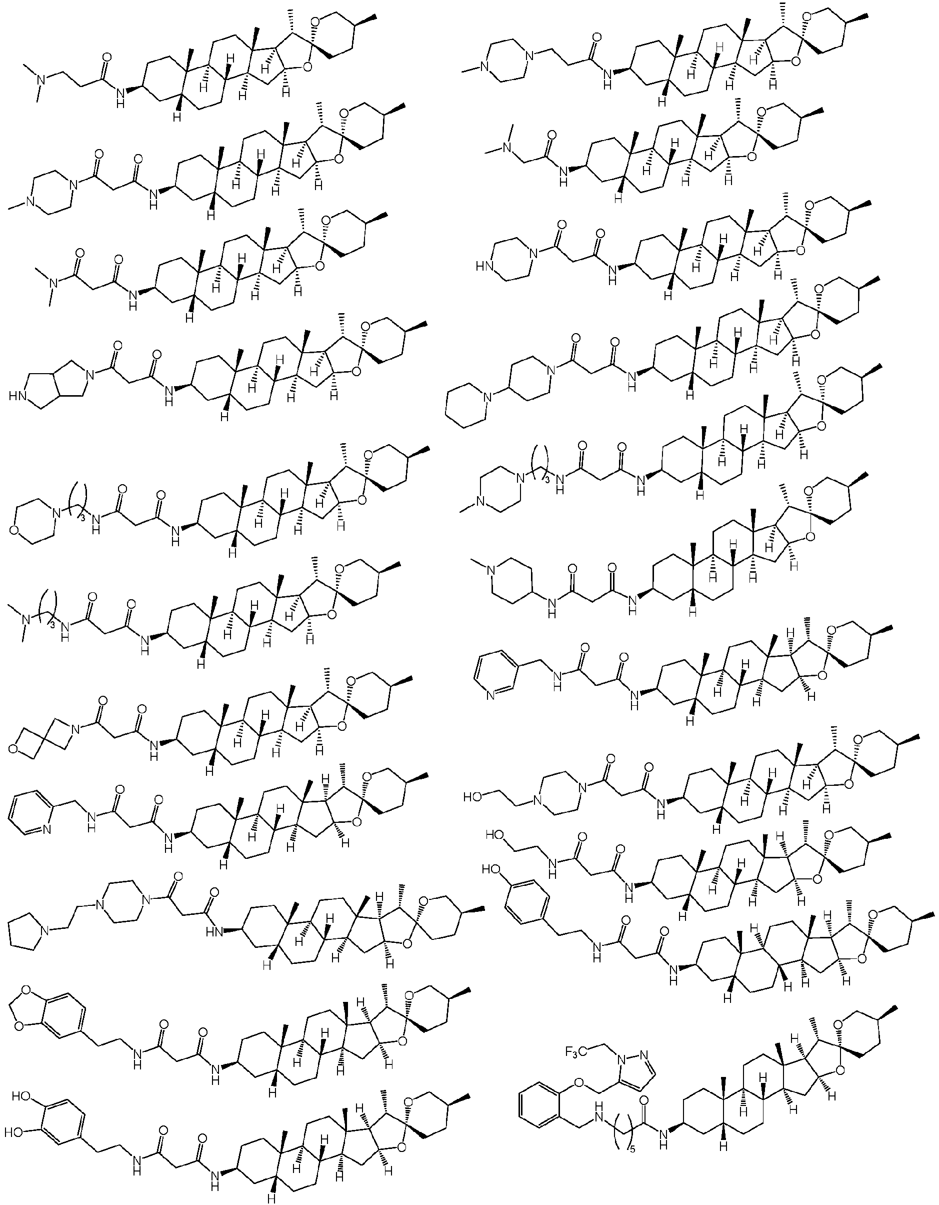

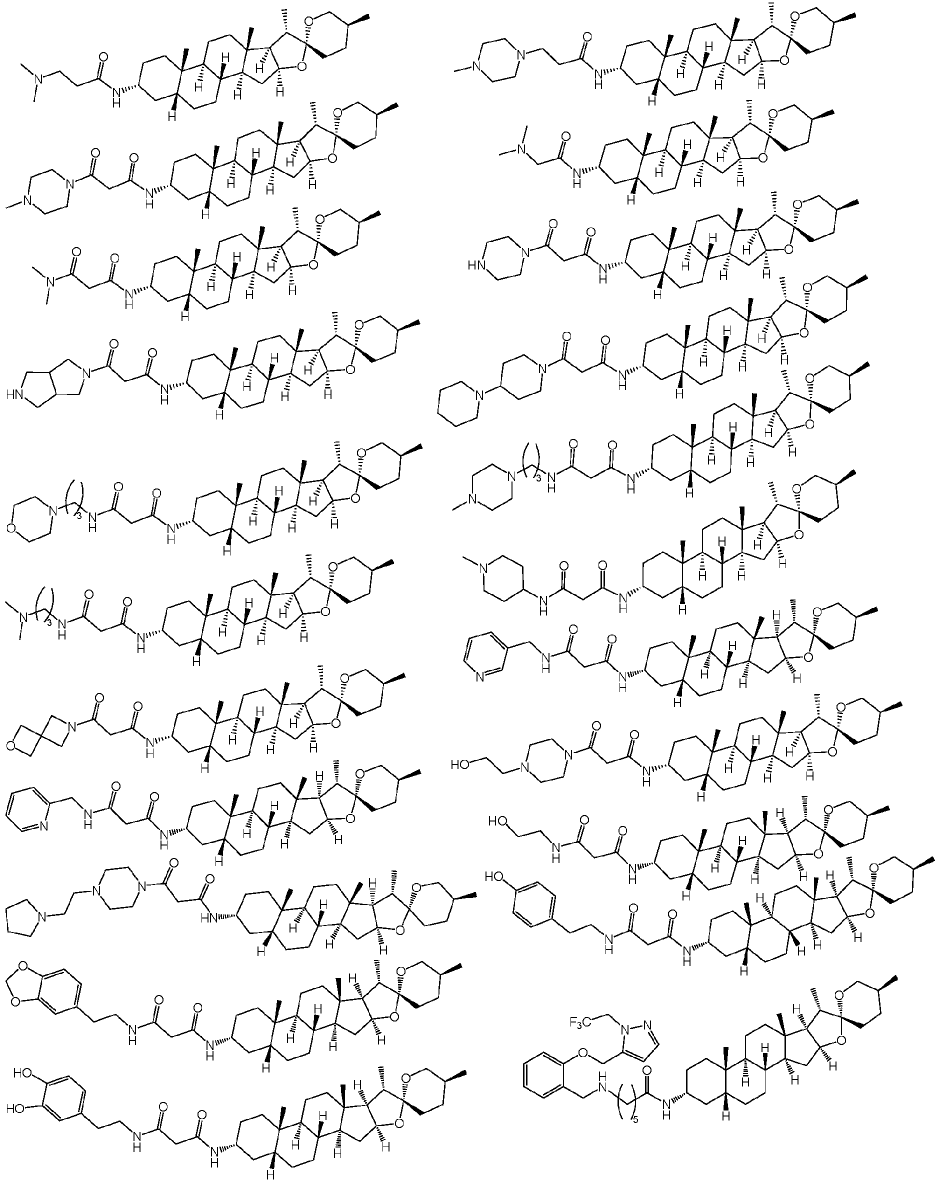

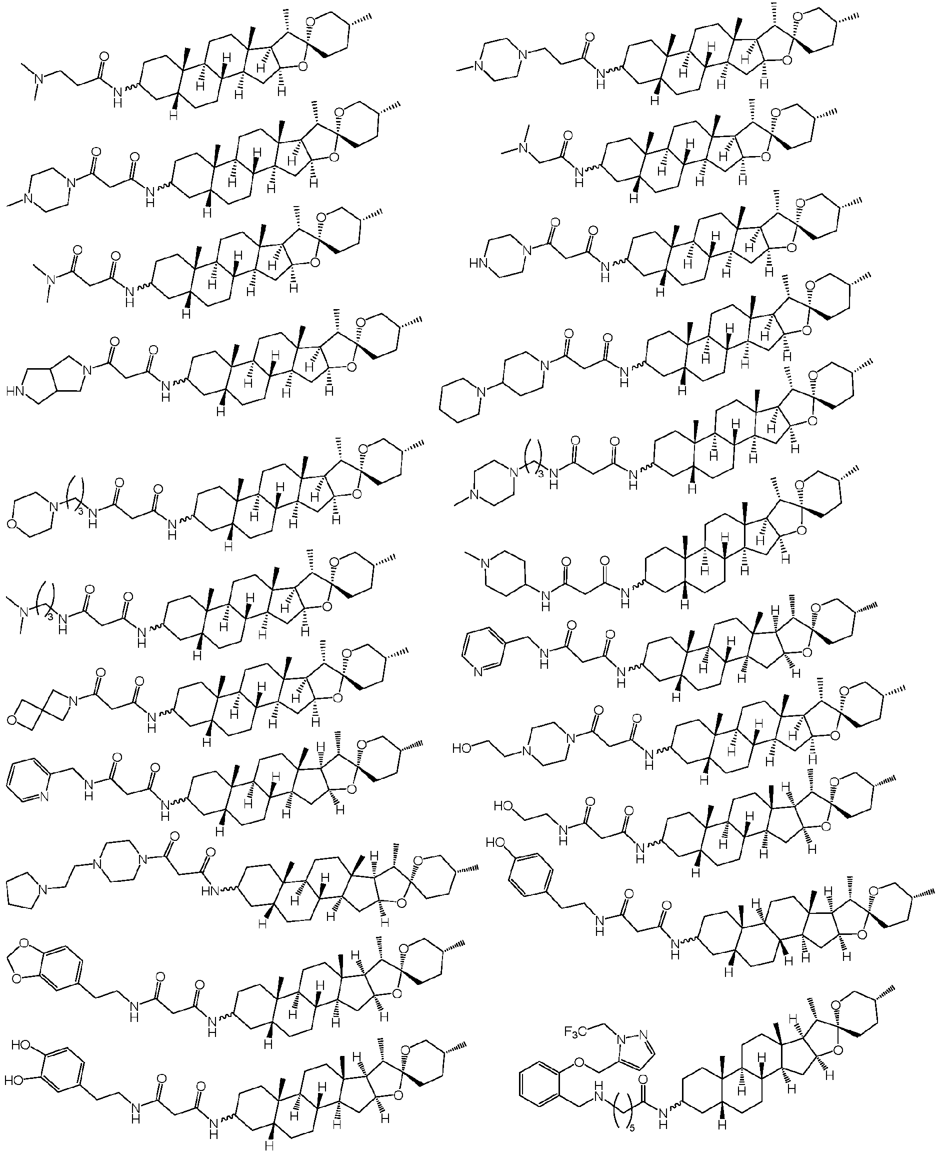

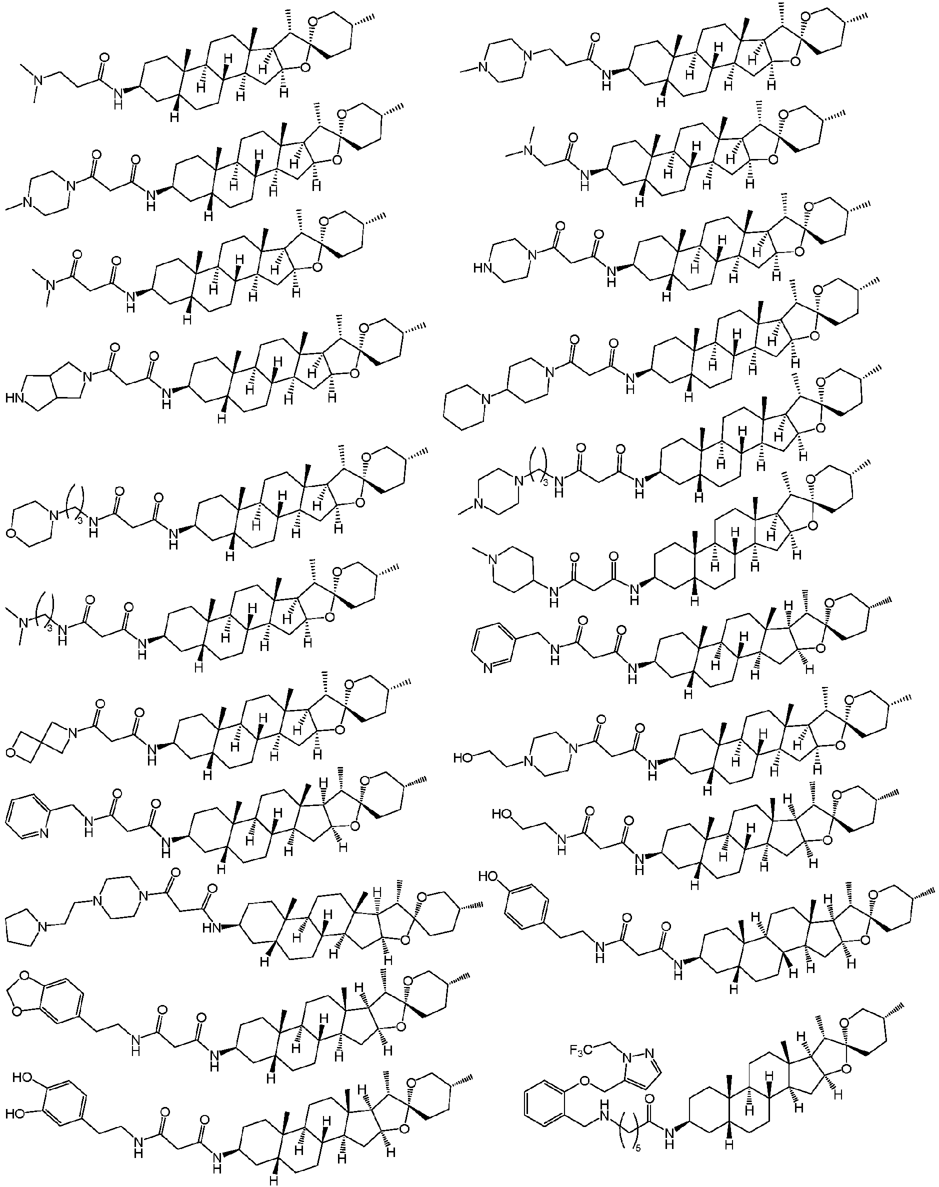

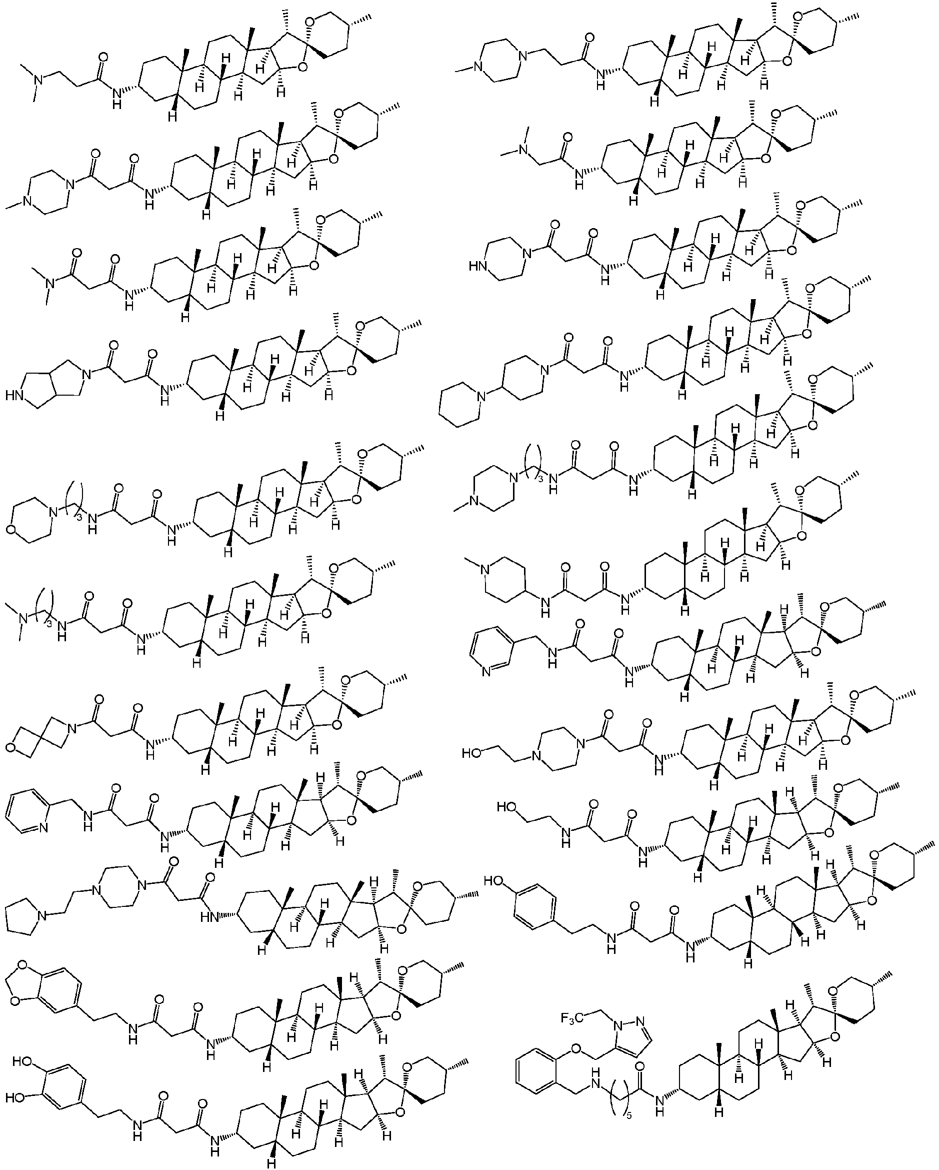

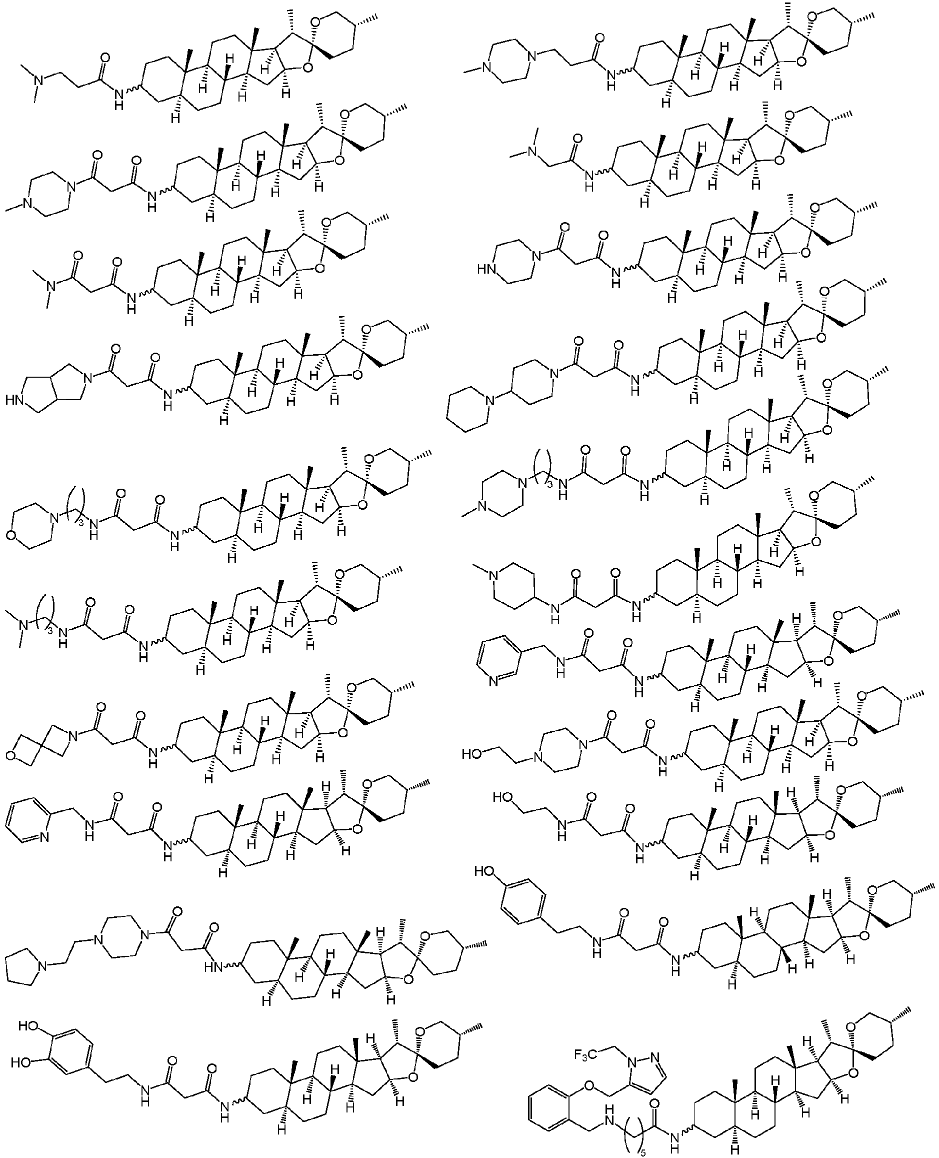

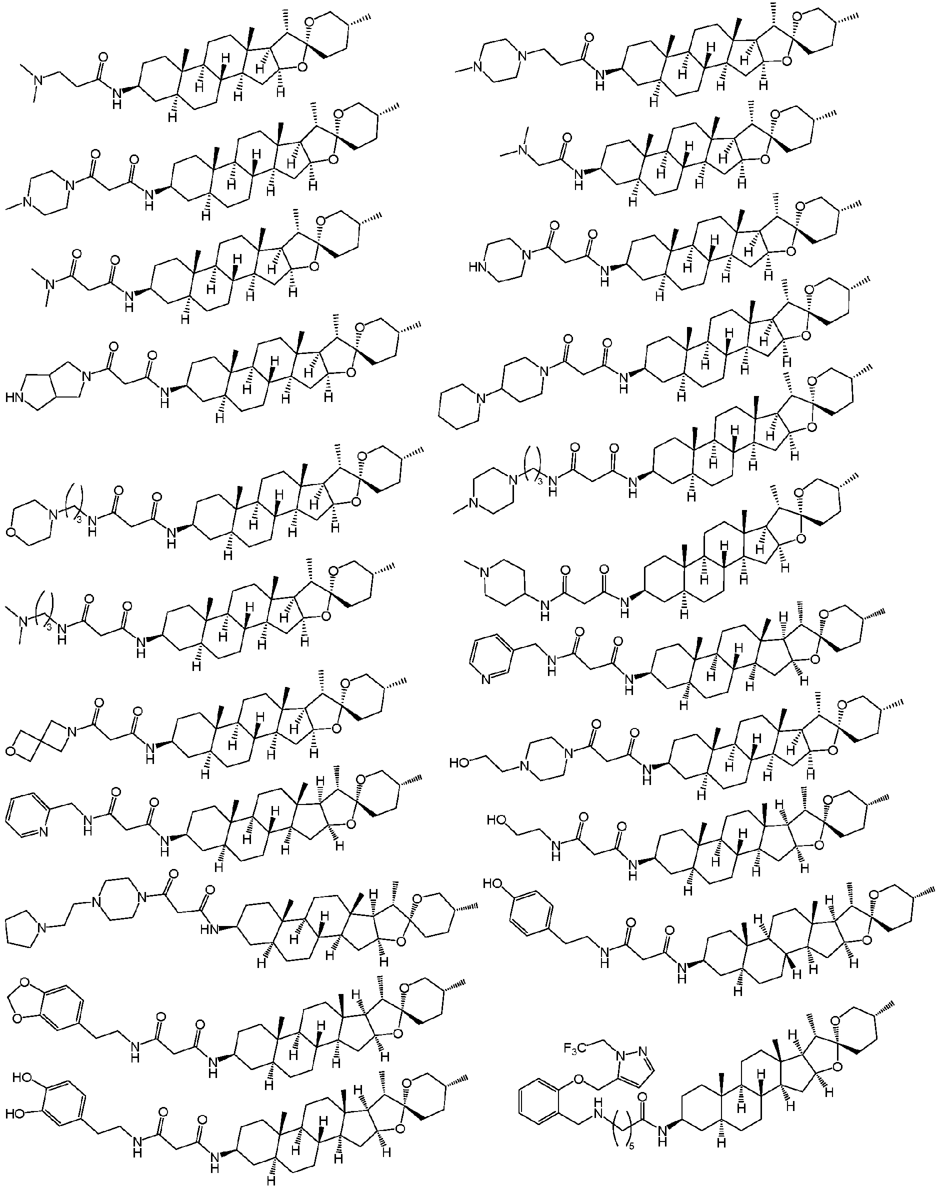

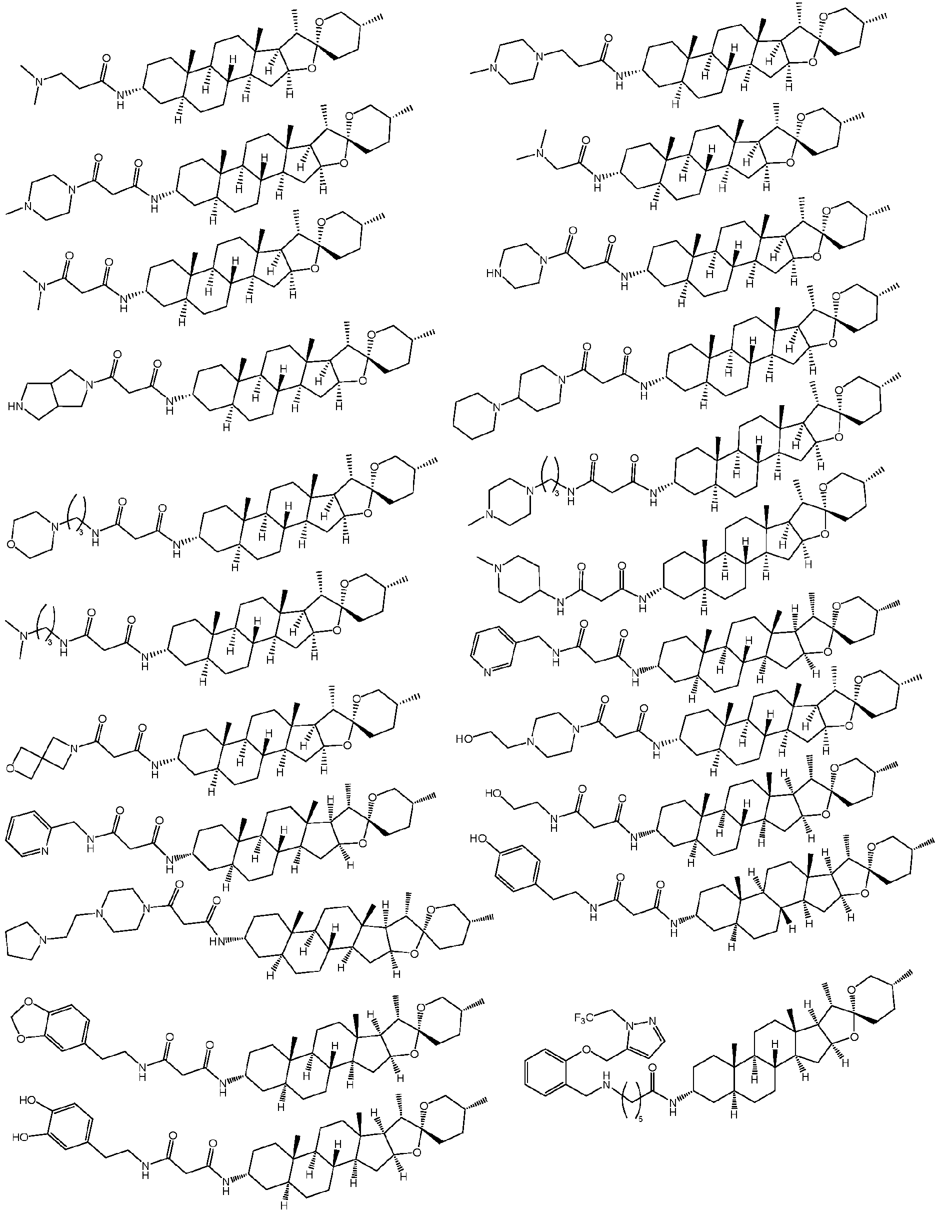

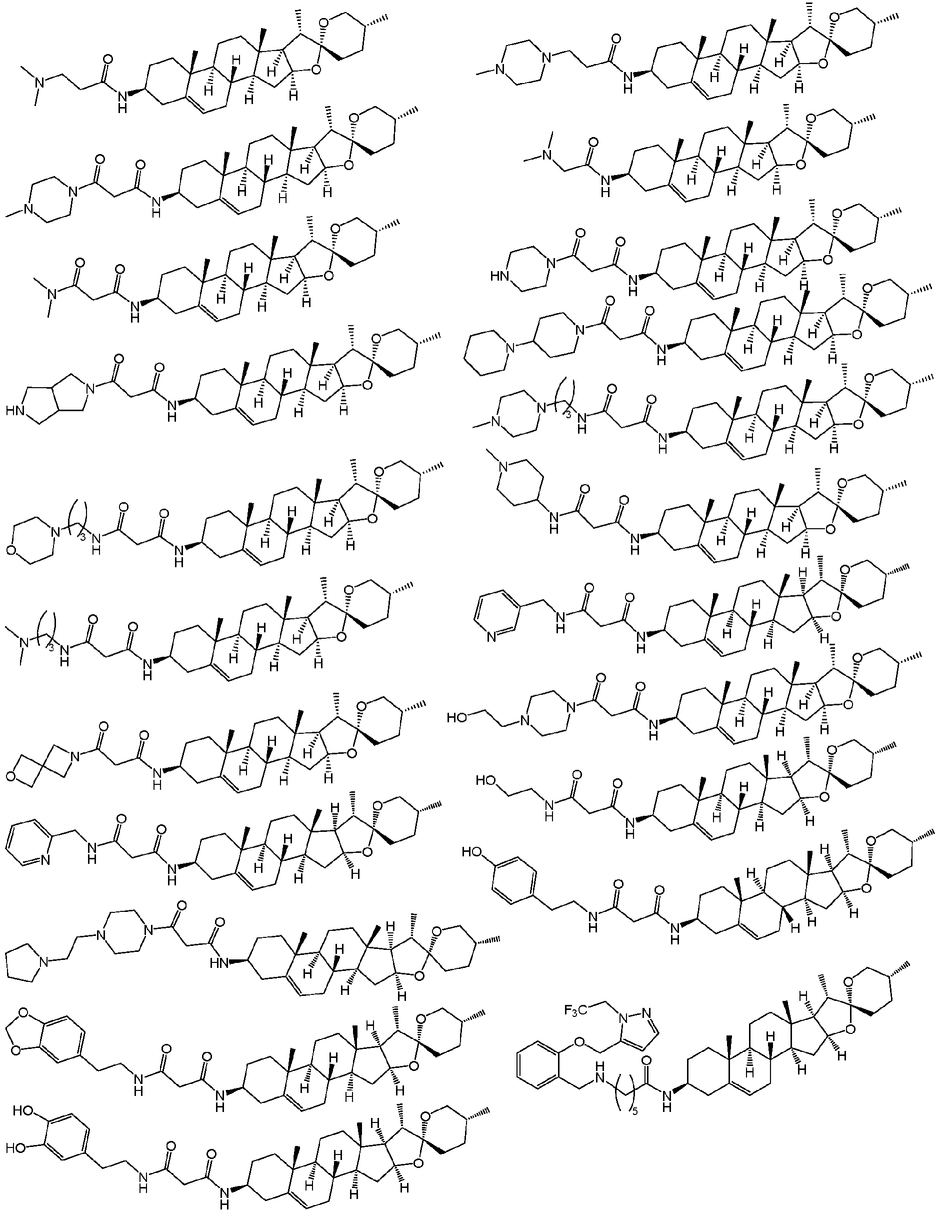

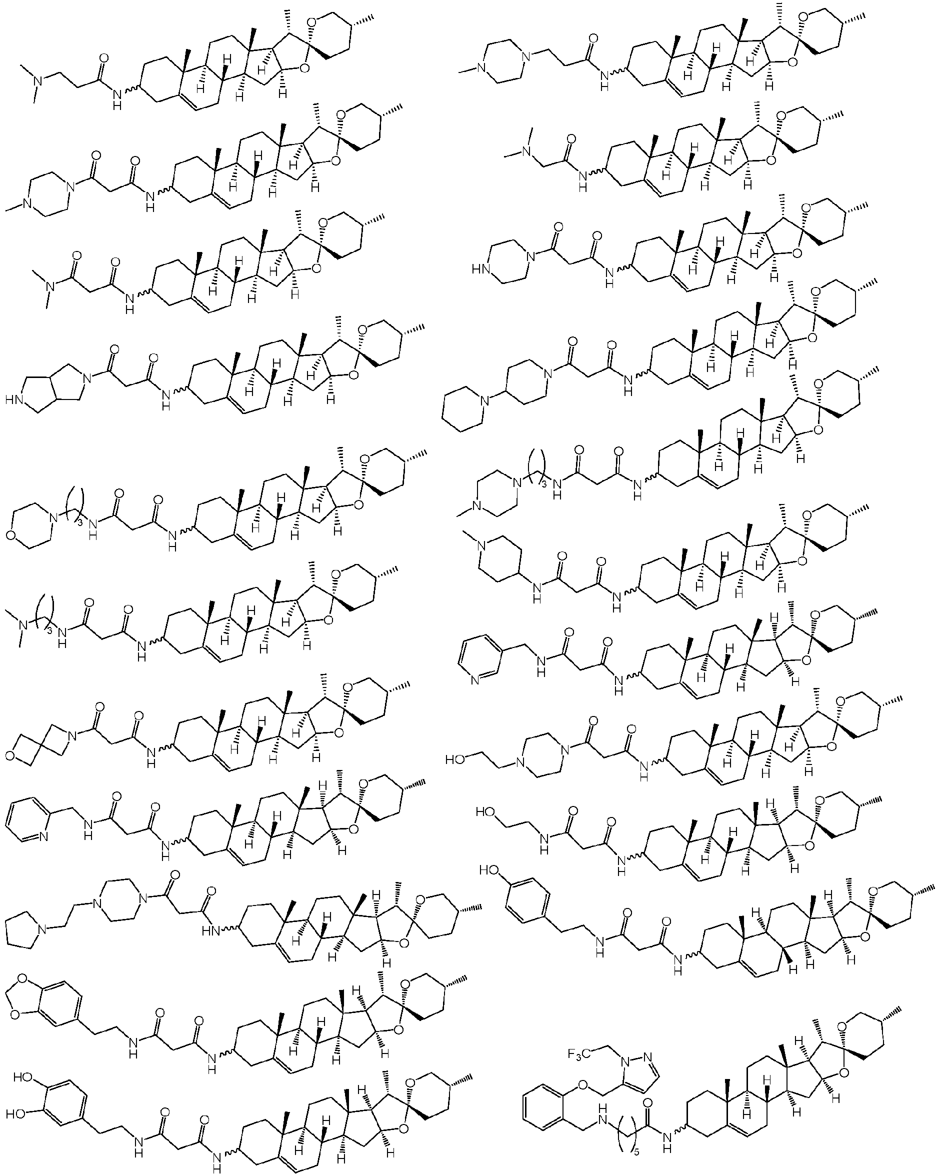

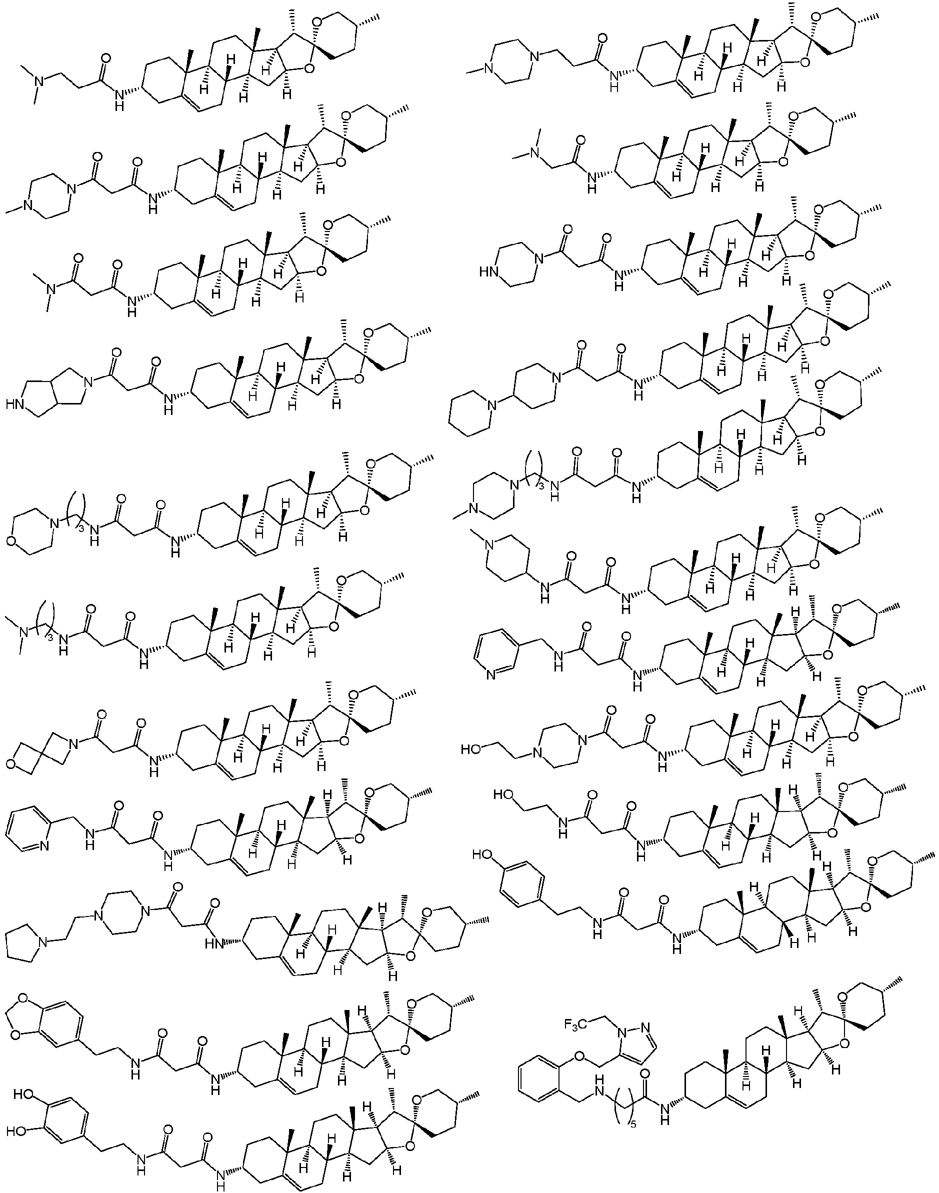

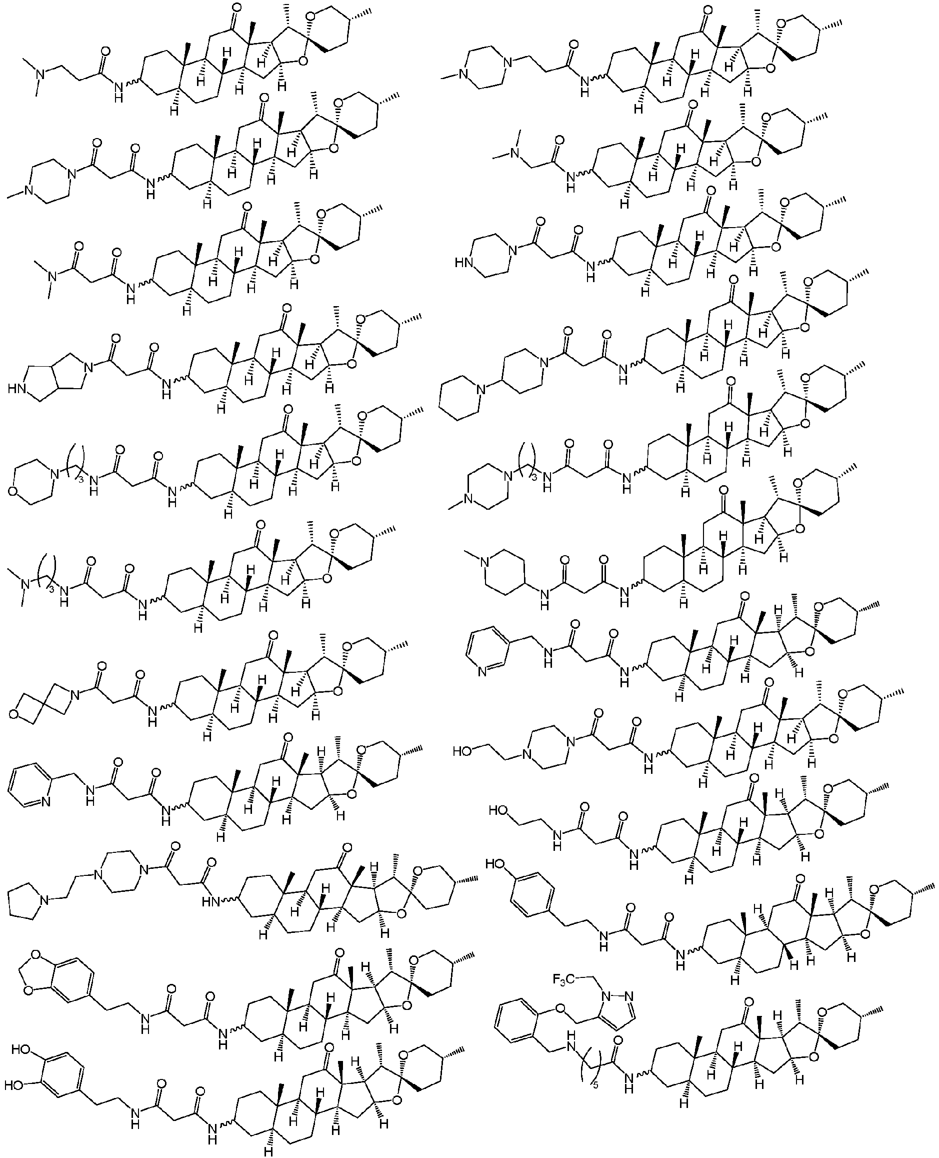

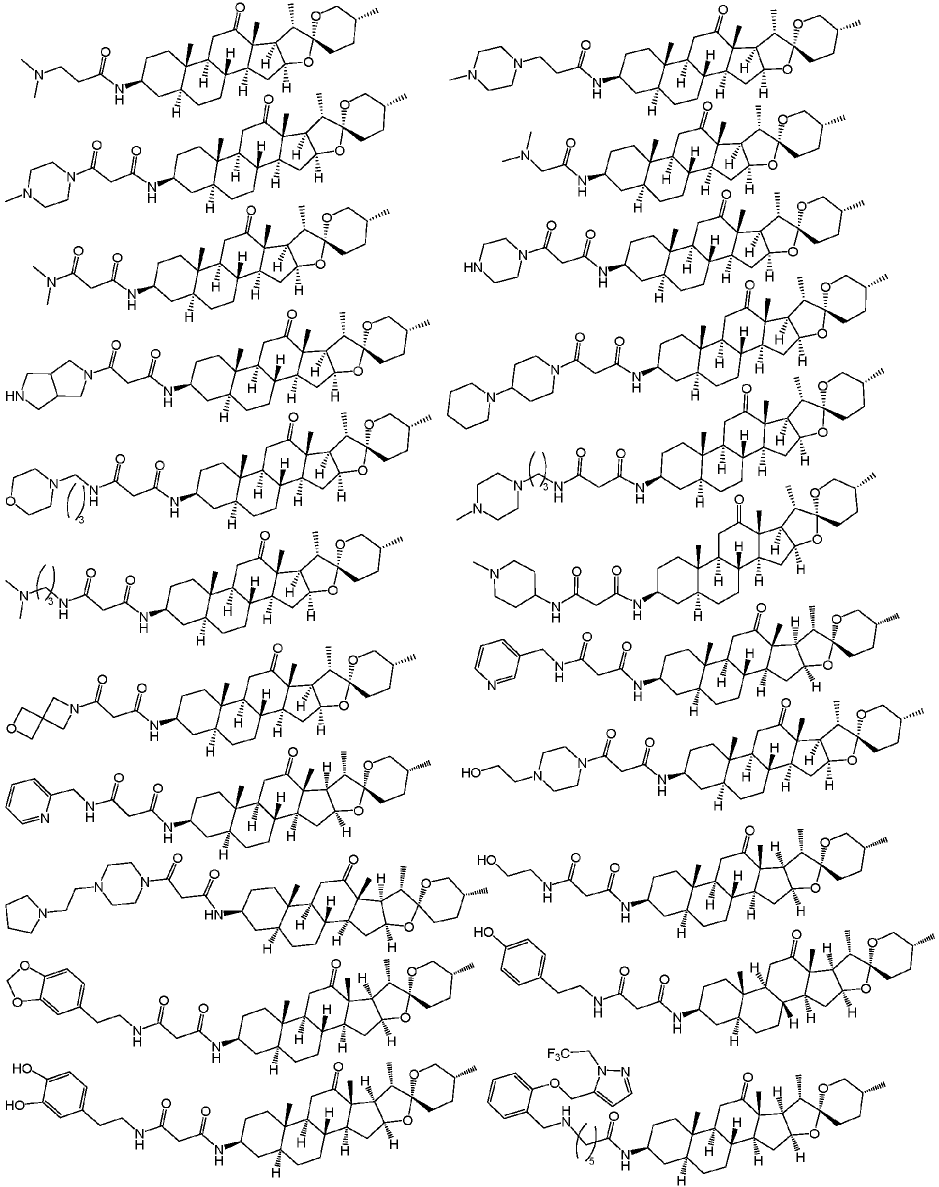

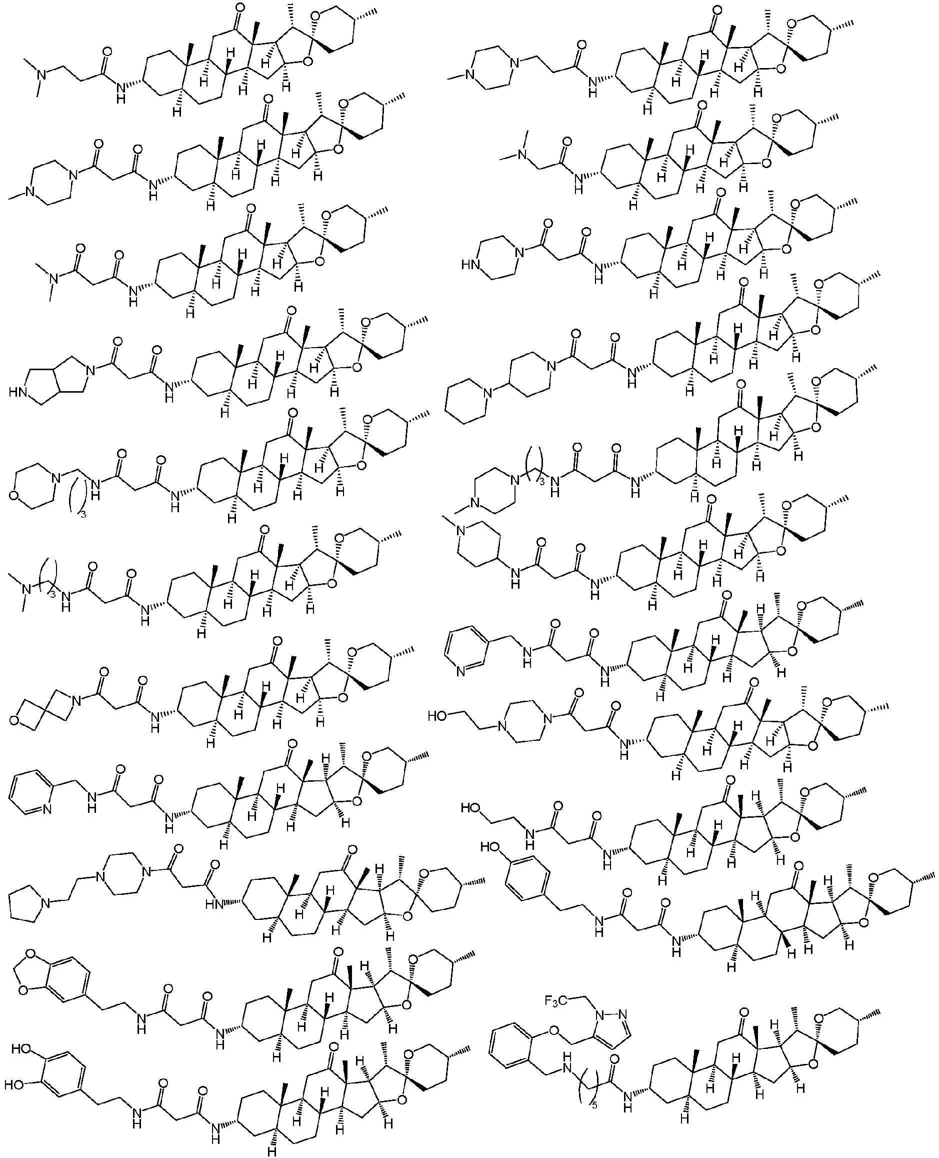

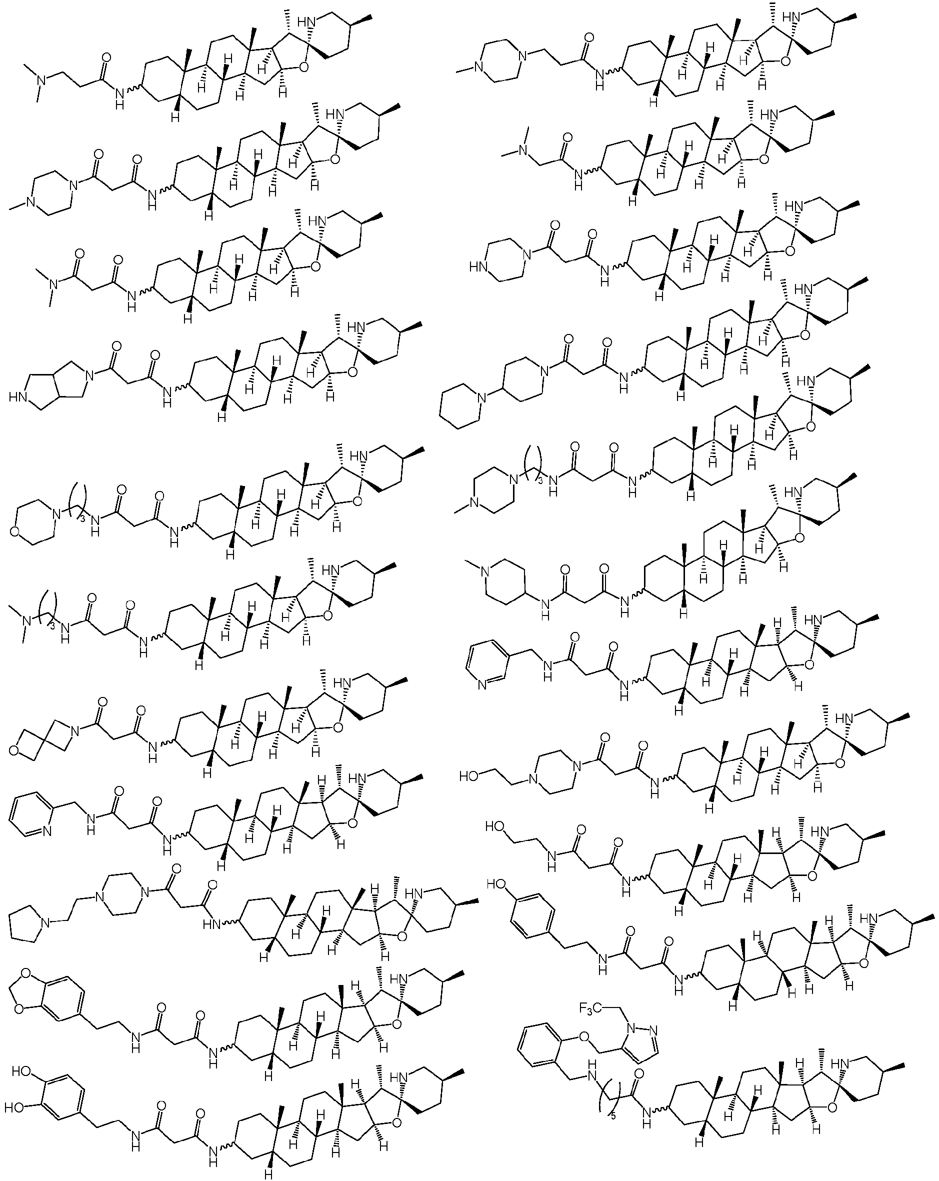

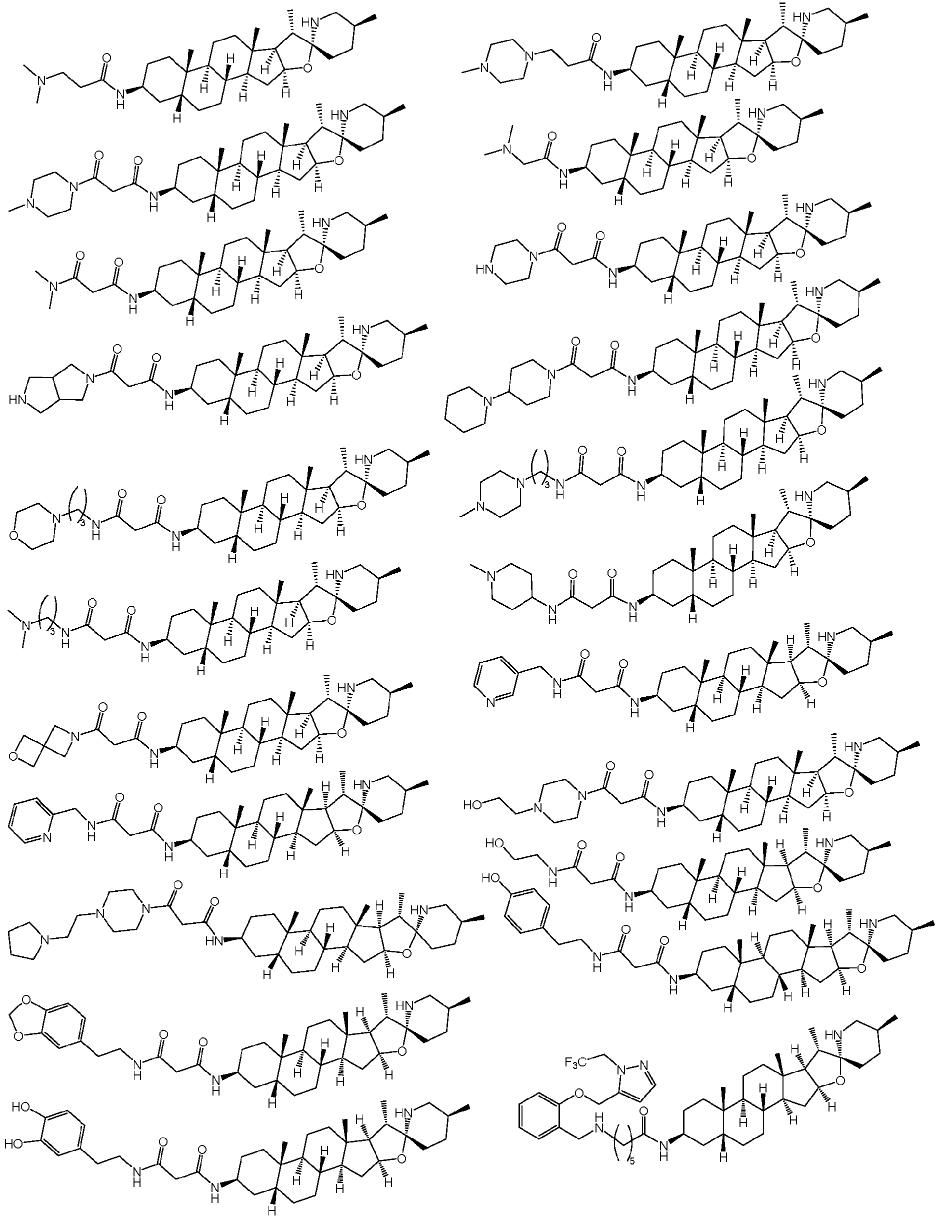

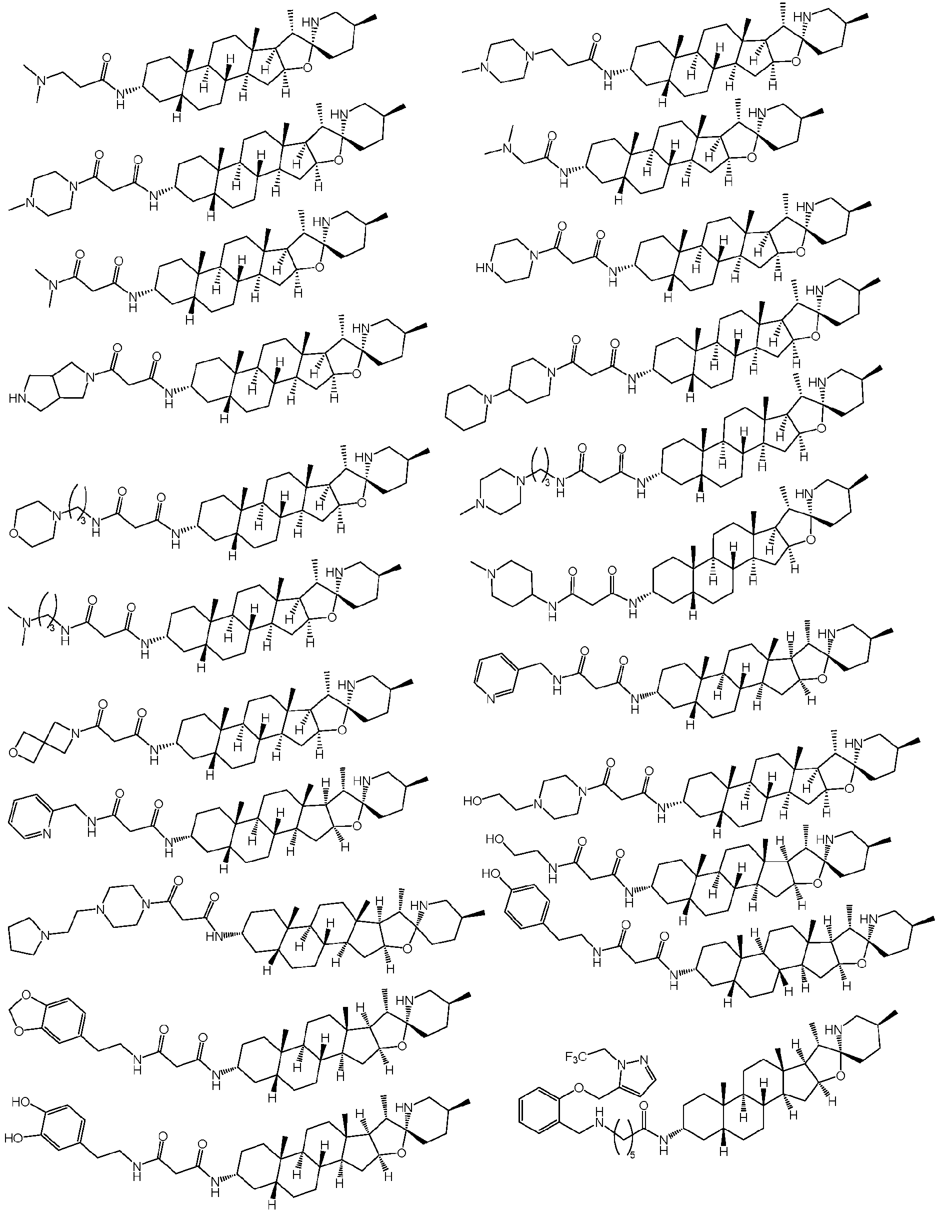

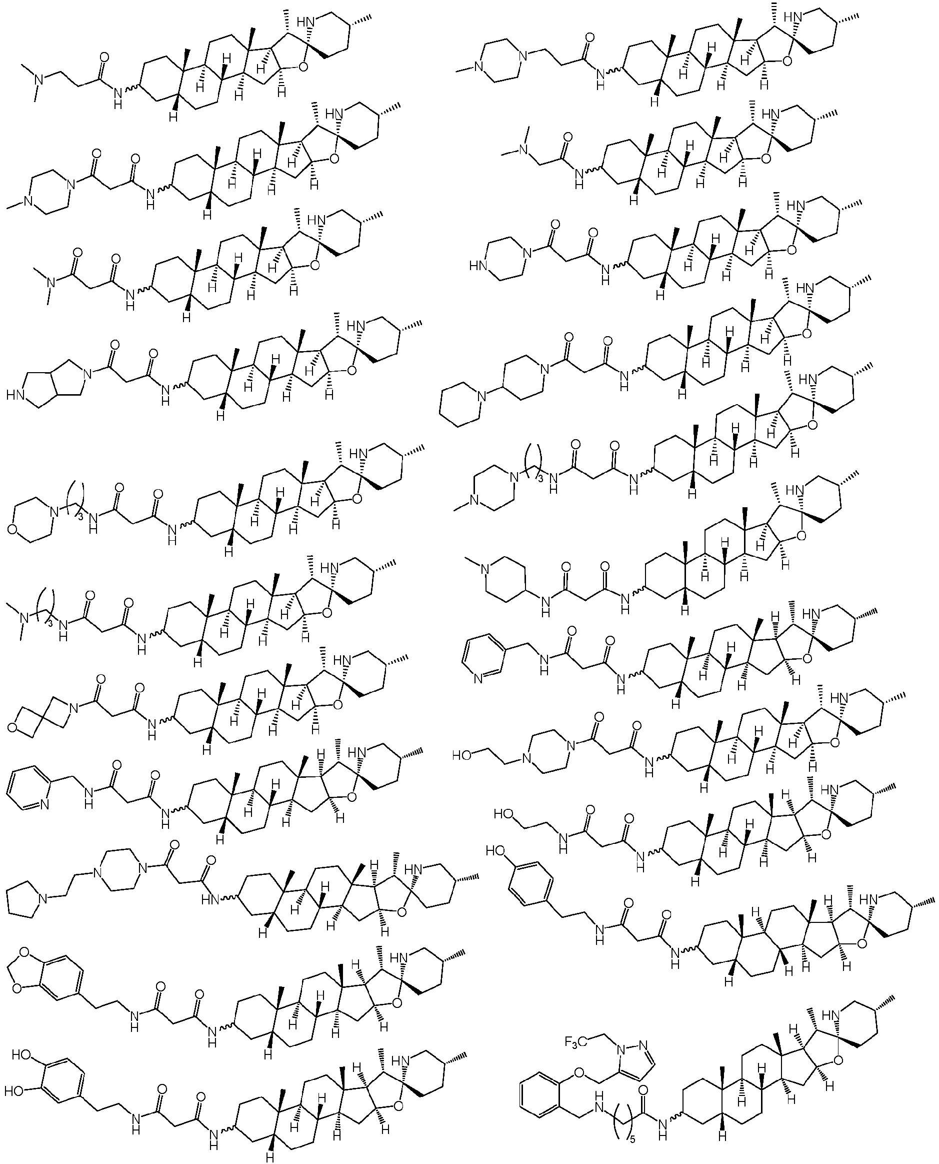

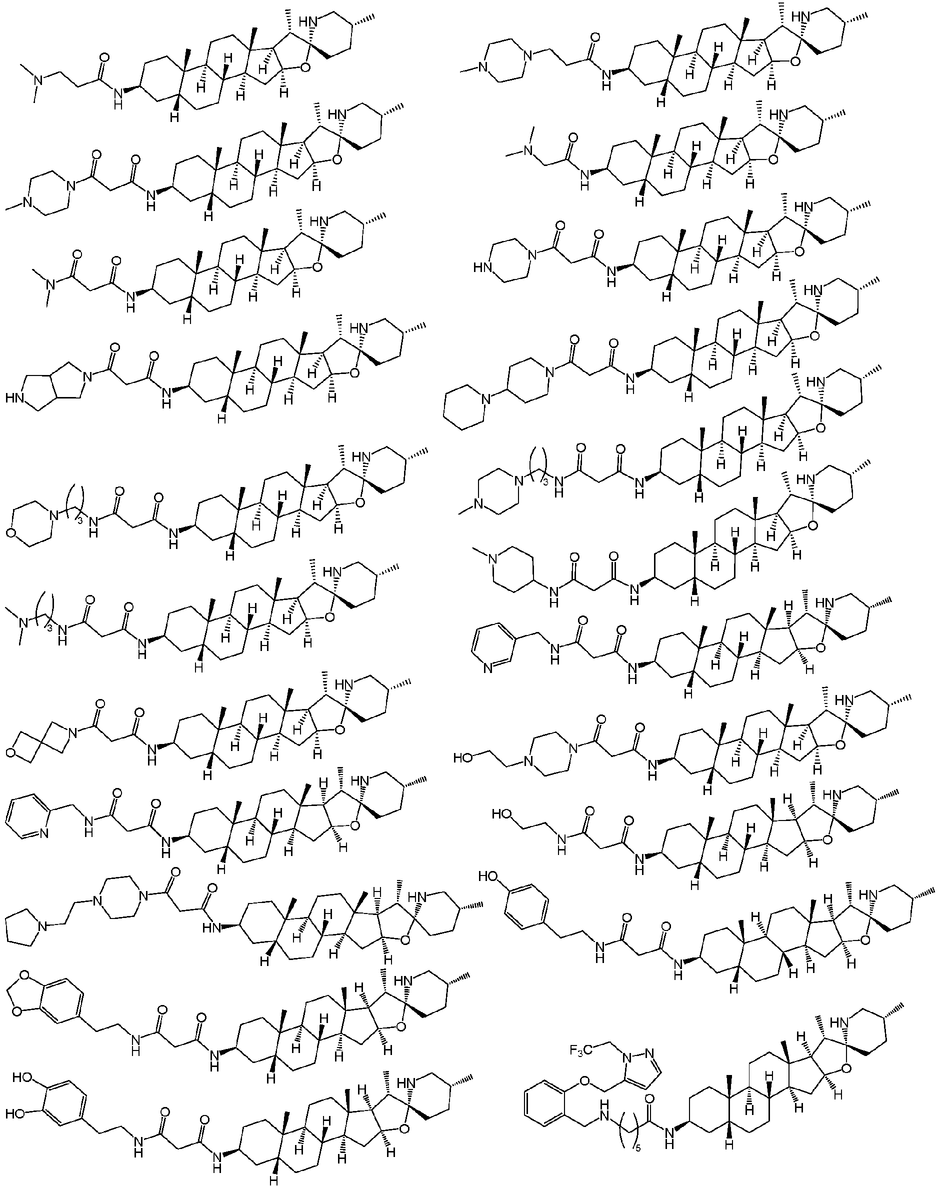

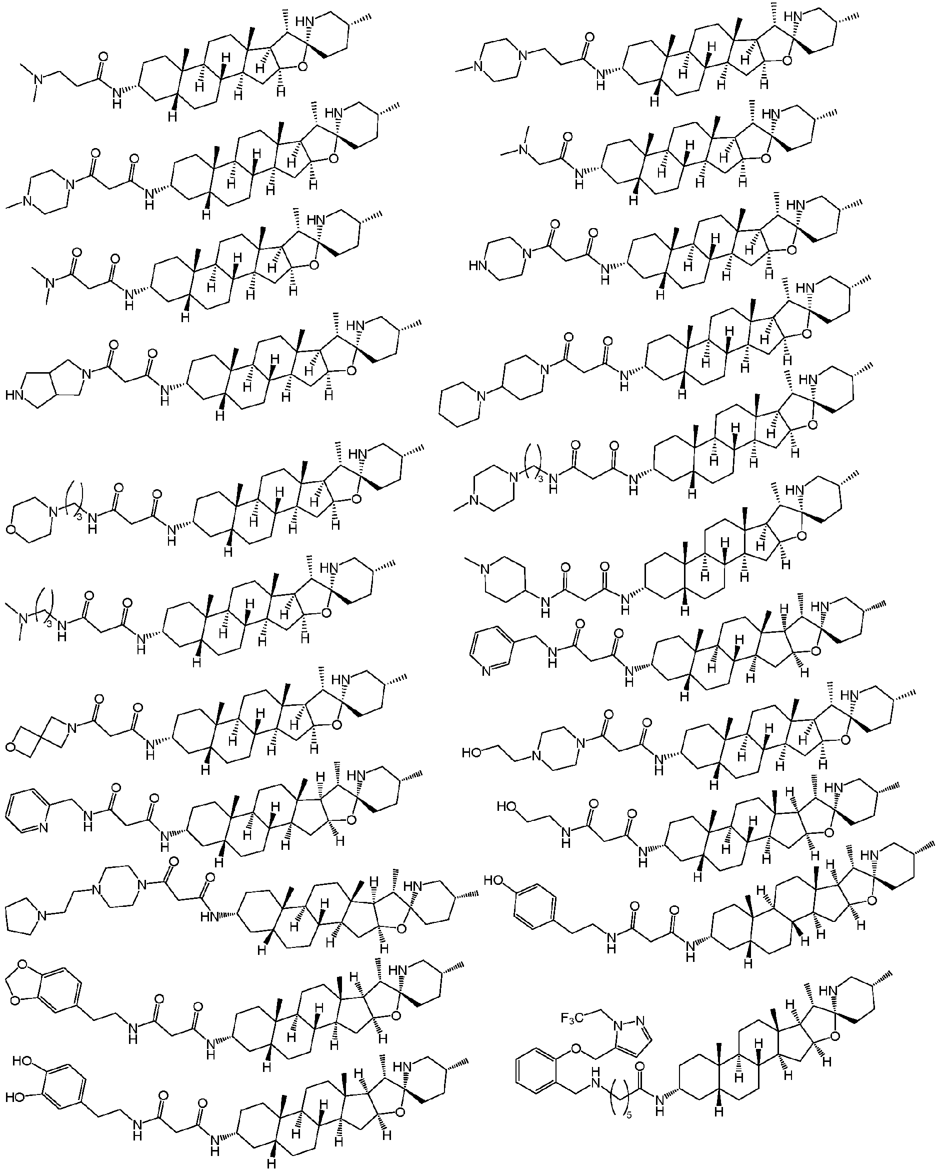

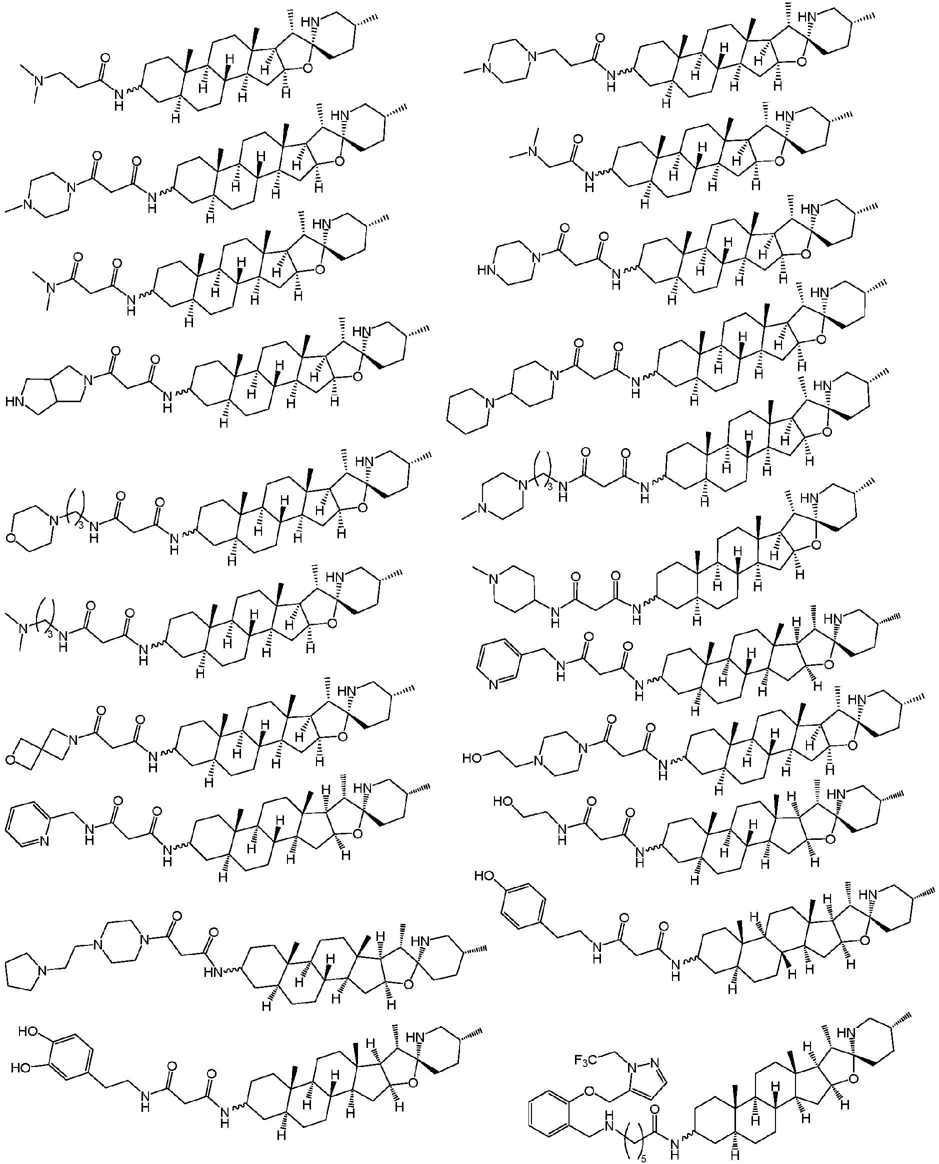

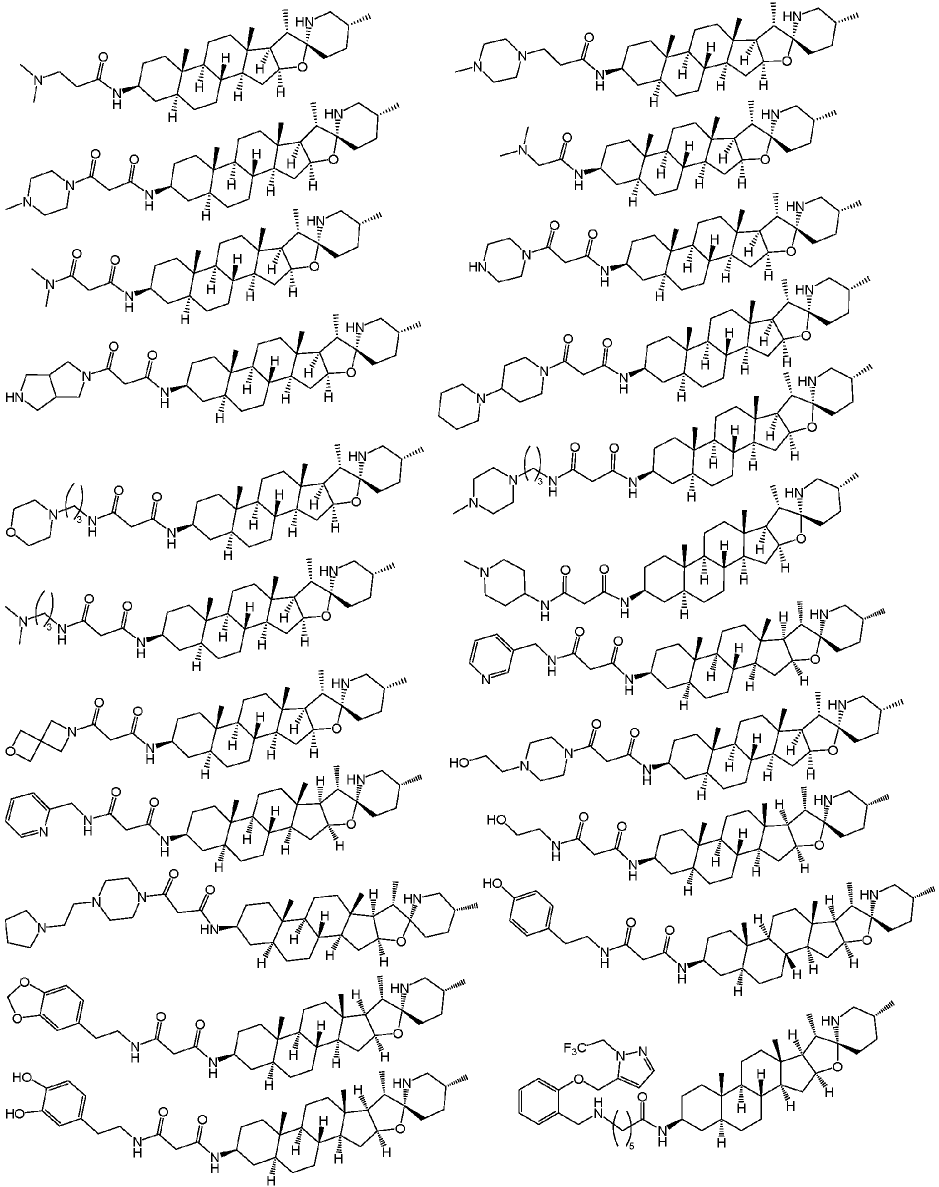

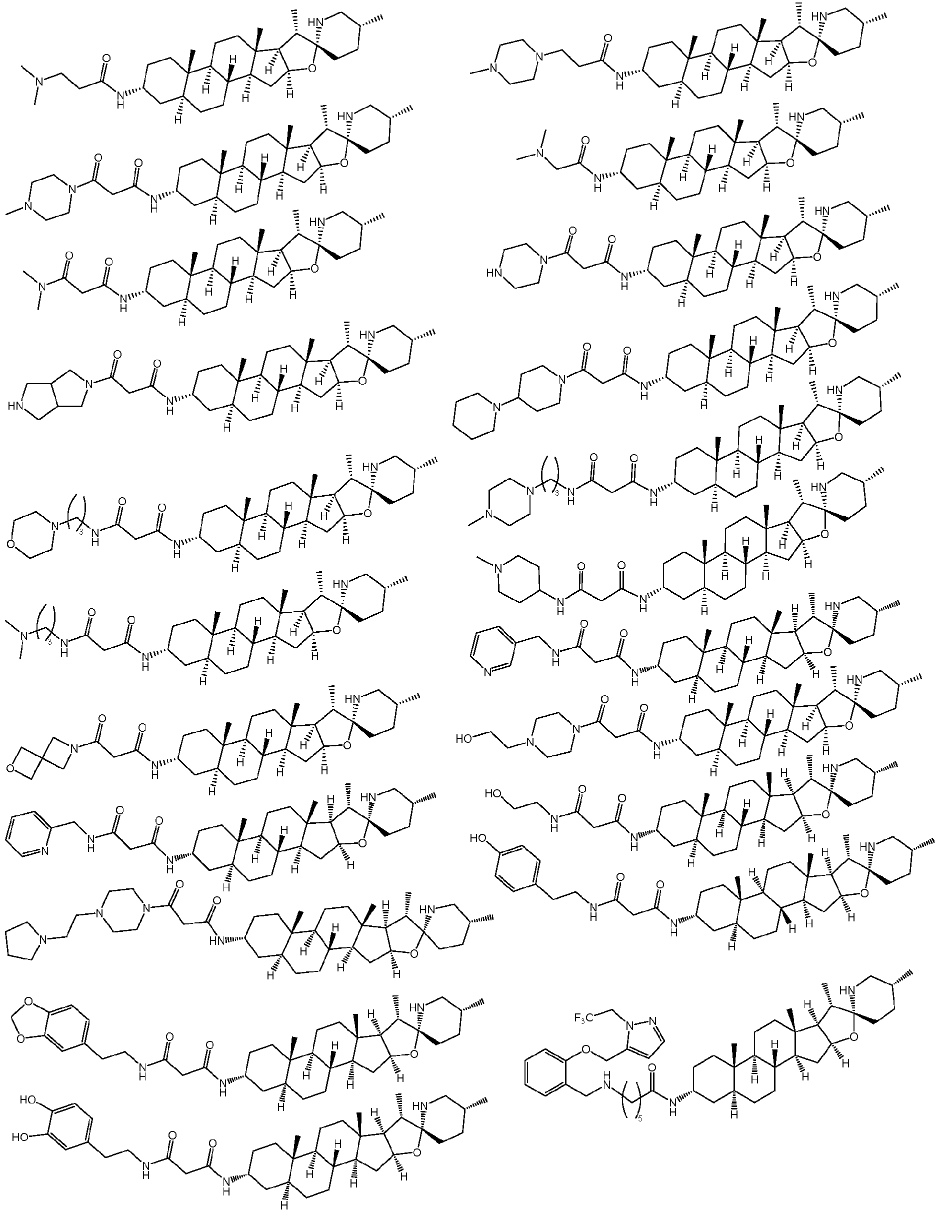

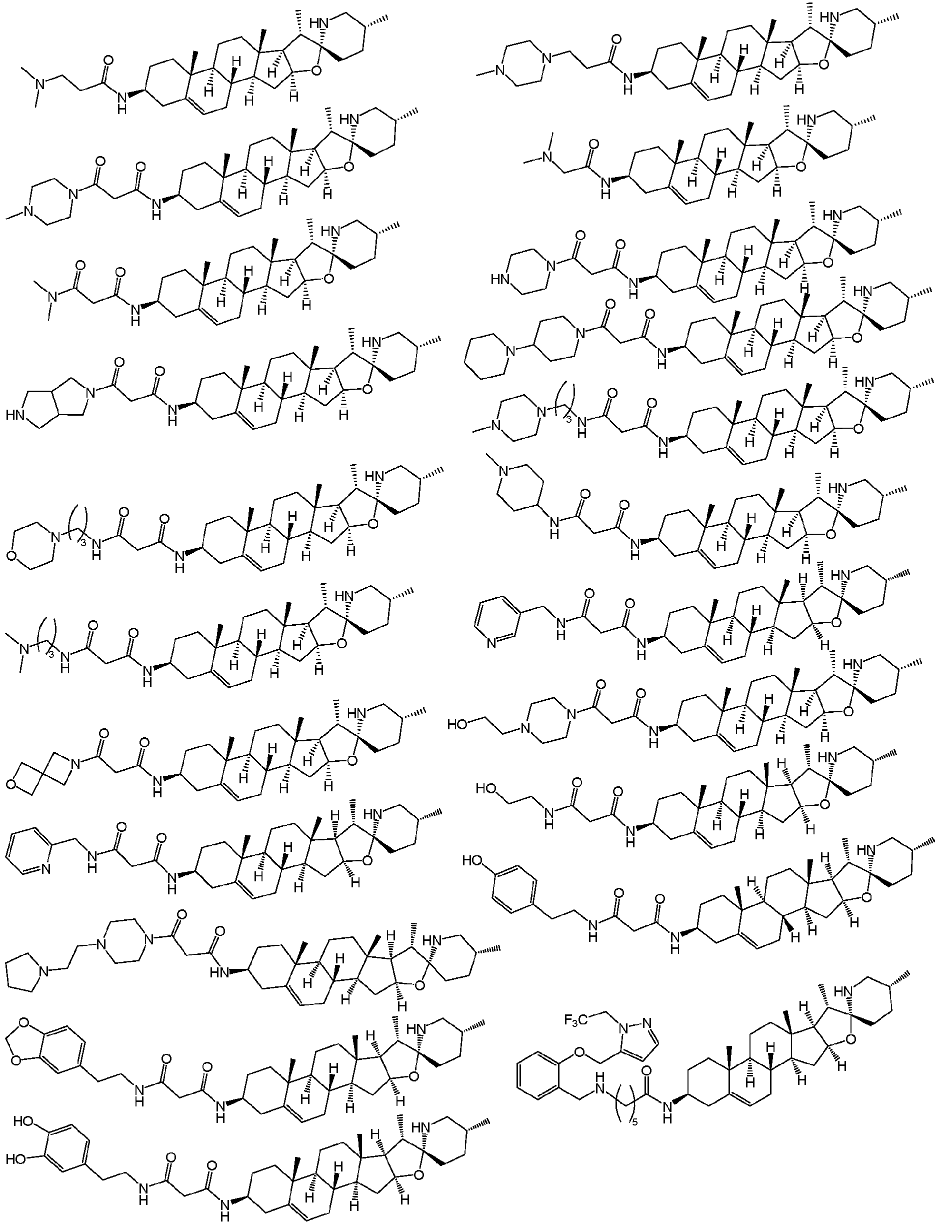

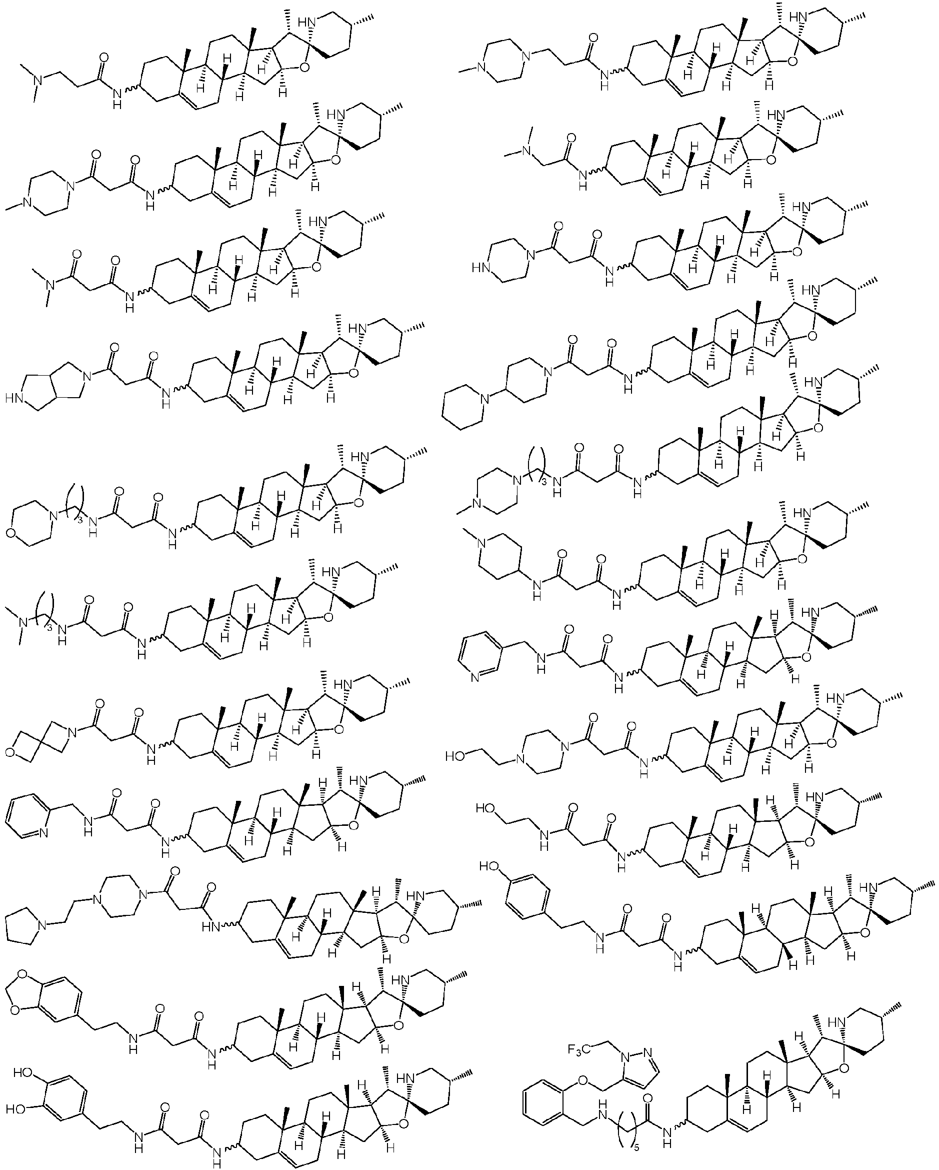

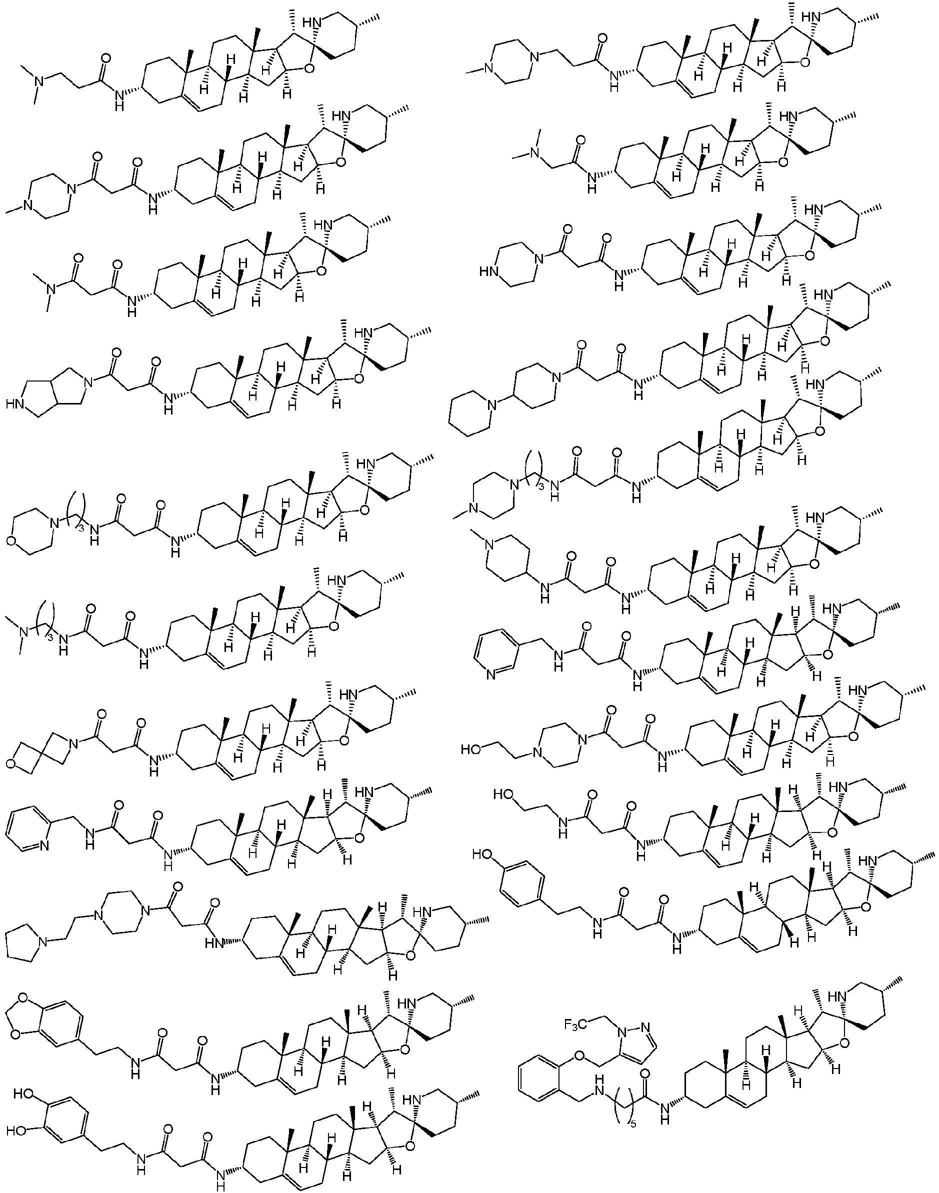

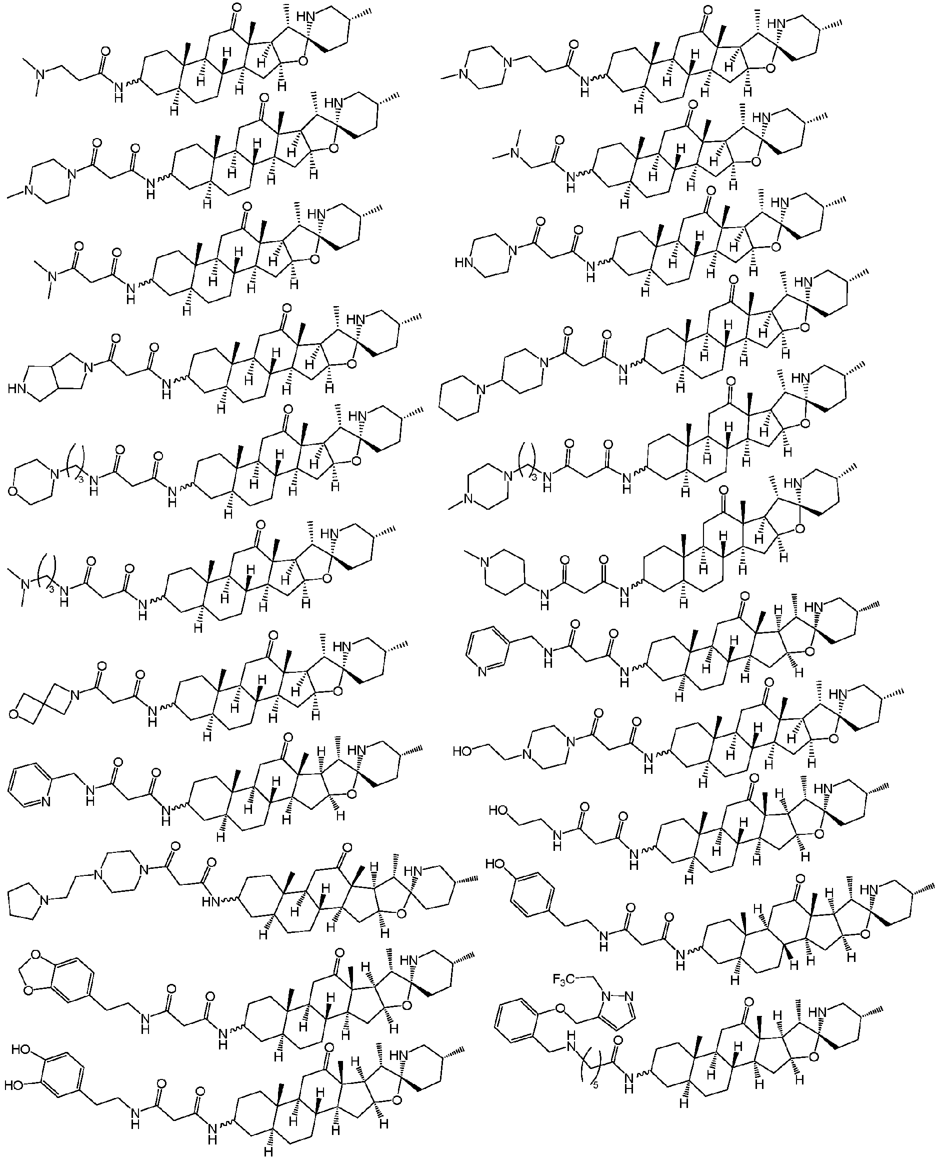

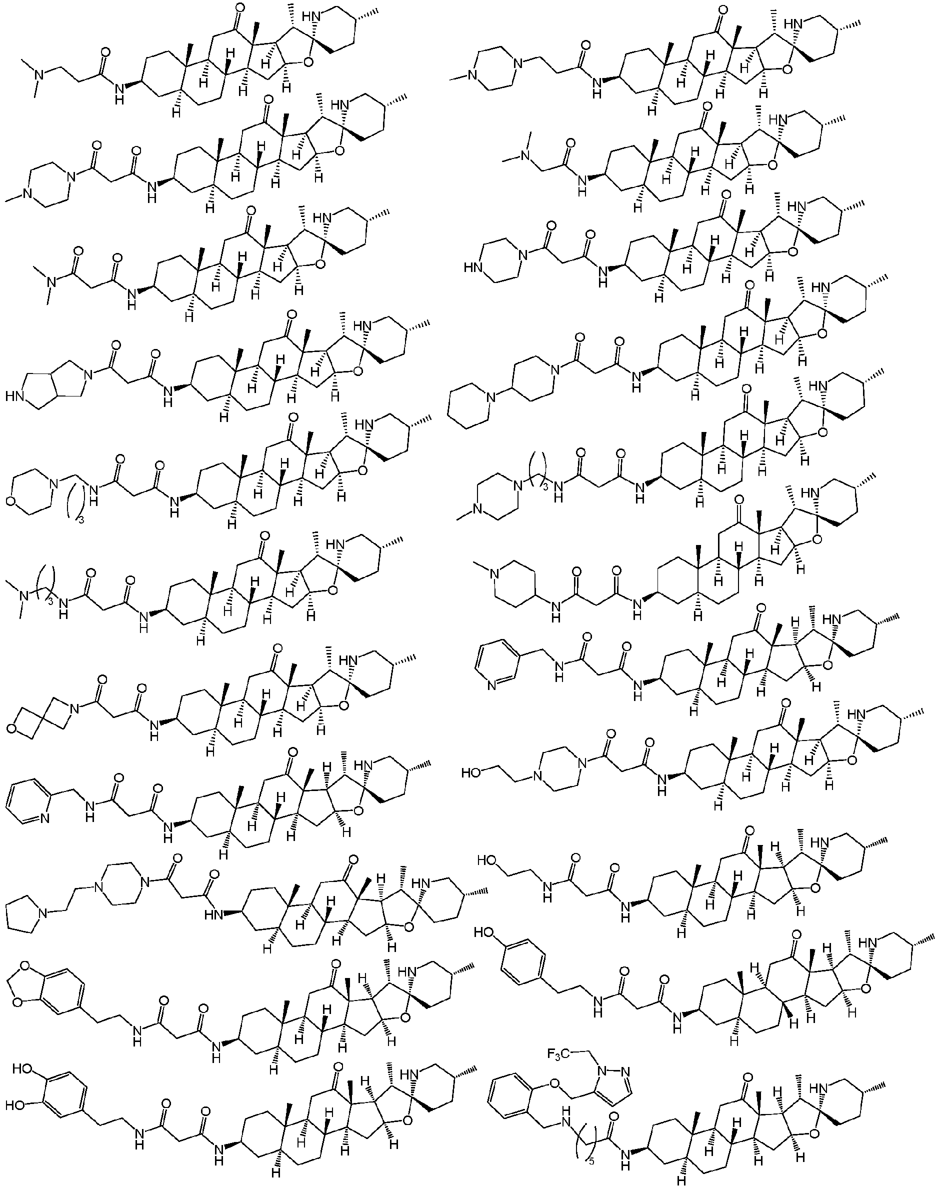

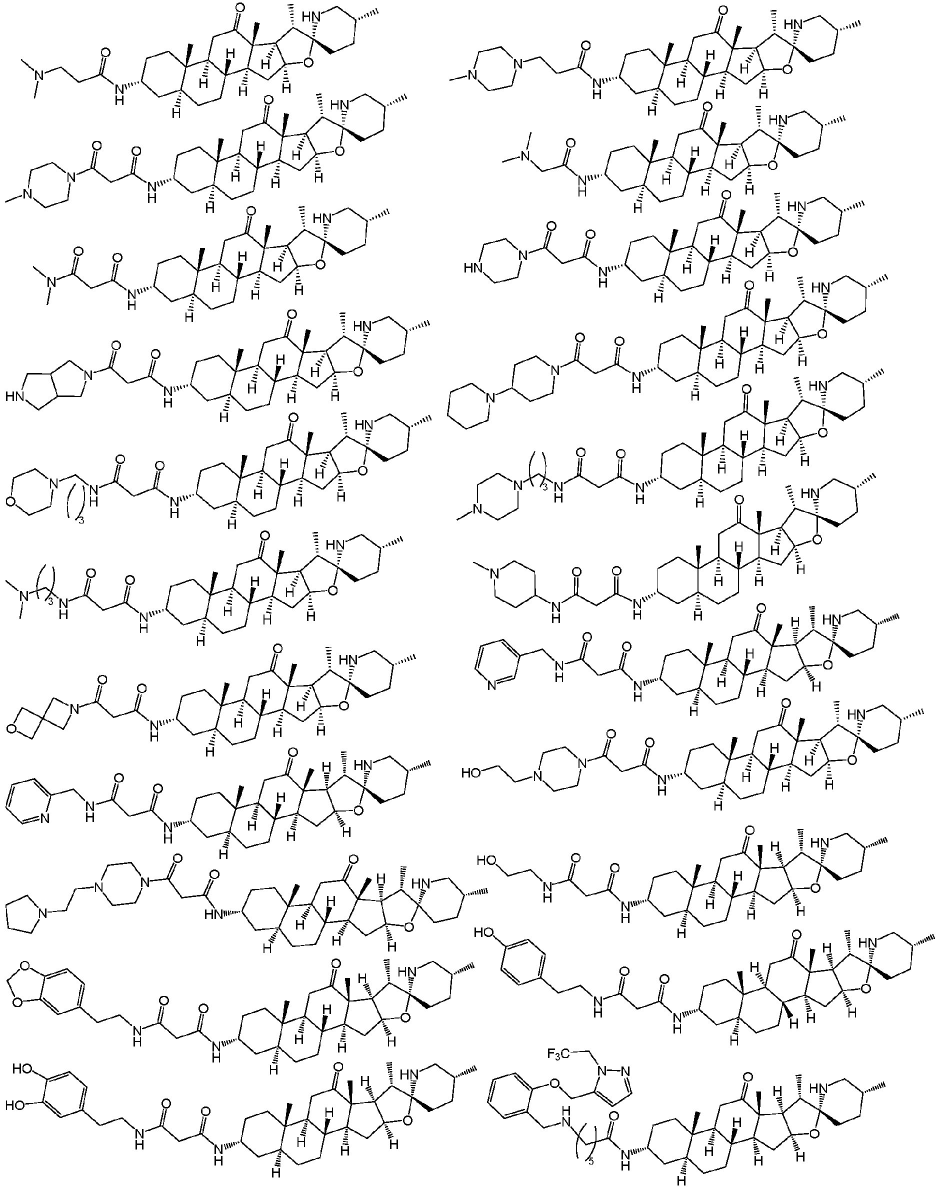

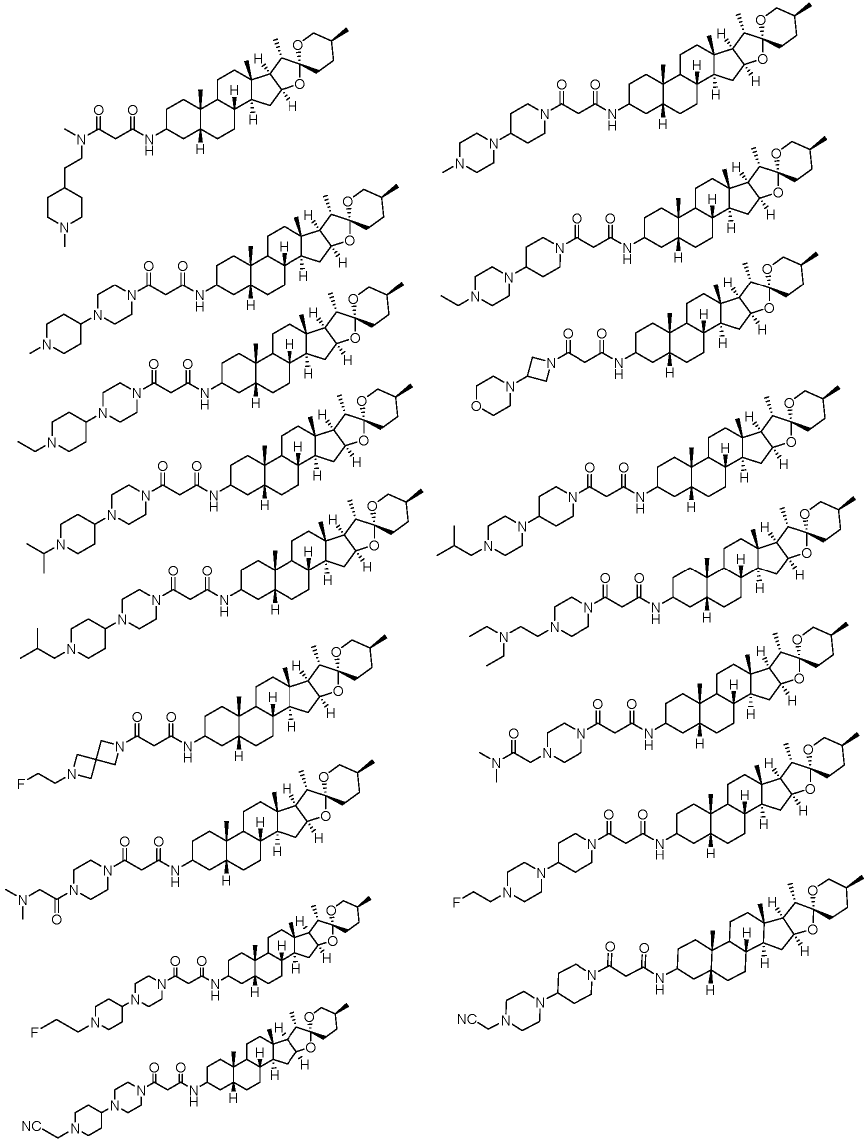

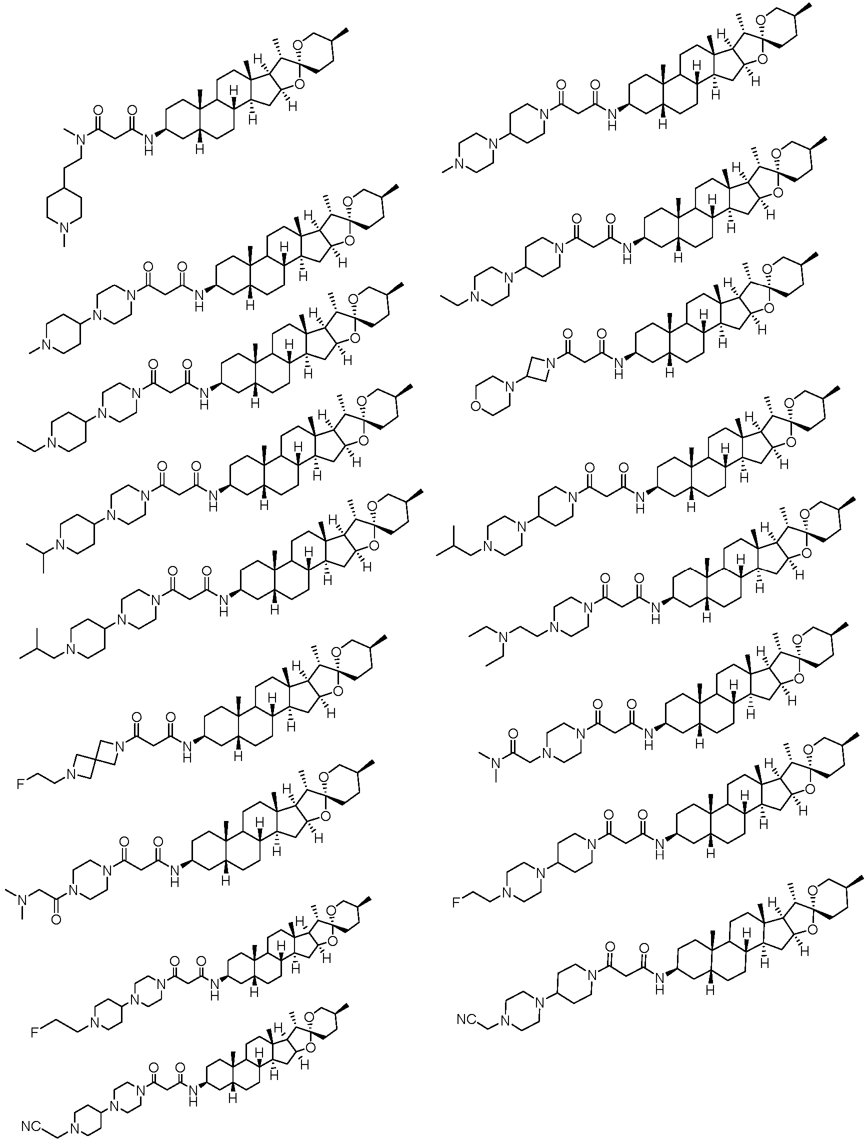

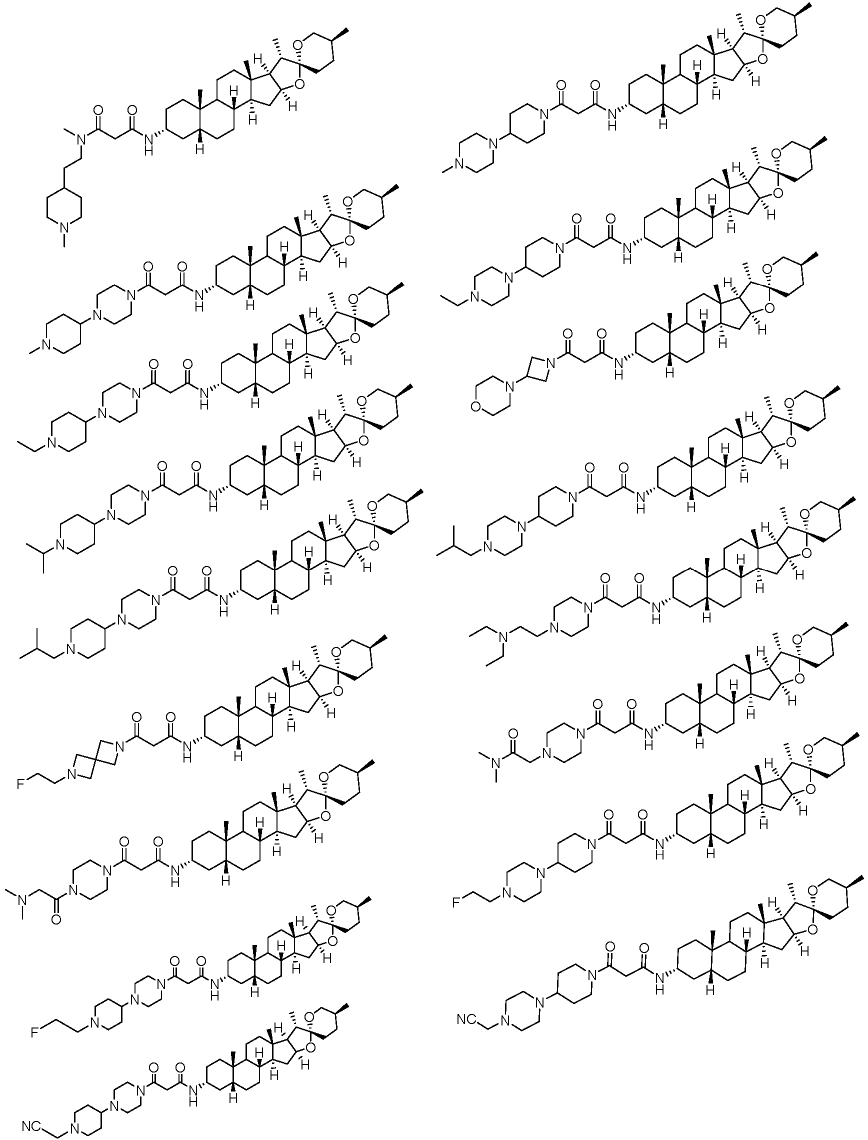

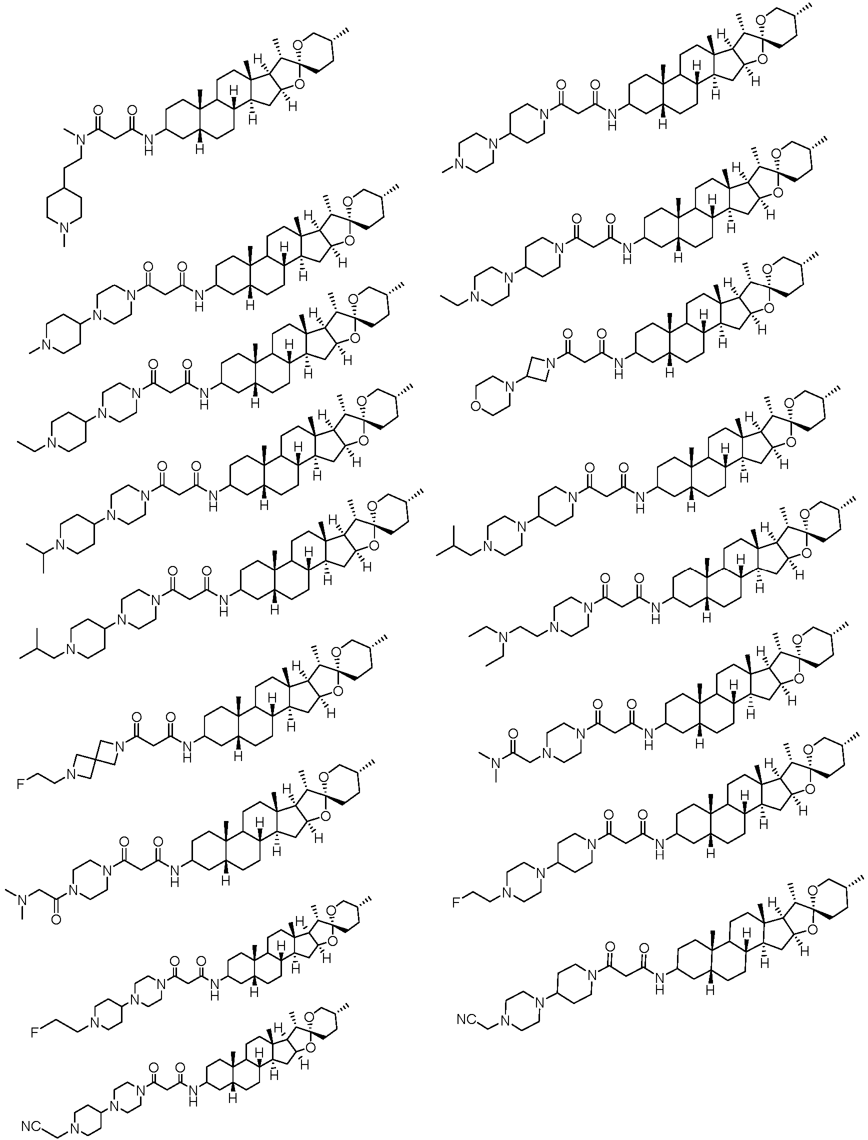

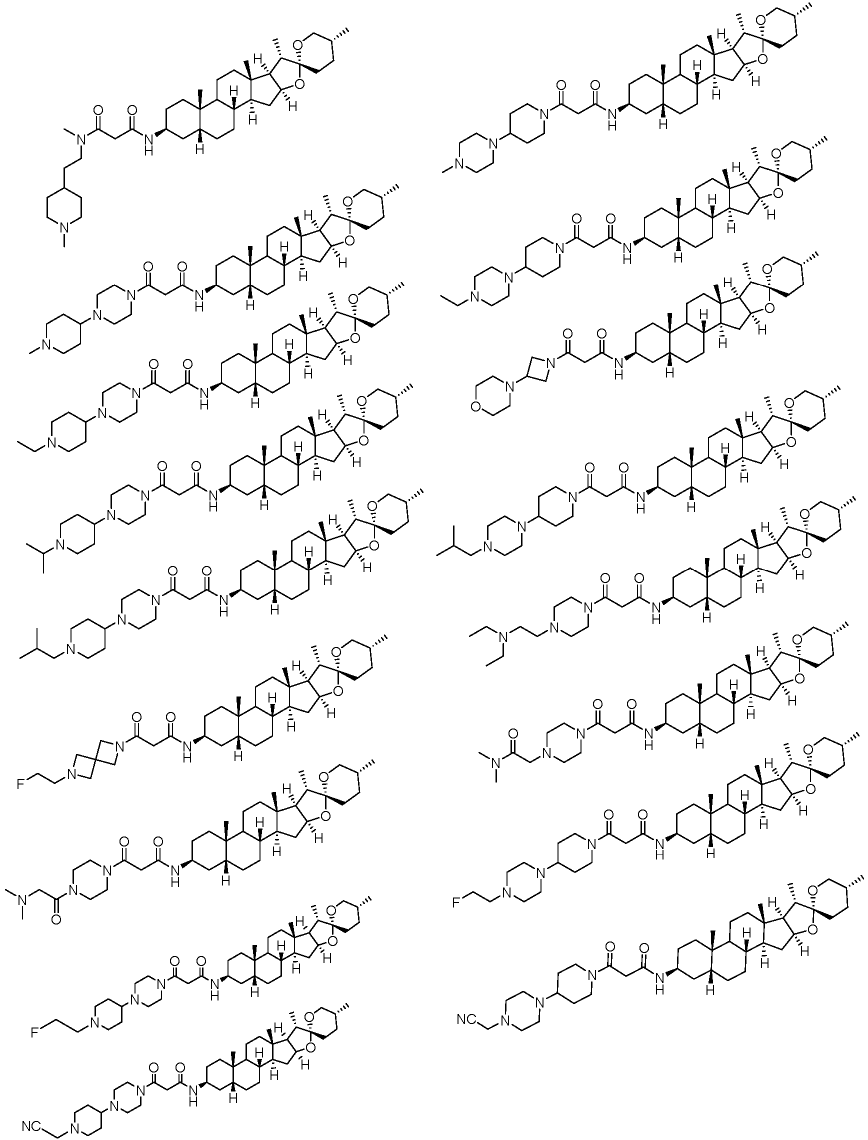

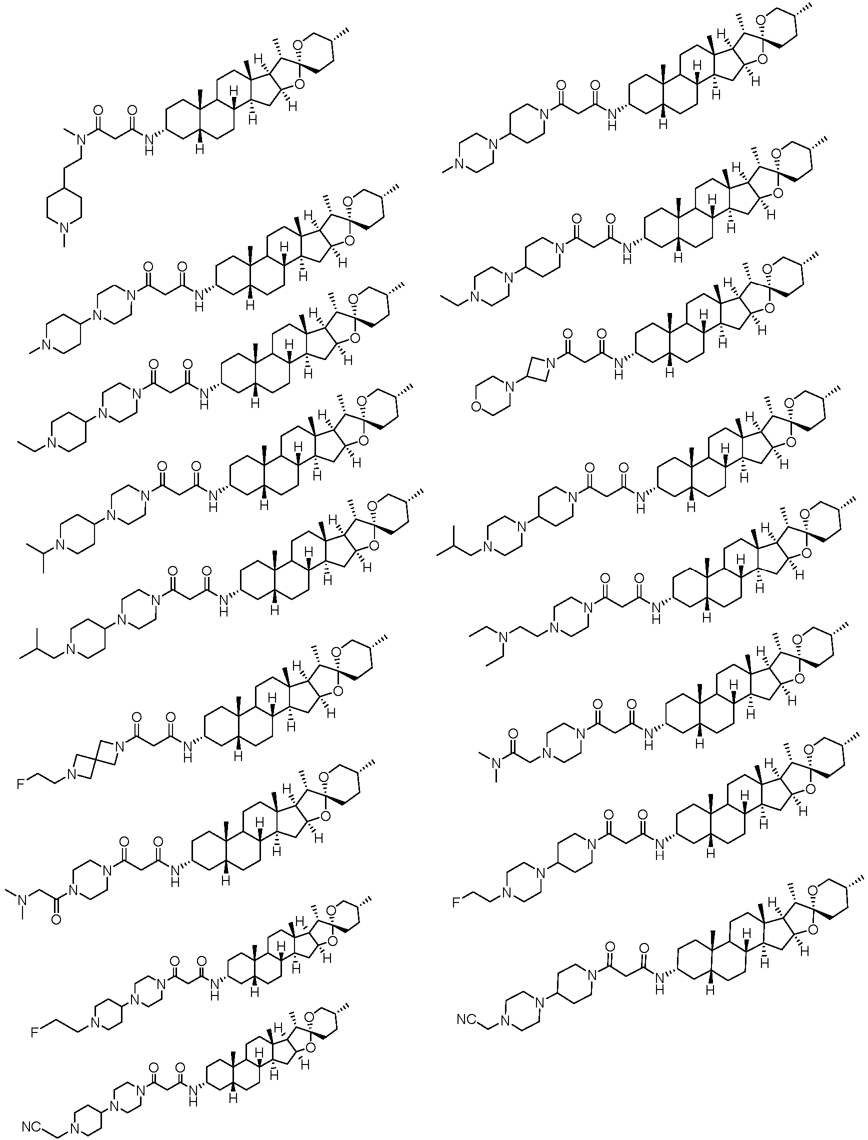

11. The use according to Claim 1, **characterized in that** one or more hydrogen atoms in the compounds are deuterium atoms.

12. A use of pharmaceutical compositions in preparation of drugs for treating related diseases caused by mitochondrial dysfunction, **characterized in that** the pharmaceutical compositions comprise a first component and pharmaceutically acceptable carriers, or a combination of the first component, a second component, and pharmaceutically acceptable carriers, the first component is compounds, pharmaceutically acceptable salts, stereoisomers, or tautomers in the use according to any of Claims 1 to 11;
the second component is additional therapeutic agents, the additional therapeutic agents include antidepressants, antimanic drugs, Parkinson's disease agents, Alzheimer's disease agents, or their combinations.

13. The use according to Claim 12, **characterized in that** the pharmaceutically acceptable salts are selected from one or more of hydrochloride, hydrobromide, sulfate, phosphate, mesylate, benzene sulfonate, p-toluenesulfonate, 1-naphthalenesulfonate, 2-naphthalene sulfonate, acetate, trifluoroacetate, malate, tartrate, citrate, lactate, oxalate, succinate, fumarate, maleate, benzoate, salicylate, phenylacetate, or mandelate;
the additional therapeutic agents are moclobemide, toloxatone, fluoxetine, paroxetine, citalopram, sertraline, venlafaxine, trimipramine, trazodone, imipramine, desipramine, clomipramine, amitriptyline, nortriptyline, doxepin, maprotiline, loxapine, amoxapine, mirtazapine, buspirone, chlormezanone, tandospirone, lithium carbonate, tacrine, huperzine a, galanthamine, donepezil, rivastigmine, memantine, pramipexole, talipexole and ropinirole, or their combinations.

14. The use according to any of Claims 1 to 13, **characterized in that** the related diseases caused by mitochondrial dysfunction include metabolic disorders, tumors, inflammations, and central nervous system disorders.

15. The use according to Claim 14, **characterized in that**
the metabolic diseases include: hyperglycemia, hyperlipidemia, high cholesterol, high low-density lipoprotein, low high-density lipoprotein, angiogenic diseases, non-alcoholic fatty liver disease, cerebrovascular accident, myocardial infarction, atherosclerosis, coronary heart disease, anti-aging, urgent and frequent urination, type I diabetes, chronic obstructive pulmonary disease;
the tumors include: benign prostatic hyperplasia, Wegener's granulomatosis, pulmonary sarcoidosis, leukemia, lymphoma, pancreatic cancer, and neurogenic tumor;
the inflammations include: peripheral neuritis, chemotherapy-induced peripheral neuritis, autoimmune diseases, conditions associated with organ transplantation, influenza virus, prevention and treatment of coronavirus infections and elimination of sequelae, acute respiratory distress syndrome, inflammatory bowel disease, Crohn's disease, ulcerative colitis, psoriasis, retinal detachment, retinitis pigmentosa, macular degeneration, pancreatitis, atopic dermatitis, rheumatoid arthritis, spondyloarthritis, gout, systemic lupus erythematosus, sjogren's syndrome, systemic sclerosis, antiphospholipid syndrome, vasculitis, osteoarthritis, autoimmune hepatitis, autoimmune liver and biliary diseases, primary sclerosing cholangitis, nephritis, celiac disease, autoimmune ITP, transplant rejection, ischemia-reperfusion injury of solid organs, sepsis, periodontitis, systemic inflammatory response syndrome, myocarditis, allergic diseases, asthma, interleukin-1 related febrile syndrome, Behcet's disease;
the central nervous system diseases include: Pick's disease, spinal cord injury repair, depression, anxiety disorder, Parkinson's disease, Alzheimer's disease, sleep disorders, ischemic stroke, hemorrhagic stroke, amyotrophic lateral sclerosis, traumatic brain injury, cerebral atrophy, Huntington's disease, schizophrenia, mania, substance withdrawal, multiple sclerosis, sleep improvement, and myasthenia.
